(19) Europäisches Patentamt
European Patent Office
Office européen des brevets

(11) **EP 3 761 034 A2**

(12) **EUROPEAN PATENT APPLICATION**

(43) Date of publication:
06.01.2021 Bulletin 2021/01

(51) Int Cl.:
*G01N 33/68* (2006.01)          *G01N 33/53* (2006.01)
*A61M 1/14* (2006.01)

(21) Application number: 20156104.0

(22) Date of filing: 20.01.2012

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 26.01.2011 US 201161436364 P

(62) Document number(s) of the earlier application(s) in accordance with Art. 76 EPC:
**12739834.5 / 2 668 497**

(71) Applicant: **University of Pittsburgh - Of the Commonwealth System of Higher Education Pittsburgh, PA 15260 (US)**

(72) Inventors:
• **SINGBARTL, Kai Scottsdale, AZ 85254 (US)**

• **KELLUM, John A. Pittsburgh, PA 15206 (US)**

(74) Representative: **Chapman, Paul Gilmour Marks & Clerk LLP 40 Torphichen Street Edinburgh EH3 8JB (GB)**

Remarks:
•The complete document including Reference Tables and the Sequence Listing can be downloaded from the EPO website
•This application was filed on 07-02-2020 as a divisional application to the application mentioned under INID code 62.
•Claims filed after the date of filing of the application / after the date of receipt of the divisional application (Rule 68(4) EPC).

(54) **URINE BIOMARKERS FOR PREDICTION OF RECOVERY AFTER ACUTE KIDNEY INJURY: PROTEOMICS**

(57)    This invention is related to the field of the prevention and treatment of kidney disease. The treatment of kidney disease may be tailored depending upon the need for, or expectation of, renal recovery. For example, renal recovery can be determined by monitoring urine biomarkers related to the development of chronic kidney disease. For example, a normalized time course of approximately fourteen Days measuring urinary proteins can be used to establish the risk of recovery versus non-recovery in patient's having suffered an acute kidney injury. Alterantively, the invention describes signature protein expression profiles to establish the probability of renal recovery and/or renal non-recovery.

Figure 1

## Description

### Statement Of Government Support

This invention was made with government support under grant #DK 070910 awarded by the National Institutes of Health. The government has certain rights in the invention.

### Field Of The Invention

This invention is related to the field of the prevention and treatment of kidney disease. The treatment of kidney disease may be tailored depending upon the need for, or expectation of, renal recovery. For example, prediction of renal recovery can be determined by monitoring urine biomarkers related to the development of chronic kidney disease. For example, differential expression platforms can be used to identify biomarker proteins in order to establish the risk of renal recovery versus renal non-recovery in patient's having suffered an acute kidney injury.

### Backgrouud

Acute Kidney Injury (AKI) has an estimated incidence rate of approximately 2000 per million population and this rate is increasing. Ali et al. "Incidence and outcomes in acute kidney injury: a comprehensive population-based study" J Am Soc Nephrol 18:1292-1298 (2007). Approximately 5% of all people admitted to intensive care units around the world develop severe AKI requiring dialysis. Uchino et al., "Acute renal failure in critically ill patients: a multinational, multicenter study" JAMA 294:813-818 (2005). A recent, multi-center study found that fewer than only about 60% patients surviving severe AKI recovered renal function by two months. Palevsky et al., "Intensity of renal support in critically ill patients with acute kidney injury" N Engl J Med 359:7-20 (2008). Thus, a large number of patients with AKI go on to have end-stage renal disease (ESRD).
However, since only a fraction of patients with AKI fail to recover renal function, interventions aimed at improving recovery or providing renal support (e.g. early dialysis) cannot be targeted appropriately without some means of determining which patients will recover and which will not. Unfortunately, clinical risk prediction for recovery after AKI is extremely limited. Research efforts to treat AKI and prevent ESRD could be tailored according to long-term-prognosis. In other words, with an accurate prediction of which patients will not recover kidney function, medical efforts could focus the development and application of aggressive treatment interventions on just these patients. Conversely, patients with a favorable prognosis would be spared from more aggressive interventions and their potential adverse effects.
Thus, development of a biomarker or biomarker panel that allows early prediction of recovery of kidney function would be an extremely valuable clinical tool. What is needed in the art are a panel of biomarkers to predict renal recovery after AKI.

### Summary

This invention is related to the field of the prevention and treatment of kidney disease. The treatment of kidney disease may be tailored depending upon the need for, or expectation of, renal recovery. For example, prediction of renal recovery can be determined by monitoring urine biomarkers related to the development of chronic kidney disease. For example, differential expression platforms can be used to identify biomarker proteins in order to establish the risk of renal recovery versus renal non-recovery in patient's having suffered an acute kidney injury.
In one embodiment, the present invention contemplates a composition comprising an renal injury biomarker, wherein said biomarker comprises at least a fragment of a protein selected from the group consisting of ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preproprotien, carbonic anhydrase, and uromodulin precursor. In one embodiment, the composition further comprises a urine sample. In one embodiment, the urine sample is collected between 1 day and 14 days after a kidney injury. In one embodiment, the urine sample is a human urine sample. In one embodiment, the biomarker is at least 2.5 fold higher as compared to an expected level in a renal recovery group. In one embodiment, the biomarker is at least 2.0 fold higher as compared to an expected level in a renal recovery group. In one embodiment, the biomarker is at least 1.5 fold higher as compared to an expected level in a renal recovery group. In one embodiment, the biomarker is at least 1.25 fold higher as compared to an expected level in a renal recovery group. In one embodiment, the biomarker is at least 2.5 fold lower as compared to an expected level in a renal recovery group. In one embodiment, the biomarker is at least 2.0 fold lower as compared to an expected level in a renal recovery group. In one embodiment, the biomarker is at least 1.5 fold lower as compared to an expected level in a renal recovery group. In one embodiment, the biomarker is at least 1.25 fold lower as compared to an expected level in a renal recovery group.

In one embodiment, the present invention contemplates a method, comprising: a) providing; i) a patient exhibiting at least one symptom of an acute renal injury; and ii) a biological fluid sample obtained from said patient, wherein said sample comprises a renal injury biomarker; b) measuring a renal recovery biomarker value; c) comparing said said renal biomarker value to an expected value from a renal recovery group; and d) predicting a probability of renal recovery for said patient based upon said comparison. In one embodiment, the probability of renal recovery is greater than 90%. In one embodiment, the probability of renal recovery is greater than 75%. In one embodiment, the probability of renal recovery is greater than 50%. In one embodiment, the probability of renal recovery is less than 50%. In one embodiment, the probability of renal recovery is less than 25%. In one embodiment, the probability of renal recovery is less than 10%. In one embodiment, the biomarker comprises at least a fragment of a protein selected from the group consisting of ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preproprotien, carbonic anhydrase, and uromodulin precusor.

In one embodiment, the present invention contemplates a kit, comprising; a) a first container comprising an antibody specifically directed to an renal injury biomarker, wherein said biomarker comprises at least a fragment of a protein selected from the group consisting of ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preproprotien, carbonic anhydrase, and uromodulin precusor; b) instructions for determining whether said biomarker is overexpressed as compared to an expected value from a renal recovery group; c) instructions for determining whether said biomarker is underexpressed as compared to an expected value from a renal recovery group; and d) instructions for determining the probability of renal recovery. In one embodiment, the antibody is a monoclonal antibody. In one embodiment, the monoclonal antibody is specifically directed to said biomarker protein fragment.

In one embodiment, the present invention contemplates a signature expression profile comprising a urinary protein biomarker panel, wherein said profile predicts renal recovery. In one embodiment, the biomarker panel comprises a plurality of overexpressed urinary proteins. In one embodiment, the biomarker panel comprises a plurality of underexpressed urinary proteins. In one embodiment, the plurality of overexpressed urinary proteins are selected from the group consisting of beta globin, catalase, alpha globin, mucin isoform precursor, ezrin, delta globin, moesin, phosphoprotein isoform, and annexin A2. In one embodiment, the plurality of underexpressed urinary proteins are selected from the group consisting of myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, and uromodulin precursor.

In one embodiment, the present invention contemplates a signature expression profile comprising a urinary protein biomarker panel, wherein said profile predicts renal non-recovery. In one embodiment, the biomarker panel comprises a plurality of overexpressed urinary proteins. In one embodiment, the biomarker panel comprises a plurality of underexpressed urinary proteins. In one embodiment, the plurality of overexpressed urinary proteins are selected from the group consisting of beta globin, catalase, alpha globin, mucin isoform precursor, ezrin, delta globin, moesin, phosphoprotein isoform, and annexin A2. In one embodiment, the plurality of underexpressed urinary proteins are selected from the group consisting of myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, and uromodulin precursor.

In one embodiment, the present invention contemplates a method, comprising: a) providing; i) a patient exhibiting at least one symptom of an acute renal injury; and ii) a biological fluid sample obtained from said patient, wherein said sample comprises a plurality of renal biomarker nucleic acids; b) expressing said plurality of renal biomarker nucleic acids, thereby creating a signature expression profile; and c) predicting a probability of renal recovery for said patient based upon said signature expression profile. In one embodiment, the signature expression profile predicts a probability of renal recovery of greater than 90%. In one embodiment, the signature expression profile predicts a probability of renal recovery of greater than 75%. In one embodiment, the signature expression profile predicts a probability of renal recovery of greater than 50%. In one embodiment, the signature expression profile predicts a probability of renal recovery of less than 50%. In one embodiment, the signature expression profile predicts a probability of renal recovery of less than 25%. In one embodiment, the signature expression profile predicts a probability of renal recovery of less than 10%. In one embodiment, the signature expression profile comprises a plurality of overexpressed urinary proteins. In one embodiment, the signature expression profile comprises a plurality of underexpressed urinary proteins. In one embodiment, the plurality of overexpressed urinary proteins are selected from the group consisting of beta globin, catalase, alpha globin, mucin isoform precursor, ezrin, delta globin, moesin, phosphoprotein isoform, and annexin A2. In one embodiment, the plurality of underexpressed urinary proteins are selected from the group consisting of myoglobin, hemopexin, serine proteinase inhibitor, serpine peptidase inhibitor, CD 14 antigen precursor, fibronectin isoform preprotein, angiotensinogen preprotein, complement component precursor, carbonic anhydrase, and uromodulin precursor.

In one embodiment, the present invention contemplates a kit, comprising; a) a first container comprising reagents for creating a signature expression profile using a biological sample, wherein said signature expression profile comprises a plurality of renal biomarker nucleic acids; b) a second container comprising monoclonal antibodies specific for said renal biomarker nucleic acids; c) a set of instructions for creating said signature expression profile; d) a set of instructions for determining overexpressed renal biomarker nucleic acids; e) a set of instructions for determining underexpressed renal biomarker nucleic acids; f) a set of instructions for predicting the probability of renal recovery; and g) a set of instructions for predicting the probabiliyt of renal non-recovery.

## Definitions

As used herein, an "injury to renal function" is an abrupt (i.e., for example, within 14 Days, preferably within 7 Days, more preferably within 72 hours, and still more preferably within 48 hours) measurable reduction in a measure of renal function. Such an injury to renal function may be identified, for example, by a decrease in glomerular filtration rate (GFR) or estimated GFR (eGFR), a reduction in urine output, an increase in serum creatinine, an increase in serum cystatin C, a requirement for renal replacement therapy (i.e., for example, dialysis), etc.

As used herein, an "improvement in renal function" is an abrupt (i.e., for example, within 14 Days, preferably within 7 Days, more preferably within 72 hours, and still more preferably within 48 hours) measurable increase in a measure of renal function. Preferred methods for measuring and/or estimating GFR are described hereinafter.

As used herein, "reduced renal function" is an abrupt (i.e., for example, within 14 Days, preferably within 7 Days, more preferably within 72 hours, and still more preferably within 48 hours) reduction in kidney function identified by an absolute increase in serum creatinine of greater than or equal to 0.1 mg/dL ($\geq 8.8$ $\mu$mol/L), a percentage increase in serum creatinine of greater than or equal to 20% (1.2-fold from baseline), or a reduction in urine output (documented oliguria of less than 0.5 ml/kg per hour).

As used herein, "acute renal failure" or "ARF" is an abrupt (i.e., for example, within 14 Days, preferably within 7 Days, more preferably within 72 hours, and still more preferably within 48 hours) reduction in kidney function identified by an absolute increase in serum creatinine of greater than or equal to 0.3 mg/dl ($\geq 26.4$ $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "relating a signal to the presence or amount" of an analyte refers to assay measurements using a standard curve calculated with known concentrations of the analyte of interest. The skilled artisan will understand that the signals obtained from an assay are often a direct result of complexes formed between, for example, one or more antibodies and a target biomolecule (i.e., for example, an analyte) and/or polypeptides containing an epitope(s) to which, for example, antibodies bind. While such assays may detect a full length biomarker and the assay result may be expressed as a concentration of a biomarker of interest, the signal from the assay is actually a result of all such "immunoreactive" polypeptides present in the sample.

As the term is used herein, an assay is "configured to detect" an analyte if an assay can generate a detectable signal indicative of the presence or amount of a physiologically relevant concentration of the analyte. For example, an antibody epitope is usually on the order of 8 amino acids, such that an immunoassay can be configured to detect a marker of interest that will also detect polypeptides related to the marker sequence, so long as those polypeptides contain the epitope(s) necessary to bind to the antibody or antibodies used in the assay.

The term "related marker" or "biomarker" as used herein with regard to a physiological substance such as one of the proteins as described herein. A related marker may also refer to one or more fragments, variants, etc., of a particular protein and/or peptide or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

The term "subject" or "patient" as used herein, refers to a human or non-human organism. Thus, the methods and compositions described herein are equally applicable to both human and veterinary disease. Further, while a subject or patient is preferably a living organism, the invention described herein may be used in post-mortem analysis as well. Preferred subjects or patients are humans, which as used herein refer to living humans that are receiving medical care for a disease or condition.

The term "analyte" as used herein, refers to any measured compound or molecule. Preferably, an analyte is measured in a sample (i.e., for example, a body fluid sample). Such a sample may be obtained from a subject or patient, or may be obtained from biological materials intended to be provided to the subject or patient. For example, a sample may be obtained from a kidney being evaluated for possible transplantation into a subject, such that an analyte measurement may be used to evaluate the kidney for preexisting damage.

The term "body fluid sample" as used herein, refers to any sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing medical condition or the effect of a treatment regimen on a medical condition. Preferred body fluid samples include but are not limited to, blood, serum, plasma, cerebrospinal fluid, urine, saliva, sputum, or pleural effusions. In addition, certain body fluid samples may be more readily analyzed following a fractionation or purification procedure, for example, separation of whole blood into serum or plasma components.

The term "diagnosis" as used herein, refers to methods by which trained medical personnel can estimate and/or determine the probability (i.e., for example, a likelihood) of whether or not a patient is suffering from a given disease or condition. In the case of the present invention, "diagnosis" includes correlating the results of an assay (i.e., for example, an immunoassay) for a renal biomarker of the present invention, optionally together with other clinical indicia, to determine the occurrence or nonoccurrence of an acute renal injury or acute renal failure for a subject or patient from which a sample was obtained and assayed. That such a diagnosis is "determined" is not meant to imply that the diagnosis is 100% accurate. Thus, for example, a measured biomarker level below a predetermined diagnostic threshold may indicate a greater likelihood of the occurrence of a disease in the subject relative to a measured biomarker level above the predetermined diagnostic threshold may indicate a lesser likelihood of the occurrence of the same disease.

The term "prognosis" as used herein, refers to a probability (i.e., for example, a likelihood) that a specific clinical outcome will occur. For example, a level or a change in level of a prognostic indicator, which in turn is associated with an increased probability of morbidity (e.g., worsening renal function, future ARF, or death) is referred to as being "indicative of an increased likelihood" of an adverse outcome in a patient.

The term "RIFLE" criteria, as used herein, refers to any quantitative clinical evaluation of renal status used to establish renal classifications of Risk, Injury, Failure, Loss, & End Stage Renal Disease based upon a uniform definition of acute kidney injury (AKI). Kellum, Crit. Care Med. 36: S141-45 (2008); and Ricci et al., Kidney Int. 73, 538-546 (2008), each hereby incorporated by reference in its entirety.

The term, "modified RIFLE criteria", as used herein, provide alternative classifications for stratifying AKI patients, and may include, Stage I, Stage II, and/or Stage III. Mehta et al., Crit. Care 11:R31 (2007), hereby incorporated by reference in its entirety.

The term,"Stage I", as used herein, refers to a risk stratification comprising a RIFLE Risk category, characterized by an increase in serum creatinine of more than or equal to 0.3 mg/dL ($\geq$ 26.4 $\mu$mol/L) and/or an increase to more than or equal to 150% (1.5-fold) from baseline. Alternatively, the category may be defined by a urine output less than 0.5 mL/kg per hour for more than 6 hours.

The term, "Stage II", as used herein, refers a risk stratification comprising a RIFLE Injury category, characterized by an increase in serum creatinine to more than 200% (> 2-fold) from baseline. Alternatively, the category may be defined by a urine output less than 0.5 mL/kg per hour for more than 12 hours.

The term, "Stage III", as used herein, refers to a risk stratification comprising a RIFLE Failure category, characterized by an increase in serum creatinine to more than 300% (> 3-fold) from baseline and/or serum creatinine $\geq$ 354 $\mu$mol/L accompanied by an acute increase of at least 44 $\mu$mol/L. Alternatively, the category may be defined by a urine output less than 0.3 mL/kg per hour for 24 hours or anuria for 12 hours.

The term "Risk category", as used herein, refers to a RIFLE classification wherein, in terms of serum creatinine, means any increase of at least 1.5 fold from baseline, or urine production of < 0.5 ml/kg body weight/hr for approximately 6 hours.

The term "Injury category" as used herein includes, refers to a RIFLE classification wherein, in terms of serum creatinine, means any increase of at least 2.0 fold from baseline or urine production <0.5 ml/kg/hr for 12 h.

The term "Failure category" as used herein includes, refers to a RIFLE classification wherein, in terms of serum creatinine means any increase of at least 3.0 fold from baseline or a urine creatinine >355 $\mu$mol/l (with a rise of >44) or urine output below 0.3 ml/kg/hr for 24 h, or anuria for at least 12 hours.

The term "Loss category" as used herein, refers to a clincial outcome risk and/or a RIFLE classification wherein the clincial outcome risk is characterized by a persistent need for renal replacement therapy for more than four weeks.

The term "End Stage Renal Disease category" or "ESRD category" as used herein, refers to a clinical outcome risk and/or a RIFLE classification characterized by a need for dialysis for more than 3 months.

The term "clinical outcome risk" as used herein, refers to a medical prognosis directed towards either renal recovery or renal non-recovery.

The term "renal biomarker" as used herein, refers to any biological compound related to the progressive development of chronic kidney disease. In particular, a renal biomarker may be a kidney injury marker. For example, a renal biomarker may comprise a urinary protein, or any metabolite and/or derivative thereof, wherein the renal biomarker is either overexpressed or underexpressed as a result of an AKI.

The term "positive going biomarker" as that term is used herein, refers to any biomarker that is determined to be elevated in subjects suffering from a disease or condition, relative to subjects not suffering from that disease or condition.

The term "negative going biomarker" as that term is used herein, refer to any biomarker that is determined to be reduced in subjects suffering from a disease or condition, relative to subjects not suffering from that disease or condition.

The term "positive going renal biomarker value" as used herein, refers to any increased likelihood (i.e., for example, increased probability) of suffering a future injury to renal function assigned to a subject when the measured biomarker concentration is above a specified threshold value, relative to a likelihood assigned when the measured biomarker concentration is below the specified threshold value. Alternatively, when the measured biomarker concentration is below a specified threshold value, an increased likelihood of a non-occurrence of an injury to renal function may be assigned to the subject relative to the likelihood assigned when the measured biomarker concentration is above the specified threshold value. Alternatively, when the measured biomarker concentration is below the threshold value, an improvement of renal function may be assigned to the subject. A positive going kidney injury marker may include, but not be limited to, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc.

The term "negative going renal biomarker value" as used herein, refers to any increased likelihood (i.e., for example, an increased probability) of suffering a future injury to renal function assigned to the subject when the measured biomarker concentration is below a specified threshold value, relative to a likelihood assigned when the measured biomarker concentration is above the threshold value. Alternatively, when the measured biomarker concentration is above the threshold value, an increased likelihood of a non-occurrence of an injury to renal function may be assigned to the subject relative to the likelihood assigned when the measured biomarker concentration is below the threshold value. Alternatively, when the measured biomarker concentration is above the threshold value, an improvement of renal function may be assigned to the subject. A negative going kidney injury marker may include, but not be limited to, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc.

The term "pre-existing" and "pre-existence" as used herein, means any risk factor (i.e., for example, a renal biomarker) existing at the time a body fluid sample is obtained from the subject.

The term "predicting" as used herein, refers to a method of forming a prognosis and/or a stratification risk assignment, wherein a medically trained person analyzes biomarker information, and optionally with relevant clincial indicia and/or demographic information.

The term "acute renal disease/failure/injury" as used herein, refers to any progressive worsening of renal function over hours to Days, resulting in the retention of nitrogenous wastes (such as urea nitrogen) and creatinine in the blood. Retention of these substances may also be referred to as, azotemia. In: Current Medical Diagnosis & Treatment 2008, 47th Ed, McGraw Hill, New York, pages 785-815, herein incorporated by reference in their entirety.

The term "chronic renal disease/failure/injury" as used herein, refers to a medical condition wherein exemplary symptoms may include, but are not limited to, hyperphosphatemia (i.e., for example, > 4.6 mg/dl) or low glomerular filtration rates (i.e., for example, < 90 ml/minute per 1.73 m2 of body surface). However, many CKD patients may have normal serum phosphate levels in conjunction with a sustained reduction in glomerular filtration rate for 3 or more months, or a normal GFR in conjunction with sustained evidence of a structural abnormality of the kidney. In some cases, patients diagnosed with chronic kidney disease are placed on hemodialysis to maintain normal blood homeostasis (i.e., for example, urea or phosphate levels). Alternatively, "chronic kidney disease" refers to a medical condition wherein a patients has either i) a sustained reduction in GFR <60 mi/min per 1.73 m2 of body surface for 3 or more months; or ii) a structural or functional abnormality of renal function for 3 or more months even in the absence of a reduced GFR. Structural or anatomical abnormalities of the kidney could be defined as, but not limited to, persistent microalbuminuria or proteinuria or hematuria or presence of renal cysts. Chronic renal failure (chronic kidney disease) may also result from an abnormal loss of renal function over months to years. In: Current Medical Diagnosis & Treatment 2008, 47th Ed, McGraw Hill, New York, pages 785-815, herein incorporated by reference in their entirety.

The term "about" as used herein in the context of any of any assay measurements refers to +/- 5% of a given measurement.

The term "asymptomatic" as used herein, refers to a patient and/or subject that does not have a renal disease and/or injury, wherein a renal disease and/or injury symptom may include, but is not limited to, having a reduced glomerular filtration rate (i.e., for example, between approximately 70 - 89 ml/min per 1.73 m2 of body surface) for less than three months.

The term "glomerular filtration rate" as used herein, refers to any measurement capable of determining kidney function. In general, a normal glomerular filtration rate ranges between approximately 120 - 90 ml/minute per 1.73 m2 of body surface. Compromised kidney function is assumed when glomerular filtration rates are less than 90 ml/minute per 1.73 m2 of body surface. Kidney failure is probable when glomerular filtration rates fall below approximately 30 ml/minute per 1.73 m2 of body surface. Dialysis is frequently initiated when glomerular filtration rates fall below approximately 15 ml/minute per 1.73 m2 of body surface.

The term "renal failure" as used herein, refers to any acute, sudden, and/or chronic loss of the ability of the kidneys to remove waste and concentrate urine without losing electrolytes.

The term "biological sample" as used herein, refers to any substance derived from a living organism. For example, a sample may be derived from blood as a urine sample, serum sample, a plasma sample, and or a whole blood sample. Alternatively, a sample may be derived from a tissue collected, for example, by a biopsy. Such a tissue sample may comprise, for example, kidney tissue, vascular tissue and/or heart tissue. A biological sample may also comprise body fluids including, but not limited to, urine, saliva, or perspiration.

The term "reagent" as used herein, refers to any substance employed to produce a chemical reaction so as to detect, measure, produce, etc., other substances. The term "antibody" as used herein refers to any peptide or polypeptide derived from, modeled after, or substantially encoded by, an immunoglobulin gene or immunoglobulin genes, or fragments thereof, capable of specifically binding an antigen or epitope. See, e.g. In: Fundamental Immunology, 3rd Edition, W.E. Paul, ed., Raven Press, N.Y. (1993); Wilson et al., J. Immunol. Methods 175:267-273 (1994); and Yarmush et al., J. Biochem. Biophys. Methods 25:85-97 (1992). The term antibody includes, but is not limited to, antigen-binding portions, i.e., "antigen binding sites" exemplified by fragments, subsequences, and/or complementarity determining regions (CDRs)) that retain capacity to bind antigen, including, but not limited to: (i) a Fab fragment, a monovalent fragment comprising VL, VH, CL or CHl domains; (ii) a F(ab')2 fragment, a bivalent fragment comprising two Fab fragments linked by a disulfide bridge at the hinge region; (iii) a Fd fragment comprising VH and CHl domains; (iv) a Fv fragment comprising VL and VH domains of a single arm of an antibody, (v) a dAb fragment (Ward et al., Nature 341:544-546 (1989)), which comprises a VH domain; or (vi) an isolated complementarity determining region (CDR). Single chain antibodies are also included by reference in the term "antibody."

The term "epitope" as used herein, refers to any antigenic determinant capable of specific binding to an antibody. Epitopes usually display chemically active surface molecules such as amino acids or sugar side chains and usually have specific three dimensional structural characteristics, as well as specific charge characteristics. Conformational and nonconformational epitopes may be distinguished in that the binding to the former but not the latter can be lost in the presence of denaturing solvents.

The term "correlating" as used herein, in reference to the use of biomarkers, refers to comparing the presence and/or amount of any biomarker(s) in a patient to its presence and/or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

## Brief Description Of The Figures

Figure 1 presents exemplary subject information relevant to the Biological Markers of Recovery for the Kidney (BioMaRK) study cohort used as the basis for some of the data analysis presented herein.

Figure 2: Representative protein biomarker familes identified by a proteomics platform

Figure 3 Representative single biomarker peptides Identified by a proteomics platform.

## Detailed Description

This invention is related to the field of the prevention and treatment of kidney disease. The treatment of kidney disease may be tailored depending upon the need for, or expectation of, renal recovery. For example, prediction of renal recovery can be determined by monitoring urine biomarkers related to the development of chronic kidney disease. For example, differential expression platforms can be used to identify biomarker proteins in order to establish the risk of renal recovery versus renal non-recovery in patient's having suffered an acute kidney injury.

Despite significant advances in the epidemiology of acute kidney injury (AKI), prognostication remains a major clinical challenge. Unfortunately, there is no reliable method to predict renal recovery. The discovery of biomarkers to aid in clinical risk prediction for recovery after AKI would represent a significant advance over current practice.

**I. Kidney Injury And/Or Disease**

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. The kidneys are located in the flank (back of the upper abdomen at either side of the spinal column). They are deep within the abdomen and are protected by the spine, lower rib cage, and the strong muscles of the back. This location protects the kidneys from many external forces. They are well-padded for a reason -- kidneys are highly vascular organs, which means that they have a large blood supply. If injury occurs, severe bleeding may result.

Kidneys may be injured by damage to the blood vessels that supply or drain them. This may be in the form of aneurysm, arteriovenous fistula, arterial blockage, or renal vein thrombosis. The extent of bleeding depends on the location and the degree of injury. Kidneys may also bleed profusely if they are damaged centrally (on the inside) -- this is a life-threatening injury. Fortunately, most kidney injuries caused by blunt trauma occur peripherally, only causing bruising of the kidney (usually a self-limiting process).

People with undiagnosed kidney conditions -- such as angiomyolipoma (benign tumor), ureteropelvic junction obstruction (congenital or acquired UPJ Obstruction), and other disorders -- are more susceptible to kidney injuries and more likely to have serious complications if they occur. Other causes of kidney injury and bleeding are medical procedures. Kidney biopsies, nephrostomy tube placements, or other surgeries can cause an abnormal connection between an artery and vein (arteriovenous fistula). This is usually a self-limiting problem, but close observation is usually needed. Injury to the kidney can also disrupt the urinary tract, causing leakage of the urine from the kidney.

Each kidney filters about 1700 liters of blood per Day and concentrates fluid and waste products into about 1 liter of urine per Day. Because of this, the kidneys receive more exposure to toxic substances in the body than almost any other organ. Therefore, they are highly susceptible to injury from toxic substances. Analgesic nephropathy is one of the most common types of toxic damage to the kidney. Exposure to lead, cleaning products, solvents, fuels, or other nephrotoxic chemicals (those which can be toxic to the kidney) can damage kidneys. Excessive buildup of body waste products, such as uric acid (that can occur with gout or with treatment of bone marrow, lymph node, or other disorders) can also damage the kidneys.

Inflammation (irritation with swelling and presence of extra immune cells) caused by immune responses to medications, infection, or other disorders may also injure the structures of the kidney, usually causing various types of glomerulonephritis or acute tubular necrosis (tissue death). Autoimmune disorders may also damage the kidneys. Injury to the kidney may result in short-term damage with minimal or no symptoms. Alternately, it can be life-threatening because of bleeding and associated shock, or it may result in acute renal failure or chronic renal failure.

Ureteral injuries (injuries to the tubes which carry urine from the kidneys to the bladder) can also be caused by trauma (blunt or penetrating), complications from medical procedures, and other diseases in the retroperitoneum such as retroperitoneal fibrosis (RPF), retroperitoneal sarcomas, or metastatic lymph node positive cancers. Medical therapies (such as OB/GYN surgeries, prior radiation or chemotherapy, and previous abdominopelvic surgeries) increase the risk for ureteral injuries.

**A. Acute Kidney Failure**

Acute (sudden) kidney failure is the sudden loss of the ability of the kidneys to remove waste and concentrate urine without losing electrolytes. There are many possible causes of kidney damage including, but are not limited to, decreased blood flow, which may occur with extremely low blood pressure caused by trauma, surgery, serious illnesses, septic shock, hemorrhage, burns, or dehydration, acute tubular necrosis (ATN), infections that directly injury the kidney such as acute pyelonephritis or septicemia, urinary tract obstruction (obstructive uropathy), autoimmune kidney disease such as interstitial nephritis or acute nephritic syndrome, disorders that cause clotting within the thin blood vessels of the kidney, idiopathic thrombocytopenic thrombotic purpura (ITTP), transfusion reaction, malignant hypertension, scleroderma, hemolytic-uremic syndrome, disorders of childbirth, such as bleeding placenta abruptio or placenta previa

Symptoms of acute kidney failure may include, but are not limited to, decrease in amount of urine (oliguria), urination stops (anuria), excessive urination at night, ankle, feet, and leg swelling, generalized swelling, fluid retention, decreased sensation, especially in the hands or feet, decreased appetite, metallic taste in mouth, persistent hiccups, changes in mental status or mood, agitation, drowsiness, lethargy, delirium or confusion, coma, mood changes, trouble paying attention, hallucinations, slow, sluggish, movements, seizures, hand tremor (shaking), nausea or vomiting, may last for Days, bruising easily, prolonged bleeding, nosebleeds, bloody stools, flank pain (between the ribs and hips), fatigue, breath odor, or high blood pressure.

Acute renal failure (ARF) may also be referred to as acute kidney injury (AKI) and may be characterized by an abrupt (i.e., for example, typically detected within about 48 hours to 1 week) reduction in glomerular filtration rate (GFR). This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in association with their respective risk factors are summarized below. See, Table 4; In: Merck Manual, 17th ed., Chapter 222, and which is hereby incorporated by reference in their entirety..

Table 4. Representative Acute Renal Failure Risk Factors

| Type of Renal Failure | Risk Factors |
| --- | --- |
| Prerenal | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| Intrinsic Renal | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulointerstitial nephritis | Drug reaction (eg, $\beta$-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |

(continued)

| Postrenal | |
|---|---|
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to Days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of Days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 Days, resolution within 1 week) rise in blood urea nitrogen and serum creatinine.

A commonly reported criteria for defining and detecting AKI is an abrupt (typically within about 2-7 Days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI.

For example, relationships between elevated serum creatinine and AKI has been reported to be associated with health risks. Praught et al., Curr Opin Nephrol Hypertens 14:265-270 (2005); and Chertow et al., J Am Soc Nephrol 16:3365-3370 (2005) (both references are herein incorporated by reference in their entirety). As described in these publications, acute worsening renal function (AKI) and increased risk of death and other detrimental outcomes are now known to be associated with very small increases in serum creatinine. These creatinine increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 Days, 3 Days, 7 Days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

Another study correlated serum creatinine levels with post-surgical mortality rates. Following heart surgery, patients with a mild fall in serum creatinine (i.e., for example, between approximately -0.1 to -0.3 mg/dL) had the lowest mortality rate, wherein patients had a larger mortality rate associated with either large falls in serum creatinine (i.e., for example, more than or equal to -0.4 mg/dL), or an increase in serum creatinine. Lassnigg et al., J Am Soc Nephrol 15:1597-1605 (2004), herein incorporated by reference in its entirety. These findings suggested that even very subtle changes in renal function, as detected by small creatinine changes within 48 hours of surgery, can be predictive of a patient's outcome.

A unified classification system using serum creatinine to define AKI in clinical trials and in clinical practice was proposed to stratify AKI patients. Bellomo et al., Crit Care 8(4):R204-212 (2004), which is herein incorporated by reference in its entirety. For example, a serum creatinine rise of 25% may define contrast-induced nephropathy. McCollough et al, Rev Cardiovasc Med. 7(4): 177-197 (2006), herein incorporated by reference in its entirety. Although various groups propose slightly different criteria for using serum creatinine to detect AKI, the consensus is that small changes in serum creatinine, such as 0.3 mg/dL (i.e., for example, approximately 25%) are sufficient to detect AKI that characterizes a worsening renal function and that the magnitude of the serum creatinine change may be an indicator of the severity of the AKI and mortality risk.

Although serial measurement of serum creatinine over a period of Days is an accepted method of detecting and diagnosing AKI patients, serum creatinine is generally regarded to have several limitations in the diagnosis, assessment and monitoring of AKI patients. The time period for serum creatinine to rise to approximately 0.3 mg/dL (25%) is considered diagnostic for AKI can be 48 hours or longer depending on the definition used.

Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Until defined by some embodiments of the present invention, there were no methods to determine whether some patients with AKI would recover fully, or whether some woudl need dialysis (either short term or long term), or whether some would have other detrimental outcomes including, but not limited to, death, major adverse cardiac events or chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

**B. Chronic Kidney Failure**

Unlike acute renal failure, chronic renal failure slowly gets worse. It most often results from any disease that causes gradual loss of kidney function. It can range from mild dysfunction to severe kidney failure. Chronic renal failure may lead to end-stage renal disease (ESRD).

Chronic renal failure usually occurs over a number of years as the internal structures of the kidney are slowly damaged. In the early stages, there may be no symptoms. In fact, progression may be so slow that symptoms do not occur until kidney function is less than one-tenth of normal.

Chronic renal failure and ESRD affect more than 2 out of 1,000 people in the United States. Diabetes and high blood pressure are the two most common causes and account for most cases. Other major causes include, but are not limited to, Alport syndrome, analgesic nephropathy, glomerulonephritis of any type (one of the most common causes), kidney stones and infection, obstructive uropathy, polycystic kidney disease, or reflux nephropathy. Chronic renal failure results in an accumulation of fluid and waste products in the body, leading to a build up of nitrogen waste products in the blood (azotemia) and general ill health. Most body systems are affected by chronic renal failure.

Initial symptoms may include, but are not limited to, fatigue, frequent hiccups, general ill feeling, generalized itching (pruritus), headache, nausea, vomiting, or unintentional weight loss. Further, later symptoms may include, but are not limited to, blood in the vomit or in stools,
decreased alertness, including drowsiness, confusion, delirium, orcoma, decreased sensation in the hands, feet, or other areas, easy bruising or bleeding, increased or decreased urine output, muscle twitching or cramps, seizures, or white crystals in and on the skin (uremic frost).

Circulating levels of cytokines and other inflammation markers are markedly elevated in patients with chronic renal failure. This could be caused by increased generation, decreased removal, or both. However, it is not well established to what extent renal function per se contributes to the uremic proinflammatory milieu. Relationships between inflammation and glomerular filtration rate (GFR) were reported in 176 patients (age, 52 +/-1 years; GFR, 6.5 +/- 0.1 mL/min) close to the initiation of renal replacement therapy. Pecoits-Filho et al., "Associations between circulating inflammatory markers and residual renal function in CRF patients" Am J Kidney Dis. 41(6): 1212-1218 (2003). For example, circulating levels of high-sensitivity C-reactive protein (hsCRP), tumor necrosis factor-alpha (TNF-alpha), interleukin-6 (IL-6), hyaluronan, and neopterin were measured after an overnight fast. Patients subsequently were subdivided into two groups according to median GFR (6.5 mL/min). Despite the narrow range of GFR (1.8 to 16.5 mL/min), hsCRP, hyaluronan, and neopterin levels were significantly greater in the subgroup with lower GFRs, and significant negative correlations were noted between GFR and IL-6 (rho = -0.18; $P < 0.05$), hyaluronan (rho = -0.25; $P < 0.001$), and neopterin (rho = -0.32; $P < 0.0005$). In a multivariate analysis, age and GFR were associated with inflammation but cardiovascular disease and diabetes mellitus were not. These results show that a low GFR per se is associated with an inflammatory state, suggesting impaired renal elimination of proinflammatory cytokines, increased generation of cytokines in uremia, or an adverse effect of inflammation on renal function.

**C. Dialysis**

Dialysis (i.e., for example, renal replacement therapy) is a method of removing toxic substances (impurities or wastes) from the blood when the kidneys are unable to do so and can be performed using several different methods. For example, peritoneal dialysis may filter waste by using the peritoneal membrane inside the abdomen. The abdomen is filled with special solutions that help remove toxins. The solutions remain in the abdomen for a time and then are drained out. This form of dialysis can be performed at home, but must be done every Day. Alternatively, hemodialysis may be performed by circulating the blood through special filters outside the body. The blood flows across a filter, along with solutions that help remove toxins.

Dialysis uses special ways of accessing the blood in the blood vessels. The access can be temporary or permanent. Temporary access takes the form of dialysis catheters -- hollow tubes placed in large veins that can support acceptable blood flows. Most catheters are used in emergency situations for short periods of time. However, catheters called tunneled catheters can be used for prolonged periods of time, often weeks to months. Permanent access is created by surgically joining an artery to a vein. This allows the vein to receive blood at high pressure, leading to a thickening of the vein's wall. This vein can handle repeated puncture and also provides excellent blood flow rates. The connection between an artery and a vein can be made using blood vessels (an arteriovenous fistula, or AVF) or a synthetic bridge (arteriovenous graft, or AVG). Blood is diverted from the access point in the body to a dialysis machine. Here, the blood flows counter-current to a special solution called the dialysate. The chemical imbalances and impurities of the blood are corrected and the blood is then returned to the body. Typically, most patients undergo hemodialysis for three sessions every week. Each session lasts 3 - 4 hours.

The purpose of dialysis is to assist kidney functions including, filters for the blood, removing waste products, regulating body water, maintaining electrolyte balance, or maintaining blood pH remains between 7.35 and 7.45. Further, dialysis may replace some of the functions for kidneys that aren't working properly that would otherwise result in the death of a patient.

Dialysis is most often used for patients who have kidney failure, but it can also quickly remove drugs or poisons in acute situations. This technique can be life saving in people with acute or chronic kidney failure.

**II. Urinary Renal Biomarkers**

Currently, no effective treatments exist to improve renal recovery, or to improve short and long-term renal outcome, after AKI. Furthermore, methods to predict recovery are also lacking. The emerging role of biomarkers for early detection of renal disease and/or renal injury may help identify new prognostic tools to predict renal clinical outcomes. Potential candidates for biomarkers of renal recovery include, but are not limited to, molecules expressed in pathways leading to regeneration and proliferation as well as markers of fibrosis and apoptosis. In addition, renal injury biomarkers may also serve to distinguish early resolution, and hence increased odds of recovery.

Acute kidney injury (AKI) has an estimated incidence rate of approximately 2000 per million population and this rate is increasing. Ali et al., "Incidence and outcomes in acute kidney injury: a comprehensive population-based study" J Am Soc Nephrol 18:1292-1298 (2007). Approximately 5% of all people admitted to intensive care units around the world develop severe AKI requiring dialysis. Uchino et al., "Acute renal failure in critically ill patients: a multinational, multicenter study" JAMA 294:813-818 (2005). A recent, United States multi-center study found that fewer than only about 60% patients surviving severe AKI recovered renal function by two months. Palevsky et al., "Intensity of renal support in critically ill patients with acute kidney injury" N Engl J Med 359:7-20 (2008). Thus, a large number of patients with AKI progress into end-stage renal disease (ESRD).

However, since only a fraction of patients with AKI fail to recover renal function, interventions aimed at improving recovery or at providing renal support (e.g. early dialysis) cannot be selectively targeted appropriately without some means of determining which patients will recover and which will not recover (i.e., for example, the availability of non-invasive biomarkers). Currently, clinical risk prediction for recovery after AKI is extremely limited. Thus, development of a non-invasive biomarker that allows early prediction of recovery of kidney function is a long felt need in the art of renal disease management.

The identification of such non-invasive biomarkers (i.e., for example, a urinary biomarker) would greatly improve long-term prognosis thereby tailoring research efforts to treat AKI and prevent ESRD. In other words, having the ability to predict which patients will not recover kidney function allows a clinician to focus limited resources on the development and application of aggressive treatment interventions on these predicted at-risk patients. Conversely, patients with a favorable prognosis would be spared from more aggressive interventions and their potential adverse effects, thereby releasing medical resources to those in need and reducing overall medical costs.

In one embodiment, the present invention contemplates methods and compositions for evaluating renal function in a subject. As described herein, measurement of various kidney injury markers described herein can be used for diagnosis, prognosis, risk stratification, staging, monitoring, categorizing and a determination of further diagnosis and treatment regimens in subjects suffering or at risk of suffering from an injury to renal function, reduced renal function, and/or acute renal failure (also called acute kidney injury).

Renal biomarkers as described herein may be used individually, or in panels, comprising a plurality of renal biomarkers, for risk stratification. In one embodiment, risk stratification identifies subjects at risk for a future: i) injury to renal function; ii) progression to reduced renal function; iii) progression to ARF; or iv) improvement in renal function, etc. In one embodiment, risk stratification diagnoses an existing disease, comprising identifying subjects who have: i) suffered an injury to renal function; ii) progressed to reduced renal function; or iii) progressed to ARF, etc.. In one embodiment, risk stratification monitors for deterioration and/or improvement of renal function. In one embodiment, risk stratification predicts a future medical outcome including, but not limited to, an improved or worsening renal function, a decreased or increased mortality risk, a decreased or increased risk that a subject will require initiation or continuation of renal replacement therapy (i.e., hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, etc.

### III. Clinical Renal Biomarker Studies

The results of a large multicenter clinical trial has recently been reported comparing two intensities of renal support for critically ill patients with acute kidney injury (AKI) in which recovery of renal function was less than 25% at 28 days and not different between the two treatment strategies. Palevsky et al., "Intensity of renal support in critically ill patients with acute kidney injury" N Engl J Med 359:7-20 (2008). These results emphasize that incomplete renal recovery is a common problem in the patients who survive severe AKI. Uchino et al., "Acute renal failure in critically ill patients: a multinational, multicenter study" JAMA, 294: 813-818 (2005). Failure to recover renal function can have tremendous negative effects on quality of life and health care costs. Manns et al., "Cost of acute renal failure requiring dialysis in the intensive care unit: clinical and resource implications of renal recovery" Crit Care Med, 31:449-455 (2003). Therefore, treatments to hasten and facilitate renal recovery are eagerly being sought by both the critical care and nephrology communities. Unfortunately, there are no effective treatments to improve renal recovery. One possible barrier to progress in this area has been the inability to forecast recovery in individual patients. The ability to prognosticate in an AKI patient population would be extremely valuable both for clinical decisions as well as to guide future research on therapy to promote recovery of renal function.

One clinical study reported that patients who recovered from AKI did not appear to differ in clinical characteristics (i.e., for example, age, gender, mechanical ventilation status, or clinical severity scores) from the non-recovery group. Bhandari et al., "Survivors of acute renal failure who do not recover renal function" QJM, 89:415-421 (1996). Secondary analysis from three randomized controlled trials (RCTs) comparing efficacy of continuous renal replacement therapy (RRT) versus intermittent RRT found that: i) APACHE III scores > 100; ii) cardiovascular instability; and iii) pre-existing renal impairment were all associated with renal non-recovery. Mehta et al., "A randomized clinical trial of continuous versus intermittent dialysis for acute renal failure" Kidney Int, 60:1154-1163 (2001); Augustine et al., "A randomized controlled trial comparing intermittent with continuous dialysis in patients with ARF" Am J Kidney Dis, 44:1000-1007 (2004); and Uehlinger et al., "Comparison of continuous and intermittent renal replacement therapy for acute renal failure" Nephrol Dial Transplant, 20:1630-1637 (2005), respectively. However, these studies did not adhere to a uniform definition of, or standard timing, to assess renal recovery.

Other studies have suggested that baseline creatinine and urine output at the time of discontinuation of RRT were most predictive of recovery. Uchino et al., "Discontinuation of continuous renal replacement therapy: a post hoc analysis of a prospective multicenter observational study" Crit Care Med, 37:2576-2582 (2009). However, urine output was analyzed after RRT had ended based on a clinical decision rather than at a fixed time point (e.g. 14 Days post AKI) to predict renal recovery. Therefore, these data are compromised to suggest that urine output was predictive of renal recovery, and further, baseline creatinine might have been less valuable because patients with stage 4 and 5 CKD were excluded.

Recently, a number of urinary biomarkers have been investigated for the purpose of early diagnosis of AKI. Since these markers correlate with renal tubular cell injury or function, their patterns in the urine, either alone or in combination, could provide new prognostic information regarding renal recovery. For example, several reports have suggested possible candidate renal biomarkers relating to three aspects of the physiology of renal recovery:

i) inflammatory markers including; a) urinary neutrophil gelatinase-associated lipocalin (uNGAL), which has been extensively studied for predicting AKI (Supavekin et al., "Differential gene expression following early renal ischemia/reperfusion" Kidney Int, 63:1714-1724 (2003); Mishra et al., "Kidney NGAL is a novel early marker of acute injury following transplantation" Pediatr Nephrol, 21:856-863 (2006); Hirsch et al., "NGAL is an early predictive biomarker of contrast-induced nephropathy in children" Pediatr Nephrol, 22: 2089-2095 (2007); and Zappitelli et al., "Urine neutrophil gelatinase-associated lipocalin is an early marker of acute kidney injury in critically ill children: a prospective cohort study" Crit Care, 11: R84 (2007); b) matrix metalloproteinase protein-9 (MMP-9), a matrix degradation enzyme which is up-regulated after ischemic injury in animal models and links to NGAL by a disulfide bond forming urinary NGAL/MMP-9 (uNGAL/MMP-9) (Ronco et al., "Matrix metalloproteinases in kidney disease progression and repair: a case of flipping the coin" Semin Nephrol, 27:352-362 (2007); and c) urinary interleukin-18 (uIL-18), an inflammatory cytokine which is found to potentiate ischemic AKI and has been tested in many clinical settings (Parikh et al., "Urine IL-18 is an early diagnostic marker for acute kidney injury and predicts mortality in the intensive care unit" J Am Soc Nephrol, 16:3046-3052 (2005); and Parikh et al., "Urinary IL-18 is an early predictive biomarker of acute kidney injury after cardiac surgery" Kidney Int, 70:199-203 (2006);

ii) growth factors including urinary hepatocyte growth factor (uHGF), a biomarker linked to renal tubular epithelial cell regeneration (Liu et al., "Hepatocyte growth factor: new arsenal in the fights against renal fibrosis? Kidney Int, 70:238-240 (2006); and

iii) filtration and tubular reabsorption markers, such as cystatin C, which is freely filtered and is normally completely reabsorbed by proximal tubular epithelial cells and urine creatinine. Herget-Rosenthal et al., "Measurement of urinary cystatin C by particle-enhanced nephelometric immunoassay: precision, interferences, stability and reference range" Ann Clin Biochem, 41:111-118 (2004).

Despite these reports, only a few suggest biomarkers having an ability to predict AKI severity. But no study has identified a biomarker as a predictor of renal recovery. Coca et al., "Biomarkers for the diagnosis and risk stratification of acute kidney injury: a systematic review" Kidney Int, 73:1008-1016 (2008). The data presented herein provide heretofore unknown renal biomarkers identified by proteomic gene expression analysis. The data was obtained from urine samples collected during a clinical study as described below.

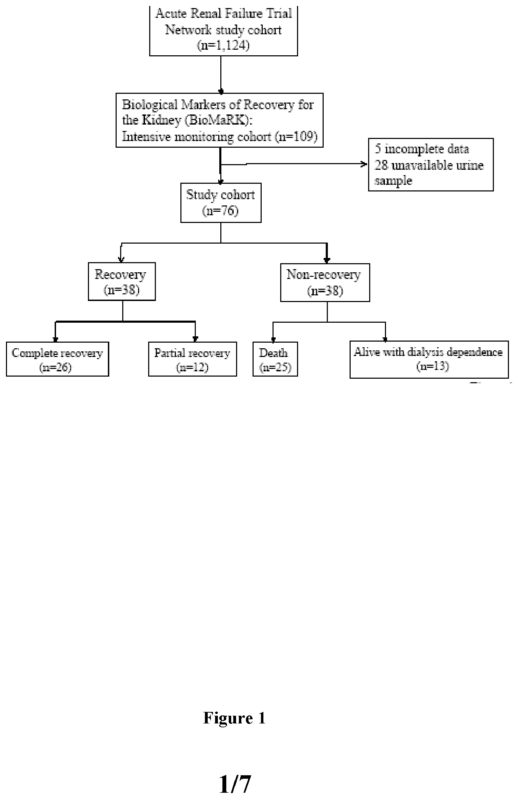

The data presented herein was collected from 109 patients in the BioMaRK clinical study where 76 patients had complete data available including urine samples. Exactly half (38 patients) recovered renal function (alive and without requirement for dialysis) by day 60. See, Figure 1. Baseline clinical characteristics of the study patients were taken. See, Table 1.

**Table 1**. Summary of baseline and clinical characteristics of the study patients

| Characteristics | All subjects (n= 76) | Recovery (n=38) | Non-recovery (n=38) | *P* value |
|---|---|---|---|---|
| Age, mean (SD), -yr | 58.4(17.0) | 52.2(15.7) | 64.7(16.2) | <0.001 |
| Gender: Female (%) | 30(39.5) | 15(39.5) | 15(39.5) | 1.00 |
| Race: White (%) | 64(84.2) | 30(79.0) | 34(89.5) | 0.21 |
| Baseline serum creatinine, mean (SD) (mg/dl) | 1.1(0.4) | 1.1(0.4) | 1.2(0.5) | 0.45 |
| BUN at initiation of RRT, mean (SD) (mg/dl) | 55.6(29.9) | 51.3(28.8) | 59.9(30.8) | 0.23 |
| Cause of acute kidney injury | | | | |
| Ischemia (%) | 66(86.8) | 29(76.3) | 37(97.4) | 0.007 |
| Nephrotoxins (%) | 16(21.3) | 10(26.3) | 6(16.2) | 0.29 |
| Sepsis (%) | 50(65.8) | 23(60.5) | 27(71.1) | 0.33 |
| Multifactorial causes (%) | 51(68.0) | 25(65.8) | 26(70.3) | 0.68 |
| Length of ICU stay before randomization-days, mean (SD) | 5.4(4.1) | 4.2(2.8) | 6.5(4.9) | 0.03 |
| Length of hospital stay before randomization-days, mean (SD) | 8.5(7.1) | 6.7(5.0) | 10.2(8.5) | 0.08 |
| Charlson comorbidity index[a], mean (SD) | 4.1(3.3) | 3.3(3.8) | 4.9(2.7) | 0.008 |
| Mechanical ventilation (%) | 69(90.8) | 34(89.5) | 35(92.1) | 1.00 |
| Sepsis[b] (%) | 47(62.7) | 22(57.9) | 25(67.6) | 0.39 |
| APACHE II score[c], mean (SD) | 23.4(7.2) | 21.8(7.2) | 25.0(6.8) | 0.06 |
| Non-renal SOFA organ-system score[d], mean (SD) | | | | |

(continued)

| Characteristics | All subjects (n= 76) | Recovery (n=38) | Non-recovery (n=38) | P value |
|---|---|---|---|---|
| Respiratory | 2.1(1.3) | 2.1(1.5) | 2.1(1.2) | 0.98 |
| Coagulation | 1.5(1.3) | 1.4(1.3) | 1.5(1.3) | 0.58 |
| Liver | 0.9(1.3) | 1.2(1.5) | 0.6(1.0) | 0.08 |
| Cardiovascular | 2.2(1.7) | 2.0(1.7) | 2.5(1.6) | 0.17 |
| Central nervous system | 2.2(1.4) | 2.3(1.3) | 2.1(1.5) | 0.45 |
| Total | 8.9(4.0) | 9.2(4.6) | 8.5(3.3) | 0.43 |
| Cleveland Clinic ICU ARF Renal Failure score[e], mean (SD) | 11.9(3.0) | 11.6(3.0) | 12.2(3.0) | 0.49 |
| Intensive strategy[f] (%) | 34(44.7) | 18(47.4) | 16(42.1) | 064 |

Abbreviations: RRT, Renal Replacement Therapy. ICU, Intensive Care Unit. APACHE II, Acute Physiology and Chronic Health Evaluation II. SOFA, Sequential Organ Failure Assessment. ARF, Acute Renal Failure.

[a] According to the method of Charlson et al.[28]

[b] Defined as sepsis plus acute organ dysfunction according to 2001 international consensus criteria for sever sepsis.[26]

[c] According to the method of Knaus et al.[29]

[d] Non renal SOFA score, excluding the renal part, assessed on the first day according to the method of Vincent et al.[30]

[e] According to the method of Thakar et al.[31]

[f] Intensive strategy, intermittent hemodialysis and sustained low-efficiency dialysis were provided six times per week (every day except Sunday), and continuous venovenous hemodiafiltration was prescribed to provide a flow rate of the total effluent (the sum of the dialysate and ultrafiltrate) of 35 ml per kilogram of body weight per hour, based on the weight before the onset of acute illness.[1]

Patients recovering from renal injury were more likely to be younger, had a shorter length of intensive care unit (ICU) stay before randomization, lower Charlson comorbidity index, and lower nonrenal SOFA score as compared to those not recovering renal function. By contrast, there were no statistical differences in gender, ethnicity, baseline serum creatinine, blood urea nitrogen (BUN) at initiation of RRT, length of hospital stay, length of ICU stay, requirement for mechanical ventilation, Cleveland Clinic ICU Acute Renal Failure (ARF) score, or intensity of RRT. The primary etiology of AKI was ischemia in both groups. However, a significantly lower percentage of ischemia (76.3%) was noted as the cause of AKI in the recovery group compared to 97.4% in non-recovery group. Of the 38 participants who recovered renal function, 26 (68.4%) had complete recovery. Among those failing to recover renal function, 25 patients (65.8%), did not survive past day 60.

### IV. Proteomics Gene Expression Platforms

In one embodiment, the present invention contemplates a method for identifying urinary biomarkers using a proteomics platform. In one embodiment, the proteomics platform detects protein expression profiles. In one embodiment, the method further comprises comparing a first protein expression profile to a second protein expression profile. In one embodiment, the comparing identifies an overexpressed protein in the first protein expression profile relative to the second protein expression profile. In one embodiment, the comparing identifies an underexpressed protein in the first protein expression profile relative to the second protein expression profile.

### A Introduction

In one embodiment, the present invention contemplates a method comprising a proteomics platform (i.e., for example, iTRAQ) capable of summarizing an analysis of relative protein biomarker expression. For example, the proteomics platform may use reporter ion peak area measurements (i.e., for example, supplied by ABI software) to estimate treatment-dependent peptide and protein biomarker relative expression. Such estimations may be accomplished using a Bayesian approach. The proteomics platform described herein includes a protein biomarker relative expression summary and a per-protein biomarker detailed analysis.

**B Experiment and Model Description**

1. Experiment Design

Proteomic platforms contemplated herein may summarize data from one or more experiments addressing a common comparison. For example, a possible experimental design for such an analysis is presented below. See, Table 5.

Table 5: Representative Proteomic Experimental Designs

|   | Experiment | Treatment | Channel | Sample |
|---|---|---|---|---|
| 1 | A | A | 113 | A1 |
| 2 | A | A | 114 | A2 |
| 3 | A | A | 115 | A3 |
| 4 | A | A | 116 | A4 |
| 5 | A | B | 117 | B1 |
| 6 | A | B | 118 | B2 |
| 7 | A | B | 119 | B4 |
| 8 | A | B | 121 | B4 |

2. Input Files

Data for proteomic analyses may be extracted from input files including but not limited to a tandem mass spectra (MSMS) summary file, such as:

| Experiment | MSMS Summary File |
|---|---|
| A | SCW_XIII_70.txt |

3. Statistical Modeling

Statistical models to estimate the treatment-dependent effects may including but not limited to: LogIntensity ~ Channel + Spectrum + Protein + Peptide + Protein: Treatment + Peptide: Treatment.

4. Data Summarization

The proteomics platform may comprise filtering the data supplied in the MSMS summary to remove unidentified proteins, contaminants, and/or peptides containing selected modifications. A representative analysis may provide a data summary as presented below. See, Table 6.

Table 6: Representative Data Summary

|  | A | Combined |
|---|---|---|
| Supplied Spectra | 4608 | 4608 |
| Unidentified Spectra | 0 | 0 |
| Disallowed Modifications | 249 | 249 |
| Spectra from Contaminants | 1210 | 1210 |
| Missing Data | 115 | 115 |
| Low Confidence Spectra | 0 | 0 |
| Degenerate Peptides | 379 | 379 |
| Remaining Spectra |  | 2655 |
| Unique Proteins |  | 360 |

(continued)

| | A | Combined |
|---|---|---|
| Unique Peptides | | 1473 |
| Model R2 | | 0.767 |

## C. Protein Biomarker Summary

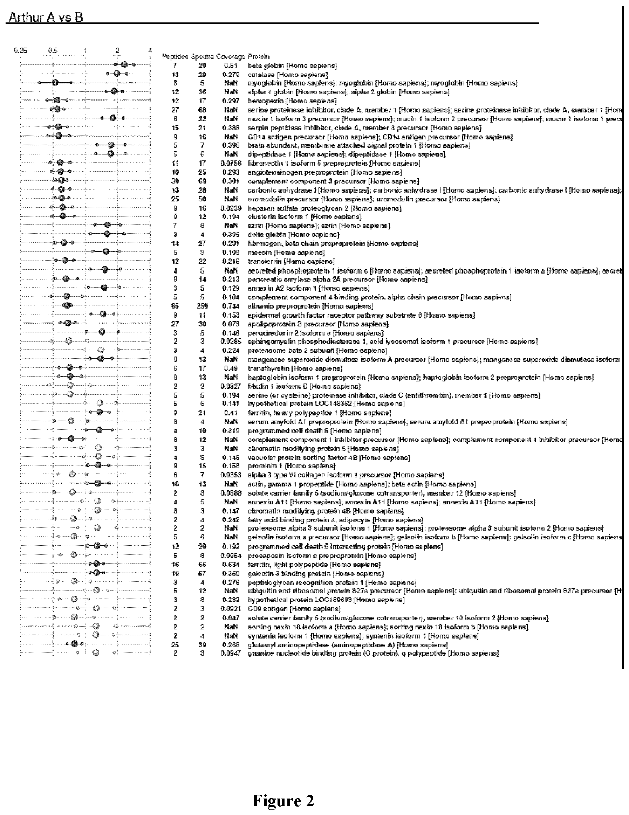

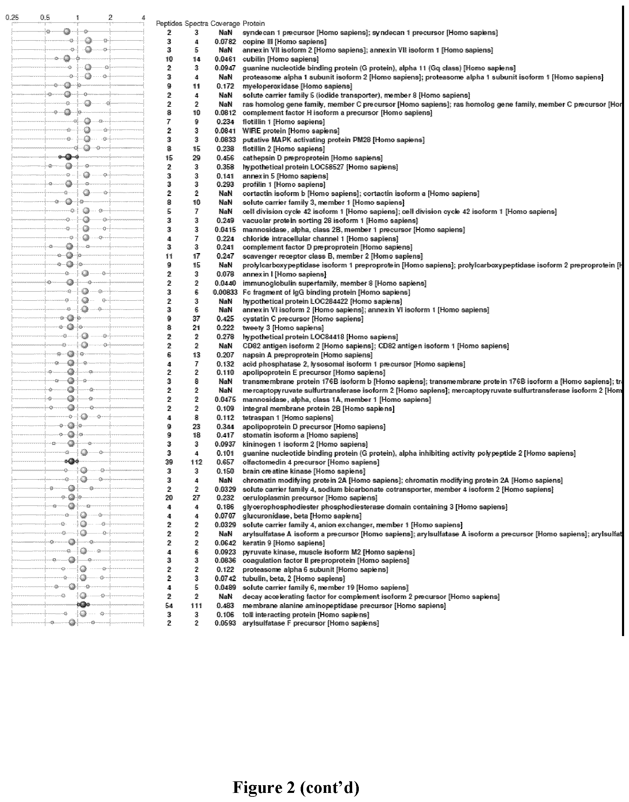

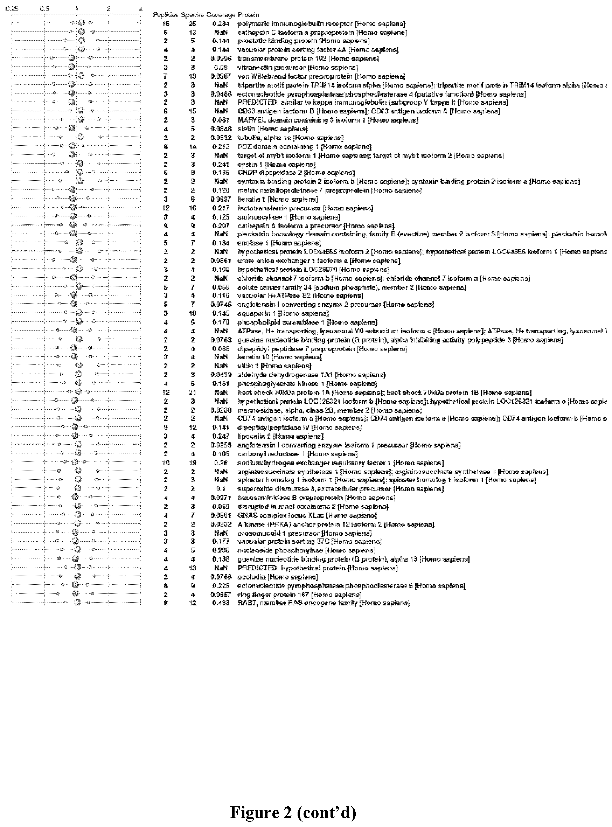

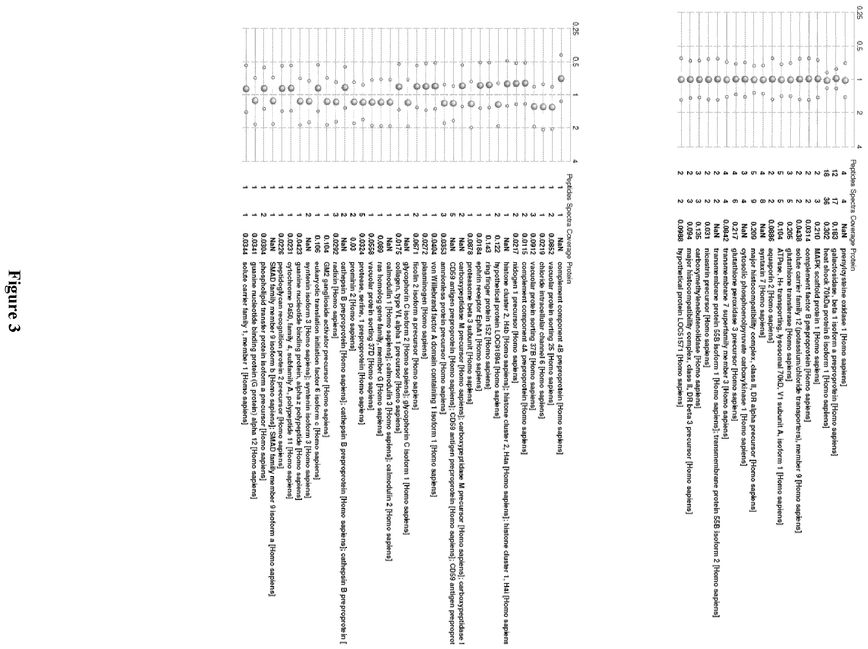

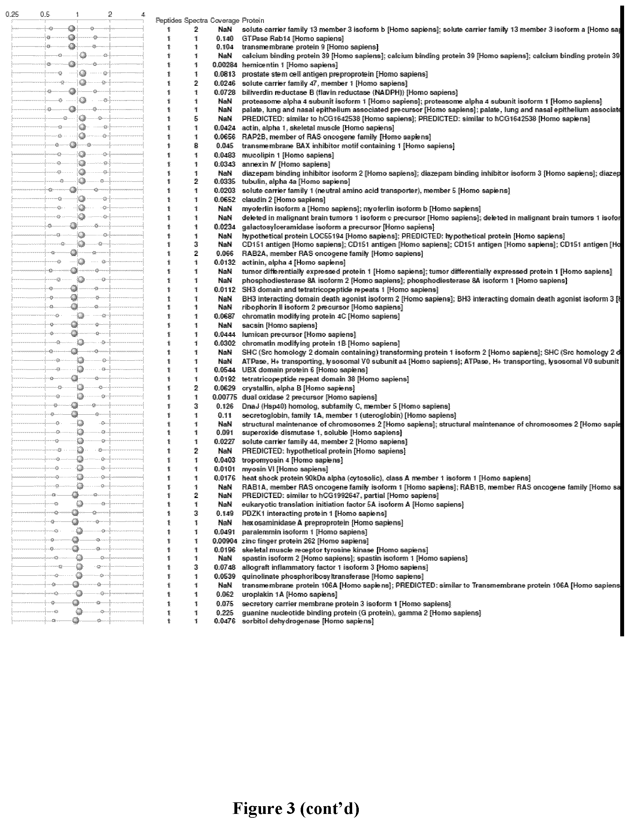

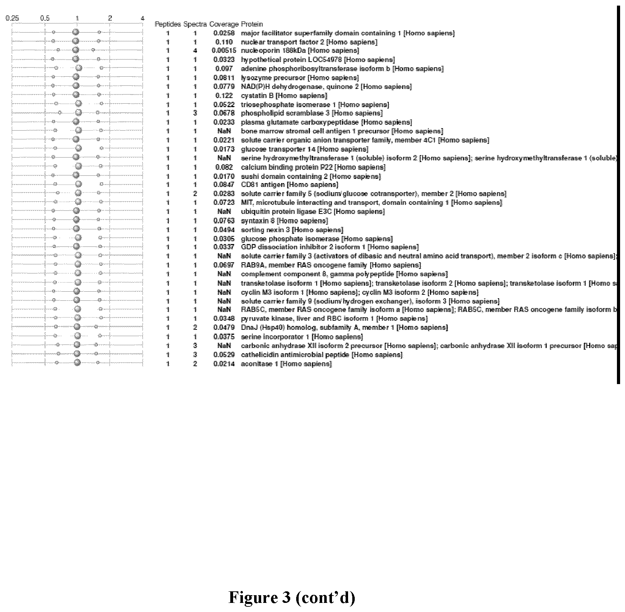

In one embodiment, the proteomics platform may identify each protein biomarker in one or more of the MSMS summaries, for example, in decreasing order of expression change magnitude. See, Figure 2. The median and estimated credible interval for each protein biomarker is given to the left in the table. Similar data is shown where protein bioarkers are identified by a single peptide. See Figure 3.

## D. Protein Biomarker Details

A detailed summary of each protein biomarker is given below, wherein each protein biomarker is designated as 5.###. These sections include protein biomarker relative expression estimates in addition to protein-level estimates.

5.1 beta globin [Homo sapiens]

| | |
|---|---|
| Protein Accession | gi\|4504349 |
| Mean Expression Ratio | 2.31 |
| Median Expression Ratio | 2.31 |
| Credible Interval | (1.9, 2.82) |
| Associated Peptides | 7 |
| Associated Spectra | 29 |
| Coverage | 0.51 |

| A | 2.5 | 50 | 97.5 | Sequence |
|---|---|---|---|---|
| 1 | 1.6 | 2.2 | 3.0 | EFTPPVQAAYQK |
| 3 | 1.8 | 2.4 | 3.1 | FESFGDLSTPDAVMGNPK |
| 4 | 1.8 | 2.4 | 3.2 | FFESFGDLSTPDAVMGNPK |
| 4 | 1.9 | 2.5 | 3.3 | GTFATLSELHCDK |
| 13 | 2.1 | 2.6 | 3.2 | LLGNVLVCVLAHHFGK |
| 3 | 1.6 | 2.2 | 2.9 | VNVDEVGGEALGR |
| 1 | 1.7 | 2.4 | 3.3 | FRLLGNVLVCVLAHHFGK |

1     M V H L T P E E K S A V T A L W G K V N V D E V G G E A L G R L L V V Y P W T Q R F F E S F G D L S T P D A V M G N P K V K A H G K K V L G A F S D G L A H L D

81     N L K G T F A T L S E L H C D K L H V D P E N F R L L G N V L V C V L A H H F G K E F T P P V Q A A Y Q K V V A G V A N A L A H K Y H

5.2 catalase [Homo sapiens]

| | | | |
|---|---|---|---|
| Protein Accession | gi|4557014 |
| Mean Expression Ratio | 1.97 |
| Median Expression Ratio | 1.97 |
| Credible Interval | (1.61, 2.40) |
| Associated Peptides | 13 |
| Associated Spectra | 20 |
| Coverage | 0.279 |

| A | 2.5 | 50 | 97.5 | Sequence |
|---|---|---|---|---|
| 2 | 1.4 | 1.8 | 2.5 | AFYVNVLNEEQR |
| 2 | 1.4 | 2.0 | 2.6 | DPILFPSFIHSQK |
| 1 | 1.5 | 2 | 2.8 | FSTVAGESGSADTVR |
| 4 | 1.7 | 2.2 | 2.9 | GPLLVQDVVFTDEMAHFDR |
| 1 | 1.6 | 2.2 | 3 | GPRGPLLVQDVVFTDEMAHFDR |
| 1 | 1.6 | 2.1 | 3.0 | IRDPILFPSFIHSQK |
| 1 | 1.5 | 2 | 2.7 | LFAYPDTHR |
| 1 | 1.4 | 1.9 | 2.6 | LSQEDPDYGIR |
| 2 | 1.4 | 2.0 | 2.7 | LVQDVVFTDEMAHFDR |
| 2 | 1.5 | 2.0 | 2.7 | NFTEVHPDYGSHIQALLDK |
| 1 | 1.4 | 2.0 | 2.7 | FAEVEQIAFDPSNMPPGIEASPDK |
| 1 | 1.4 | 1.9 | 2.7 | VFEHIGK |
| 1 | 1.4 | 2.0 | 2.7 | FNTANDDNVTQVR |

1 M A D S R D P A S D Q M Q H W K E Q R A A Q K A D V L T T G A G N P V G D K L N V I T V G P R G P L L V Q D V V F T D E M A H F D R E R I P E R V V H A K G A G

81 A F G Y F E V T H D I T K Y S K A K V F E H I G K K T P I A V R F S T V A G E S G S A D T V R D P R G F A V K F Y T E D G N W D L V G N N T P I F F I R D P I L

161 F P S F I H S Q K R N P Q T H L K D P D M V W D F W S L R P E S L H Q V S F L F S D R G I P D G H R H M N G Y G S H T F K L V N A N G E A V Y C K F H Y K T D Q

241 G I K N L S V E D A A R L S Q E D P D Y G I R D L F N A I A T G K Y P S W T F Y I Q V M T F N Q A E T F P F N P F D L T K V W P H K D Y P L I P V G K L V L N R

321 N P V N Y F A E V E Q I A F D P S N M P P G I E A S P D K M L Q G R L F A Y P D T H R H R L G P N Y L H I P V N C P Y R A R V A N Y Q R D G P M C M Q D N Q G G

401 A P N Y Y P N S F G A P E Q Q P S A L E H S I Q Y S G E V R R F N T A N D D N V T Q V R A F Y V N V L N E E Q R K R L C E N I A G H L K D A Q I F I Q K K A V K

481 N F T E V H P D Y G S H I Q A L L D K Y N A E K P K N A I H T F V Q S G S H L A A R E K A N L

5.3 myoglobin [Homo sapiens]; myoglobin [Homo sapiens]; myoglobin [Homo sapiens]

Protein Accession gi|4885477 gi|44955888 gi|44955885
Mean Expression Ratio 0.525
Median Expression Ratio 0.526
Credible Interval (0.373, 0.736)
Associated Peptides 3
Associated Spectra 5
Coverage NaN

| A | 2.5 | 50 | 97.5 | Sequence |
|---|-----|-----|------|----------|
| 1 | 0.35 | 0.53 | 0.8 | GLSDGEWQLVLNVWGK |
| 2 | 0.36 | 0.53 | 0.77 | HGATVLTALGGILK |
| 2 | 0.31 | 0.45 | 0.65 | VEADIPGHGQEVLIR |

5.4 alpha 1 globin [Homo sapiens]; alpha 2 globin [Homo sapiens]

Protein Accession gi|4504347 gi|4504345
Mean Expression Ratio 1.86
Median Expression Ratio 1.86
Credible Interval (1.56, 2.22)
Associated Peptides 12
Associated Spectra 36
Coverage NaN

| A | 2.5 | 50 | 97.5 | Sequence |
|---|-----|-----|------|----------|
| 2 | 1.6 | 2.2 | 2.9 | AAHLPAEFTPAVHASLDK |
| 1 | 1.4 | 1.9 | 2.7 | ALSALSDLHAHK |
| 1 | 1.4 | 1.9 | 2.6 | FLSFPTTK |
| 1 | 1.4 | 1.9 | 2.6 | FPHFDLSHGSAQVK |
| 1 | 1.4 | 1.9 | 2.7 | MFLSFPTTK |
| 7 | 1.6 | 2.1 | 2.6 | TYFPHFDLSHGSAQVK |
| 3 | 1.4 | 1.8 | 2.4 | VADALTNAVAHVDDMPNAL |
| 2 | 1.3 | 1.7 | 2.3 | VADALTNAVAHVDDMPNALSALSDLHAH |
| 1 | 1.5 | 2 | 2.7 | VDDMPNALSALSDLHAHK |
| 9 | 1.2 | 1.5 | 1.9 | VGAHAGEYGAEALER |

(continued)

| A | 2.5 | 50 | 97.5 | Sequence |
|---|-----|-----|------|----------|
| 6 | 1.5 | 1.9 | 2.4 | VADALTNAVAHVDDMPNALSALSDLHAHK |
| 2 | 1.4 | 1.8 | 2.4 | VDPVNFK |

5.5 hemopexin [Homo sapiens]

Protein Accession gi|11321561
Mean Expression Ratio 0.547
Median Expression Ratio 0.547
Credible Interval (0.444, 0.676)
Associated Peptides 12
Associated Spectra 17
Coverage 0.297

| A | 2.5 | 50 | 97.5 | Sequence |
|---|-----|-----|------|----------|
| 1 | 0.4 | 0.55 | 0.77 | DYFMPCPGR |
| 1 | 0.4 | 0.55 | 0.76 | EWFWDLATGTMK |
| 1 | 0.40 | 0.56 | 0.79 | FDPVRGEVPPR |
| 1 | 0.39 | 0.54 | 0.76 | FQGDREWFWDLATGTMK |
| 4 | 0.35 | 0.46 | 0.62 | LLQDEFPGIPSPLDAAVECHR |
| 1 | 0.4 | 0.56 | 0.77 | NFPSPVDAAFR |
| 2 | 0.4 | 0.55 | 0.75 | RLWWLDLK |
| 1 | 0.39 | 0.55 | 0.77 | SGAQATWTELPWPHEK |
| 2 | 0.4 | 0.55 | 0.75 | VAEGETKPDPDVTER |
| 1 | 0.39 | 0.54 | 0.75 | YYCFQGNQFLR |
| 1 | 0.39 | 0.54 | 0.77 | GIILDSVDAAFICPGSSR |
| 1 | 0.37 | 0.52 | 0.73 | LWWLDLK |

1 M A R V L G A P V A L G L W S L C W S L A I A T P L P P T S A H G N V A E G E T K P D P D V T E R C S D G W S F D A T T L D D N G T M L F F K G E F V W K S H K

81 W D R E L I S E R W K N F P S P V D A A F R Q G H N S V F L I K G D K V W V Y P P E K K E K G Y P K L L Q D E F P G I P S P L D A A V E C H R G E C Q A E G V L

161 F F Q G D R E W F W D L A T G T M K E R S W P A V G N C S S A L R W L G R Y Y C F Q G N Q F L R F D P V R G E V P P R Y P R D V R D Y F M P C P G R G H G H R N

241 G T G H G N S T H H G P E Y M R C S P H L V L S A L T S D N H G A T Y A F S G T H Y W R L D T S R D G W H S W P I A H Q W P Q G P S A V D A A F S W E E K L Y L

321 V Q G T Q V Y V F L T K G G Y T L V S G Y P K R L E K E V G T P H G I I L D S V
D A A F I C P G S S R L H I M A G R R L W W L D L K S G A Q A T W T E L P W P H

401 E K V D G A L C M E K S L G P N S C S A N G P G L Y L I H G P N L Y C Y S D V
E K L N A A K A L P Q P Q N V T S L L G C T H

5.6 serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]; serine proteinase inhibitor, clade A, member 1 [Homo sapiens]

Protein Accession gi|50363221 gi|50363219 gi|50363217 gi|189163542 gi|189163540 gi|189163538 gi|189163536
Mean Expression Ratio 0.551
Median Expression Ratio 0.551
Credible Interval (0.482, 0.627)
Associated Peptides 27
Associated Spectra 68
Coverage NaN

| A | 2.5 | 50 | 97.5 | Sequence |
|---|---|---|---|---|
| 9 | 0.46 | 0.57 | 0.7 | DTEEEDFHVDQVTTVK |
| 1 | 0.43 | 0.57 | 0.77 | DTVFALVNYIFFK |
| 2 | 0.40 | 0.53 | 0.71 | ELDRDTVFALVNYIFFK |
| 1 | 0.42 | 0.56 | 0.76 | GTEAAGAMFLEAIPMSIPPEVK |
| 1 | 0.4 | 0.54 | 0.72 | ITPNLAEFAFSLYR |
| 2 | 0.43 | 0.57 | 0.75 | KLSSWVLLMK |
| 2 | 0.44 | 0.58 | 0.77 | KQINDYVEK |
| 1 | 0.43 | 0.57 | 0.77 | LAEFAFSLYR |
| 4 | 0.4 | 0.52 | 0.67 | LGMFNIQHCK |
| 18 | 0.39 | 0.46 | 0.55 | LQHLENELTHDIITK |
| 5 | 0.42 | 0.53 | 0.68 | L VDKFLEDVK |
| 2 | 0.42 | 0.56 | 0.74 | QINDYVEK |
| 2 | 0.44 | 0.59 | 0.78 | SASLHLPK |
| 1 | 0.4 | 0.54 | 0.73 | SVLGQLGITK |
| 1 | 0.42 | 0.57 | 0.77 | TLNQPDSQLQLTTGNGLFLSEGLK |
| 1 | 0.4 | 0.54 | 0.73 | TVNFGDTEEAKK |
| 3 | 0.41 | 0.53 | 0.69 | VFSNGADLSGVTEEAPLK |
| 2 | 0.39 | 0.52 | 0.69 | WERPFEVK |
| 1 | 0.41 | 0.54 | 0.73 | LVDKFLEDVKK |
| 1 | 0.39 | 0.53 | 0.71 | GKWERPFEVK |
| 1 | 0.42 | 0.57 | 0.76 | AVLTIDEK |

(continued)

| A | 2.5 | 50 | 97.5 | Sequence |
|---|---|---|---|---|
| 1 | 0.4 | 0.54 | 0.73 | FLEDVKK |
| 1 | 0.41 | 0.56 | 0.75 | LSITGTYDLK |
| 2 | 0.42 | 0.56 | 0.73 | FLEDVK |
| 1 | 0.39 | 0.53 | 0.71 | EEEDFHVDQVTTVK |
| 1 | 0.43 | 0.58 | 0.77 | GLFLSEGLK |
| 1 | 0.41 | 0.55 | 0.74 | FNKPFVF |

5.7 mucin 1 isoform 3 precursor [Homo sapiens]; mucin 1 isoform 2 precursor [Homo sapiens]; mucin 1 isoform 1 precursor [Homo sapiens]

Protein Accession gi|67189069 gi|67189007 gi|65301117
Mean Expression Ratio 1.81
Median Expression Ratio 1.81
Credible Interval (1.45, 2.27)
Associated Peptides 6
Associated Spectra 22
Coverage NaN

| A | 2.5 | 50 | 97.5 | Sequence |
|---|---|---|---|---|
| 1 | 1.3 | 1.8 | 2.5 | DISEMFLQIYK |
| 2 | 1.4 | 1.9 | 2.6 | HDVETQFNQYK |
| 2 | 1.3 | 1.7 | 2.4 | KNYGQLDIFPAR |
| 4 | 1.5 | 1.9 | 2.6 | NYGQLDIFPAR |
| 1 | 1.3 | 1.8 | 2.4 | QGGFLGLSNIK |
| 12 | 1.6 | 2.0 | 2.5 | EGTINVHDVETQFNQYK |

5.8 serpin peptidase inhibitor, clade A, member 3 precursor [Homo sapiens]

Protein Accession gi|50659080
Mean Expression Ratio 0.563
Median Expression Ratio 0.563
Credible Interval (0.463, 0.681)
Associated Peptides 15
Associated Spectra 21
Coverage 0.388

| A | 2.5 | 50 | 97.5 | Sequence |
|---|---|---|---|---|
| 2 | 0.41 | 0.55 | 0.74 | AVLDVFEEGTEASAATAVK |
| 1 | 0.39 | 0.54 | 0.75 | DYNLNDILLQLGIEEAFTSK |
| 1 | 0.42 | 0.58 | 0.8 | EIGELYLPK |

(continued)

| A | 2.5 | 50 | 97.5 | Sequence |
|---|---|---|---|---|
| 2 | 0.37 | 0.51 | 0.68 | HPNSPLDEENLTQENQDR |
| 1 | 0.38 | 0.53 | 0.73 | ITLLSALVETR |
| 1 | 0.42 | 0.57 | 0.8 | KLINDYVK |
| 1 | 0.4 | 0.55 | 0.76 | LYGSEAFATDFQDSAAAK |
| 3 | 0.45 | 0.6 | 0.8 | MEEVEAMLLPETLK |
| 1 | 0.42 | 0.57 | 0.79 | MEEVEAMLLPETLKR |
| 2 | 0.42 | 0.57 | 0.77 | NLAVSQVVHK |
| 2 | 0.42 | 0.56 | 0.76 | NSPLDEENLTQENQDR |
| 1 | 0.40 | 0.55 | 0.76 | SPLDEENL TQENQDR |
| 1 | 0.41 | 0.57 | 0.77 | WEMPFDPQDTHQSR |
| 1 | 0.4 | 0.55 | 0.76 | WRDSLEFR |
| 1 | 0.41 | 0.57 | 0.78 | EQLSLLDRFTEDAK |

1 M E R M L P L L A L G L L A A G F C P A V L C H P N S P L D E E N L T Q E N Q D R G T H V D L G L A S A N V D F A F S L Y K Q L V L K A P D K N V I F S P L S I

81 S T A L A F L S L G A H N T T L T E I L K G L K F N L T E T S E A E I H Q S F Q H L L R T L N Q S S D E L Q L S M G N A M F V K E Q L S L L D R F T E D A K R L

161 Y G S E A F A T D F Q D S A A A K K L I N D Y V K N G T R G K I T D L I K D L D S Q T M M V L V N Y I F F K A K W E M P F D P Q D T H Q S R F Y L S K K K W V M

241 V P M M S L H H L T I P Y F R D E E L S C T V V E L K Y T G N A S A L F I L P D Q D K M E E V E A M L L P E T L K R W R D S L E F R E I G E L Y L P K F S I S R

321 D Y N L N D I L L Q L G I E E A F T S K A D L S G I T G A R N L A V S Q V V H K A V L D V F E E G T E A S A A T A V K I T L L S A L V E T R T I V R F N R P F L

401 MIIVPTDTQNIFFMSKVTNPKQA

5.9 CD14 antigen precursor [Homo sapiens]; CD14 antigen precursor [Homo sapiens]

Protein Accession gi|91105159 gi|4557417
Mean Expression Ratio 0.567
Median Expression Ratio 0.567
Credible Interval (0.452, 0.707)
Associated Peptides 9
Associated Spectra 16
Coverage NaN

| A | 2.5 | 50 | 97.5 | Sequence |
|---|---|---|---|---|
| 4 | 0.4 | 0.53 | 0.71 | AFPALTSLDLSDNPGLGER |
| 1 | 0.41 | 0.58 | 0.81 | GLMAALCPHK |
| 1 | 0.42 | 0.58 | 0.8 | GLMAALCPHKFP AIQNL |
| 1 | 0.40 | 0.56 | 0.79 | GLMAALCPHKFP AIQNLALR |
| 2 | 0.43 | 0.58 | 0.8 | ITGTMPPLPLEATGLALSSLR |
| 3 | 0.39 | 0.53 | 0.72 | LTVGAAQVPAQLLVGALR |
| 1 | 0.4 | 0.56 | 0.78 | SWLAELQQWLKPGLK |
| 2 | 0.41 | 0.56 | 0.77 | TTPEPCELDDEDFR |
| 1 | 0.39 | 0.54 | 0.76 | VLSIAQAHSPAFSCEQVR |

5.10 brain abundant, membrane attached signal protein 1 [Homo sapiens]

Protein Accession gi|30795231
Mean Expression Ratio 1.75
Median Expression Ratio 1.75
Credible Interval (1.30, 2.35)
Associated Peptides 5
Associated Spectra 7
Coverage 0.396

| A | 2.5 | 50 | 97.5 | Sequence |
|---|---|---|---|---|
| 1 | 1.2 | 1.8 | 2.7 | AAEAAAAPAESAAPAAGEEPSKEEGEPK |
| 1 | 1.2 | 1.7 | 2.5 | AEPPKAPEQEQAAPGPAAGGEAPK |
| 2 | 1.3 | 1.8 | 2.6 | APEQEQAAPGPAAGGEAPK |
| 1 | 1.2 | 1.8 | 2.7 | AQGPAASAEEPKPVEAPAANSDQTVTVK |
| 2 | 1.3 | 1.8 | 2.6 | EKPDQDAEGK |

1 M G G K L S K K K K G Y N V N D E K A K E K D K K A E G A A T E E E G T P K E S E P Q A A A E P A E A K E G K E K P D Q D A E G K A E E K E G E K D A A A A K E

81 E A P K A E P E K T E G A A E A K A E P P K A P E Q E Q A A P G P A A G G E A P K A A E A A A A P A E S A A P A A G E E P S K E E G E P K K T E A P A A P A A Q

161 E T K S D G A P A S D S K P G S S E A A P S S K E T P A A T E A P S S T P K A Q G P A A S A E E P K P V E A P A A N S D Q T V T V K E

5.11 dipeptidase 1 [Homo sapiens]; dipeptidase 1 [Homo sapiens]

Protein Accession gi|4758190 gi|189458885

Mean Expression Ratio 1.72
Median Expression Ratio 1.72
Credible Interval (1.25, 2.37)
Associated Peptides 5
Associated Spectra 6
Coverage NaN

| A | 2.5 | 50 | 97.5 | Sequence |
|---|-----|-----|------|----------|
| 1 | 1.2 | 1.7 | 2.6 | A VGFGGDFDGVPR |
| 1 | 1.2 | 1.8 | 2.6 | DSPVIDGHNDLPWQLLDMFNNR |
| 1 | 1.2 | 1.8 | 2.7 | VPEGLEDVSKYPDLIAELLR |
| 1 | 1.2 | 1.8 | 2.7 | YPDLIAELLR |
| 2 | 1.2 | 1.8 | 2.5 | VASLIGVEGGHSIDSSLGVLR |

5.12 fibronectin 1 isoform 5 preproprotein [Homo sapiens]

Protein Accession gi|47132553
Mean Expression Ratio 0.586
Median Expression Ratio 0.585
Credible Interval (0.475, 0.724)
Associated Peptides 11
Associated Spectra 17
Coverage 0.0758

| A | 2.5 | 50 | 97.5 | Sequence |
|---|-----|------|------|----------|
| 3 | 0.40 | 0.55 | 0.74 | A VEENQESTPVVIQQETTGTPR |
| 2 | 0.43 | 0.58 | 0.79 | EESPLLIGQQSTVSDVPR |
| 2 | 0.41 | 0.57 | 0.77 | ESVPISDTIIPAVPPPTDLR |
| 1 | 0.45 | 0.63 | 0.88 | LISWDAPAVTVR |
| 1 | 0.42 | 0.58 | 0.81 | QVDAVPANGQTPIQR |
| 1 | 0.43 | 0.59 | 0.82 | SSPVVIDASTAIDAPSNLR |
| 2 | 0.43 | 0.59 | 0.81 | TETITGFQVDAVPANGQTPIQR |
| 2 | 0.42 | 0.57 | 0.78 | VDVIPVNLPGEHGQR |
| 1 | 0.43 | 0.6 | 0.84 | VTWAPPPSIDLTNFLVR |
| 1 | 0.43 | 0.6 | 0.83 | DLEVVAATPTSLLISWDAPAVTVR |
| 1 | 0.37 | 0.51 | 0.7 | IISCHPVGTDEEPLQFR |

1 M L R G P G P G L L L L A V Q C L G T A V P S T G A S K S K R Q A Q Q M V Q P Q S P V A V S Q S K P G C Y D N G K H Y Q I N Q Q W E R T Y L G N A L V C T C Y G

81 G S R G F N C E S K P E A E E T C F D K Y T G N T Y R V G D T Y E R P K D S M I W D C T C I G A G R G R I S C T I A N R C H E G G Q S Y K I G D T W R R P H E T

161 GGYMLECVCLGNGKGEWTCKPIAEKCFDHAAGTSYVVG
ETWEKPYQGWMMVDCTCLGEGSGRITCTSRNRCNDQDTRT
SY

241 RIGDTWSKKDNRGNLLQCICTGNGREWKCERHTSVQTT
SSGSGPFTDVRAAVYQPQPHPQPPPYGHCVTDSGVVYSVGM

321 QWLKTQGNKQMLCTCLGNGVSCQETAVTQTYGGNSNGE
PCVLPFTYNGRTFYSCTTEGRQDGHLWCSTTSNYEQDQKYS
F

401 CTDHTVLVQTRGGNSNGALCHFPFLYNNHNYTDCTSEGR
RDNMKWCGTTQNYDADQKFGFCPMAAHEEICTTNEGVMYR
I

481 GDQWDKQHDMGHMMRCTCVGNGREWTCIAYSQLRDQ
CIVDDITYNVNDTFHKRHEEGHMLNCTCFGQGRGRWKCDP
VDQ

561 CQDSETGTFYQIGDSWEKYVHGVRYQCYCYGRGIGEWH
CQPLQTYPSSSGPVEVFITETPSQPNSHPIQWNAPQPSHISK

641 YILRWRPKNSVGRWKEATIPGHLNSYTIKGLKPGVVYEG
QLISIQQYGHQEVTRFDFTTTSTSTPVTSNTVTGETTPFSP

721 LVATSESVTEITASSFVVSWVSASDTVSGFRVEYELSEEG
DEPQYLDLPSTATSVNIPDLLPGRKYIVNVYQISEDGEQS

801 LILSTSQTTAPDAPPDPTVDQVDDTSIVVRWSRPQAPITG
YRIVYSPSVEGSSTELNLPETANSVTLSDLQPGVQYNITI

881 YAVEENQESTPVVIQQETTGTPRSDTVPSPRDLQFVEVTD
VKVTIMWTPPESAVTGYRVDVIPVNLPGEHGQRLPISRNT

961 FAEVTGLSPGVTYYFKVFAVSHGRESKPLTAQQTTKLDA
PTNLQFVNETDSTVLVRWTPPRAQITGYRLTVGLTRRGQPR

1041 QYNVGPSVSKYPLRNLQPASEYTVSLVAIKGNQESPKAT
GVFTTLQPGSSIPPYNTEVTETTIVITWTPAPRIGFKLGVR

1121 PSQGGEAPREVTSDSGSIVVSGLTPGVEYVVYTIQVLRDG
QERDAPIVNKVVTPLSPPTNLHLEANPDTGVLTVSWERSTT

1201 P D I T G Y R I T T T P T N G Q Q G N S L E E V V H A D Q S S C T F D N L S P G
L E Y N V S V Y T V K D D K E S V P I S D T I I P A V P P P T D L R F T N I G P

1281 D T M R V T W A P P P S I D L T N F L V R Y S P V K N E E D V A E L S I S P S D
N A V V L T N L L P G T E Y V V S V S S V Y E Q H E S T P L R G R Q K T G L D S

1361 P T G I D F S D I T A N S F T V H W I A P R A T I T G Y R I R H H P E H F S G R P
R E D R V P H S R N S I T L T N L T P G T E Y V V S I V A L N G R E E S P L L

1441 I G Q Q S T V S D V P R D L E V V A A T P T S L L I S W D A P A V T V R Y Y R
I T Y G E T G G N S P V Q E F T V P G S K S T A T I S G L K P G V D Y T I T V Y A

1521 V T G R G D S P A S S K P I S I N Y R T E I D K P S Q M Q V T D V Q D N S I S V
K W L P S S S P V T G Y R V T T T P K N G P G P T K T K T A G P D Q T E M T I E

1601 G L Q P T V E Y V V S V Y A Q N P S G E S Q P L V Q T A V T T I P A P T D L K
F T Q V T P T S L S A Q W T P P N V Q L T G Y R V R V T P K E K T G P M K E I N L

1681 A P D S S S V V V S G L M V A T K Y E V S V Y A L K D T L T S R P A Q G V V
T T L E N V S P P R R A R V T D A T E T T I T I S W R T K T E T I T G F Q V D A V P

1761 A N G Q T P I Q R T I K P D V R S Y T I T G L Q P G T D Y K I Y L Y T L N D N A
R S S P V V I D A S T A I D A P S N L R F L A T T P N S L L V S W Q P P R A R I

1841 T G Y I I K Y E K P G S P P R E V V P R P R P G V T E A T I T G L E P G T E Y T I
Y V I A L K N N Q K S E P L I G R K K T D E L P Q L V T L P H P N L H G P E I

1921 L D V P S T V Q K T P F V T H P G Y D T G N G I Q L P G T S G Q Q P S V G Q Q
M I F E E H G F R R T T P P T T A T P I R H R P R P Y P P N V G E E I Q I G H I P

2001 R E D V D Y H L Y P H G P G L N P N A S T G Q E A L S Q T T I S W A P F Q D T
S E Y I I S C H P V G T D E E P L Q F R V P G T S T S A T L T G L T R G A T Y N I

2081 I V E A L K D Q Q R H K V R E E V V T V G N S V N E G L N Q P T D D S C F D
P Y T V S H Y A V G D E W E R M S E S G F K L L C Q C L G F G S G H F R C D S S R
W

2161 C H D N G V N Y K I G E K W D R Q G E N G Q M M S C T C L G N G K G E F K
C D P H E A T C Y D D G K T Y H V G E Q W Q K E Y L G A I C S C T C F G G Q R G
W R C

2241 D N C R R P G G E P S P E G T T G Q S Y N Q Y S Q R Y H Q R T N T N V N C P I
E C F M P L D V Q A D R E D S R E

5.13 angiotensinogen preproprotein [Homo sapiens]

Protein Accession gi|4557287
Mean Expression Ratio 0.59
Median Expression Ratio 0.59
Credible Interval (0.482, 0.718)
Associated Peptides 10
Associated Spectra 25
Coverage 0.293

| A | 2.5 | 50 | 97.5 | Sequence |
|---|-----|-----|------|----------|
| 2 | 0.42 | 0.57 | 0.77 | AAMVGMLANFLGFR |
| 2 | 0.4 | 0.54 | 0.73 | ADSQAQLLLSTVVGVFTAPGLHLK |
| 1 | 0.44 | 0.6 | 0.83 | ALQDQLVLVAAK |
| 2 | 0.46 | 0.62 | 0.85 | DPTFIPAPIQAK |
| 1 | 0.45 | 0.63 | 0.87 | FMQAVTGWK |
| 1 | 0.42 | 0.56 | 0.78 | QPFVQGLALYTPVVLPR |
| 2 | 0.46 | 0.62 | 0.85 | VGEVLNSIFFELEADER |
| 2 | 0.43 | 0.58 | 0.79 | VLSALQAVQGLLVAQGR |
| 11 | 0.4 | 0.5 | 0.61 | DRVYIHPFHL |
| 1 | 0.44 | 0.6 | 0.84 | LQAILGVPWK |

1 MRKRAPQSEMAPAGVSLRATILCLLAWAGLAAGDRVYIHP FHLVIHNESTCEQLAKANAGKPKDPTFIPAPIQAKTSPVD

81 EKALQDQLVLVAAKLDTEDKLRAAMVGMLANFLGFRIYG MHSELWGVVHGATVLSPTAVFGTLASLYLGALDHTADRLQ A

161 ILGVPWKDKNCTSRLDAHKVLSALQAVQGLLVAQGRAD SQAQLLLSTVVGVFTAPGLHLKQPFVQGLALYTPVVLPRSL D

241 FTELDVAAEKIDRFMQAVTGWKTGCSLMGASVDSTLAFN TYVHFQGKMKGFSLLAEPQEFWVDNSTSVSVPMLSGMGTFQ

321 HWSDIQDNFSVTQVPFTESACLLLIQPHYASDLDKVEGLT FQQNSLNWMKKLSPRTIHLTMPQLVLQGSYDLQDLLAQAE

401 LPAILHTELNLQKLSNDRIRVGEVLNSIFFELEADEREPTE STQQLNKPEVLEVTLNRPFLFAVYDQSATALHFLGRVAN

481 PLSTA

5.14 complement component 3 precursor [Homo sapiens]

Protein Accession gi|115298678
Mean Expression Ratio 0.601
Median Expression Ratio 0.602
Credible Interval (0.532, 0.678)
Associated Peptides 39
Associated Spectra 69
Coverage 0.301

| A | 2.5 | 50 | 97.5 | Sequence |
|---|---|---|---|---|
| 1 | 0.46 | 0.61 | 0.82 | CAEENCFIQK |
| 1 | 0.45 | 0.6 | 0.8 | DAPDHQELNLDVSLQLPSR |
| 2 | 0.44 | 0.58 | 0.77 | DFDFVPPVVR |
| 1 | 0.45 | 0.6 | 0.81 | DTWVEHWPEEDECQDEENQK |
| 11 | 0.35 | 0.43 | 0.53 | EDIPPADLSDQVPDTESETR |
| 3 | 0.45 | 0.59 | 0.76 | EPGQDLVVLPLSITTDFIPSFR |
| 1 | 0.45 | 0.6 | 0.81 | EYVLPSFEVIVEPTEK |
| 1 | 0.46 | 0.6 | 0.81 | FYHPEKEDGK |
| 1 | 0.46 | 0.61 | 0.83 | HQQTVTIPPK |
| 1 | 0.46 | 0.61 | 0.82 | IHWESASLLR |
| 5 | 0.44 | 0.56 | 0.71 | ILLQGTPVAQMTEDAVDAER |
| 3 | 0.48 | 0.62 | 0.81 | IPIEDGSGEVVLSR |
| 1 | 0.44 | 0.59 | 0.79 | IWDVVEK |
| 2 | 0.46 | 0.61 | 0.8 | KQELSEAEQATR |
| 2 | 0.47 | 0.62 | 0.83 | LESEETMVLEAHDAQGDVPVTVTVHDFPGK |
| 1 | 0.46 | 0.61 | 0.82 | LMNIFLK |
| 3 | 0.48 | 0.62 | 0.81 | QKPDGVFQEDAPVIHQEMIGGLR |
| 1 | 0.47 | 0.63 | 0.85 | SGQSEDRQPVPGQQMTLK |
| 1 | 0.45 | 0.59 | 0.8 | SITTDFIPSFR |
| 2 | 0.45 | 0.59 | 0.78 | SNLDEDIIAEENIVSR |
| 1 | 0.44 | 0.59 | 0.79 | SWDIPELVNMGQWK |
| 1 | 0.45 | 0.61 | 0.81 | TELRPGETLNVNF |
| 2 | 0.46 | 0.61 | 0.81 | TELRPGETLNVNFLLR |
| 2 | 0.47 | 0.63 | 0.83 | TVMVNIENPEGIPVK |
| 1 | 0.46 | 0.61 | 0.81 | VFVTNPDGSPAYR |
| 1 | 0.45 | 0.61 | 0.82 | VIFGIQDGEQR |
| 1 | 0.45 | 0.6 | 0.8 | VLLDGVQNPR |
| 3 | 0.44 | 0.58 | 0.75 | VQLSNDFDEYIMAIEQTIK |
| 3 | 0.45 | 0.58 | 0.76 | VTIKPAPETEK |
| 1 | 0.46 | 0.61 | 0.81 | AGDFLEANYMNLQR |

(continued)

| A | 2.5 | 50 | 97.5 | Sequence |
|---|-----|----|------|----------|
| 1 | 0.45 | 0.6 | 0.8 | GICVADPFEVTVMQDFFIDLR |
| 1 | 0.46 | 0.61 | 0.81 | LKGPLLNK |
| 1 | 0.41 | 0.54 | 0.72 | FYYIYNEK |
| 1 | 0.47 | 0.63 | 0.84 | QPVPGQQMTLK |
| 1 | 0.46 | 0.62 | 0.83 | RIPIEDGSGEVVLSR |
| 1 | 0.47 | 0.62 | 0.83 | VSHSEDDCLAFK |
| 1 | 0.45 | 0.6 | 0.8 | LSINTHPSQKPL |
| 1 | 0.45 | 0.6 | 0.8 | GLSSDFWGEKPNLSY |
| 1 | 0.48 | 0.64 | 0.85 | QIHFTK |

1 MGPTSGPSLLLLLLLTHLPLALGSPMYSIITPNILRLESEETM
VLEAHDAQGDVPVTVTVHDFPGKKLVLSSEKTVLTPAT

81 NHMGNVTFTIPANREFKSEKGRNKFVTVQATFGTQVVEK
VVLVSLQSGYLFIQTDKTIYTPGSTVLYRIFTVNHKLLPVG

161 RTVMVNIENPEGIPVKQDSLSSQNQLGVLPLSWDIPELVN
MGQWKIRAYYENSPQQVFSTEFEVKEYVLPSFEVIVEPTE

241 KFYYIYNEKGLEVTITARFLYGKKVEGTAFVIFGIQDGEQ
RISLPESLKRIPIEDGSGEVVLSRKVLLDGVQNPRAEDLV

321 GKSLYVSATVILHSGSDMVQAERSGIPIVTSPYQIHFTKTP
KYFKPGMPFDLMVFVTNPDGSPAYRVPVAVQGEDTVQSL

401 TQGDGVAKLSINTHPSQKPLSITVRTKKQELSEAEQATRT
MQALPYSTVGNSNNYLHLSVLRTELRPGETLNVNFLLRMD

481 RAHEAKIRYYTYLIMNKGRLLKAGRQVREPGQDLVVLPL
SITTDFIPSFRLVAYYTLIGASGQREVVADSVWVDVKDSCV

561 GSLVVKSGQSEDRQPVPGQQMTLKIEGDHGARVVLVAVD
KGVFVLNKKNKLTQSKIWDVVEKADIGCTPGSGKDYAGVFS

641 DAGLTFTSSSGQQTAQRAELQCPQPAARRRRSVQLTEKR
MDKVGKYPKELRKCCEDGMRENPMRFSCQRRTRFISLGEAC

721 KKVFLDCCNYITELRRQHARASHLGLARSNLDEDIIAEEN
IVSRSEFPESWLWNVEDLKEPPKNGISTKLMNIFLKDSIT

801 T W E I L A V S M S D K K G I C V A D P F E V T V M Q D F F I D L R L P Y S V V R N E Q V E I R A V L Y N Y R Q N Q E L K V R V E L L H N P A F C S L A T T K R

881 R H Q Q T V T I P P K S S L S V P Y V I V P L K T G L Q E V E V K A A V Y H H F I S D G V R K S L K V V P E G I R M N K T V A V R T L D P E R L G R E G V Q K E

961 D I P P A D L S D Q V P D T E S E T R I L L Q G T P V A Q M T E D A V D A E R L K H L I V T P S G C G E Q N M I G M T P T V I A V H Y L D E T E Q W E K F G L E

1041 K R Q G A L E L I K K G Y T Q Q L A F R Q P S S A F A A F V K R A P S T W L T A Y V V K V F S L A V N L I A I D S Q V L C G A V K W L I L E K Q K P D G V F Q E

1121 D A P V I H Q E M I G G L R N N N E K D M A L T A F V L I S L Q E A K D I C E E Q V N S L P G S I T K A G D F L E A N Y M N L Q R S Y T V A I A G Y A L A Q M G

1201 R L K G P L L N K F L T T A K D K N R W E D P G K Q L Y N V E A T S Y A L L A L L Q L K D F D F V P P V V R W L N E Q R Y Y G G G Y G S T Q A T F M V F Q A L A

1281 Q Y Q K D A P D H Q E L N L D V S L Q L P S R S S K I T H R I H W E S A S L L R S E E T K E N E G F T V A E G K G Q G T L S V V T M Y H A K A K D Q L T C N K

1361 F D L K V T I K P A P E T E K R P Q D A K N T M I L E I C T R Y R G D Q D A T M S I L D I S M M T G F A P D T D D L K Q L A N G V D R Y I S K Y E L D K A F S D

1441 R N T L I I Y L D K V S H S E D D C L A F K V H Q Y F N V E L I Q P G A V K V Y A Y Y N L E E S C T R F Y H P E K E D G K L N K L C R D E L C R C A E E N C F I

1521 Q K S D D K V T L E E R L D K A C E P G V D Y V Y K T R L V K V Q L S N D F D E Y I M A I E Q T I K S G S D E V Q V G Q Q R T F I S P I K C R E A L K L E E K K

1601 H Y L M W G L S S D F W G E K P N L S Y I I G K D T W V E H W P E E D E C Q D E E N Q K Q C Q D L G A F T E S M V V F G C P N

5.15 carbonic anhydrase I [Homo sapiens]; carbonic anhydrase I [Homo sapiens]; carbonic anhydrase I [Homo sapiens]; carbonic anhydrase I [Homo sapiens]

Protein Accession gi|4502517 gi|192447434 gi|192447432 gi|192447430 Mean Expression Ratio 0.602
Median Expression Ratio 0.604
Credible Interval (0.499, 0.718)
Associated Peptides 13
Associated Spectra 28
Coverage NaN

| A | 2.5 | 50 | 97.5 | Sequence |
|---|---|---|---|---|
| 2 | 0.47 | 0.63 | 0.85 | ADGLAVIGVLMK |
| 3 | 0.47 | 0.63 | 0.83 | EIINVGHSFHVNFEDNDNR |
| 1 | 0.43 | 0.59 | 0.82 | ESISVSSEQLAQFR |
| 1 | 0.43 | 0.58 | 0.8 | HDTSLKPISVSYNPATAK |
| 1 | 0.43 | 0.59 | 0.8 | LFQFHF |
| 6 | 0.45 | 0.58 | 0.74 | LYPIANGNNQSPVDIK |
| 2 | 0.45 | 0.6 | 0.81 | NGPEQWSK |
| 2 | 0.46 | 0.62 | 0.83 | SFHVNFEDNDNR |
| 1 | 0.43 | 0.6 | 0.82 | SLLSNVEGDNAVPMQHN |
| 1 | 0.41 | 0.57 | 0.78 | VLDALQAIK |
| 2 | 0.45 | 0.61 | 0.81 | YSAELHVAHW |
| 1 | 0.46 | 0.63 | 0.87 | YSAELHVAHWNSAK |
| 5 | 0.41 | 0.53 | 0.68 | SLLSNVEGDNAVPMQHNNRPTQPLK |

5.16 uromodulin precursor [Homo sapiens]; uromodulin precursor [Homo sapiens]

Protein Accession gi|59850812 gi|56550049
Mean Expression Ratio 0.608
Median Expression Ratio 0.607
Credible Interval (0.528, 0.701)
Associated Peptides 25
Associated Spectra 50
Coverage NaN

| A | 2.5 | 50 | 97.5 | Sequence |
|---|---|---|---|---|
| 1 | 0.47 | 0.63 | 0.86 | ACSYPLDMK |
| 9 | 0.51 | 0.64 | 0.79 | DSTIQVVENGESSQGR |
| 3 | 0.46 | 0.6 | 0.8 | DWVSVVTPAR |
| 1 | 0.45 | 0.59 | 0.8 | FSVQMFR |
| 1 | 0.44 | 0.59 | 0.8 | INF ACSYPLDMK |
| 4 | 0.47 | 0.6 | 0.78 | LADEIIIR |
| 1 | 0.45 | 0.62 | 0.83 | LECGANDMK |
| 1 | 0.46 | 0.61 | 0.83 | LSPGLGCTDVDECAEPGLSHCH |
| 2 | 0.46 | 0.61 | 0.8 | LYVGTMLDGGDLSR |
| 5 | 0.45 | 0.58 | 0.73 | MAETCVPVLR |
| 1 | 0.44 | 0.59 | 0.8 | SCVCPEGFR |
| 1 | 0.43 | 0.59 | 0.79 | SNTLYLADEIIIR |
| 3 | 0.46 | 0.6 | 0.79 | STEYGEGY ACDTDLR |
| 1 | 0.45 | 0.6 | 0.8 | TALQPMVSALNIR |

(continued)

| A | 2.5 | 50 | 97.5 | Sequence |
|---|---|---|---|---|
| 2 | 0.43 | 0.57 | 0.76 | TLDEYWR |
| 1 | 0.44 | 0.6 | 0.81 | VFMYLSDSR |
| 1 | 0.45 | 0.61 | 0.82 | VGTMLDGGDLSR |
| 1 | 0.47 | 0.64 | 0.86 | VLNLGPITR |
| 1 | 0.43 | 0.58 | 0.79 | WHCQCK |
| 5 | 0.46 | 0.58 | 0.74 | YFIIQDR |
| 1 | 0.48 | 0.64 | 0.86 | SLGFDKVFMYLSDSR |
| 1 | 0.46 | 0.61 | 0.83 | SLGFDK |
| 1 | 0.46 | 0.62 | 0.83 | SYPLDMK |
| 1 | 0.45 | 0.6 | 0.8 | CSGFNDR |
| 1 | 0.46 | 0.61 | 0.82 | DLNIK |

5.17 heparan sulfate proteoglycan 2 [Homo sapiens]

Protein Accession gi|126012571

Mean Expression Ratio 0.614

Median Expression Ratio 0.614

Credible Interval (0.488, 0.761)

Associated Peptides 9

Associated Spectra 16

Coverage 0.0239

| A | 2.5 | 50 | 97.5 | Sequence |
|---|---|---|---|---|
| 2 | 0.42 | 0.58 | 0.79 | DFISLGLQDGHLVFR |
| 2 | 0.43 | 0.6 | 0.82 | FHDDGFLAFPGHVF |
| 1 | 0.42 | 0.6 | 0.83 | FHDDGFLAFPGHVF SR |
| 1 | 0.45 | 0.63 | 0.9 | GSIQVDGEELVSGR |
| 2 | 0.47 | 0.64 | 0.88 | GSVYIGGAPDVATLTGGR |
| 2 | 0.42 | 0.59 | 0.8 | LV SEDPINDGEWHR |
| 2 | 0.44 | 0.61 | 0.83 | PGAPPPQPLDLQHR |
| 3 | 0.44 | 0.59 | 0.8 | SLPEVPETIELEVR |
| 1 | 0.44 | 0.63 | 0.87 | IGGAPDVATLTGGR |

MGWRAAGALLLALLLHGRLLAVTHGLRAYDGLSLPEDIET VTASQMRWTHSYLSDDEDMLADSISGDDLGSGDLGSGDFQ

81 MVYFRALVNFTRSIEYSPQLEDAGSREFREVSEAVVDTLE
SEYLKIPGDQVVSVVFIKELDGWVFVELDVGSEGNADGAQ

161 IQEMLLRVISSGSVASYVTSPQGFQFRRLGTVPQFPRACT
EAEFACHSYNECVALEYRCDRRPDCRDMSDELNCEEPVLG

241 ISPTFSLLVETTSLPPRPETTIMRQPPVTHAPQPLLPGSVRP
LPCGPQEAACRNGHCIPRDYLCDGQEDCEDGSDELDCG

321 PPPPCEPNEFPCGNGHCALKLWRCDGDFDCEDRTDEANC
PTKRPEEVCGPTQFRCVSTNMCIPASFHCDEESDCPDRSDE

401 FGCMPPQVVTPPRESIQASRGQTVTFTCVAIGVPTPIINWR
LNWGHIPSHPRVTVTSEGGRGTLIIRDVKESDQGAYTCE

481 AMNARGMVFGIPDGVLELVPQRGPCPDGHFYLEHSAACL
PCFCFGITSVCQSTRRFRDQIRLRFDQPDDFKGVNVTMPAQ

561 PGTPPLSSTQLQIDPSLHEFQLVDLSRRFLVHDSFWALPE
QFLGNKVDSYGGSLRYNVRYELARGMLEPVQRPDVVLMGA

641 GYRLLSRGHTPTQPGALNQRQVQFSEEHWVHESGRPVQR
AELLQVLQSLEAVLIQTVYNTKMASVGLSDIAMDTTVTHAT

721 SHGRAHSVEECRCPIGYSGLSCESCDAHFTRVPGGPYLGT
CSGCNCNGHASSCDPVYGHCLNCQHNTEGPQCNKCKAGFF

801 GDAMKATATSCRPCPCPYIDASRRFSDTCFLDTDGQATC
DACAPGYTGRRCESCAPGYEGNPIQPGGKCRPVNQEIVRCD

881 ERGSMGTSGEACRCKNNVVGRLCNECADGSFHLSTRNPD
GCLKCFCMGVSRHCTSSSWSRAQLHGASEEPGHFSLTNAAS

961 THTTNEGIFSPTPGELGFSSFHRLLSGPYFWSLPSRFLGDK
VTSYGGELRFTVTQRSQPGSTPLHGQPLVVLQGNNIILE

1041 HHVAQEPSPGQPSTFIVPFREQAWQRPDGQPATREHLLM
ALAGIDTLLIRASYAQQPAESRVSGISMDVAVPEETGQDPA

1121 LEVEQCSCPPGYRGPSCQDCDTGYTRTPSGLYLGTCERC
SCHGHSEACEPETGACQGCQHHTEGPRCEQCQPGYYGDAQR

1201 G T P Q D C Q L C P C Y G D P A A G Q A A H T C F L D T D G H P T C D A C S
P G H S G R H C E R C A P G Y Y G N P S Q G Q P C Q R D S Q V P G P I G C N C D P
Q

1281 G S V S S Q C D A A G Q C Q C K A Q V E G L T C S H C R P H H F H L S A S N
P D G C L P C F C M G I T Q Q C A S S A Y T R H L I S T H F A P G D F Q G F A L V N

1361 P Q R N S R L T G E F T V E P V P E G A Q L S F G N F A Q L G H E S F Y W Q L
P E T Y Q G D K V A A Y G G K L R Y T L S Y T A G P Q G S P L S D P D V Q I T G N

1441 N I M L V A S Q P A L Q G P E R R S Y E I M F R E E F W R R P D G Q P A T R E
H L L M A L A D L D E L L I R A T F S S V P L A A S I S A V S L E V A Q P G P S N

1521 R P R A L E V E E C R C P P G Y I G L S C Q D C A P G Y T R T G S G L Y L G H
C E L C E C N G H S D L C H P E T G A C S Q C Q H N A A G E F C E L C A P G Y Y G

1601 D A T A G T P E D C Q P C A C P L T N P E N M F S R T C E S L G A G G Y R C T
A C E P G Y T G Q Y C E Q C G P G Y V G N P S V Q G G Q C L P E T N Q A P L V V E

1681 V H P A R S I V P Q G G S H S L R C Q V S G S P P H Y F Y W S R E D G R P V P
S G T Q Q R H Q G S E L H F P S V Q P S D A G V Y I C T C R N L H Q S N T S R A E

1761 L L V T E A P S K P I T V T V E E Q R S Q S V R P G A D V T F I C T A K S K S P
A Y T L V W T R L H N G K L P T R A M D F N G I L T I R N V Q L S D A G T Y V C

1841 T G S N M F A M D Q G T A T L H V Q A S G T L S A P V V S I H P P Q L T V Q P
G Q L A E F R C S A T G S P T P T L E W T G G P G G Q L P A K A Q I H G G I L R L

1921 P A V E P T D Q A Q Y L C R A H S S A G Q Q V A R A V L H V H G G G G P R V
Q V S P E R T Q V H A G R T V R L Y C R A A G V P S A T I T W R K E G G S L P P Q
A

2001 R S E R T D I A T L L I P A I T T A D A G F Y L C V A T S P A G T A Q A R I Q V
V V L S A S D A S P P P V K I E S S S P S V T E G Q T L D L N C V V A G S A H A

2081 Q V T W Y R R G G S L P P H T Q V H G S R L R L P Q V S P A D S G E Y V C R
V E N G S G P K E A S I T V S V L H G T H S G P S Y T P V P G S T R P I R I E P S S

2161 S H V A E G Q T L D L N C V V P G Q A H A Q V T W H K R G G S L P A R H Q T
H G S L L R L H Q V T P A D S G E Y V C H V V G T S G P L E A S V L V T I E A S V I

2241 P G P I P P V R I E S S S S T V A E G Q T L D L S C V V A G Q A H A Q V T W Y
K R G G S L P A R H Q V R G S R L Y I F Q A S P A D A G Q Y V C R A S N G M E A S

2321 I T V T V T G T Q G A N L A Y P A G S T Q P I R I E P S S S Q V A E G Q T L D L
N C V V P G Q S H A Q V T W H K R G G S L P V R H Q T H G S L L R L Y Q A S P A

2401 D S G E Y V C R V L G S S V P L E A S V L V T I E P A G S V P A L G V T P T V
R I E S S S S Q V A E G Q T L D L N C L V A G Q A H A Q V T W H K R G G S L P A R

2481 H Q V H G S R L R L L Q V T P A D S G E Y V C R V V G S S G T Q E A S V L V
T I Q Q R L S G S H S Q G V A Y P V R I E S S S A S L A N G H T L D L N C L V A S Q

2561 A P H T I T W Y K R G G S L P S R H Q I V G S R L R I P Q V T P A D S G E Y V
C H V S N G A G S R E T S L I V T I Q G S G S S H V P S V S P P I R I E S S S P T

2641 V V E G Q T L D L N C V V A R Q P Q A I I T W Y K R G G S L P S R H Q T H G S
H L R L H Q M S V A D S G E Y V C R A N N N I D A L E A S I V I S V S P S A G S P

2721 S A P G S S M P I R I E S S S S H V A E G E T L D L N C V V P G Q A H A Q V T
W H K R G G S L P S H H Q T R G S R L R L H H V S P A D S G E Y V C R V M G S S G

2801 P L E A S V L V T I E A S G S S A V H V P A P G G A P P I R I E P S S S R V A E
G Q T L D L K C V V P G Q A H A Q V T W H K R G G N L P A R H Q V H G P L L R L

2881 N Q V S P A D S G E Y S C Q V T G S S G T L E A S V L V T I E P S S P G P I P A
P G L A Q P I Y I E A S S S H V T E G Q T L D L N C V V P G Q A H A Q V T W Y K

2961 R G G S L P A R H Q T H G S Q L R L H L V S P A D S G E Y V C R A A S G P G P
E Q E A S F T V T V P P S E G S S Y R L R S P V I S I D P P S S T V Q Q G Q D A S

3041 F K C L I H D G A A P I S L E W K T R N Q E L E D N V H I S P N G S I I T I V G
T R P S N H G T Y R C V A S N A Y G V A Q S V V N L S V H G P P T V S V L P E G

3121 P V W V K V G K A V T L E C V S A G E P R S S A R W T R I S S T P A K L E Q R
T Y G L M D S H A V L Q I S S A K P S D A G T Y V C L A Q N A L G T A Q K Q V E V

3201 I V D T G A M A P G A P Q V Q A E E A E L T V E A G H T A T L R C S A T G S P
A P T I H W S K L R S P L P W Q H R L E G D T L I I P R V A Q Q D S G Q Y I C N A

3281 T S P A G H A E A T I I L H V E S P P Y A T T V P E H A S V Q A G E T V Q L Q
C L A H G T P P L T F Q W S R V G S S L P G R A T A R N E L L H F E R A A P E D S

3361 G R Y R C R V T N K V G S A E A F A Q L L V Q G P P G S L P A T S I P A G S T
P T V Q V T P Q L E T K S I G A S V E F H C A V P S D R G T Q L R W F K E G G Q L

3441 P P G H S V Q D G V L R I Q N L D Q S C Q G T Y I C Q A H G P W G K A Q A S A Q L V I Q A L P S V L I N I R T S V Q T V V V G H A V E F E C L A L G D P K P Q V

3521 T W S K V G G H L R P G I V Q S G G V V R I A H V E L A D A G Q Y R C T A T N A A G T T Q S H V L L L V Q A L P Q I S M P Q E V R V P A G S A A V F P C I A S G

3601 Y P T P D I S W S K L D G S L P P D S R L E N N M L M L P S V R P Q D A G T Y V C T A T N R Q G K V K A F A H L Q V P E R V V P Y F T Q T P Y S F L P L P T I K

3681 D A Y R K F E I K I T F R P D S A D G M L L Y N G Q K R V P G S P T N L A N R Q P D F I S F G L V G G R P E F R F D A G S G M A T I R H P T P L A L G H F H T V

3761 T L L R S L T Q G S L I V G D L A P V N G T S Q G K F Q G L D L N E E L Y L G G Y P D Y G A I P K A G L S S G F I G C V R E L R I Q G E E I V F H D L N L T A H

3841 G I S H C P T C R D R P C Q N G G Q C H D S E S S S Y V C V C P A G F T G S R C E H S Q A L H C H P E A C G P D A T C V N R P D G R G Y T C R H L G R S G L R

3921 C E E G V T V T T P S L S G A G S Y L A L P A L T N T H H E L R L D V E F K P L A P D G V L L F S G G K S G P V E D F V S L A M V G G H L E F R Y E L G S G L A

4001 V L R S A E P L A L G R W H R V S A E R L N K D G S L R V N G G R P V L R S S P G K S Q G L N L H T L L Y L G G V E P S V P L S P A T N M S A H F R G C V G E V

4081 S V N G K R L D L T Y S F L G S Q G I G Q C Y D S S P C E R Q P C Q H G A T C M P A G E Y E F Q C L C R D G F K G D L C E H E E N P C Q L R E P C L H G G T C Q

4161 G T R C L C L P G F S G P R C Q Q G S G H G I A E S D W H L E G S G G N D A P G Q Y G A Y F H D D G F L A F P G H V F S R S L P E V P E T I E L E V R T S T A S

4241 G L L L W Q G V E V G E A G Q G K D F I S L G L Q D G H L V F R Y Q L G S G E A R L V S E D P I N D G E W H R V T A L R E G R R G S I Q V D G E E L V S G R S P

4321 G P N V A V N A K G S V Y I G G A P D V A T L T G G R F S S G I T G C V K N L V L H S A R P G A P P P Q P L D L Q H R A Q A G A N T R P C P S

5.18 clusterin isoform 1 [Homo sapiens]

Protein Accession gi|42716297
Mean Expression Ratio 0.62
Median Expression Ratio 0.619
Credible Interval (0.484, 0.788)
Associated Peptides 9

Associated Spectra 12
Coverage 0.194

| A | 2.5 | 50 | 97.5 | Sequence |
|---|---|---|---|---|
| 2 | 0.42 | 0.59 | 0.82 | ASSIIDELFQDR |
| 1 | 0.45 | 0.63 | 0.89 | ASSIIDELFQDRF |
| 1 | 0.44 | 0.62 | 0.87 | EILSVDCSTNNPSQAK |
| 1 | 0.45 | 0.63 | 0.9 | ELDESLQVAER |
| 3 | 0.42 | 0.57 | 0.77 | LFDSDPITVTVPVEVSR |
| 1 | 0.44 | 0.63 | 0.89 | MLDVMQDHF |
| 1 | 0.44 | 0.63 | 0.89 | PITVTVPVEVSR |
| 1 | 0.44 | 0.62 | 0.88 | VTTVASHTSDSDVPSGVTEVVVK |
| 1 | 0.43 | 0.6 | 0.85 | RPHFFFPK |

1 M Q V C S Q P Q R G C V R E Q S A I N T A P P S A H N A A S P G G A R G H R V P L T E A C K D S R I G G M M K T L L L F V G L L L T W E S G Q V L G D Q T V S D

81 N E L Q E M S N Q G S K Y V N K E I Q N A V N G V K Q I K T L I E K T N E E R K T L L S N L E E A K K K K E D A L N E T R E S E T K L K E L P G V C N E T M M A

161 L W E E C K P C L K Q T C M K F Y A R V C R S G S G L V G R Q L E E F L N Q S S P F Y F W M N G D R I D S L L E N D R Q Q T H M L D V M Q D H F S R A S S I I D

241 E L F Q D R F F T R E P Q D T Y H Y L P F S L P H R R P H F F F P K S R I V R S L M P F S P Y E P L N F H A M F Q P F L E M I H E A Q Q A M D I H F H S P A F Q

321 H P P T E F I R E G D D D R T V C R E I R H N S T G C L R M K D Q C D K C R E I L S V D C S T N N P S Q A K L R R E L D E S L Q V A E R L T R K Y N E L L K S Y

401 Q W K M L N T S S L L E Q L N E Q F N W V S R L A N L T Q G E D Q Y Y L R V T T V A S H T S D S D V P S G V T E V V V K L F D S D P I T V T V P V E V S R K N P

481 KFMETVAEKALQEYRKKHREE

5.19 ezrin [Homo sapiens]; ezrin [Homo sapiens]

Protein Accession gi|21614499 gi|161702986
Mean Expression Ratio 1.62
Median Expression Ratio 1.61
Credible Interval (1.23, 2.13)
Associated Peptides 7
Associated Spectra 8
Coverage NaN

| A | 2.5 | 50 | 97.5 | Sequence |
|---|---|---|---|---|
| 2 | 1.2 | 1.7 | 2.5 | FYPEDVAEELIQDITQK |
| 1 | 1.1 | 1.6 | 2.3 | KEDEVEEWQHR |
| 1 | 1.1 | 1.6 | 2.4 | QLLTLSSELSQAR |
| 1 | 1.1 | 1.6 | 2.4 | QQLETEK |
| 1 | 1.2 | 1.7 | 2.4 | GFPTWLK |
| 1 | 1.1 | 1.6 | 2.3 | IAQDLEMYGINYFEIK |
| 1 | 1.1 | 1.6 | 2.3 | ILQLCMGNHELYMR |

5.20 delta globin [Homo sapiens]

Protein Accession gi|4504351
Mean Expression Ratio 1.60
Median Expression Ratio 1.60
Credible Interval (1.12, 2.29)
Associated Peptides 3
Associated Spectra 4
Coverage 0.306

| A | 2.5 | 50 | 97.5 | Sequence |
|---|---|---|---|---|
| 2 | 1.2 | 1.7 | 2.6 | FFESFGDLSSPDAVMGNPK |
| 1 | 1.1 | 1.6 | 2.5 | VNVDAVGGEALGR |
| 1 | 1.1 | 1.6 | 2.5 | GTFSQLSELHCDK |

1 M V H L T P E E K T A V N A L W G K V N V D A V G G E A L G R L L V V Y P W T Q R F F E S F G D L S S P D A V M G N P K V K A H G K K V L G A F S D G L A H L D

81 N L K G T F S Q L S E L H C D K L H V D P E N F R L L G N V L V C V L A R N F G K E F T P Q M Q A A Y Q K V V A G V A N A L A H K Y H

5.21 fibrinogen, beta chain preproprotein [Homo sapiens]

Protein Accession gi|70906435
Mean Expression Ratio 0.633
Median Expression Ratio 0.633
Credible Interval (0.527, 0.76)
Associated Peptides 14
Associated Spectra 27
Coverage 0.291

| A | 2.5 | 50 | 97.5 | Sequence |
|---|---|---|---|---|
| 3 | 0.49 | 0.66 | 0.87 | AHYGGFTVQNEANK |
| 1 | 0.46 | 0.62 | 0.85 | DNENVVNEYSSELEK |

(continued)

| A | 2.5 | 50 | 97.5 | Sequence |
|---|---|---|---|---|
| 1 | 0.5 | 0.68 | 0.92 | ETVNSNIPTNLR |
| 1 | 0.47 | 0.64 | 0.88 | GGETSEMYLIQPDSSVKPYR |
| 7 | 0.47 | 0.59 | 0.75 | HQLYIDETVNSNIPTNLR |
| 1 | 0.47 | 0.65 | 0.9 | IDETVNSNIPTNLR |
| 2 | 0.45 | 0.6 | 0.8 | LIQPDSSVKPYR |
| 2 | 0.44 | 0.59 | 0.8 | MGPTELLIEMEDWK |
| 1 | 0.45 | 0.62 | 0.85 | NYCGLPGEYWLGNDK |
| 1 | 0.45 | 0.62 | 0.86 | QGFGNVATNTDGK |
| 1 | 0.46 | 0.63 | 0.87 | YIDETVNSNIPTNLR |
| 4 | 0.45 | 0.6 | 0.78 | QGVNDNEEGFF SAR |
| 1 | 0.47 | 0.65 | 0.9 | LESDVSAQMEYCR |
| 1 | 0.46 | 0.64 | 0.88 | SILENLR |

1 MKRMVSWSFHKLKTMKHLLLLLLLCVFLVKSQGVNDNEEG FFSARGHRPLDKKREEAPSLRPAPPPISGGGYRARPAKAAA

81 TQKKVERKAPDAGGCLHADPDLGVLCPTGCQLQEALLQQ ERPIRNSVDELNNNVEAVSQTSSSSFQYMYLLKDLWQKRQK

161 QVKDNENVVNEYSSELEKHQLYIDETVNSNIPTNLRVLRS ILENLRSKIQKLESDVSAQMEYCRTPCTVSCNIPVVSGKE

241 CEEIIRKGGETSEMYLIQPDSSVKPYRVYCDMNTENGGW TVIQNRQDGSVDFGRKWDPYKQGFGNVATNTDGKNYCGLP G

321 EYWLGNDKISQLTRMGPTELLIEMEDWKGDKVKAHYGG FTVQNEANKYQISVNKYRGTAGNALMDGASQLMGENRTMT IH

401 NGMFFSTYDRDNDGWLTSDPRKQCSKEDGGGWWYNRCH AANPNGRYYWGGQYTWDMAKHGTDDGVVWMNWKGSWYS MRKM

481 SMKIRPFFPQQ

5.22 moesin [Homo sapiens]

Protein Accession gi|4505257
Mean Expression Ratio 1.57
Median Expression Ratio 1.57

Credible Interval (1.18, 2.08)
Associated Peptides 5
Associated Spectra 9

| A | 2.5 | 50 | 97.5 | Sequence |
|---|-----|----|------|----------|
| 4 | 1.3 | 1.8 | 2.4 | FYPEDVSEELIQDITQR |
| 2 | 1.1 | 1.5 | 2.2 | GSELWLGVDALGLNIYEQNDR |
| 1 | 1.1 | 1.6 | 2.3 | TQEQLALEMAELTAR |
| 1 | 1.1 | 1.6 | 2.3 | ERQEAEEAK |
| 1 | 1.1 | 1.6 | 2.3 | KTQEQLALEMAELTAR |

1 MPKTISVRVTTMDAELEFAIQPNTTGKQLFDQVVKTIGLRE VWFFGLQYQDTKGFSTWLKLNKKVTAQDVRKESPLLFKF

81 RAKFYPEDVSEELIQDITQRLFFLQVKEGILNDDIYCPPET AVLLASYAVQSKYGDFNKEVHKSGYLAGDKLLPQRVLEQ

161 HKLNKDQWEERIQVWHEEHRGMLREDAVLEYLKIAQDL EMYGVNYFSIKNKKGSELWLGVDALGLNIYEQNDRLTPKIG F

241 PWSEIRNISFNDKKFVIKPIDKKAPDFVFYAPRLRINKRIL ALCMGNHELYMRRRKPDTIEVQQMKAQAREEKHQKQMER 321 AMLENEKKKREMAEKEKEKIEREKEELMERLKQIEEQTK KAQQELEEQTRRALELEQERKAQSEAEKLAKERQEAEEAK

401 EALLQASRDQKKTQEQLALEMAELTARISQLEMARQKKE SEAVEWQQKAQMVQEDLEKTRAELKTAMSTPHVAEPAENE Q

481 DEQDENGAEASADLRADAMAKDRSEEERTTEAEKNERV QKHLKALTSELANARDESKKTANDMIHAENMRLGRDKYKT LR

561 QIRQGNTKQRIDEFESM

5.23 transferrin [Homo sapiens]

Protein Accession gi|4557871
Mean Expression Ratio 0.648
Median Expression Ratio 0.648
Credible Interval (0.535, 0.786)
Associated Peptides 12
Associated Spectra 22
Coverage 0.216

| A | 2.5 | 50 | 97.5 | Sequence |
|---|-----|-----|------|----------|
| 1 | 0.46 | 0.64 | 0.88 | CSTSSLLEACTFR |
| 1 | 0.46 | 0.63 | 0.88 | DCHLAQVPSHTVVAR |
| 3 | 0.47 | 0.62 | 0.83 | EDLIWELLNQAQEHFGK |
| 2 | 0.45 | 0.61 | 0.83 | EGTCPEAPTDECKPVK |
| 3 | 0.53 | 0.7 | 0.93 | HQTVPQNTGGK |
| 2 | 0.47 | 0.63 | 0.85 | HSTIFENLANK |
| 3 | 0.48 | 0.64 | 0.86 | KPVEEYANCHLAR |
| 1 | 0.47 | 0.64 | 0.9 | MYLGYEYVTAIR |
| 1 | 0.48 | 0.66 | 0.9 | NIPIGLLYCDLPEPR |
| 2 | 0.47 | 0.64 | 0.87 | NLNEKDYELLCLDGTR |
| 2 | 0.47 | 0.63 | 0.86 | SAGWNIPIGLLYCDLPEPR |
| 1 | 0.48 | 0.66 | 0.91 | YLGEEYVK |

1 MRLAVGALLVCAVLGLCLAVPDKTVRWCAVSEHEATKCQ SFRDHMKSVIPSDGPSVACVKKASYLDCIRAIAANEADAVT

81 LDAGLVYDAYLAPNNLKPVVAEFYGSKEDPQTFYYAVAV VKKDSGFQMNQLRGKKSCHTGLGRSAGWNIPIGLLYCDLPE

161 PRKPLEKAVANFFSGSCAPCADGTDFPQLCQLCPGCGCST LNQYFGYSGAFKCLKDGAGDVAFVKHSTIFENLANKADRD

241 QYELLCLDNTRKPVDEYKDCHLAQVPSHTVVARSMGGK EDLIWELLNQAQEHFGKDKSKEFQLFSSPHGKDLLFKDSAH G

321 FLKVPPRMDAKMYLGYEYVTAIRNLREGTCPEAPTDECK PVKWCALSHHERLKCDEWSVNSVGKIECVSAETTEDCIAKI

401 MNGEADAMSLDGGFVYIAGKCGLVPVLAENYNKSDNCE DTPEAGYFAVAVVKKSASDLTWDNLKGKKSCHTAVGRTAG WNGLNLCEPNNKEGYYGYTGAFRCLVEKGDVAFVKHQTVP QN

561 TGGKNPDPWAKNLNEKDYELLCLDGTRKPVEEYANCHL ARAPNHAVVTRKDKEACVHKILRQQQHLFGSNVTDCSGNFC L

641 FRSETKDLLFRDDTVCLAKLHDRNTYEKYLGEEYVKAVG NLRKCSTSSLLEACTFRRP

5.24 secreted phosphoprotein 1 isoform c [Homo sapiens]; secreted phosphoprotein 1 isoform a [Homo sapiens]; secreted phosphoprotein 1 isoform b [Homo sapiens]

Protein Accession gi|91598939 gi|91206462 gi|4759166
Mean Expression Ratio 1.53
Median Expression Ratio 1.52
Credible Interval (1.10, 2.13)
Associated Peptides 4
Associated Spectra 5
Coverage NaN

| A | 2.5 | 50 | 97.5 | Sequence |
|---|-----|-----|------|----------|
| 2 | 1 | 1.5 | 2.1 | AIPVAQDLNAPSDWDSR |
| 1 | 1.1 | 1.7 | 2.5 | GKDSYETSQLDDQSAETHSHK |
| 1 | 1.0 | 1.5 | 2.3 | KANDESNEHSDVIDSQELSK |
| 1 | 1.1 | 1.6 | 2.4 | SKEEDK |

5.25 pancreatic amylase alpha 2A precursor [Homo sapiens]

Protein Accession gi|4502085
Mean Expression Ratio 0.664
Median Expression Ratio 0.664
Credible Interval (0.527, 0.841)
Associated Peptides 8
Associated Spectra 14
Coverage 0.213

| A | 2.5 | 50 | 97.5 | Sequence |
|---|-----|-----|------|----------|
| 2 | 0.46 | 0.63 | 0.86 | ALVFVDNHDNQR |
| 1 | 0.49 | 0.69 | 0.97 | EVTINPDTTCGNDWVCEHR |
| 5 | 0.48 | 0.64 | 0.84 | IAEYMNHLIDIGVAGFR |
| 1 | 0.47 | 0.66 | 0.95 | LTGLLDLALEK |
| 2 | 0.46 | 0.64 | 0.89 | LTGLLDLALEKDYVR |
| 1 | 0.48 | 0.68 | 0.95 | NVVDGQPFTNWYDNGSNQVAFGR |
| 1 | 0.47 | 0.66 | 0.93 | NWGEGWGFVPSDR |
| 1 | 0.46 | 0.66 | 0.93 | WVDIALECER |

1 M K F F L L L L F T I G F C W A Q Y S P N T Q Q G R T S I V H L F E W R W V D I A L
E C E R Y L A P K G F G G V Q V S P P N E N V A I Y N P F R P W W E R Y Q P V

81 S Y K L C T R S G N E D E F R N M V T R C N N V G V R I Y V D A V I N H M C G
N A V S A G T S S T C G S Y F N P G S R D F P A V P Y S G W D F N D G K C K T G S

161 GDIENYNDATQVRDCRLTGLLDLALEKDYVRSKIAEYMN HLIDIGVAGFRLDASKHMWPGDIKAILDKLHNLNSNWFPAG

241 SKPFIYQEVIDLGGEPIKSSDYFGNGRVTEFKYGAKLGTV IRKWNGEKMSYLKNWGEGWGFVPSDRALVFVDNHDNQRGH

321 GAGGASILTFWDARLYKMAVGFMLAHPYGFTRVMSSYR WPRQFQNGNDVNDWVGPPNNNGVIKEVTINPDTTCGNDWVCE

401 HRWRQIRNMVIFRNVVDGQPFTNWYDNGSNQVAFGRGN RGFIVFNNDDWSFSLTLQTGLPAGTYCDVISGDKINGNCTGI

481 KIYVSDDGKAHFSISNSAEDPFIAIHAESKL

5.26 annexin A2 isoform 1 [Homo sapiens]

Protein Accession gi|50845388
Mean Expression Ratio 1.50
Median Expression Ratio 1.5
Credible Interval (1.07, 2.11)
Associated Peptides 3
Associated Spectra 5
Coverage 0.129

| A | 2.5 | 50 | 97.5 | Sequence |
|---|-----|----|------|----------|
| 2 | 1.1 | 1.6 | 2.4 | GLGTDEDSLIEIICSR |
| 2 | 1.0 | 1.5 | 2.2 | RAEDGSVIDYELIDQDAR |
| 1 | 1.0 | 1.5 | 2.4 | SYSPYDMLESIR |

1 MGRQLAGCGDAGKKASFKMSTVHEILCKLSLEGDHSTPPS AYGSVKAYTNFDAERDALNIETAIKTKGVDEVTIVNILTN

81 RSNAQRQDIAFAYQRRTKKELASALKSALSGHLETVILGL LKTPAQYDASELKASMKGLGTDEDSLIEIICSRTNQELQE

161 INRVYKEMYKTDLEKDIISDTSGDFRKLMVALAKGRRAE DGSVIDYELIDQDARDLYDAGVKRKGTDVPKWISIMTERSV

241 PHLQKVFDRYKSYSPYDMLESIRKEVKGDLENAFLNLVQ CIQNKPLYFADRLYDSMKGKGTRDKVLIRIMVSRSEVDMLK

321 IRSEFKRKYGKSLYYYIQQDTKGDYQKALLYLCGGDD

5.27 complement component 4 binding protein, alpha chain precursor [Homo sapiens]

Protein Accession gi|4502503
Mean Expression Ratio 0.673
Median Expression Ratio 0.675
Credible Interval (0.48, 0.94)
Associated Peptides 5
Associated Spectra 5
Coverage 0.104

| A | 2.5 | 50 | 97.5 | Sequence |
|---|-----|-----|------|----------|
| 1 | 0.44 | 0.66 | 0.99 | EDVYVVGTVLR |
| 1 | 0.42 | 0.64 | 0.95 | LSLEIEQLELQR |
| 1 | 0.45 | 0.68 | 1.0 | FSAICQGDGTWSPR |
| 1 | 0.44 | 0.67 | 1 | YTCLPGYVR |
| 1 | 0.44 | 0.66 | 1 | CEWETPEGCEQVLTGK |

1 MHPPKTPSGALHRKRKMAAWPFSRLWKVSDPILFQMTLIA ALLPAVLGNCGPPPTLSFAAPMDITLTETRFKTGTTLKYT

81 CLPGYVRSHSTQTLTCNSDGEWVYNTFCIYKRCRHPGELR NGQVEIKTDLSFGSQIEFSCSEGFFLIGSTTSRCEVQDRG

161 VGWSHPLPQCEIVKCKPPPDIRNGRHSGEENFYAYGFSVT YSCDPRFSLLGHASISCTVENETIGVWRPSPPTCEKITCR

241 KPDVSHGEMVSGFGPIYNYKDTIVFKCQKGFVLRGSSVIH CDADSKWNPSPPACEPNSCINLPDIPHASWETYPRPTKED

321 VYVVGTVLRYRCHPGYKPTTDEPTTVICQKNLRWTPYQG CEALCCPEPKLNNGEITQHRKSRPANHCVYFYGDEISFSCH

401 ETSRFSAICQGDGTWSPRTPSCGDICNFPPKIAHGHYKQS SSYSFFKEEIIYECDKGYILVGQAKLSCSYSHWSAPAPQC

481 KALCRKPELVNGRLSVDKDQYVEPENVTIQCDSGYGVVG PQSITCSGNRTWYPEVPKCEWETPEGCEQVLTGKRLMQCLP

561 NPEDVKMALEVYKLSLEIEQLELQRDSARQSTLDKEL

5.28 albumin preproprotein [Homo sapiens]

Protein Accession gi|4502027

Mean Expression Ratio 0.681
Median Expression Ratio 0.681
Credible Interval (0.626, 0.74)
Associated Peptides 65
Associated Spectra 259
Coverage 0.744

| A | 2.5 | 50 | 97.5 | Sequence |
|---|-----|-----|------|----------|
| 1 | 0.49 | 0.64 | 0.86 | AAFTECCQAADK |
| 5 | 0.46 | 0.58 | 0.74 | ADDKETCFAEEGK |
| 1 | 0.49 | 0.64 | 0.85 | ADDKETCFAEEGKK |
| 3 | 0.55 | 0.7 | 0.9 | AEFAEVSK |
| 10 | 0.56 | 0.68 | 0.83 | ALVLIAFAQYLQQCPFEDHVK |
| 1 | 0.52 | 0.69 | 0.9 | AQYLQQCPFEDHVK |
| 9 | 0.51 | 0.63 | 0.77 | AVMDDFAAFVEK |
| 1 | 0.5 | 0.66 | 0.87 | CCTESLVNR |
| 5 | 0.62 | 0.78 | 0.99 | CIAEVENDEMPADLPSLAADFVESK |
| 7 | 0.61 | 0.76 | 0.94 | CTAFHDNEETFLK |
| 1 | 0.5 | 0.66 | 0.87 | DDNPNLPR |
| 2 | 0.52 | 0.69 | 0.9 | DLGEENFK |
| 2 | 0.48 | 0.63 | 0.82 | ECCEKPLLEK |
| 2 | 0.56 | 0.74 | 0.96 | ETCFAEEGK |
| 2 | 0.5 | 0.65 | 0.85 | ETCFAEEGKK |
| 1 | 0.51 | 0.68 | 0.89 | ETYGEMADCCAK |
| 4 | 0.49 | 0.63 | 0.8 | FKDLGEENFK |
| 3 | 0.49 | 0.64 | 0.82 | FQNALLVR |
| 1 | 0.52 | 0.69 | 0.9 | FSALEVDETYVPK |
| 1 | 0.53 | 0.7 | 0.92 | FYAPELLFFAK |
| 1 | 0.54 | 0.72 | 0.96 | HDNEETFLK |
| 2 | 0.53 | 0.7 | 0.91 | HPDYSVVLLLR |
| 1 | 0.55 | 0.72 | 0.95 | IAEVENDEMPADLPSLAADFVESK |
| 4 | 0.54 | 0.68 | 0.87 | KQTALVELVK |
| 15 | 0.55 | 0.65 | 0.79 | KVPQVSTPTLVEVSR |
| 2 | 0.5 | 0.65 | 0.85 | KYLYEIAR |
| 2 | 0.51 | 0.67 | 0.87 | LCTVATLR |
| 3 | 0.52 | 0.66 | 0.86 | LDELRDEGK |
| 1 | 0.48 | 0.64 | 0.85 | LKECCEKPLLEK |
| 4 | 0.52 | 0.67 | 0.85 | LVNEVTEFAK |
| 7 | 0.52 | 0.64 | 0.8 | LVRPEVDVMCTAFHDNEETFLK |
| 2 | 0.54 | 0.7 | 0.9 | LVTDLTK |
| 3 | 0.51 | 0.66 | 0.86 | MPCAEDYLSVVLNQLCVLHEK |

(continued)

| A | 2.5 | 50 | 97.5 | Sequence |
|---|---|---|---|---|
| 7 | 0.6 | 0.74 | 0.92 | NECFLQHK |
| 1 | 0.54 | 0.7 | 0.93 | PLVEEPQNLIK |
| 4 | 0.51 | 0.65 | 0.83 | QEPERNECFLQHK |
| 2 | 0.53 | 0.7 | 0.91 | QLCVLHEK |
| 2 | 0.54 | 0.7 | 0.91 | QTALVELVK |
| 4 | 0.49 | 0.63 | 0.8 | RPCFSALEVDETYVPK |
| 1 | 0.52 | 0.69 | 0.91 | SALEVDETYVPK |
| 18 | 0.5 | 0.59 | 0.7 | SHCIAEVENDEMPADLPSLAADFVESK |
| 23 | 0.64 | 0.75 | 0.88 | SLHTLFGDK |
| 1 | 0.52 | 0.69 | 0.9 | TAFHDNEETFLK |
| 2 | 0.52 | 0.69 | 0.9 | TCVADESAENCDK |
| 2 | 0.52 | 0.68 | 0.9 | TFHADICTLSEK |
| 1 | 0.53 | 0.7 | 0.93 | VFDEFKPL |
| 1 | 0.51 | 0.68 | 0.9 | VFDEFKPLVEEPQNL |
| 46 | 0.59 | 0.67 | 0.75 | VFDEFKPLVEEPQNLIK |
| 4 | 0.52 | 0.67 | 0.86 | VHTECCHGDLLECADDR |
| 4 | 0.57 | 0.72 | 0.93 | VPQVSTPTLVEVSR |
| 1 | 0.52 | 0.7 | 0.93 | VSTPTLVEVSR |
| 1 | 0.52 | 0.69 | 0.91 | YLYEIAR |
| 1 | 0.52 | 0.69 | 0.91 | YTKKVPQVSTPTLVEVSR |
| 9 | 0.62 | 0.76 | 0.94 | DVFLGMFLYEYAR |
| 2 | 0.51 | 0.67 | 0.87 | QNCELFEQLGEYK |
| 2 | 0.54 | 0.71 | 0.92 | YICENQDSISSK |
| 1 | 0.52 | 0.68 | 0.91 | CASLQK |
| 1 | 0.51 | 0.68 | 0.9 | KLVAASQAALGL |
| 1 | 0.51 | 0.67 | 0.89 | LQQCPFEDHVK |
| 3 | 0.52 | 0.67 | 0.86 | FYAPELLFFAKR |
| 1 | 0.52 | 0.7 | 0.92 | AACLLPKLDELR |
| 1 | 0.53 | 0.7 | 0.92 | VFDEFK |
| 1 | 0.51 | 0.67 | 0.9 | RHPDYSVVLLLR |
| 1 | 0.51 | 0.68 | 0.9 | RHPYFYAPELLFFAK |
| 1 | 0.52 | 0.69 | 0.91 | AACLLPK |

1 MKWVTFISLLFLFSSAYSRGVFRRDAHKSEVAHRFKDLGEE NFKALVLIAFAQYLQQCPFEDHVKLVNEVTEFAKTCVAD

81 ESAENCDKSLHTLFGDKLCTVATLRETYGEMADCCAKQE PERNECFLQHKDDNPNLPRLVRPEVDVMCTAFHDNEETFLK

161 K Y L Y E I A R R H P Y F Y A P E L L F F A K R Y K A A F T E C C Q A A D K A A C L L P K L D E L R D E G K A S S A K Q R L K C A S L Q K F G E R A F K A W A V

241 A R L S Q R F P K A E F A E V S K L V T D L T K V H T E C C H G D L L E C A D D R A D L A K Y I C E N Q D S I S S K L K E C C E K P L L E K S H C I A E V E N D

321 E M P A D L P S L A A D F V E S K D V C K N Y A E A K D V F L G M F L Y E Y A R R H P D Y S V V L L L R L A K T Y E T T L E K C C A A A D P H E C Y A K V F D E

401 F K P L V E E P Q N L I K Q N C E L F E Q L G E Y K F Q N A L L V R Y T K K V P Q V S T P T L V E V S R N L G K V G S K C C K H P E A K R M P C A E D Y L S V V

481 L N Q L C V L H E K T P V S D R V T K C C T E S L V N R R P C F S A L E V D E T Y V P K E F N A E T F T F H A D I C T L S E K E R Q I K K Q T A L V E L V K H K

561 P K A T K E Q L K A V M D D F A A F V E K C C K A D D K E T C F A E E G K K L V A A S Q A A L G L

5.29 epidermal growth factor receptor pathway substrate 8 [Homo sapiens]

Protein Accession gi|56682953
Mean Expression Ratio 1.46
Median Expression Ratio 1.46
Credible Interval (1.13, 1.86)
Associated Peptides 9
Associated Spectra 11
Coverage 0.153

| A | 2.5 | 50 | 97.5 | Sequence |
|---|---|---|---|---|
| 1 | 1.1 | 1.6 | 2.3 | APAPAPPGTVTQVDVR |
| 1 | 1.1 | 1.5 | 2.1 | EQFIPPYVPR |
| 2 | 1.1 | 1.5 | 2.0 | ISAAASDSGVESFDEGSSH |
| 1 | 1.0 | 1.5 | 2.1 | NILDIVRPPESGLGR |
| 1 | 1.0 | 1.4 | 2.0 | SQMEEVQDELIHR |
| 1 | 1 | 1.4 | 2.0 | VWTQDMILQVDDR |
| 1 | 1 | 1.4 | 2 | DDILEILDDR |
| 2 | 1.0 | 1.4 | 2.0 | MISNADPSIPPPPR |
| 1 | 1.0 | 1.5 | 2.0 | ANLISEDIESAISDSK |

1 M N G H I S N H P S S F G M Y P S Q M N G Y G S S P T F S Q T D R E H G S K T S A K A L Y E Q R K N Y A R D S V S S V S D I S Q Y R V E H L T T F V L D R K D A

81 M I T V D D G I R K L K L L D A K G K V W T Q D M I L Q V D D R A V S L I D L E S K N E L E N F P L N T I Q H C Q A V M H S C S Y D S V L A L V C K E P T Q N K

161 P D L H L F Q C D E V K A N L I S E D I E S A I S D S K G G K Q K R R P D A L R M I S N A D P S I P P P P R A P A P A P P G T V T Q V D V R S R V A A W S A W A

241 A D Q G D F E K P R Q Y H E Q E E T P E M M A A R I D R D V Q I L N H I L D D I E F F I T K L Q K A A E A F S E L S K R K K N K K G K R K G P G E G V L T L R A

321 K P P P P D E F L D C F Q K F K H G F N L L A K L K S H I Q N P S A A D L V H F L F T P L N M V V Q A T G G P E L A S S V L S P L L N K D T I D F L N Y T V N G

401 D E R Q L W M S L G G T W M K A R A E W P K E Q F I P P Y V P R F R N G W E P P M L N F M G A T M E Q D L Y Q L A E S V A N V A E H Q R K Q E I K R L S T E H S

481 S V S E Y H P A D G Y A F S S N I Y T R G S H L D Q G E A A V A F K P T S N R H I D R N Y E P L K T Q P K K Y A K S K Y D F V A R N N S E L S V L K D D I L E I

561 L D D R K Q W W K V R N A S G D S G F V P N N I L D I V R P P E S G L G R A D P P Y T H T I Q K Q R M E Y G P R P A D T P P A P S P P P T P A P V P V P L P P S

641 T P A P V P V S K V P A N I T R Q N S S S S D S G G S I V R D S Q R H K Q L P V D R R K S Q M E E V Q D E L I H R L T I G R S A A Q K K F H V P R Q N V P V I N

721 I T Y D S T P E D V K T W L Q S K G F N P V T V N S L G V L N G A Q L F S L N K D E L R T V C P E G A R V Y S Q I T V Q K A A L E D S S G S S E L Q E I M R R R

801 Q E K I S A A A S D S G V E S F D E G S S H

5.30 apolipoprotein B precursor [Homo sapiens]

Protein Accession gi|105990532
Mean Expression Ratio 0.688
Median Expression Ratio 0.688
Credible Interval (0.585, 0.815)
Associated Peptides 27
Associated Spectra 30
Coverage 0.073

| A | 2.5 | 50 | 97.5 | Sequence |
|---|---|---|---|---|
| 1 | 0.5 | 0.68 | 0.92 | AHLDIAGSLEGHLR |
| 1 | 0.48 | 0.65 | 0.88 | ATFQTPDFIVPLTDLR |
| 1 | 0.47 | 0.65 | 0.88 | DKDQEVLLQTFLDDASPGDKR |

(continued)

| A | 2.5 | 50 | 97.5 | Sequence |
|---|-----|----|----|----------|
| 1 | 0.5 | 0.69 | 0.93 | ELPVLHVPR |
| 1 | 0.51 | 0.7 | 0.95 | FLDMLIK |
| 1 | 0.52 | 0.7 | 0.96 | HSITNPLAVLCEFISQSIK |
| 2 | 0.5 | 0.68 | 0.9 | ILGEELGFASLHDLQLLGK |
| 1 | 0.5 | 0.69 | 0.94 | LELELRPTGEIEQY |
| 1 | 0.52 | 0.7 | 0.96 | LHVAGNLK |
| 1 | 0.51 | 0.69 | 0.94 | LIDVISMYR |
| 1 | 0.49 | 0.67 | 0.92 | LVALIPEPSAQQLR |
| 2 | 0.51 | 0.67 | 0.9 | NMGLPDFHIPENLFLK |
| 1 | 0.51 | 0.69 | 0.94 | QSMTLSSEVQIPDFDVDLGTILR |
| 1 | 0.5 0. | 68 | 0.93 | QTIIVVLENVQR |
| 1 | 0.49 | 0.67 | 0.9 | SSEVQIPDFDVDLGTILR |
| 1 | 0.5 | 0.68 | 0.92 | TEVIPPLIENR |
| 1 | 0.51 | 0.69 | 0.95 | TLQGIPQMIGEVIR |
| 1 | 0.52 | 0.7 | 0.96 | TLSSEVQIPDFDVDLGTILR |
| 1 | 0.49 | 0.67 | 0.92 | TQFNNNEYSQDLDAYNTK |
| 2 | 0.53 | 0.72 | 0.95 | VAWHYDEEK |
| 1 | 0.49 | 0.68 | 0.92 | YTYNYEAESSSGVPGTADSR |
| 1 | 0.51 | 0.7 | 0.95 | QSFDLSVK |
| 1 | 0.51 | 0.69 | 0.94 | VTNMGIIIPDFAR |
| 1 | 0.51 | 0.7 | 0.95 | HIQNIDIQHLAGK |
| 1 | 0.53 | 0.72 | 0.99 | VLLDQLGTTISFER |
| 1 | 0.52 | 0.71 | 0.97 | LALWGEHTGQLY |
| 1 | 0.51 | 0.68 | 0.94 | MGIIIPDFAR |

1 MDPPRPALLALLALPALLLLLLAGARAEEEMLENVSLVCP
KDATRFKHLRKYTYNYEAESSSGVPGTADSRSATRINCKV

81 ELEVPQLCSFILKTSQCTLKEVYGFNPEGKALLKKTKNSEE
FAAAMSRYELKLAIPEGKQVFLYPEKDEPTYILNIKRGI

161 ISALLVPPETEEAKQVLFLDTVYGNCSTHFTVKTRKGNVA
TEISTERDLGQCDRFKPIRTGISPLALIKGMTRPLSTLIS

241 SSQSCQYTLDAKRKHVAEAICKEQHLFLPFSYKNKYGMV
AQVTQTLKLEDTPKINSRFFGEGTKKMGLAFESTKSTSPPK

321 QAEAVLKTLQELKKLTISEQNIQRANLFNKLVTELRGLSD
EAVTSLLPQLIEVSSPITLQALVQCGQPQCSTHILQWLKR

401 V H A N P L L I D V V T Y L V A L I P E P S A Q Q L R E I F N M A R D Q R S R A
T L Y A L S H A V N N Y H K T N P T G T Q E L L D I A N Y L M E Q I Q D D C T G

481 D E D Y T Y L I L R V I G N M G Q T M E Q L T P E L K S S I L K C V Q S T K P S
L M I Q K A A I Q A L R K M E P K D K D Q E V L L Q T F L D D A S P G D K R L A

561 A Y L M L M R S P S Q A D I N K I V Q I L P W E Q N E Q V K N F V A S H I A N I
L N S E E L D I Q D L K K L V K E A L K E S Q L P T V M D F R K F S R N Y Q L Y

641 K S V S L P S L D P A S A K I E G N L I F D P N N Y L P K E S M L K T T L T A F
G F A S A D L I E I G L E G K G F E P T L E A L F G K Q G F F P D S V N K A L Y

721 W V N G Q V P D G V S K V L V D H F G Y T K D D K H E Q D M V N G I M L S V
E K L I K D L K S K E V P E A R A Y L R I L G E E L G F A S L H D L Q L L G K L L L

801 M G A R T L Q G I P Q M I G E V I R K G S K N D F F L H Y I F M E N A F E L P T
G A G L Q L Q I S S S G V I A P G A K A G V K L E V A N M Q A E L V A K P S V S

881 V E F V T N M G I I I P D F A R S G V Q M N T N F F H E S G L E A H V A L K A G
K L K F I I P S P K R P V K L L S G G N T L H L V S T T K T E V I P P L I E N R

961 Q S W S V C K Q V F P G L N Y C T S G A Y S N A S S T D S A S Y Y P L T G D T
R L E L E L R P T G E I E Q Y S V S A T Y E L Q R E D R A L V D T L K F V T Q A E

1041 G A K Q T E A T M T F K Y N R Q S M T L S S E V Q I P D F D V D L G T I L R V
N D E S T E G K T S Y R L T L D I Q N K K I T E V A L M G H L S C D T K E E R K I

1121 K G V I S I P R L Q A E A R S E I L A H W S P A K L L L Q M D S S A T A Y G S
T V S K R V A W H Y D E E K I E F E W N T G T N V D T K K M T S N F P V D L S D
Y

1201 P K S L H M Y A N R L L D H R V P Q T D M T F R H V G S K L I V A M S S W L
Q K A S G S L P Y T Q T L Q D H L N S L K E F N L Q N M G L P D F H I P E N L F L K

1281 S D G R V K Y T L N K N S L K I E I P L P F G G K S S R D L K M L E T V R T P
A L H F K S V G F H L P S R E F Q V P T F T I P K L Y Q L Q V P L L G V L D L S T

1361 N V Y S N L Y N W S A S Y S G G N T S T D H F S L R A R Y H M K A D S V V D
L L S Y N V Q G S G E T T Y D H K N T F T L S C D G S L R H K F L D S N I K F S H V

1441 E K L G N N P V S K G L L I F D A S S S W G P Q M S A S V H L D S K K K Q H L
F V K E V K I D G Q F R V S S F Y A K G T Y G L S C Q R D P N T G R L N G E S N L

1521 R F N S S Y L Q G T N Q I T G R Y E D G T L S L T S T S D L Q S G I I K N T A S
L K Y E N Y E L T L K S D T N G K Y K N F A T S N K M D M T F S K Q N A L L R S

1601 E Y Q A D Y E S L R F F S L L S G S L N S H G L E L N A D I L G T D K I N S G A
H K A T L R I G Q D G I S T S A T T N L K C S L L V L E N E L N A E L G L S G A

1681 S M K L T T N G R F R E H N A K F S L D G K A A L T E L S L G S A Y Q A M I L
G V D S K N I F N F K V S Q E G L K L S N D M M G S Y A E M K F D H T N S L N I A

1761 G L S L D F S S K L D N I Y S S D K F Y K Q T V N L Q L Q P Y S L V T T L N S
D L K Y N A L D L T N N G K L R L E P L K L H V A G N L K G A Y Q N N E I K H I Y

1841 A I S S A A L S A S Y K A D T V A K V Q G V E F S H R L N T D I A G L A S A I
D M S T N Y N S D S L H F S N V F R S V M A P F T M T I D A H T N G N G K L A L W

1921 G E H T G Q L Y S K F L L K A E P L A F T F S H D Y K G S T S H H L V S R K S
I S A A L E H K V S A L L T P A E Q T G T W K L K T Q F N N N E Y S Q D L D A Y N

2001 T K D K I G V E L T G R T L A D L T L L D S P I K V P L L L S E P I N I I D A L E
M R D A V E K P Q E F T I V A F V K Y D K N Q D V H S I N L P F F E T L Q E Y

2081 F E R N R Q T I I V V L E N V Q R N L K H I N I D Q F V R K Y R A A L G K L P
Q Q A N D Y L N S F N W E R Q V S H A K E K L T A L T K K Y R I T E N D I Q I A L

2161 D D A K I N F N E K L S Q L Q T Y M I Q F D Q Y I K D S Y D L H D L K I A I A
N I I D E I I E K L K S L D E H Y H I R V N L V K T I H D L H L F I E N I D F N K

2241 S G S S T A S W I Q N V D T K Y Q I R I Q I Q E K L Q Q L K R H I Q N I D I Q H
L A G K L K Q H I E A I D V R V L L D Q L G T T I S F E R I N D V L E H V K H F

2321 V I N L I G D F E V A E K I N A F R A K V H E L I E R Y E V D Q Q I Q V L M D
K L V E L A H Q Y K L K E T I Q K L S N V L Q Q V K I K D Y F E K L V G F I D D A

2401 V K K L N E L S F K T F I E D V N K F L D M L I K K L K S F D Y H Q F V D E T
N D K I R E V T Q R L N G E I Q A L E L P Q K A E A L K L F L E E T K A T V A V Y

2481 L E S L Q D T K I T L I I N W L Q E A L S S A S L A H M K A K F R E T L E D T R
D R M Y Q M D I Q Q E L Q R Y L S L V G Q V Y S T L V T Y I S D W W T L A A K N

2561 L T D F A E Q Y S I Q D W A K R M K A L V E Q G F T V P E I K T I L G T M P A
F E V S L Q A L Q K A T F Q T P D F I V P L T D L R I P S V Q I N F K D L K N I K

2641 IPSRFSTPEFTILNTFHIPSFTIDFVEMKVKIIRTIDQMLNS ELQWPVPDIYLRDLKVEDIPLARITLPDFRLPEIAIPE

2721 FIIPTLNLNDFQVPDLHIPEFQLPHISHTIEVPTFGKLYSIL KIQSPLFTLDANADIGNGTTSANEAGIAASITAKGESK

2801 LEVLNFDFQANAQLSNPKINPLALKESVKFSSKYLRTEH GSEMLFFGNAIEGKSNTVASLHTEKNTLELSNGVIVKINNQ

2881 LTLDSNTKYFHKLNIPKLDFSSQADLRNEIKTLLKAGHIA WTSSGKGSWKWACPRFSDEGTHESQISFTIEGPLTSFGLS

2961 NKINSKHLRVNQNLVYESGSLNFSKLEIQSQVDSQHVGH SVLTAKGMALFGEGKAEFTGRHDAHLNGKVIGTLKNSLFFS

3041 AQPFEITASTNNEGNLKVRFPLRLTGKIDFLNNYALFLSP SAQQASWQVSARFNQYKYNQNFSAGNNENIMEAHVGINGE

3121 ANLDFLNIPLTIPEMRLPYTIITTPPLKDFSLWEKTGLKEF LKTTKQSFDLSVKAQYKKNKHRHSITNPLAVLCEFISQS

3201 IKSFDRHFEKNRNNALDFVTKSYNETKIKFDKYKAEKSH DELPRTFQIPGYTVPVVNVEVSPFTIEMSAFGYVFPKAVSM

3281 PSFSILGSDVRVPSYTLILPSLELPVLHVPRNLKLSLPDFK ELCTISHIFIPAMGNITYDFSFKSSVITLNTNAELFNQS

3361 DIVAHLLSSSSSVIDALQYKLEGTTRLTRKRGLKLATALS LSNKFVEGSHNSTVSLTTKNMEVSVATTTKAQIPILRMNF

3441 KQELNGNTKSKPTVSSSMEFKYDFNSSMLYSTAKGAVD HKLSLESLTSYFSIESSTKGDVKGSVLSREYSGTIASEANTY

3521 LNSKSTRSSVKLQGTSKIDDIWNLEVKENFAGEATLQRI YSLWEHSTKNHLQLEGLFFTNGEHTSKATLELSPWQMSALV

3601 QVHASQPSSFHDFPDLGQEVALNANTKNQKIRWKNEVRI HSGSFQSQVELSNDQEKAHLDIAGSLEGHLRFLKNIILPVY

3681 DKSLWDFLKLDVTTSIGRRQHLRVSTAFVYTKNPNGYSF SIPVKVLADKFIIPGLKLNDLNSVLVMPTFHVPFTDLQVPS

3761 C K L D F R E I Q I Y K K L R T S S F A L N L P T L P E V K F P E V D V L T K Y S Q P E D S L I P F F E I T V P E S Q L T V S Q F T L P K S V S D G I A A L D L

3841 N A V A N K I A D F E L P T I I V P E Q T I E I P S I K F S V P A G I V I P S F Q A L T A R F E V D S P V Y N A T W S A S L K N K A D Y V E T V L D S T C S S T

3921 V Q F L E Y E L N V L G T H K I E D G T L A S K T K G T F A H R D F S A E Y E E D G K Y E G L Q E W E G K A H L N I K S P A F T D L H L R Y Q K D K K G I S T S

4001 A A S P A V G T V G M D M D E D D D F S K W N F Y Y S P Q S S P D K K L T I F K T E L R V R E S D E E T Q I K V N W E E E A A S G L L T S L K D N V P K A T G V

4081 L Y D Y V N K Y H W E H T G L T L R E V S S K L R R N L Q N N A E W V Y Q G A I R Q I D D I D V R F Q K A A S G T T G T Y Q E W K D K A Q N L Y Q E L L T Q E G

4161 Q A S F Q G L K D N V F D G L V R V T Q E F H M K V K H L I D S L I D F L N F P R F Q F P G K P G I Y T R E E L C T M F I R E V G T V L S Q V Y S K V H N G S E

4241 I L F S Y F Q D L V I T L P F E L R K H K L I D V I S M Y R E L L K D L S K E A Q E V F K A I Q S L K T T E V L R N L Q D L L Q F I F Q L I E D N I K Q L K E M

4321 K F T Y L I N Y I Q D E I N T I F S D Y I P Y V F K L L K E N L C L N L H K F N E F I Q N E L Q E A S Q E L Q Q I H Q Y I M A L R E E Y F D P S I V G W T V K Y

4401 Y E L E E K I V S L I K N L L V A L K D F H S E Y I V S A S N F T S Q L S S Q V E Q F L H R N I Q E Y L S I L T D P D G K G K E K I A E L S A T A Q E I I K S Q

4481 A I A T K K I I S D Y H Q Q F R Y K L Q D F S D Q L S D Y Y E K F I A E S K R L I D L S I Q N Y H T F L I Y I T E L L K K L Q S T T V M N P Y M K L A P G E L T

4561 IIL

5.31 peroxiredoxin 2 isoform a [Homo sapiens]

Protein Accession gi|32189392
Mean Expression Ratio 1.44
Median Expression Ratio 1.43
Credible Interval (1.03, 2.04)
Associated Peptides 3
Associated Spectra 5
Coverage 0.146

| A | 2.5 | 50 | 97.5 | Sequence |
|---|---|---|---|---|
| 3 | 1.1 | 1.5 | 2.2 | EGGLGPLNIPLLADVTR |
| 1 | 0.99 | 1.5 | 2.2 | KEGGLGPLNIPLLADVTR |
| 1 | 0.96 | 1.5 | 2.2 | QITVNDLPVGR |

MASGNARIGKPAPDFKATAVVDGAFKEVKLSDYKGKYVVLFFYPLDFTFVCPT
EIIAFSNRAEDFRKLGCEVLGVSVDSQ

FTHLAWINTPRKEGGLGPLNIPLLADVTRRLSEDYGVLKTDEGIAYRGLFIIDG
KGVLRQITVNDLPVGRSVDEALRLVQ

AFQYTDEHGEVCPAGWKPGSDTIKPNVDDSKEYFSKHN

5.32 sphingomyelin phosphodiesterase 1, acid lysosomal isoform 1 precursor [Homo sapiens]

Protein Accession gi|56117840
Mean Expression Ratio 0.703
Median Expression Ratio 0.702
Credible Interval (0.479, 1.03)
Associated Peptides 2
Associated Spectra 3
Coverage 0.0285

| A | 2.5 | 50 | 97.5 | Sequence |
|---|---|---|---|---|
| 2 | 0.43 | 0.65 | 0.98 | AWEPWLPAEALR |
| 1 | 0.45 | 0.69 | 1.1 | LCNLLK |

MPRYGASLRQSCPRSGREQGQDGTAGAPGLLWMGLVLAL
ALALALALALSDSRVLWAPAEAHPLSPQGHPARLHRIVPRL

RDVFGWGNLTCPICKGLFTAINLGLKKEPNVARVGSVAIK
LCNLLKIAPPAVCQSIVHLFEDDMVEVWRRSVLSPSEACG

LLLGSTCGHWDIFSSWNISLPTVPKPPPKPPSPPAPGAPVS
RILFLTDLHWDHDYLEGTDPDCADPLCCRRGSGLPPASR

PGAGYWGEYSKCDLPLRTLESLLSGLGPAGPFDMVYWTG
DIPAHDVWHQTRQDQLRALTTVTALVRKFLGPVPVYPAVGN

HESTPVNSFPPPFIEGNHSSRWLYEAMAKAWEPWLPAEA
LRTLRIGGFYALSPYPGLRLISLNMNFCSRENFWLLINSTD

PAGQLQWLVGELQAAEDRGDKVHIIGHIPPGHCLKSWSW
NYYRIVARYENTLAAQFFGHTHVDEFEVFYDEETLSRPLAV

481 A F L A P S A T T Y I G L N P G Y R V Y Q I D G N Y S G S S H V V L D H E T Y I
L N L T Q A N I P G A I P H W Q L L Y R A R E T Y G L P N T L P T A W H N L V Y

561 R M R G D M Q L F Q T F W F L Y H K G H P P S E P C G T P C R L A T L C A Q L
S A R A D S P A L C R H L M P D G S L P E A Q S L W P R P L F C

5.33 proteasome beta 2 subunit [Homo sapiens]

Protein Accession gi|4506195
Mean Expression Ratio 1.41
Median Expression Ratio 1.41
Credible Interval (0.987, 2.04)
Associated Peptides 3
Associated Spectra 4
Coverage 0.224

| A | 2.5 | 50 | 97.5 | Sequence |
|---|---|---|---|---|
| 1 | 0.97 | 1.5 | 2.3 | FILNLPTFSVR |
| 2 | 1.0 | 1.5 | 2.2 | LIGIQGPDYVLVASDR |
| 1 | 0.91 | 1.4 | 2.1 | HVNLLLAGYDEHEGPALY |

1 M E Y L I G I Q G P D Y V L V A S D R V A A S N I V Q M K D D H D K M F K M S E
K I L L L C V G E A G D T V Q F A E Y I Q K N V Q L Y K M R N G Y E L S P T A A

81 A N F T R R N L A D C L R S R T P Y H V N L L L A G Y D E H E G P A L Y Y M D
Y L A A L A K A P F A A H G Y G A F L T L S I L D R Y Y T P T I S R E R A V E L L

161 R K C L E E L Q K R F I L N L P T F S V R I I D K N G I H D L D N I S F P K Q G S

5.34 complement component 4B preproprotein [Homo sapiens]

Protein Accession gi|178557739
Mean Expression Ratio 0.712
Median Expression Ratio 0.711
Credible Interval (0.435, 1.15)
Associated Peptides 1
Associated Spectra 1
Coverage NaN

| A | 2.5 | 50 | 97.5 | Sequence |
|---|---|---|---|---|
| 1 | 0.39 | 0.65 | 1.1 | LLSQQQADGSFQDLSPVIHR |

5.35 manganese superoxide dismutase isoform A precursor [Homo sapiens]; manganese superoxide dismutase isoform A precursor [Homo sapiens]

Protein Accession gi|67782307 gi|67782305
Mean Expression Ratio 1.40
Median Expression Ratio 1.40
Credible Interval (1.10, 1.77)
Associated Peptides 9
Associated Spectra 13
Coverage NaN

| A | 2.5 | 50 | 97.5 | Sequence |
|---|-----|-----|------|----------|
| 2 | 1.0 | 1.4 | 1.9 | AIWNVINWENVTER |
| 2 | 1.1 | 1.5 | 2.1 | DFGSFDKFK |
| 1 | 1.0 | 1.4 | 2.0 | FNGGGHINHSIFW |
| 1 | 1 | 1.4 | 2 | GDVTAQIALQPALK |
| 2 | 1.0 | 1.4 | 2 | GELLEAIKR |
| 1 | 0.97 | 1.4 | 1.9 | HHAAYVNNLNVTEEK |
| 2 | 1 | 1.4 | 1.9 | NVRPDYLK |
| 1 | 1.0 | 1.4 | 2 | GWLGFNK |
| 1 | 0.96 | 1.4 | 1.9 | YQEALAK |

5.36 transthyretin [Homo sapiens]

Protein Accession gi|4507725
Mean Expression Ratio 0.718
Median Expression Ratio 0.718
Credible Interval (0.572, 0.9)
Associated Peptides 6
Associated Spectra 17
Coverage 0.49

| A | 2.5 | 50 | 97.5 | Sequence |
|---|-----|-----|------|----------|
| 3 | 0.54 | 0.73 | 1 | ALGISPFHEHAEVVF |
| 2 | 0.56 | 0.76 | 1.0 | ALGISPFHEHAEVVFTANDSGPR |
| 6 | 0.48 | 0.61 | 0.8 | GSPAINVAVHVFR |
| 2 | 0.5 | 0.7 | 0.95 | KAADDTWEPFASGK |
| 3 | 0.54 | 0.74 | 1 | TSESGELHGLTTEEEFVEGIYK |
| 1 | 0.51 | 0.72 | 1 | AADDTWEPFASGK |

1 M A S H R L L L L C L A G L V F V S E A G P T G T G E S K C P L M V K V L D A V
R G S P A I N V A V H V F R K A A D D T W E P F A S G K T S E S G E L H G L T T

81 E E E F V E G I Y K V E I D T K S Y W K A L G I S P F H E H A E V V F T A N D S G P R R Y T I A A L L S P Y S Y S T T A V V T N P K E

5.37 haptoglobin isoform 1 preproprotein [Homo sapiens]; haptoglobin isoform 2 preproprotein [Homo sapiens]

Protein Accession gi|4826762 gi|186910296
Mean Expression Ratio 0.721
Median Expression Ratio 0.72
Credible Interval (0.565, 0.918)
Associated Peptides 9
Associated Spectra 13
Coverage NaN

| A | 2.5 | 50 | 97.5 | Sequence |
|---|-----|-----|------|----------|
| 1 | 0.52 | 0.74 | 1.1 | AVHDLEEDTWYATGILSFDK |
| 1 | 0.51 | 0.73 | 1.0 | GSFPWQAK |
| 1 | 0.5 | 0.71 | 1.0 | HYEGSTVPEKK |
| 2 | 0.54 | 0.74 | 1.0 | ILGGHLDAK |
| 1 | 0.48 | 0.67 | 0.95 | SPVGVQPILNEHTFCAGMSK |
| 1 | 0.52 | 0.72 | 1.0 | VMPICLPSKDYAEVGR |
| 1 | 0.52 | 0.73 | 1.0 | VTSIQDWVQK |
| 3 | 0.51 | 0.69 | 0.93 | YVMLPVADQDQCIR |
| 2 | 0.5 | 0.7 | 0.97 | FTDHLK |

5.38 fibulin 1 isoform D [Homo sapiens]

Protein Accession gi|34734066
Mean Expression Ratio 0.726
Median Expression Ratio 0.728
Credible Interval (0.464, 1.12)
Associated Peptides 2
Associated Spectra 2
Coverage 0.0327

| A | 2.5 | 50 | 97.5 | Sequence |
|---|-----|-----|------|----------|
| 1 | 0.42 | 0.68 | 1.1 | EFTRPEEIIFLR |
| 1 | 0.45 | 0.73 | 1.2 | IIFDITEGNLR |

1 M E R A A P S R R V P L P L L L L G G L A L L A A G V D A D V L L E A C C A D G H R M A T H Q K D C S L P Y A T E S K E C R M V Q E Q C C H S Q L E E L H C A T

GISLANEQDRCATPHGDNASLEATFVKRCCHCCLLGRAAQ
AQGQSCEYSLMVGYQCGQVFRACCVKSQETGDLDVGGLQE

TDKIIEVEEEQEDPYLNDRCRGGGPCKQQCRDTGDEVVC
SCFVGYQLLSDGVSCEDVNECITGSHSCRLGESCINTVGSF

RCQRDSSCGTGYELTEDNSCKDIDECESGIHNCLPDFICQ
NTLGSFRCRPKLQCKSGFIQDALGNCIDINECLSISAPCP

IGHTCINTEGSYTCQKNVPNCGRGYHLNEEGTRCVDVDE
CAPPAEPCGKGHRCVNSPGSFRCECKTGYYFDGISRMCVDV

NECQRYPGRLCGHKCENTLGSYLCSCSVGFRLSVDGRSC
EDINECSSSPCSQECANVYGSYQCYCRRGYQLSDVDGVTCE

DIDECALPTGGHICSYRCINIPGSFQCSCPSSGYRLAPNGR
NCQDIDECVTGIHNCSINETCFNIQGGFRCLAFECPENY

RRSAATLQQEKTDTVRCIKSCRPNDVTCVFDPVHTISHTV
ISLPTFREFTRPEEIIFLRAITPPHPASQANIIFDITEGN

LRDSFDIIKRYMDGMTVGVVRQVRPIVGPFHAVLKLEMN
YVVGGVVSHRNVVNVHIFVSEYWF

5.39 serine (or cysteine) proteinase inhibitor, clade C (antithrombin), member 1 [Homo sapiens]

Protein Accession gi|4502261
Mean Expression Ratio 0.73
Median Expression Ratio 0.73
Credible Interval (0.529, 1.00)
Associated Peptides 5
Associated Spectra 5
Coverage 0.194

| A | 2.5 | 50 | 97.5 | Sequence |
|---|-----|----|------|----------|
| 1 | 0.48 | 0.72 | 1.1 | AFLEVNEEGSEAAASTAVVIAGR |
| 1 | 0.49 | 0.74 | 1.1 | EVPLNTIIFMGR |
| 1 | 0.48 | 0.72 | 1.1 | VAEGTQVLELPFK |
| 1 | 0.49 | 0.72 | 1.1 | ELTPEVLQEWLDELEEMMLVVHMPR |
| 1 | 0.47 | 0.7 | 1.0 | ATEDEGSEQKIPEATNR |

MYSNVIGTVTSGKRKVYLLSLLLIGFWDCVTCHGSPVDICT
AKPRDIPMNPMCIYRSPEKKATEDEGSEQKIPEATNRRV

81 W E L S K A N S R F A T T F Y Q H L A D S K N D N D N I F L S P L S I S T A F A
M T K L G A C N D T L Q Q L M E V F K F D T I S E K T S D Q I H F F F A K L N C

161 R L Y R K A N K S S K L V S A N R L F G D K S L T F N E T Y Q D I S E L V Y G
A K L Q P L D F K E N A E Q S R A A I N K W V S N K T E G R I T D V I P S E A I N

241 E L T V L V L V N T I Y F K G L W K S K F S P E N T R K E L F Y K A D G E S C S
A S M M Y Q E G K F R Y R R V A E G T Q V L E L P F K G D D I T M V L I L P K P

321 E K S L A K V E K E L T P E V L Q E W L D E L E E M M L V V H M P R F R I E D
G F S L K E Q L Q D M G L V D L F S P E K S K L P G I V A E G R D D L Y V S D A F

401 H K A F L E V N E E G S E A A A S T A V V I A G R S L N P N R V T F K A N R P F
L V F I R E V P L N T I I F M G R V A N P C V K

5.40 hypothetical protein LOC148362 [Homo sapiens]

Protein Accession gi|124244088
Mean Expression Ratio 1.37
Median Expression Ratio 1.37
Credible Interval (0.994, 1.92)
Associated Peptides 5
Associated Spectra 5
Coverage 0.141

| A | 2.5 | 50 | 97.5 | Sequence |
|---|---|---|---|---|
| 1 | 0.92 | 1.4 | 2.0 | LLELFTDLSCNPEMMK |
| 1 | 0.94 | 1.4 | 2.1 | NAADSYFSLLQGFINSLDESTQESK |
| 1 | 0.93 | 1.4 | 2.1 | SLLQGFINSLDESTQESK |
| 1 | 0.94 | 1.4 | 2.1 | YIQNFK |
| 1 | 0.92 | 1.4 | 2.1 | IPTEAPQLELK |

1 M T H W F H R N P L K A T A P V S F N Y Y G V V T G P S A S K I C N D L R S S R
A R L L E L F T D L S C N P E M M K N A A D S Y F S L L Q G F I N S L D E S T Q

81 E S K L R Y I Q N F K W T D T L Q G Q V P S A Q Q D A V F E L I S M G F N V A L
W Y T K Y A S R L A G K E N I T E D E A K E V H R S L K I A A G I F K H L K E S

161 H L P K L I T P A E K G R D L E S R L I E A Y V I Q C Q A E A Q E V T I A R A I E
L K H A P G L I A A L A Y E T A N F Y Q K A D H T L S S L E P A Y S A K W R K

241 Y L H L K M C F Y T A Y A Y C Y H G E T L L A S D K C G E A I R S L Q E A E K
L Y A K A E A L C K E Y G E T K G P G P T V K P S G H L F F R K L G N L V K N T L

321 E K C Q R E N G F I Y F Q K I P T E A P Q L E L K A N Y G L V E P I P F E F P P T
S V Q W T P E T L A A F D L T K R P K D D S T K P K P E E E V K P V K E P D I

401 K P Q K D T G C Y I S

5.41 ferritin, heavy polypeptide 1 [Homo sapiens]

Protein Accession gi|56682959
Mean Expression Ratio 1.36
Median Expression Ratio 1.36
Credible Interval (1.12, 1.67)
Associated Peptides 9
Associated Spectra 21
Coverage 0.41

| A | 2.5 | 50 | 97.5 | Sequence |
|---|---|---|---|---|
| 3 | 1.0 | 1.4 | 1.9 | ELGDHVTNLR |
| 1 | 1.1 | 1.5 | 2.1 | GGRIFLQDIK |
| 2 | 0.9 | 1.2 | 1.6 | LCDFIETHYLNEQVK |
| 2 | 1.0 | 1.4 | 1.9 | MGAPESGLAEYLFDK |
| 3 | 1.1 | 1.5 | 1.9 | NDPHLCDFIETHYLNEQVK |
| 5 | 1.1 | 1.4 | 1.9 | NVNQSLLELHK |
| 1 | 0.97 | 1.3 | 1.8 | YFLHQSHEER |
| 1 | 0.96 | 1.3 | 1.8 | LCDFIETHY |
| 3 | 1.0 | 1.4 | 1.9 | IFLQDIK |

1 M T T A S T S Q V R Q N Y H Q D S E A A I N R Q I N L E L Y A S Y V Y L S M S Y
Y F D R D D V A L K N F A K Y F L H Q S H E E R E H A E K L M K L Q N Q R G G R

81 I F L Q D I K K P D C D D W E S G L N A M E C A L H L E K N V N Q S L L E L H K
L A T D K N D P H L C D F I E T H Y L N E Q V K A I K E L G D H V T N L R K M G

161 APE S G LA E Y L F D K H T L G D S D N E S

5.42 serum amyloid A1 preproprotein [Homo sapiens]; serum amyloid A1 preproprotein [Homo sapiens]

Protein Accession gi|40316912 gi|40316910
Mean Expression Ratio 0.738
Median Expression Ratio 0.737
Credible Interval (0.512, 1.06)
Associated Peptides 3
Associated Spectra 4
Coverage NaN

| A | 2.5 | 50 | 97.5 | Sequence |
|---|-----|-----|------|----------|
| 2 | 0.47 | 0.69 | 1.0 | FFGHGAEDSLADQAANEWGR |
| 1 | 0.48 | 0.73 | 1.1 | SFFSFLGEAFDGAR |
| 1 | 0.48 | 0.74 | 1.1 | GPGGAWAAEVISDAR |

5.43 programmed cell death 6 [Homo sapiens]

Protein Accession gi|7019485
Mean Expression Ratio 1.34
Median Expression Ratio 1.34
Credible Interval (1.02, 1.77)
Associated Peptides 4
Associated Spectra 10
Coverage 0.319

| A | 2.5 | 50 | 97.5 | Sequence |
|---|-----|-----|------|----------|
| 4 | 0.99 | 1.3 | 1.8 | LSDQFHDILIR |
| 4 | 1.0 | 1.4 | 1.9 | SGVISDTELQQALSNGTWTPFNPVTVR |
| 1 | 0.99 | 1.4 | 2.1 | YITDWQNVFR |
| 1 | 0.93 | 1.3 | 2.0 | AGVNFSEFTGVWK |

1 MAAYSYRPGPGAGPGPAAGAALPDQSFLWNVFQRVDKDR
SGVISDTELQQALSNGTWTPFNPVTVRSIISMFDRENKAGV

81 NFSEFTGVWKYITDWQNVFRTYDRDNSGMIDKNELKQAL
SGFGYRLSDQFHDILIRKFDRQGRGQIAFDDFIQGCIVLQR

161 LTDIFRRYDTDQDGWIQVSYEQYLSMVFSIV

5.44 complement component 1 inhibitor precursor [Homo sapiens]; complement component 1 inhibitor precursor [Homo sapiens]

Protein Accession gi|73858570 gi|73858568
Mean Expression Ratio 0.745
Median Expression Ratio 0.744
Credible Interval (0.58, 0.953)
Associated Peptides 8
Associated Spectra 12
Coverage NaN

| A | 2.5 | 50 | 97.5 | Sequence |
|---|-----|-----|------|----------|
| 3 | 0.5 | 0.69 | 0.93 | GVTSVSQIFHSPDLAIR |
| 2 | 0.55 | 0.77 | 1.1 | KYPV AHFIDQTLK |

(continued)

| A | 2.5 | 50 | 97.5 | Sequence |
|---|-----|-----|------|----------|
| 1 | 0.52 | 0.74 | 1.0 | LEDMEQALSPSVFK |
| 1 | 0.52 | 0.75 | 1.0 | LLDSLPSDTR |
| 1 | 0.53 | 0.75 | 1.1 | L VLLNAIYLSAK |
| 1 | 0.52 | 0.74 | 1.1 | QHQTVLELTETGVEAAAASAISVAR |
| 2 | 0.53 | 0.73 | 1.0 | QTVLELTETGVEAAAASAISVAR |
| 1 | 0.53 | 0.76 | 1.1 | DFTCVHQALK |

5.45 chromatin modifying protein 5 [Homo sapiens]

Protein Accession gi|189409150
Mean Expression Ratio 1.34
Median Expression Ratio 1.34
Credible Interval (0.915, 2.00)
Associated Peptides 3
Associated Spectra 3
Coverage NaN

| A | 2.5 | 50 | 97.5 | Sequence |
|---|-----|-----|------|----------|
| 1 | 0.89 | 1.4 | 2.1 | APPPSLTDCIGTVDSR |
| 1 | 0.88 | 1.4 | 2.1 | IDQIEDLQDQLEDMMEDANEIQEALSR |
| 1 | 0.9 | 1.4 | 2.1 | YKDQIK |

5.46 vacuolar protein sorting factor 4B [Homo sapiens]

Protein Accession gi|17865802
Mean Expression Ratio 1.33
Median Expression Ratio 1.33
Credible Interval (0.961, 1.84)
Associated Peptides 4
Associated Spectra 5
Coverage 0.146

| A | 2.5 | 50 | 97.5 | Sequence |
|---|-----|-----|------|----------|
| 1 | 0.9 | 1.4 | 2.0 | ENKPSIIFIDEIDSLCGSR |
| 1 | 0.88 | 1.3 | 2.0 | LLEPVVSMSDMLR |
| 2 | 0.94 | 1.4 | 2.0 | LQNQLQGAIVIERPNVK |
| 1 | 0.9 | 1.3 | 2 | SLSNTKPTVNEHDLLK |

1 M S S T S P N L Q K A I D L A S K A A Q E D K A G N Y E E A L Q L Y Q H A V Q Y F L H V V K Y E A Q G D K A K Q S I R A K C T E Y L D R A E K L K E Y L K N K E

81 K K A Q K P V K E G Q P S P A D E K G N D S D G E G E S D D P E K K K L Q N Q L Q G A I V I E R P N V K W S D V A G L E G A K E A L K E A V I L P I K F P H L F

161 T G K R T P W R G I L L F G P P G T G K S Y L A K A V A T E A N N S T F F S I S S S D L V S K W L G E S E K L V K N L F Q L A R E N K P S I I F I D E I D S L C

241 G S R S E N E S E A A R R I K T E F L V Q M Q G V G V D N D G I L V L G A T N I P W V L D S A I R R R F E K R I Y I P L P E P H A R A A M F K L H L G T T Q N S

321 L T E A D F R E L G R K T D G Y S G A D I S I I V R D A L M Q P V R K V Q S A T H F K K V R G P S R A D P N H L V D D L L T P C S P G D P G A I E M T W M D V P

401 G D K L L E P V V S M S D M L R S L S N T K P T V N E H D L L K L K K F T E D F G Q E G

5.47 prominin 1 [Homo sapiens]

Protein Accession gi|5174387
Mean Expression Ratio 1.32
Median Expression Ratio 1.32
Credible Interval (1.05, 1.66)
Associated Peptides 9
Associated Spectra 15
Coverage 0.158

| A | 2.5 | 50 | 97.5 | Sequence |
|---|---|---|---|---|
| 3 | 11. | 4 | 1.8 | AFTDLNSINSVLGGGILDR |
| 1 | 0.96 | 1.3 | 1.9 | LLNEDWEYYLSGK |
| 1 | 0.96 | 1.3 | 1.9 | LTFEQVYSDCK |
| 3 | 0.95 | 1.3 | 1.7 | QLPPVDAELDNVNNVLR |
| 1 | 0.96 | 1.3 | 1.9 | SLHQQSTQLSSSLTSVK |
| 3 | 1.0 | 1.4 | 1.9 | TDLDGLVQQGYQSLNDIPDR |
| 1 | 0.94 | 1.3 | 1.8 | TLLNETPEQIK |
| 1 | 0.92 | 1.3 | 1.8 | VLPIEQSLSTLYQSVK |
| 1 | 0.94 | 1.3 | 1.9 | VNLNIFLLGAAGR |

1 M A L V L G S L L L L G L C G N S F S G G Q P S S T D A P K A W N Y E L P A T N Y E T Q D S H K A G P I G I L F E L V H I F L Y V V Q P R D F P E D T L R K F L

81 Q K A Y E S K I D Y D K P E T V I L G L K I V Y Y E A G I I L C C V L G L L F I I L M P L V G Y F F C M C R C C N K C G G E M H Q R Q K E N G P F L R K C F A I

SLLVICIIISIGIFYGFVANHQVRTRIKRSRKLADSNFKDLR
TLLNETPEQIKYILAQYNTTKDKAFTDLNSINSVLGGG

ILDRLRPNIIPVLDEIKSMATAIKETKEALENMNSTLKSLH
QQSTQLSSSLTSVKTSLRSSLNDPLCLVHPSSETCNSIR

LSLSQLNSNPELRQLPPVDAELDNVNNVLRTDLDGLVQQ
GYQSLNDIPDRVQRQTTTVVAGIKRVLNSIGSDIDNVTQRL

PIQDILSAFSVYVNNTESYIHRNLPTLEEYDSYWWLGGLV
ICSLLTLIVIFYYLGLLCGVCGYDRHATPTTRGCVSNTGG

VFLMVGVGLSFLFCWILMIIVVLTFVFGANVEKLICEPYT
SKELFRVLDTPYLLNEDWEYYLSGKLFNKSKMKLTFEQVY

SDCKKNRGTYGTLHLQNSFNISEHLNINEHTGSISSELESL
KVNLNIFLLGAAGRKNLQDFAACGIDRMNYDSYLAQTGK

SPAGVNLLSFAYDLEAKANSLPPGNLRNSLKRDAQTIKTI
HQQRVLPIEQSLSTLYQSVKILQRTGNGLLERVTRILASL

DFAQNFITNNTSSVIIEETKKYGRTIIGYFEHYLQWIEFSIS
EKVASCKPVATALDTAVDVFLCSYIIDPLNLFWFGIGK

ATVFLLPALIFAVKLAKYYRRMDSEDVYDDVETIPMKNM
ENGNNGYHKDHVYGIHNPVMTSPSQH

5.48 alpha 3 type VI collagen isoform 1 precursor [Homo sapiens]

Protein Accession gi|55743098
Mean Expression Ratio 0.756
Median Expression Ratio 0.757
Credible Interval (0.563, 1.02)
Associated Peptides 6
Associated Spectra 7
Coverage 0.0353

| A | 2.5 | 50 | 97.5 | Sequence |
|---|-----|----|------|----------|
| 1 | 0.49 | 0.71 | 1.0 | AAPLQGMLPGLLAPLR |
| 1 | 0.51 | 0.75 | 1.1 | GADQAELEEIAFDSSLVFIPAEFR |
| 1 | 0.5 | 0.73 | 1.1 | GAQGPAGPAGPPGLIGEQGISGPR |
| 1 | 0.51 | 0.75 | 1.1 | ITEGVPQLLIVLTADR |
| 1 | 0.51 | 0.75 | 1.1 | LVDYLDVGFDTTR |

(continued)

| A | 2.5 | 50 | 97.5 | Sequence |
|---|-----|-----|------|----------|
| 2 | 0.56 | 0.8 | 1.1 | MKPLDGSALYTGSALDFVR |

1 MRKHRHLPLVAVFCLFLSGFPTTHAQQQQADVKNGAAADI
IFLVDSSWTIGEEHFQLVREFLYDVVKSLAVGENDFHFAL

81 VQFNGNPHTEFLLNTYRTKQEVLSHISNMSYIGGTNQTGK
GLEYIMQSHLTKAAGSRAGDGVPQVIVVLTDGHSKDGLAL

161 PSAELKSADVNVFAIGVEDADEGALKEIASEPLNMHMFN
LENFTSLHDIVGNLVSCVHSSVSPERAGDTETLKDITAQDS

241 ADIIFLIDGSNNTGSVNFAVILDFLVNLLEKLPIGTQQIRV
GVVQFSDEPRTMFSLDTYSTKAQVLGAVKALGFAGGELA

321 NIGLALDFVVENHFTRAGGSRVEEGVPQVLVLISAGPSSD
EIRYGVVALKQASVFSFGLGAQAASRAELQHIATDDNLVF

401 TVPEFRSFGDLQEKLLPYIVGVAQRHIVLKPPTIVTQVIEV
NKRDIVFLVDGSSALGLANFNAIRDFIAKVIQRLEIGQD

481 LIQVAVAQYADTVRPEFYFNTHPTKREVITAVRKMKPLD
GSALYTGSALDFVRNNLFTSSAGYRAAEGIPKLLVLITGGK

561 SLDEISQPAQELKRSSIMAFAIGNKGADQAELEEIAFDSSL
VFIPAEFRAAPLQGMLPGLLAPLRTLSGTPEVHSNKRDI

641 IFLLDGSANVGKTNFPYVRDFVMNLVNSLDIGNDNIRVGL
VQFSDTPVTEFSLNTYQTKSDILGHLRQLQLQGGSGLNTG

721 SALSYVYANHFTEAGGSRIREHVPQLLLLLTAGQSEDSYL
QAANALTRAGILTFCVGASQANKAELEQIAFNPSLVYLMD

801 DFSSLPALPQQLIQPLTTYVSGGVEEVPLAQPESKRDILFL
FDGSANLVGQFPVVRDFLYKIIDELNVKPEGTRIAVAQY

881 SDDVKVESRFDEHQSKPEILNLVKRMKIKTGKALNLGYA
LDYAQRYIFVKSAGSRIEDGVLQFLVLLVAGRSSDRVDGPA

961 SNLKQSGVVPFIFQAKNADPAELEQIVLSPAFILAAESLPK
IGDLHPQIVNLLKSVHNGAPAPVSGEKDVVFLLDGSEGV

1041 R S G F P L L K E F V Q R V V E S L D V G Q D R V R V A V V Q Y S D R T R P
E F Y L N S Y M N K Q D V V N A V R Q L T L L G G P T P N T G A A L E F V L R N I
L

1121 V S S A G S R I T E G V P Q L L I V L T A D R S G D D V R N P S V V V K R G G
A V P I G I G I G N A D I T E M Q T I S F I P D F A V A I P T F R Q L G T V Q Q V

1201 I S E R V T Q L T R E E L S R L Q P V L Q P L P S P G V G G K R D V V F L I D G
S Q S A G P E F Q Y V R T L I E R L V D Y L D V G F D T T R V A V I Q F S D D P

1281 K V E F L L N A H S S K D E V Q N A V Q R L R P K G G R Q I N V G N A L E Y
V S R N I F K R P L G S R I E E G V P Q F L V L I S S G K S D D E V D D P A V E L K

1361 Q F G V A P F T I A R N A D Q E E L V K I S L S P E Y V F S V S T F R E L P S L
E Q K L L T P I T T L T S E Q I Q K L L A S T R Y P P P A V E S D A A D I V F L

1441 I D S S E G V R P D G F A H I R D F V S R I V R R L N I G P S K V R V G V V Q F
S N D V F P E F Y L K T Y R S Q A P V L D A I R R L R L R G G S P L N T G K A L

1521 E F V A R N L F V K S A G S R I E D G V P Q H L V L V L G G K S Q D D V S R F
A Q V I R S S G I V S L G V G D R N I D R T E L Q T I T N D P R L V F T V R E F R

1601 E L P N I E E R I M N S F G P S A A T P A P P G V D T P P P S R P E K K K A D I
V F L L D G S I N F R R D S F Q E V L R F V S E I V D T V Y E D G D S I Q V G L

1681 V Q Y N S D P T D E F F L K D F S T K R Q I I D A I N K V V Y K G G R H A N T
K V G L E H L R V N H F V P E A G S R L D Q R V P Q I A F V I T G G K S V E D A Q

1761 D V S L A L T Q R G V K V F A V G V R N I D S E E V G K I A S N S A T A F R V
G N V Q E L S E L S E Q V L E T L H D A M H E T L C P G V T D A A K A C N L D V I

1841 L G F D G S R D Q N V F V A Q K G F E S K V D A I L N R I S Q M H R V S C S G
G R S P T V R V S V V A N T P S G P V E A F D F D E Y Q P E M L E K F R N M R S Q

1921 H P Y V L T E D T L K V Y L N K F R Q S S P D S V K V V I H F T D G A D G D L
A D L H R A S E N L R Q E G V R A L I L V G L E R V V N L E R L M H L E F G R G F

2001 M Y D R P L R L N L L D L D Y E L A E Q L D N I A E K A C C G V P C K C S G
Q R G D R G P I G S I G P K G I P G E D G Y R G Y P G D E G G P G E R G P P G V N G

2081 T Q G F Q G C P G Q R G V K G S R G F P G E K G E V G E I G L D G L D G E D
G D K G L P G S S G E K G N P G R R G D K G P R G E K G E R G D V G I R G D P G N
P

2161 G Q D S Q E R G P K G E T G D L G P M G V P G R D G V P G G P G E T G K N G G F G R R G P P G A K G N K G G P G Q P G F E G E Q G T R G A Q G P A G P A G P P G

2241 L I G E Q G I S G P R G S G G A A G A P G E R G R T G P L G R K G E P G E P G P K G G I G N R G P R G E T G D D G R D G V G S E G R R G K K G E R G F P G Y P G

2321 P K G N P G E P G L N G T T G P K G I R G R R G N S G P P G I V G Q K G D P G Y P G P A G P K G N R G D S I D Q C A L I Q S I K D K C P C C Y G P L E C P V F P

2401 T E L A F A L D T S E G V N Q D T F G R M R D V V L S I V N D L T I A E S N C P R G A R V A V V T Y N N E V T T E I R F A D S K R K S V L L D K I K N L Q V A L

2481 T S K Q Q S L E T A M S F V A R N T F K R V R N G F L M R K V A V F F S N T P T R A S P Q L R E A V L K L S D A G I T P L F L T R Q E D R Q L I N A L Q I N N T

2561 A V G H A L V L P A G R D L T D F L E N V L T C H V C L D I C N I D P S C G F G S W R P S F R D R R A A G S D V D I D M A F I L D S A E T T T L F Q F N E M K K

2641 Y I A Y L V R Q L D M S P D P K A S Q H F A R V A V V Q H A P S E S V D N A S M P P V K V E F S L T D Y G S K E K L V D F L S R G M T Q L Q G T R A L G S A I E

2721 Y T I E N V F E S A P N P R D L K I V V L M L T G E V P E Q Q L E E A Q R V I L Q A K C K G Y F F V V L G I G R K V N I K E V Y T F A S E P N D V F F K L V D K

2801 S T E L N E E P L M R F G R L L P S F V S S E N A F Y L S P D I R K Q C D W F Q G D Q P T K N L V K F G H K Q V N V P N N V T S S P T S N P V T T T K P V T T T

2881 K P V T T T T K P V T T T T K P V T I I N Q P S V K P A A A K P A P A K P V A A K P V A T K M A T V R P P V A V K P A T A A K P V A A K P A A V R P P A A A A

2961 K P V A T K P E V P R P Q A A K P A A T K P A T T K P M V K M S R E V Q V F E I T E N S A K L H W E R A E P P G P Y F Y D L T V T S A H D Q S L V L K Q N L T V

3041 T D R V I G G L L A G Q T Y H V A V V C Y L R S Q V R A T Y H G S F S T K K S Q P P P P Q P A R S A S S S T I N L M V S T E P L A L T E T D I C K L P K D E G T

3121 C R D F I L K W Y Y D P N T K S C A R F W Y G G C G G N E N K F G S Q K E C E K V C A P V L A K P G V I S V M G T

5.49 actin, gamma 1 propeptide [Homo sapiens]; beta actin [Homo sapiens]

Protein Accession gi|4501887 gi|4501885
Mean Expression Ratio 1.31
Median Expression Ratio 1.31
Credible Interval (1.05, 1.67)
Associated Peptides 10
Associated Spectra 13
Coverage NaN

| A | 2.5 | 50 | 97.5 | Sequence |
|---|---|---|---|---|
| 1 | 0.9 | 1.3 | 1.8 | ANTVLSGGTTMYPGIADR |
| 1 | 0.95 | 1.4 | 1.9 | DLYANTVLSGGTTMYPGIADR |
| 1 | 1 | 1.4 | 1.9 | KDLYANTVLSGGTTMYPGIADR |
| 2 | 0.97 | 1.3 | 1.8 | LCYVALDFEQEMATAASSSSLEK |
| 2 | 0.92 | 1.3 | 1.8 | TTGIVMDSGDGVTHTVPIYEGY |
| 1 | 0.95 | 1.3 | 1.9 | TTGIVMDSGDGVTHTVPIYEGYALPHAILR |
| 1 | 0.93 | 1.3 | 1.8 | TVLSGGTTMYPGIADR |
| 1 | 0.91 | 1.3 | 1.8 | VALDFEQEMATAAS SSSLEK |
| 1 | 0.98 | 1.4 | 2.0 | VAPEEHPVLLTEAPLNPK |
| 2 | 0.97 | 1.3 | 1.8 | YPIEHGIVTNWDDMEK |

5.50 solute carrier family 5 (sodium/glucose cotransporter), member 12 [Homo sapiens]

Protein Accession gi|157671931

Mean Expression Ratio 0.762

Median Expression Ratio 0.764

Credible Interval (0.515, 1.12)

Associated Peptides 2

Associated Spectra 3

Coverage 0.0388

| A | 2.5 | 50 | 97.5 | Sequence |
|---|---|---|---|---|
| 2 | 0.44 | 0.68 | 1 | LHIFDFDVDPLR |
| 1 | 0.52 | 0.8 | 1.2 | QGAESVLQNGLR |

1 M E V K N F A V W D Y V V F A A L F F I S S G I G V F F A I K E R K K A T S R E F
L V G G R Q M S F G P V G L S L T A S F M S A V T V L G T P S E V Y R F G A S

81 F L V F F I A Y L F V I L L T S E L F L P V F Y R S G I T S T Y E Y L Q L R F N K P
V R Y A A T V I Y I V Q T I L Y T G V V V Y A P A L A L N Q V T G F D L W G

161 S V F A T G I V C T F Y C T L G G L K A V V W T D A F Q M V V M I V G F L T V L I Q G S T H A G G F H N V L E Q S T N G S R L H I F D F D V D P L R R H T F W T

241 I T V G G T F T W L G I Y G V N Q S T I Q R C I S C K T E K H A K L A L Y F N L L G L W I I L V C A V F S G L I M Y S H F K D C D P W T S G I I S A P D Q L M P

321 Y F V M E I F A T M P G L P G L F V A C A F S G T L S T V A S S I N A L A T V T F E D F V K S C F P H L S D K L S T W I S K G L C L L F G V M C T S M A V A A S

401 V M G G V V Q A S L S I H G M C G G P M L G L F S L G I V F P F V N W K G A L G G L L T G I T L S F W V A I G A F I Y P A P A S K T W P L P L S T D Q C I K S N

481 V T A T G P P V L S S R P G I A D T W Y S I S Y L Y Y S A V G C L G C I V A G V I I S L I T G R Q R G E D I Q P L L I R P V C N L F C F W S K K Y K T L C W C G

561 V Q H D S G T E Q E N L E N G S A R K Q G A E S V L Q N G L R R E S L V H V P G Y D P K D K S Y N N M A F E T T H F

5.51 vacuolar protein sorting 25 [Homo sapiens]

Protein Accession gi|14150155
Mean Expression Ratio 1.31
Median Expression Ratio 1.30
Credible Interval (0.845, 2.04)
Associated Peptides 1
Associated Spectra 2
Coverage 0.0852

| A | 2.5 | 50 | 97.5 | Sequence |
|---|-----|-----|------|----------|
| 2 | 0.9 | 1.4 | 2.2 | KLPVESIQIVLEELR |

1 M A M S F E W P W Q Y R F P P F F T L Q P N V D T R Q K Q L A A W C S L V L S F C R L H K Q S S M T V M E A Q E S P L F N N V K L Q R K L P V E S I Q I V L E E

81 L R K K G N L E W L D K S K S S F L I M W R R P E E W G K L I Y Q W V S R S G Q N N S V F T L Y E L T N G E D T E D E E F H G L D E A T L L R A L Q A L Q Q E H

161 K A E I I T V S D G R G V K F F

5.52 annexin A11 [Homo sapiens]; annexin A11 [Homo sapiens]; annexin A11 [Homo sapiens]

Protein Accession gi|4557317 gi|22165433 gi|22165431
Mean Expression Ratio 1.3

Median Expression Ratio 1.30
Credible Interval (0.93, 1.82)
Associated Peptides 4
Associated Spectra 5
Coverage NaN

| A | 2.5 | 50 | 97.5 | Sequence |
|---|-----|-----|------|----------|
| 1 | 0.88 | 1.3 | 2.0 | AHLVAVFNEYQR |
| 1 | 0.9 | 1.3 | 2 | GFGTDEQAIIDCLGSR |
| 1 | 0.9 | 1.3 | 2 | GTITDAPGFDPLR |
| 2 | 0.9 | 1.3 | 1.9 | GVGTDEACLIEILASR |

5.53 chromatin modifying protein 4B [Homo sapiens]

Protein Accession gi|28827795
Mean Expression Ratio 1.3
Median Expression Ratio 1.30
Credible Interval (0.882, 1.89)
Associated Peptides 3
Associated Spectra 3
Coverage 0.147

| A | 2.5 | 50 | 97.5 | Sequence |
|---|-----|-----|------|----------|
| 1 | 0.87 | 1.3 | 2.1 | QLAQIDGTLSTIEFQR |
| 1 | 0.85 | 1.3 | 2.0 | KIEQEL T AAK |
| 1 | 0.87 | 1.3 | 2.1 | KQEFLEK |

1 M S V F G K L F G A G G G K A G K G G P T P Q E A I Q R L R D T E E M L S K K Q E F L E K K I E Q E L T A A K K H G T K N K R A A L Q A L K R K K R Y E K Q L A

81 Q I D G T L S T I E F Q R E A L E N A N T N T E V L K N M G Y A A K A M K A A H D N M D I D K V D E L M Q D I A D Q Q E L A E E I S T A I S K P V G F G E E F D

161 E D E L M A E L E E L E Q E E L D K N L L E I S G P E T V P L P N V P S I A L P S K P A K K K E E E D D D M K E L E N W A G S M

5.54 fatty acid binding protein 4, adipocyte [Homo sapiens]

Protein Accession gi|4557579
Mean Expression Ratio 0.769
Median Expression Ratio 0.771
Credible Interval (0.526, 1.12)
Associated Peptides 2
Associated Spectra 4

Coverage 0.242

| A | 2.5 | 50 | 97.5 | Sequence |
|---|-----|-----|------|----------|
| 3 | 0.49 | 0.73 | 1.1 | NTEISFILGQEFDEVTADDR |
| 1 | 0.49 | 0.76 | 1.2 | LVSSENFDDYMK |

1 MCDAFVGTWKLVSSENFDDYMKEVGVGFATRKVAGMAKP
NMIISVNGDVITIKSESTFKNTEISFILGQEFDEVTADDRK
81 VKSTITLDGGVLVHVQKWDGKSTTIKRKREDDKLVVECV
MKGVTSTRVYERA

5.55 chloride intracellular channel 6 [Homo sapiens]

Protein Accession gi|27894378
Mean Expression Ratio 1.29
Median Expression Ratio 1.29
Credible Interval (0.8, 2.07)
Associated Peptides 1
Associated Spectra 1
Coverage 0.0219

| A | 2.5 | 50 | 97.5 | Sequence |
|---|-----|-----|------|----------|
| 1 | 0.82 | 1.4 | 2.2 | VGDGPQQEPGEDEER |

1 MAEAAEPEGVAPGPQGPPEVPAPLAERPGEPGAAGGEAEG
PEGSEGAEEAPRGAAAVKEAGGGGPDRGPEAEARGTRGAH

81 GETEAEEGAPEGAEVPQGGEETSGAQQVEGASPGRGAQG
EPRGEAQREPEDSAAPERQEEAEQRPEVPEGSASGEAGDSV

161 DAEGPLGDNIEAEGPAGDSVEAEGRVGDSVDAEGPAGDS
VDAEGPLGDNIQAEGPAGDSVDAEGRVGDSVDAEGPAGDSV

241 DAEGRVGDSVEAGDPAGDGVEAGVPAGDSVEAEGPAGD
SMDAEGPAGRARRVSGEPQQSGDGSLSPQAEAIEVAAGESA
G

321 RSPGELAWDAAEEAEVPGVKGSEEAAPGDARADAGEDR
VGDGPQQEPGEDEERRERSPEGPREEEAAGGEEESPDSSPHG

401 EASRGAAEPEAQLSNHLAEEGPAEGSGEAARVNGRREDG
EASEPRALGQEHDITLFVKAGYDGESIGNCPFSQRLFMILW

481 L K G V I F N V T T V D L K R K P A D L Q N L A P G T N P P F M T F D G E V K T D V N K I E E F L E E K L A P P R Y P K L G T Q H P E S N S A G N D V F A K F S

561 A F I K N T K K D A N E I H E K N L L K A L R K L D N Y L N S P L P D E I D A Y S T E D V T V S G R K F L D G D E L T L A D C N L L P K L H I I K I V A K K Y R

641 D F E F P S E M T G I W R Y L N N A Y A R D E F T N T C P A D Q E I E H A Y S D V A K R M K

5.56 proteasome alpha 3 subunit isoform 1 [Homo sapiens]; proteasome alpha 3 subunit isoform 2 [Homo sapiens]

Protein Accession gi|4506183 gi|23110939
Mean Expression Ratio 1.29
Median Expression Ratio 1.29
Credible Interval (0.844, 1.97)
Associated Peptides 2
Associated Spectra 2
Coverage NaN

| A | 2.5 | 50 | 97.5 | Sequence |
|---|-----|-----|------|----------|
| 1 | 0.85 | 1.4 | 2.2 | HVGMAVAGLLADAR |
| 1 | 0.82 | 1.3 | 2.1 | AFELELSWVGELTNGR |

5.57 gelsolin isoform a precursor [Homo sapiens]; gelsolin isoform b [Homo sapiens]; gelsolin isoform c [Homo sapiens] ; gelsolin isoform c [Homo sapiens]; gelsolin isoform b [Homo sapiens]; gelsolin isoform b [Homo sapiens]; gelsolin isoform b [Homo sapiens]; gelsolin isoform b [Homo sapiens]

Protein Accession gi|4504165 gi|38044288 gi|189083782 gi|189083780 gi|189083778 gi|189083776 gi|189083774
Mean Expression Ratio 0.776
Median Expression Ratio 0.777
Credible Interval (0.57, 1.05)
Associated Peptides 5
Associated Spectra 6
Coverage NaN

| A | 2.5 | 50 | 97.5 | Sequence |
|---|-----|-----|------|----------|
| 2 | 0.56 | 0.8 | 1.2 | AAQHGMDDDGTGQK |
| 1 | 0.53 | 0.77 | 1.1 | AQPVQVAEGSEPDGFWEALGGK |
| 1 | 0.53 | 0.78 | 1.1 | DSQEEEKTEALTSAK |
| 1 | 0.49 | 0.73 | 1.1 | NWQGAQSTQDEVAASAILTAQLDEELGGTPVQSR |
| 1 | 0.52 | 0.77 | 1.1 | TAQLDEELGGTPVQSR |

5.58 vacuolar protein sorting 37B [Homo sapiens]

Protein Accession gi|13375926
Mean Expression Ratio 1.29
Median Expression Ratio 1.28
Credible Interval (0.848, 1.94)
Associated Peptides 1
Associated Spectra 3
Coverage 0.0912

| A | 2.5 | 50 | 97.5 | Sequence |
|---|---|---|---|---|
| 3 0. | 93 | 1.4 | 2.0 | LPELAPTAPLPYPAPEASGPPAVAPR |

1 M A G A G S E A R F A G L S L V Q L N E L L E D E G Q L T E M V Q K M E E T Q
N V Q L N K E M T L A S N R S L A E G N L L Y Q P Q L D T L K A R L T Q K Y Q E L

81 Q V L F E A Y Q I K K T K L D R Q S S S A S L E T L L A L L Q A E G A K I E E D T
E N M A E K F L D G E L P L D S F I D V Y Q S K R K L A H M R R V K I E K L Q

161 E M V L K G Q R L P Q A L A P L P P R L P E L A P T A P L P Y P A P E A S G P P
A V A P R R I P P P P P P V P A G R L A T P F T A A M S S G Q A V P Y P G L Q C

241 P P L P P R V G L P T Q Q G F S S Q F V S P Y P P P L P Q R P P P R L P P H Q P G
F I L Q

5.59 programmed cell death 6 interacting protein [Homo sapiens]

Protein Accession gi|22027538
Mean Expression Ratio 1.28
Median Expression Ratio 1.28
Credible Interval (1.05, 1.57)
Associated Peptides 12
Associated Spectra 20
Coverage 0.192

| A | 2.5 | 50 | 97.5 | Sequence |
|---|---|---|---|---|
| 2 | 1 | 1.3 | 1.8 | EPSAPSIPTPAYQSSPAGGHAPTPPTPAPR |
| 1 | 0.9 | 1.3 | 1.7 | FIQQTYPSGGEEQAQYCR |
| 3 | 1 | 1.3 | 1.8 | FLTALAQDGVINEEALSVTELDR |
| 2 | 0.97 | 1.3 | 1.8 | FYNELTEILVR |
| 1 | 0.92 | 1.3 | 1.7 | HEGALETLLR |
| 2 | 0.93 | 1.3 | 1.7 | LQHAAELIK |
| 1 | 0.93 | 1.3 | 1.8 | NIQVSHQEFSK |

(continued)

| A | 2.5 | 50 | 97.5 | Sequence |
|---|-----|-----|------|----------|
| 3 | 0.98 | 1.3 | 1.7 | NLATAYDNFVELVANLK |
| 2 | 0.97 | 1.3 | 1.8 | SVIEQGGIQTVDQLIK |
| 1 | 0.88 | 1.2 | 1.7 | FTDLFEK |
| 1 | 0.95 | 1.3 | 1.8 | KQEGLLK |
| 1 | 0.91 | 1.3 | 1.7 | YDEYVNVK |

MATFISVQLKKTSEVDLAKPLVKFIQQTYPSGGEEQAQYCRAAEELSKLRRAAVGRPLDKHEGALETLLRYYDQICSIEP

KFPFSENQICLTFTWKDAFDKGSLFGGSVKLALASLGYEKSCVLFNCAALASQIAAEQNLDNDEGLKIAAKHYQFASGAF

LHIKETVLSALSREPTVDISPDTVGTLSLIMLAQAQEVFFLKATRDKMKDAIIAKLANQAADYFGDAFKQCQYKDTLPKE

VFPVLAAKHCIMQANAEYHQSILAKQQKKFGEEIARLQHAAELIKTVASRYDEYVNVKDFSDKINRALAAAKKDNDFIYH

DRVPDLKDLDPIGKATLVKSTPVNVPISQKFTDLFEKMVPVSVQQSLAAYNQRKADLVNRSIAQMREATTLANGVLASLN

LPAAIEDVSGDTVPQSILTKSRSVIEQGGIQTVDQLIKELPELLQRNREILDESLRLLDEEEATDNDLRAKFKERWQRTP

SNELYKPLRAEGTNFRTVLDKAVQADGQVKECYQSHRDTIVLLCKPEPELNAAIPSANPAKTMQGSEVVNVLKSLLSNLD

EVKKEREGLENDLKSVNFDMTSKFLTALAQDGVINEEALSVTELDRVYGGLTTKVQESLKKQEGLLKNIQVSHQEFSKMK

QSNNEANLREEVLKNLATAYDNFVELVANLKEGTKFYNELTEILVRFQNKCSDIVFARKTERDELLKDLQQSIAREPSAP

SIPTPAYQSSPAGGHAPTPPTPAPRTMPPTKPQPPARPPPPVLPANRAPSATAPSPVGAGTAAPAPSQTPGSAPPPQAQG

PPYPTYPGYPGYCQMPMPMGYNPYAYGQYNMPYPPVYHQSPGQAPYPGPQQPSYPFPQPPQQSYYPQQ

5.60 prosaposin isoform a preproprotein [Homo sapiens]

Protein Accession gi|11386147
Mean Expression Ratio 0.78
Median Expression Ratio 0.78
Credible Interval (0.587, 1.04)
Associated Peptides 5
Associated Spectra 8
Coverage 0.0954

| A | 2.5 | 50 | 97.5 | Sequence |
|---|-----|-----|------|----------|
| 2 | 0.51 | 0.72 | 1.0 | EIVDSYLPVILDIIK |
| 1 | 0.55 | 0.8 | 1.2 | GCSFLPDPYQK |
| 1 | 0.55 | 0.79 | 1.1 | LVGYLDR |
| 3 | 0.55 | 0.77 | 1.1 | QEILAALEK |
| 1 | 0.54 | 0.78 | 1.1 | SLPCDICK |

1 MYALFLLASLLGAALAGPVLGLKECTRGSAVWCQNVKTAS
DCGAVKHCLQTVWNKPTVKSLPCDICKDVVTAAGDMLKDN

81 ATEEEILVYLEKTCDWLPKPNMSASCKEIVDSYLPVILDII
KGEMSRPGEVCSALNLCESLQKHLAELNHQKQLESNKIP

161 ELDMTEVVAPFMANIPLLLYPQDGPRSKPQPKDNGDVCQ
DCIQMVTDIQTAVRTNSTFVQALVEHVKEECDRLGPGMADI

241 CKNYISQYSEIAIQMMMHMQPKEICALVGFCDEVKEMPM
QTLVPAKVASKNVIPALELVEPIKKHEVPAKSDVYCEVCEF

321 LVKEVTKLIDNNKTEKEILDAFDKMCSKLPKSLSEECQEV
VDTYGSSILSILLEEVSPELVCSMLHLCSGTRLPALTVHV

401 TQPKDGGFCEVCKKLVGYLDRNLEKNSTKQEILAALEKG
CSFLPDPYQKQCDQFVAEYEPVLIEILVEVMDPSFVCLKIG

481 ACPSAHKPLLGTEKCIWGPSYWCQNTETAAQCNAVEHCK
RHVWN

5.61 ferritin, light polypeptide [Homo sapiens]

Protein Accession gi|20149498
Mean Expression Ratio 1.28
Median Expression Ratio 1.28

Credible Interval (1.11, 1.47)
Associated Peptides 16
Associated Spectra 66
Coverage 0.634

| A | 2.5 | 50 | 97.5 | Sequence |
|---|-----|-----|------|----------|
| 1 | 0.97 | 1.3 | 1.7 | AAMALEK |
| 5 | 1.0 | 1.3 | 1.7 | ALFQDIK |
| 3 | 0.98 | 1.3 | 1.7 | DDVALEGVSHFFR |
| 3 | 0.93 | 1.2 | 1.6 | DLHALGSAR |
| 1 | 0.96 | 1.3 | 1.7 | FDRDDVALEGVSHFFR |
| 1 | 0.98 | 1.3 | 1.8 | KLNQALLDLHALGSAR |
| 3 | 1.0 | 1.3 | 1.7 | KPAEDEWGK |
| 4 | 0.92 | 1.2 | 1.5 | LCDFLETHFLDEEVK |
| 12 | 0.93 | 1.1 | 1.4 | LGGPEAGLGEYLFER |
| 1 | 1.0 | 1.4 | 1.9 | LGGPEAGLGEYLFERLTLK |
| 2 | 1.1 | 1.5 | 2.0 | LGGPEAGLGEYLFERLTLKHD |
| 12 | 1 | 1.2 | 1.5 | LNQALLDLHALGSAR |
| 1 | 0.96 | 1.3 | 1.7 | MGDHLTNLHR |
| 1 | 0.85 | 1.1 | 1.5 | QALLDLHALGSAR |
| 15 | 1.1 | 1.4 | 1.7 | TDPHLCDFLETHFLDEEVK |
| 1 | 0.96 | 1.3 | 1.7 | ELAEEK |

1 MSSQIRQNYSTDVEAAVNSLVNLYLQASYTYLSLGFYFDR
DDVALEGVSHFFRELAEEKREGYERLLKMQNQRGGRALFQ
81 DIKKPAEDEWGKTPDAMKAAMALEKKLNQALLDLHALGS
ARTDPHLCDFLETHFLDEEVKLIKKMGDHLTNLHRLGGPEA
161 GLGEYLFERLTLKHD

5.62 galectin 3 binding protein [Homo sapiens]

Protein Accession gi|5031863
Mean Expression Ratio 1.28
Median Expression Ratio 1.28
Credible Interval (1.11, 1.48)
Associated Peptides 19
Associated Spectra 57
Coverage 0.369

| A | 2.5 | 50 | 97.5 | Sequence |
|---|-----|-----|------|----------|
| 3 | 1.0 | 1.3 | 1.8 | AAFGQGSGPIMLDEVQCTGTEASLADCK |
| 2 | 0.93 | 1.2 | 1.6 | ALMLCEGLFVADVTDFEGWK |

(continued)

| A | 2.5 | 50 | 97.5 | Sequence |
|---|---|---|---|---|
| 3 | 1.0 | 1.3 | 1.7 | ASHEEVEGLVEK |
| 15 | 1.0 | 1.2 | 1.5 | ELSEALGQIFDSQR |
| 8 | 0.96 | 1.2 | 1.5 | GQWGTVCDNLWDLTDASVVCR |
| 1 | 0.95 | 1.3 | 1.7 | IDITLSSVK |
| 1 | 0.92 | 1.2 | 1.7 | IYTSPTWSAFVTDSSWSAR |
| 1 | 0.95 | 1.3 | 1.7 | QSFQTPQHPSFL |
| 3 | 1.0 | 1.3 | 1.8 | QSFQTPQHPSFLFQDK |
| 3 | 1 | 1.3 | 1.7 | QTPQHPSFLFQDK |
| 1 | 0.94 | 1.3 | 1.7 | RIDITLSSVK |
| 2 | 0.96 | 1.3 | 1.7 | SDLAVPSELALLK |
| 6 | 1.2 | 1.5 | 1.9 | STSSFPCPAGHFNGFR |
| 1 | 0.97 | 1.3 | 1.8 | TIAYENK |
| 1 | 0.97 | 1.3 | 1.7 | TLQALEFHTVPF |
| 1 | 0.92 | 1.3 | 1.7 | VADVTDFEGWK |
| 2 | 0.92 | 1.2 | 1.6 | WSHEALFQK |
| 2 | 0.9 | 1.2 | 1.6 | YSSDYFQAPSDYR |
| 1 | 0.96 | 1.3 | 1.8 | VEIFYR |

1 M T P P R L F W V W L L V A G T Q G V N D G D M R L A D G G A T N Q G R V E I F Y R G Q W G T V C D N L W D L T D A S V V C R A L G F E N A T Q A L G R A A F G

81 Q G S G P I M L D E V Q C T G T E A S L A D C K S L G W L K S N C R H E R D A G V V C T N E T R S T H T L D L S R E L S E A L G Q I F D S Q R G C D L S I S V N

161 V Q G E D A L G F C G H T V I L T A N L E A Q A L W K E P G S N V T M S V D A E C V P M V R D L L R Y F Y S R R I D I T L S S V K C F H K L A S A Y G A R Q L Q

241 G Y C A S L F A I L L P Q D P S F Q M P L D L Y A Y A V A T G D A L L E K L C L Q F L A W N F E A L T Q A E A W P S V P T D L L Q L L L P R S D L A V P S E L A

321 L L K A V D T W S W G E R A S H E E V E G L V E K I R F P M M L P E E L F E L Q F N L S L Y W S H E A L F Q K K T L Q A L E F H T V P F Q L L A R Y K G L N L T

401 E D T Y K P R I Y T S P T W S A F V T D S S W S A R K S Q L V Y Q S R R G P L V K Y S S D Y F Q A P S D Y R Y Y P Y Q S F Q T P Q H P S F L F Q D K R V S W S L

481 V Y L P T I Q S C W N Y G F S C S S D E L P V L G L T K S G G S D R T I A Y E N K A L M L C E G L F V A D V T D F E G W K A A I P S A L D T N S S K S T S S F P

561 CPAGHFNGFRTVIRPFYLTNSSGVD

5.63 peptidoglycan recognition protein 1 [Homo sapiens]

Protein Accession gi|4827036
Mean Expression Ratio 0.784
Median Expression Ratio 0.786
Credible Interval (0.55, 1.12)
Associated Peptides 3
Associated Spectra 4
Coverage 0.276

| A | 2.5 | 50 | 97.5 | Sequence |
|---|-----|-----|------|----------|
| 1 | 0.51 | 0.77 | 1.2 | AAQGLLACGVAQGALR |
| 2 | 0.52 | 0.76 | 1.1 | ALASECAQHLSLPLR |
| 1 | 0.51 | 0.77 | 1.2 | TLGWCDVGYNFLIGEDGLVYEGR |

1 M S R R S M L L A W A L P S L L R L G A A Q E T E D P A C C S P I V P R N E W K
A L A S E C A Q H L S L P L R Y V V V S H T A G S S C N T P A S C Q Q Q A R N V

81 Q H Y H M K T L G W C D V G Y N F L I G E D G L V Y E G R G W N F T G A H S
G H L W N P M S I G I S F M G N Y M D R V P T P Q A I R A A Q G L L A C G V A Q G
A

161 LRSNYVLKGHRDVQRTLSPGNQLYHLIQNWPHYRSP

5.64 ubiquitin and ribosomal protein S27a precursor [Homo sapiens]; ubiquitin and ribosomal protein S27a precursor [Homo sapiens]

Protein Accession gi|4506713 gi|208022622
Mean Expression Ratio 1.27
Median Expression Ratio 1.27
Credible Interval (0.99, 1.64)
Associated Peptides 5
Associated Spectra 12
Coverage NaN

| A | 2.5 | 50 | 97.5 | Sequence |
|---|-----|-----|------|----------|
| 3 | 0.95 | 1.3 | 1.8 | ESTLHLVLR |
| 4 | 1.0 | 1.4 | 1.8 | TITLEVEPSDTIENVK |
| 1 | 0.9 | 1.3 | 1.9 | TLSDYNIQK |
| 1 | 0.9 | 1.3 | 1.8 | LIFAGK |
| 3 | 0.89 | 1.2 | 1.7 | MQIFVK |

5.65 complement component 4A preproprotein [Homo sapiens]

Protein Accession gi|67190748
Mean Expression Ratio 0.787
Median Expression Ratio 0.786
Credible Interval (0.511, 1.21)
Associated Peptides 1
Associated Spectra 2
Coverage 0.0115

| A | 2.5 | 50 | 97.5 | Sequence |
|---|-----|----|------|----------|
| 2 | 0.48 | 0.74 | 1.2 | LLSQQQADGSFQDPCPVLDR |

1 M R L L W G L I W A S S F F T L S L Q K P R L L L F S P S V V H L G V P L S V G V
Q L Q D V P R G Q V V K G S V F L R N P S R N N V P C S P K V D F T L S S E R

81 D F A L L S L Q V P L K D A K S C G L H Q L L R G P E V Q L V A H S P W L K D S
L S R T T N I Q G I N L L F S S R R G H L F L Q T D Q P I Y N P G Q R V R Y R V

161 F A L D Q K M R P S T D T I T V M V E N S H G L R V R K K E V Y M P S S I F Q
D D F V I P D I S E P G T W K I S A R F S D G L E S N S S T Q F E V K K Y V L P N

241 F E V K I T P G K P Y I L T V P G H L D E M Q L D I Q A R Y I Y G K P V Q G V A
Y V R F G L L D E D G K K T F F R G L E S Q T K L V N G Q S H I S L S K A E F Q

321 D A L E K L N M G I T D L Q G L R L Y V A A A I I E S P G G E M E E A E L T S
W Y F V S S P F S L D L S K T K R H L V P G A P F L L Q A L V R E M S G S P A S G

401 I P V K V S A T V S S P G S V P E V Q D I Q Q N T D G S G Q V S I P I I I P Q T I S
E L Q L S V S A G S P H P A I A R L T V A A P P S G G P G F L S I E R P D S

481 R P P R V G D T L N L N L R A V G S G A T F S H Y Y Y M I L S R G Q I V F M N
R E P K R T L T S V S V F V D H H L A P S F Y F V A F Y Y H G D H P V A N S L R V

561 D V Q A G A C E G K L E L S V D G A K Q Y R N G E S V K L H L E T D S L A L V
A L G A L D T A L Y A A G S K S H K P L N M G K V F E A M N S Y D L G C G P G G
G

641 D S A L Q V F Q A A G L A F S D G D Q W T L S R K R L S C P K E K T T R K K R
N V N F Q K A I N E K L G Q Y A S P T A K R C C Q D G V T R L P M M R S C E Q R
A

721 A R V Q Q P D C R E P F L S C C Q F A E S L R K K S R D K G Q A G L Q R A L E I L Q E E D L I D E D D I P V R S F F P E N W L W R V E T V D R F Q I L T L W L P

801 D S L T T W E I H G L S L S K T K G L C V A T P V Q L R V F R E F H L H L R L P M S V R R F E Q L E L R P V L Y N Y L D K N L T V S V H V S P V E G L C L A G G

881 G G L A Q Q V L V P A G S A R P V A F S V V P T A A A A V S L K V V A R G S F E F P V G D A V S K V L Q I E K E G A I H R E E L V Y E L N P L D H R G R T L E I

961 P G N S D P N M I P D G D F N S Y V R V T A S D P L D T L G S E G A L S P G G V A S L L R L P R G C G E Q T M I Y L A P T L A A S R Y L D K T E Q W S T L P P E

1041 T K D H A V D L I Q K G Y M R I Q Q F R K A D G S Y A A W L S R D S S T W L T A F V L K V L S L A Q E Q V G G S P E K L Q E T S N W L L S Q Q Q A D G S F Q D P

1121 C P V L D R S M Q G G L V G N D E T V A L T A F V T I A L H H G L A V F Q D E G A E P L K Q R V E A S I S K A N S F L G E K A S A G L L G A H A A A I T A Y A L

1201 T L T K A P V D L L G V A H N N L M A M A Q E T G D N L Y W G S V T G S Q S N A V S P T P A P R N P S D P M P Q A P A L W I E T T A Y A L L H L L L H E G K A E

1281 M A D Q A S A W L T R Q G S F Q G G F R S T Q D T V I A L D A L S A Y W I A S H T T E E R G L N V T L S S T G R N G F K S H A L Q L N N R Q I R G L E E E L Q F

1361 S L G S K I N V K V G G N S K G T L K V L R T Y N V L D M K N T T C Q D L Q I E V T V K G H V E Y T M E A N E D Y E D Y E Y D E L P A K D D P D A P L Q P V T P

1441 L Q L F E G R R N R R R R E A P K V V E E Q E S R V H Y T V C I W R N G K V G L S G M A I A D V T L L S G F H A L R A D L E K L T S L S D R Y V S H F E T E G P

1521 H V L L Y F D S V P T S R E C V G F E A V Q E V P V G L V Q P A S A T L Y D Y Y N P E R R C S V F Y G A P S K S R L L A T L C S A E V C Q C A E G K C P R Q R R

1601 A L E R G L Q D E D G Y R M K F A C Y Y P R V E Y G F Q V K V L R E D S R A A F R L F E T K I T Q V L H F T K D V K A A A N Q M R N F L V R A S C R L R L E P G

1681 K E Y L I M G L D G A T Y D L E G H P Q Y L L D S N S W I E E M P S E R L C R S T R Q R A A C A Q L N D F L Q E Y G T Q G C Q V

5.66 hypothetical protein LOC169693 [Homo sapiens]

Protein Accession gi|23397518
Mean Expression Ratio 0.788
Median Expression Ratio 0.788
Credible Interval (0.572, 1.07)
Associated Peptides 3
Associated Spectra 8
Coverage 0.282

| A | 2.5 | 50 | 97.5 | Sequence |
|---|-----|-----|------|----------|
| 6 | 0.56 | 0.75 | 1 | ESIAGLVVTAISEDAQR |
| 1 | 0.52 | 0.77 | 1.1 | QHLAHGALPVATVDRPDFYPPAYEESLEVEK |
| 1 | 0.53 | 0.8 | 1.2 | QHLAHGALPVATVDRPDF |

1 MQNRTGLILCALALLMGFLMVCLGAFFISWGSIFDCQGSLI
AAYLLLPLGFVILLSGIFWSNYRQVTESKGVLRHMLRQH

81 LAHGALPVATVDRPDFYPPAYEESLEVEKQSCPAEREASG
IPPPLYTETGLEFQDGNDSHPEAPPSYRESIAGLVVTAIS

161 EDAQRRGQEC

5.67 nidogen 1 precursor [Homo sapiens]

Protein Accession gi|115298674
Mean Expression Ratio 0.79
Median Expression Ratio 0.788
Credible Interval (0.513, 1.22)
Associated Peptides 1
Associated Spectra 2
Coverage 0.0217

| A | 2.5 | 50 | 97.5 | Sequence |
|---|-----|-----|------|----------|
| 2 | 0.48 | 0.75 | 1.2 | GPGQGDLELEDGDDFVSPALELSGALR |

1 MLASSSRIRAAWTRALLLPLLLAGPVGCLSRQELFPFGPGQ
GDLELEDGDDFVSPALELSGALRFYDRSDIDAVYVTTNG

81 IIATSEPPAKESHPGLFPPTFGAVAPFLADLDTTDGLGKVY
YREDLSPSITQRAAECVHRGFPEISFQPSSAVVVTWESV

161 A P Y Q G P S R D P D Q K G K R N T F Q A V L A S S D S S S Y A I F L Y P E D G
L Q F H T T F S K K E N N Q V P A V V A F S Q G S V G F L W K S N G A Y N I F A

241 N D R E S V E N L A K S S N S G Q Q G V W V F E I G S P A T T N G V V P A D V
I L G T E D G A E Y D D E D E D Y D L A T T R L G L E D V G T T P F S Y K A L R R

321 G G A D T Y S V P S V L S P R R A A T E R P L G P P T E R T R S F Q L A V E T F
H Q Q H P Q V I D V D E V E E T G V V F S Y N T D S R Q T C A N N R H Q C S V H

401 A E C R D Y A T G F C C S C V A G Y T G N G R Q C V A E G S P Q R V N G K V
K G R I F V G S S Q V P I V F E N T D L H S Y V V M N H G R S Y T A I S T I P E T V

481 G Y S L L P L A P V G G I I G W M F A V E Q D G F K N G F S I T G G E F T R Q A
E V T F V G H P G N L V I K Q R F S G I D E H G H L T I D T E L E G R V P Q I P

561 F G S S V H I E P Y T E L Y H Y S T S V I T S S S T R E Y T V T E P E R D G A S P
S R I Y T Y Q W R Q T I T F Q E C V H D D S R P A L P S T Q Q L S V D S V F V

641 L Y N Q E E K I L R Y A L S N S I G P V R E G S P D A L Q N P C Y I G T H G C D
T N A A C R P G P R T Q F T C E C S I G F R G D G R T C Y D I D E C S E Q P S V

721 C G S H T I C N N H P G T F R C E C V E G Y Q F S D E G T C V A V V D Q R P I N
Y C E T G L H N C D I P Q R A Q C I Y T G G S S Y T C S C L P G F S G D G Q A C

801 Q D V D E C Q P S R C H P D A F C Y N T P G S F T C Q C K P G Y Q G D G F R C
V P G E V E K T R C Q H E R E H I L G A A G A T D P Q R P I P P G L F V P E C D A

881 H G H Y A P T Q C H G S T G Y C W C V D R D G R E V E G T R T R P G M T P P
C L S T V A P P I H Q G P A V P T A V I P L P P G T H L L F A Q T G K I E R L P L E

961 G N T M R K T E A K A F L H V P A K V I I G L A F D C V D K M V Y W T D I T E
P S I G R A S L H G G E P T T I I R Q D L G S P E G I A V D H L G R N I F W T D S

1041 N L D R I E V A K L D G T Q R R V L F E T D L V N P R G I V T D S V R G N L Y
W T D W N R D N P K I E T S Y M D G T N R R I L V Q D D L G L P N G L T F D A F S

1121 S Q L C W V D A G T N R A E C L N P S Q P S R R K A L E G L Q Y P F A V T S
Y G K N L Y F T D W K M N S V V A L D L A I S K E T D A F Q P H K Q T R L Y G I T
T

1201 A L S Q C P Q G H N Y C S V N N G G C T H L C L A T P G S R T C R C P D N T L
G V D C I E Q K

5.68 CD9 antigen [Homo sapiens]

Protein Accession gi|4502693
Mean Expression Ratio 1.27
Median Expression Ratio 1.27
Credible Interval (0.856, 1.88)
Associated Peptides 2
Associated Spectra 3
Coverage 0.0921

| A | 2.5 | 50 | 97.5 | Sequence |
|---|-----|-----|------|----------|
| 2 | 0.91 | 1.4 | 2.1 | EVQEFYK |
| 1 | 0.8 | 1.2 | 2.0 | AGGVEQFISDICPK |

1 MPVKGGTKCIKYLLFGFNFIFWLAGIAVLAIGLWLRFDSQT
KSIFEQETNNNNSSFYTGVYILIGAGALMMLVGFLGCCG

81 AVQESQCMLGLFFGFLLVIFAIEIAAAIWGYSHKDEVIKEV
QEFYKDTYNLKTKDEPQRETLKAIHYALNCCGLAGGVE

161 QFISDICPKKDVLETFTVKSCPDAIKEVFDNKFHIIGAVGI
GIAVVMIFGMIFSMILCCAIRRNREMV

5.69 solute carrier family 5 (sodium/glucose cotransporter), member 10 isoform 2 [Homo sapiens]

Protein Accession gi|109659836
Mean Expression Ratio 0.79
Median Expression Ratio 0.79
Credible Interval (0.519, 1.22)
Associated Peptides 2
Associated Spectra 2
Coverage 0.047

| A | 2.5 | 50 | 97.5 | Sequence |
|---|-----|-----|------|----------|
| 1 | 0.47 | 0.76 | 1.2 | ALFPDDVGCVVPSECLR |
| 1 | 0.48 | 0.78 | 1.2 | LVMELMPIGLR |

1 MAANSTSDLHTPGTQLSVADIIVITVYFALNVAVGIWSSCR
ASRNTVNGYFLAGRDMTWWPIGASLFASSEGSGLFIGLA

81 GSGAAGGLAVAGFEWNATYVLLALAWVFVPIYISSEIVTL
PEYIQKRYGGQRIRMYLSVLSLLLSVFTKISLDLYAGALF

161 VHICLGWNFYLSTILTLGITALYTIAGGLAAVIYTDALQT
LIMVVGAVILTIKAFDQIGGYGQLEAAYAQAIPSRTIANT

241 TCHLPRTDAMHMFRDPHTGDLPWTGMTFGLTIMATWYW
CTDQVIVQRSLSARDLNHAKAGSILASYLKMLPMGLIIMPG
M

321 ISRALFPDDVGCVVPSECLRACGAEVGCSNIAYPKLVMEL
MPIGLRGLMIAVMLAALMSSLTSIFNSSSTLFTMDIWRRL

401 RPRSGERELLLVGRLVIVALIGVSVAWIPVLQDSNSGQLF
IYMQSVTSSLAPPVTAVFVLGVFWRRANEQGAFWGLIAGL

481 VVGATRLVLEFLNPAPPCGEPDTRPAVLGSIHYLHFAVAL
FALSGAVVVAGSLLTPPPQSVQIENLTWWTLAQDVPLGTK

561 AGDGQTPQKHAFWARVCGFNAILLMCVNIFFYAYFA

5.70 sorting nexin 18 isoform a [Homo sapiens]; sorting nexin 18 isoform b [Homo sapiens]

Protein Accession gi|157057545 gi|157057543
Mean Expression Ratio 1.26
Median Expression Ratio 1.26
Credible Interval (0.82, 1.91)
Associated Peptides 2
Associated Spectra 2
Coverage NaN

| A | 2.5 | 50 | 97.5 | Sequence |
|---|-----|----|------|----------|
| 1 | 0.86 | 1.4 | 2.2 | APEPGPAGDGGPGAPAR |
| 1 | 0.78 | 1.2 | 2.0 | LVPTHTQVPVHR |

5.71 syntenin isoform 1 [Homo sapiens]; syntenin isoform 1 [Homo sapiens]

Protein Accession gi|56243522 gi|55749490
Mean Expression Ratio 1.26
Median Expression Ratio 1.26
Credible Interval (0.87, 1.83)
Associated Peptides 2
Associated Spectra 4
Coverage NaN

| A | 2.5 | 50 | 97.5 | Sequence |
|---|-----|----|------|----------|
| 3 | 0.94 | 1.4 | 2.0 | ANVAVVSGAPLQGQLVAR |

(continued)

| A | 2.5 | 50 | 97.5 | Sequence |
|---|-----|-----|------|----------|
| 1 | 0.82 | 1.2 | 1.9 | VAVVSGAPLQGQLVAR |

5.72 glutamyl aminopeptidase (aminopeptidase A) [Homo sapiens]

Protein Accession gi|132814467
Mean Expression Ratio 0.795
Median Expression Ratio 0.794
Credible Interval (0.682, 0.932)
Associated Peptides 25
Associated Spectra 39
Coverage 0.268

| A | 2.5 | 50 | 97.5 | Sequence |
|---|-----|-----|------|----------|
| 1 | 0.6 | 0.8 | 1.1 | AQLLDYK |
| 1 | 0.58 | 0.78 | 1.1 | ASLIDDAF ALAR |
| 2 | 0.62 | 0.81 | 1.1 | AVHQFDSVK |
| 1 | 0.63 | 0.84 | 1.1 | EESVDDKWTR |
| 1 | 0.6 | 0.8 | 1.1 | ETNLLYDPK |
| 1 | 0.61 | 0.83 | 1.1 | EYGALSNMPVAK |
| 4 | 0.65 | 0.84 | 1.1 | IAIPDFGTGAMENWGLITYR |
| 6 | 0.6 | 0.76 | 0.98 | INPDHIGFYR |
| 1 | 0.59 | 0.8 | 1.1 | KSFPCFDEPNKK |
| 1 | 0.59 | 0.8 | 1.1 | LLYGLASVK |
| 1 | 0.58 | 0.79 | 1.1 | LPDFVNPVHY |
| 1 | 0.57 | 0.77 | 1.0 | NMAWNWIQLNWDYLVNR |
| 1 | 0.58 | 0.79 | 1.1 | NNIEWLK |
| 1 | 0.61 | 0.83 | 1.1 | QMGYPVLNVNGVK |
| 1 | 0.57 | 0.77 | 1.1 | SFPCFDEPNKK |
| 1 | 0.56 | 0.75 | 1.0 | SIVATDHEPTDAR |
| 1 | 0.57 | 0.78 | 1.0 | SSHPIIVTVTTPDEITSVFDGISY |
| 3 | 0.57 | 0.75 | 0.99 | TSDFWAALEEASR |
| 1 | 0.61 | 0.82 | 1.1 | TSDFWAALEEASRLPVK |
| 2 | 0.62 | 0.82 | 1.1 | YLWLHLR |
| 3 | 0.58 | 0.77 | 1.0 | YPQAGAGEKPR |
| 1 | 0.58 | 0.8 | 1.1 | MLEDWIKPENFQK |
| 1 | 0.58 | 0.78 | 1.1 | IQLNWDYLVNR |
| 1 | 0.57 | 0.77 | 1.0 | YLDLLK |
| 1 | 0.58 | 0.78 | 1.1 | QMESFFAK |

MNFAEREGSKRYCIQTKHVAILCAVVVGVGLIVGLAVGLT
RSCDSSGDGGPGTAPAPSHLPSSTASPSGPPAQDQDICPA

SEDESGQWKNFRLPDFVNPVHYDLHVKPLLEEDTYTGTVS
ISINLSAPTRYLWLHLRETRITRLPELKRPSGDQVQVRRC

FEYKKQEYVVVEAEEELTPSSGDGLYLLTMEFAGWLNGS
LVGFYRTTYTENGQVKSIVATDHEPTDARKSFPCFDEPNKK

ATYTISITHPKEYGALSNMPVAKEESVDDKWTRTTFEKSV
PMSTYLVCFAVHQFDSVKRISNSGKPLTIYVQPEQKHTAE

YAANITKSVFDYFEEYFAMNYSLPKLDKIAIPDFGTGAME
NWGLITYRETNLLYDPKESASSNQQRVATVVAHELVHQWF

GNIVTMDWWEDLWLNEGFASFFEFLGVNHAETDWQMRD
QMLLEDVLPVQEDDSLMSSHPIIVTVTTPDEITSVFDGISYS

KGSSILRMLEDWIKPENFQKGCQMYLEKYQFKNAKTSDF
WAALEEASRLPVKEVMDTWTRQMGYPVLNVNGVKNITQKR
F

LLDPRANPSQPPSDLGYTWNIPVKWTEDNITSSVLFNRSE
KEGITLNSSNPSGNAFLKINPDHIGFYRVNYEVATWDSIA

TALSLNHKTFSSADRASLIDDAFALARAQLLDYKVALNL
TKYLKREENFLPWQRVISAVTYIISMFEDDKELYPMIEEYF

QGQVKPIADSLGWNDAGDHVTKLLRSSVLGFACKMGDR
EALNNASSLFEQWLNGTVSLPVNLRLLVYRYGMQNSGNEIS
W

NYTLEQYQKTSLAQEKEKLLYGLASVKNVTLLSRYLDLL
KDTNLIKTQDVFTVIRYISYNSYGKNMAWNWIQLNWDYLV
N

RYTLNNRNLGRIVTIAEPFNTELQLWQMESFFAKYPQAG
AGEKPREQVLETVKNNIEWLKQHRNTIREWFFNLLESG

5.73 histone cluster 2, H4b [Homo sapiens]; histone cluster 2, H4a [Homo sapiens]; histone cluster 1, H4i [Homo sapiens]; histone cluster 1, H41 [Homo sapiens]; histone cluster 1, H4e [Homo sapiens]; histone cluster 1, H4b [Homo sapiens]; histone cluster 1, H4h [Homo sapiens]; histone cluster 1, H4c [Homo sapiens]; histone cluster 1, H4k [Homo sapiens]; histone cluster 1, H4f [Homo sapiens]; histone cluster 1, H4d [Homo sapiens]; histone cluster 1, H4a [Homo sapiens]; histone cluster 4, H4 [Homo sapiens]; histone cluster 1, H4j [Homo sapiens]

Protein Accession gi|77539758 gi|4504323 gi|4504321 gi|4504317 gi|4504315 gi|4504313 gi|4504311 gi|4504309
Mean Expression Ratio 0.791
Median Expression Ratio 0.794
Credible Interval (0.489, 1.26)
Associated Peptides 1
Associated Spectra 1
Coverage NaN

| A | 2.5 | 50 | 97.5 | Sequence |
|---|-----|----|------|----------|
| 1 | 0.45 | 0.75 | 1.3 | VFLENVIR |

5.74 guanine nucleotide binding protein (G protein), q polypeptide [Homo sapiens]

Protein Accession gi|40254462
Mean Expression Ratio 1.25
Median Expression Ratio 1.26
Credible Interval (0.847, 1.88)
Associated Peptides 2
Associated Spectra 3
Coverage 0.0947

| A | 2.5 | 50 | 97.5 | Sequence |
|---|-----|----|------|----------|
| 2 | 0.86 | 1.3 | 1.9 | VADPAYLPTQQDVLR |
| 1 | 0.83 | 1.3 | 2 | VPTTGIIEYPFDLQSVIFR |

1 M T L E S I M A C C L S E E A K E A R R I N D E I E R Q L R R D K R D A R R E L K L L L L G T G E S G K S T F I K Q M R I I H G S G Y S D E D K R G F T K L V Y

81 Q N I F T A M Q A M I R A M D T L K I P Y K Y E H N K A H A Q L V R E V D V E K V S A F E N P Y V D A I K S L W N D P G I Q E C Y D R R R E Y Q L S D S T K Y Y

161 L N D L D R V A D P A Y L P T Q Q D V L R V R V P T T G I I E Y P F D L Q S V I F R M V D V G G Q R S E R R K W I H C F E N V T S I M F L V A L S E Y D Q V L V

241 E S D N E N R M E E S K A L F R T I I T Y P W F Q N S S V I L F L N K K D L L E E K I M Y S H L V D Y F P E Y D G P Q R D A Q A A R E F I L K M F V D L N P D S

321 DKIIYSHFTCATDTENIRFVFAAVKDTILQLNLKEYNLV

5.75 syndecan 1 precursor [Homo sapiens]; syndecan 1 precursor [Homo sapiens]

Protein Accession gi|55749480 gi|29568086
Mean Expression Ratio 0.797
Median Expression Ratio 0.795
Credible Interval (0.536, 1.19)

Associated Peptides 2
Associated Spectra 3
Coverage NaN

| A | 2.5 | 50 | 97.5 | Sequence |
|---|-----|-----|------|----------|
| 2 | 0.49 | 0.74 | 1.1 | EGEAVVLPEVEPGLTAR |
| 1 | 0.52 | 0.8 | 1.3 | NQSPVDQGATGASQGLLDR |

5.76 copine III [Homo sapiens]

Protein Accession gi|4503015
Mean Expression Ratio 1.26
Median Expression Ratio 1.26
Credible Interval (0.877, 1.80)
Associated Peptides 3
Associated Spectra 4
Coverage 0.0782

| A | 2.5 | 50 | 97.5 | Sequence |
|---|-----|-----|------|----------|
| 2 | 0.9 | 1.3 | 1.9 | DIVQFVPFR |
| 1 | 0.83 | 1.3 | 1.9 | LYGPTNFSPIINHVAR |
| 1 | 0.82 | 1.3 | 1.9 | VLLIITDGVITDLDETR |

1 M A A Q C V T K V A L N V S C A N L L D K D I G S K S D P L C V L F L N T S G Q
Q W Y E V E R T E R I K N C L N P Q F S K T F I I D Y Y F E V V Q K L K F G V Y

81 D I D N K T I E L S D D D F L G E C E C T L G Q I V S S K K L T R P L V M K T G R
P A G K G S I T I S A E E I K D N R V V L F E M E A R K L D N K D L F G K S D

161 P Y L E F H K Q T S D G N W L M V H R T E V V K N N L N P V W R P F K I S L N
S L C Y G D M D K T I K V E C Y D Y D N D G S H D L I G T F Q T T M T K L K E A S

241 R S S P V E F E C I N E K K R Q K K K S Y K N S G V I S V K Q C E I T V E C T F
L D Y I M G G C Q L N F T V G V D F T G S N G D P R S P D S L H Y I S P N G V N

321 E Y L T A L W S V G L V I Q D Y D A D K M F P A F G F G A Q I P P Q W Q V S H
E F P M N F N P S N P Y C N G I Q G I V E A Y R S C L P Q I K L Y G P T N F S P I

401 I N H V A R F A A A A T Q Q Q T A S Q Y F V L L I I T D G V I T D L D E T R Q A
I V N A S R L P M S I I I V G V G G A D F S A M E F L D G D G G S L R S P L G E

481 V A I R D I V Q F V P F R Q F Q N A P K E A L A Q C V L A E I P Q Q V V G Y F N
T Y K L L P P K N P A T K Q Q K Q

5.77 annexin VII isoform 2 [Homo sapiens]; annexin VII isoform 1 [Homo sapiens]

Protein Accession gi|4809279 gi|4502111
Mean Expression Ratio 1.26
Median Expression Ratio 1.26
Credible Interval (0.897, 1.79)
Associated Peptides 3
Associated Spectra 5
Coverage NaN

| A | 2.5 | 50 | 97.5 | Sequence |
|---|---|---|---|---|
| 3 | 0.9 | 1.3 | 1.8 | DENQSINHQMAQEDAQR |
| 1 | 0.86 | 1.3 | 2 | GFGTDEQAIVDVV ANR |
| 1 | 0.83 | 1.3 | 1.9 | VLIEILCTR |

5.78 cubilin [Homo sapiens]

Protein Accession gi|126091152
Mean Expression Ratio 0.798
Median Expression Ratio 0.798
Credible Interval (0.637, 1.01)
Associated Peptides 10
Associated Spectra 14
Coverage 0.0461

| A | 2.5 | 50 | 97.5 | Sequence |
|---|---|---|---|---|
| 1 | 0.56 | 0.79 | 1.1 | CNFDVLEIYGGPDFHSPR |
| 4 | 0.56 | 0.75 | 1 | DFVEILDGGHEDAPLR |
| 1 | 0.58 | 0.81 | 1.1 | EQLANPIVSSGNSLFLR |
| 1 | 0.57 | 0.8 | 1.1 | LQVLLLTDGVGR |
| 1 | 0.57 | 0.8 | 1.1 | NGGSPESPIIGQYCGNSNPR |
| 2 | 0.58 | 0.78 | 1.1 | SPENPMQVSSTGNELAIR |
| 1 | 0.57 | 0.8 | 1.1 | SPFFPNVYPGER |
| 1 | 0.55 | 0.78 | 1.1 | VPGQSGVVESIGHPTLPYR |
| 1 | 0.57 | 0.8 | 1.1 | LNDEDLSECLHQIQK |
| 1 | 0.59 | 0.82 | 1.2 | YCGNTIPDSIDTSSNTAVVR |

1 M M N M S L P F L W S L L T L L I F A E V N G E A G E L E L Q R Q K R S I N L Q
Q P R M A T E R G N L V F L T G S A Q N I E F R T G S L G K I K L N D E D L S E

81 C L H Q I Q K N K E D I I E L K G S A I G L P Q N I S S Q I Y Q L N S K L V D L E R
K F Q G L Q Q T V D K K V C S S N P C Q N G G T C L N L H D S F F C I C P P

161 Q W K G P L C S A D V N E C E I Y S G T P L S C Q N G G T C V N T M G S Y S C
H C P P E T Y G P Q C A S K Y D D C E G G S V A R C V H G I C E D L M R E Q A G E

241 P K Y S C V C D A G W M F S P N S P A C T L D R D E C S F Q P G P C S T L V Q
C F N T Q G S F Y C G A C P T G W Q G N G Y I C E D I N E C E I N N G G C S V A P

321 P V E C V N T P G S S H C Q A C P P G Y Q G D G R V C T L T D I C S V S N G G
C H P D A S C S S T L G S L P L C T C L P G Y T G N G Y G P N G C V Q L S N I C L

401 S H P C L N G Q C I D T V S G Y F C K C D S G W T G V N C T E N I N E C L S N P
C L N G G T C V D G V D S F S C E C T R L W T G A L C Q V P Q Q V C G E S L S G

481 I N G S F S Y R S P D V G Y V H D V N C F W V I K T E M G K V L R I T F T F F R
L E S M D N C P H E F L Q V Y D G D S S A F Q L G R F C G S S L P H E L L S S

561 D N A L Y F H L Y S E H L R N G R G F T V R W E T Q Q P E C G G I L T G P Y G
S I K S P G Y P G N Y P P G R D C V W I V V T S P D L L V T F T F G T L S L E H H

641 D D C N K D Y L E I R D G P L Y Q D P L L G K F C T T F S V P P L Q T T G P F A
R I H F H S D S Q I S D Q G F H I T Y L T S P S D L R C G G N Y T D P E G E L F

721 L P E L S G P F T H T R Q C V Y M M K Q P Q G E Q I Q I N F T H V E L Q C Q S D
S S Q N Y I E V R D G E T L L G K V C G N G T I S H I K S I T N S V W I R F K I

801 D A S V E K A S F R A V Y Q V A C G D E L T G E G V I R S P F F P N V Y P G E R
T C R W T I H Q P Q S Q V I L L N F T V F E I G S S A H C E T D Y V E I G S S S

881 I L G S P E N K K Y C G T D I P S F I T S V Y N F L Y V T F V K S S S T E N H G F
M A K F S A E D L A C G E I L T E S T G T I Q S P G H P N V Y P H G I N C T W

961 H I L V Q P N H L I H L M F E T F H L E F H Y N C T N D Y L E V Y D T D S E T S
L G R Y C G K S I P P S L T S S G N S L M L V F V T D S D L A Y E G F L I N Y E

1041 A I S A A T A C L Q D Y T D D L G T F T S P N F P N N Y P N N W E C I Y R I T
V R T G Q L I A V H F T N F S L E E A I G N Y Y T D F L E I R D G G Y E K S P L L

1121 G I F Y G S N L P P T I I S H S N K L W L K F K S D Q I D T R S G F S A Y W D G
S S T G C G G N L T T S S G T F I S P N Y P M P Y Y H S S E C Y W W L K S S H G

1201 S A F E L E F K D F H L E H H P N C T L D Y L A V Y D G P S S N S H L L T Q L
C G D E K P P L I R S S G D S M F I K L R T D E G Q Q G R G F K A E Y R Q T C E N

1281 V V I V N Q T Y G I L E S I G Y P N P Y S E N Q H C N W T I R A T T G N T V N
Y T F L A F D L E H H I N C S T D Y L E L Y D G P R Q M G R Y C G V D L P P P G S

1361 T T S S K L Q V L L L T D G V G R R E K G F Q M Q W F V Y G C G G E L S G A
T G S F S S P G F P N R Y P P N K E C I W Y I R T D P G S S I Q L T I H D F D V E Y

1441 H S R C N F D V L E I Y G G P D F H S P R I A Q L C T Q R S P E N P M Q V S S T
G N E L A I R F K T D L S I N G R G F N A S W Q A V T G G C G G I F Q A P S G E

1521 I H S P N Y P S P Y R S N T D C S W V I R V D R N H R V L L N F T D F D L E P
Q D S C I M A Y D G L S S T M S R L A R T C G R E Q L A N P I V S S G N S L F L R

1601 F Q S G P S R Q N R G F R A Q F R Q A C G G H I L T S S F D T V S S P R F P A N
Y P N N Q N C S W I I Q A Q P P L N H I T L S F T H F E L E R S T T C A R D F V

1681 E I L D G G H E D A P L R G R Y C G T D M P H P I T S F S S A L T L R F V S D S
S I S A G G F H T T V T A S V S A C G G T F Y M A E G I F N S P G Y P D I Y P P

1761 N V E C V W N I V S S P G N R L Q L S F I S F Q L E D S Q D C S R D F V E I R E
G N A T G H L V G R Y C G N S F P L N Y S S I V G H T L W V R F I S D G S G S G

1841 T G F Q A T F M K I F G N D N I V G T H G K V A S P F W P E N Y P H N S N Y Q
W T V N V N A S H V V H G R I L E M D I E E I Q N C Y Y D K L R I Y D G P S I H A

1921 R L I G A Y C G T Q T E S F S S T G N S L T F H F Y S D S S I S G K G F L L E W
F A V D A P D G V L P T I A P G A C G G F L R T G D A P V F L F S P G W P D S Y

2001 S N R V D C T W L I Q A P D S T V E L N I L S L D I E S H R T C A Y D S L V I R
D G D N N L A Q Q L A V L C G R E I P G P I R S T G E Y M F I R F T S D S S V T

2081 R A G F N A S F H K S C G G Y L H A D R G I I T S P K Y P E T Y P S N L N C S
W H V L V Q S G L T I A V H F E Q P F Q I P N G D S S C N Q G D Y L V L R N G P D

2161 I C S P P L G P P G G N G H F C G S H A S S T L F T S D N Q M F V Q F I S D H S
N E G Q G F K I K Y E A K S L A C G G N V Y I H D A D S A G Y V T S P N H P H N

2241 Y P P H A D C I W I L A A P P E T R I Q L Q F E D R F D I E V T P N C T S N Y L
E L R D G V D S D A P I L S K F C G T S L P S S Q W S S G E V M Y L R F R S D N

2321 S P T H V G F K A K Y S I A Q C G G R V P G Q S G V V E S I G H P T L P Y R D
N L F C E W H L Q G L S G H Y L T I S F E D F N L Q N S S G C E K D F V E I W D N

2401 H T S G N I L G R Y C G N T I P D S I D T S S N T A V V R F V T D G S V T A S G
F R L R F E S S M E E C G G D L Q G S I G T F T S P N Y P N P N P H G R I C E W

2481 R I T A P E G R R I T L M F N N L R L A T H P S C N N E H V I V F N G I R S N S
P Q L E K L C S S V N V S N E I K S S G N T M K V I F F T D G S R P Y G G F T A

2561 S Y T S S E D A V C G G S L P N T P E G N F T S P G Y D G V R N Y S R N L N C
E W T L S N P N Q G N S S I S I H F E D F Y L E S H Q D C Q F D V L E F R V G D A

2641 D G P L M W R L C G P S K P T L P L V I P Y S Q V W I H F V T N E R V E H I G
F H A K Y S F T D C G G I Q I G D S G V I T S P N Y P N A Y D S L T H C S S L L E

2721 A P Q G H T I T L T F S D F D I E P H T T C A W D S V T V R N G G S P E S P I I
G Q Y C G N S N P R T I Q S G S N Q L V V T F N S D H S L Q G G G F Y A T W N T

2801 Q T L G C G G I F H S D N G T I R S P H W P Q N F P E N S R C S W T A I T H K S
K H L E I S F D N N F L I P S G D G Q C Q N S F V K V W A G T E E V D K A L L A

2881 T G C G N V A P G P V I T P S N T F T A V F Q S Q E A P A Q G F S A S F V S R C
G S N F T G P S G Y I I S P N Y P K Q Y D N N M N C T Y V I E A N P L S V V L L

2961 T F V S F H L E A R S A V T G S C V N D G V H I I R G Y S V M S T P F A T V C
G D E M P A P L T I A G P V L L N F Y S N E Q I T D F G F K F S Y R I I S C G G V

3041 F N F S S G I I T S P A Y S Y A D Y P N D M H C L Y T I T V S D D K V I E L K F
S D F D V V P S T S C S H D Y L A I Y D G A N T S D P L L G K F C G S K R P P N

3121 V K S S N N S M L L V F K T D S F Q T A K G W K M S F R Q T L G P Q Q G C G
G Y L T G S N N T F A S P D S D S N G M Y D K N L N C V W I I I A P V N K V I H L T

3201 F N T F A L E A A S T R Q R C L Y D Y V K L Y D G D S E N A N L A G T F C G
S T V P A P F I S S G N F L T V Q F I S D L T L E R E G F N A T Y T I M D M P C G G

3281 T Y N A T W T P Q N I S S P N S S D P D V P F S I C T W V I D S P P H Q Q V K I
T V W A L Q L T S Q D C T Q N Y L Q L Q D S P Q G H G N S R F Q F C G R N A S A

3361 V P V F Y S S M S T A M V I F K S G V V N R N S R M S F T Y Q I A D C N R D Y
H K A F G N L R S P G W P D N Y D N D K D C T V T L T A P Q N H T I S L F F H S L

3441 G I E N S V E C R N D F L E V R N G S N S N S P L L G K Y C G T L L P N P V F S
Q N N E L Y L R F K S D S V T S D R G Y E I I W T S S P S G C G G T L Y G D R G

3521 S F T S P G Y P G T Y P N N T Y C E W V L V A P A G R L V T I N F Y F I S I D D P G D C V Q N Y L T L Y D G P N A S S P S S G P Y C G G D T S I A P F V A S S N

3601 QVFIKFHADYARRPSAFRLTWDS

5.79 guanine nucleotide binding protein (G protein), alpha 11 (Gq class) [Homo sapiens]

Protein Accession gi|115511049
Mean Expression Ratio 1.25
Median Expression Ratio 1.25
Credible Interval (0.849, 1.85)
Associated Peptides 2
Associated Spectra 3
Coverage 0.0947

| A | 2.5 | 50 | 97.5 | Sequence |
|---|---|---|---|---|
| 1 | 0.8 | 1.2 | 1.9 | SHLVDYFPEFDGPQR |
| 2 | 0.88 | 1.3 | 2 | VPTTGIIEYPFDLENIIFR |

1 M T L E S M M A C C L S D E V K E S K R I N A E I E K Q L R R D K R D A R R E L K L L L G T G E S G K S T F I K Q M R I I H G A G Y S E E D K R G F T K L V Y

81 Q N I F T A M Q A M I R A M E T L K I L Y K Y E Q N K A N A L L I R E V D V E K V T T F E H Q Y V S A I K T L W E D P G I Q E C Y D R R R E Y Q L S D S A K Y Y

161 L T D V D R I A T L G Y L P T Q Q D V L R V R V P T T G I I E Y P F D L E N I I F R M V D V G G Q R S E R R K W I H C F E N V T S I M F L V A L S E Y D Q V L V

241 E S D N E N R M E E S K A L F R T I I T Y P W F Q N S S V I L F L N K K D L L E D K I L Y S H L V D Y F P E F D G P Q R D A Q A A R E F I L K M F V D L N P D S

321 DKIIYSHFTCATDTENIRFVFAAVKDTILQLNLKEYNLV

5.80 proteasome alpha 1 subunit isoform 2 [Homo sapiens]; proteasome alpha 1 subunit isoform 1 [Homo sapiens]

Protein Accession gi|4506179 gi|23110935
Mean Expression Ratio 1.25
Median Expression Ratio 1.25
Credible Interval (0.869, 1.78)
Associated Peptides 3
Associated Spectra 4
Coverage NaN

| A | 2.5 | 50 | 97.5 | Sequence |
|---|-----|----|------|----------|
| 2 | 0.9 | 1.3 | 2.0 | DLEFTIYDDDDVSPFLEGLEER |
| 1 | 0.82 | 1.2 | 1.9 | ILHVDNHIGISIAGLTADAR |
| 1 | 0.84 | 1.3 | 1.9 | DLEFTIYDDDDVSPFLEGLEERPQR |

5.81 myeloperoxidase [Homo sapiens]

Protein Accession gi|4557759
Mean Expression Ratio 0.805
Median Expression Ratio 0.805
Credible Interval (0.627, 1.04)
Associated Peptides 9
Associated Spectra 11
Coverage 0.172

| A | 2.5 | 50 | 97.5 | Sequence |
|---|-----|----|------|----------|
| 2 | 0.55 | 0.78 | 1.1 | FCGLPQPETVGQLGTVLR |
| 1 | 0.56 | 0.8 | 1.1 | FPTDQLTPDQER |
| 2 | 0.56 | 0.78 | 1.1 | IGLDLP ALNMQR |
| 1 | 0.56 | 0.8 | 1.1 | IVGAMVQIITYR |
| 1 | 0.57 | 0.83 | 1.2 | NIDIWMGGVSEPLK |
| 1 | 0.56 | 0.8 | 1.1 | NQINALTSFVDASMVYGSEEPLAR |
| 1 | 0.58 | 0.81 | 1.2 | QALAQISLPR |
| 1 | 0.55 | 0.79 | 1.1 | VVLEGGIDPILR |
| 1 | 0.57 | 0.81 | 1.2 | VGPLLACIIGTQFR |

1 M G V P F F S S L R C M V D L G P C W A G G L T A E M K L L L A L A G L L A I L
A T P Q P S E G A A P A V L G E V D T S L V L S S M E E A K Q L V D K A Y K E R

81 R E S I K Q R L R S G S A S P M E L L S Y F K Q P V A A T R T A V R A A D Y L H
V A L D L L E R K L R S L W R R P F N V T D V L T P A Q L N V L S K S S G C A Y

161 Q D V G V T C P E Q D K Y R T I T G M C N N R R S P T L G A S N R A F V R W L
P A E Y E D G F S L P Y G W T P G V K R N G F P V A L A R A V S N E I V R F P T D

241 Q L T P D Q E R S L M F M Q W G Q L L D H D L D F T P E P A A R A S F V T G V
N C E T S C V Q Q P P C F P L K I P P N D P R I K N Q A D C I P F F R S C P A C P

321 G S N I T I R N Q I N A L T S F V D A S M V Y G S E E P L A R N L R N M S N Q L
G L L A V N Q R F Q D N G R A L L P F D N L H D D P C L L T N R S A R I P C F L

401 A G D T R S S E M P E L T S M H T L L L R E H N R L A T E L K S L N P R W D G E R L Y Q E A R K I V G A M V Q I I T Y R D Y L P L V L G P T A M R K Y L P T Y R

481 S Y N D S V D P R I A N V F T N A F R Y G H T L I Q P F M F R L D N R Y Q P M E P N P R V P L S R V F F A S W R V V L E G G I D P I L R G L M A T P A K L N R Q

561 N Q I A V D E I R E R L F E Q V M R I G L D L P A L N M Q R S R D H G L P G Y N A W R R F C G L P Q P E T V G Q L G T V L R N L K L A R K L M E Q Y G T P N N I

641 D I W M G G V S E P L K R K G R V G P L L A C I I G T Q F R K L R D G D R F W W E N E G V F S M Q Q R Q A L A Q I S L P R I I C D N T G I T T V S K N N I F M S

721 NSYPRDFVNCSTLPALNLASWREAS

5.82 solute carrier family 5 (iodide transporter), member 8 [Homo sapiens]

Protein Accession gi|167466278
Mean Expression Ratio 0.808
Median Expression Ratio 0.806
Credible Interval (0.554, 1.18)
Associated Peptides 2
Associated Spectra 4
Coverage NaN

| A | 2.5 | 50 | 97.5 | Sequence |
|---|-----|-----|------|----------|
| 1 | 0.54 | 0.84 | 1.3 | EDFLSNFDIFK |
| 3 | 0.51 | 0.74 | 1.1 | SHPVEDGGTDNPAFNHIELNSDQSGK |

5.83 ras homolog gene family, member C precursor [Homo sapiens]; ras homolog gene family, member C precursor [Homo sapiens]; ras homolog gene family, member C precursor [Homo sapiens]; ras homolog gene family, member A [Homo sapiens]

Protein Accession gi|28395033 gi|111494251 gi|111494248 gi|10835049 Mean Expression Ratio 1.24
Median Expression Ratio 1.24
Credible Interval (0.815, 1.90)
Associated Peptides 2
Associated Spectra 2
Coverage NaN

| A | 2.5 | 50 | 97.5 | Sequence |
|---|-----|-----|------|----------|
| 1 | 0.78 | 1.3 | 2 | HFCPNVPIILVGNK |
| 1 | 0.82 | 1.3 | 2.0 | QVELALWDTAGQEDYDR |

5.84 complement factor H isoform a precursor [Homo sapiens]

Protein Accession gi|62739186
Mean Expression Ratio 0.806
Median Expression Ratio 0.807
Credible Interval (0.627, 1.04)
Associated Peptides 8
Associated Spectra 10
Coverage 0.0812

| A | 2.5 | 50 | 97.5 | Sequence |
|---|-----|-----|------|----------|
| 1 | 0.56 | 0.8 | 1.1 | EQVQSCGPPPELLNGNVK |
| 2 | 0.55 | 0.76 | 1.0 | KGEWVALNPLR |
| 1 | 0.57 | 0.81 | 1.2 | LIILEEHLK |
| 1 | 0.55 | 0.78 | 1.1 | RPYFPVAVGK |
| 1 | 0.59 | 0.85 | 1.2 | SCDNPYIPNGDYSPLR |
| 2 | 0.57 | 0.79 | 1.1 | SSNLIILEEHLK |
| 1 | 0.58 | 0.83 | 1.2 | TCNEGYQLLGEINYR |
| 1 | 0.55 | 0.79 | 1.1 | TDCLSLPSFENAIPMGEK |

```
1   MRLLAKIICLMLWAICVAEDCNELPPRRNTEILTGSWSDQT
    YPEGTQAIYKCRPGYRSLGNVIMVCRKGEWVALNPLRKC

81  QKRPCGHPGDTPFGTFTLTGGNVFEYGVKAVYTCNEGYQ
    LLGEINYRECDTDGWTNDIPICEVVKCLPVTAPENGKIVSS

161 AMEPDREYHFGQAVRFVCNSGYKIEGDEEMHCSDDGFW
    SKEKPKCVEISCKSPDVINGSPISQKIIYKENERFQYKCNMG

241 YEYSERGDAVCTESGWRPLPSCEEKSCDNPYIPNGDYSPL
    RIKHRTGDEITYQCRNGFYPATRGNTAKCTSTGWIPAPRC

321 TLKPCDYPDIKHGGLYHENMRRPYFPVAVGKYYSYYCDE
    HFETPSGSYWDHIHCTQDGWSPAVPCLRKCYFPYLENGYNQ

401 NHGRKFVQGKSIDVACHPGYALPKAQTTVTCMENGWSPT
    PRCIRVKTCSKSSIDIENGFISESQYTYALKEKAKYQCKLG

481 YVTADGETSGSITCGKDGWSAQPTCIKSCDIPVFMNARTK
    NDFTWFKLNDTLDYECHDGYESNTGSTTGSIVCGYNGWSD
```

561 L P I C Y E R E C E L P K I D V H L V P D R K K D Q Y K V G E V L K F S C K P G F T I V G P N S V Q C Y H F G L S P D L P I C K E Q V Q S C G P P P E L L N G N

641 V K E K T K E E Y G H S E V V E Y Y C N P R F L M K G P N K I Q C V D G E W T T L P V C I V E E S T C G D I P E L E H G W A Q L S S P P Y Y Y G D S V E F N C S

721 E S F T M I G H R S I T C I H G V W T Q L P Q C V A I D K L K K C K S S N L I I L E E H L K N K K E F D H N S N I R Y R C R G K E G W I H T V C I N G R W D P E

801 V N C S M A Q I Q L C P P P P Q I P N S H N M T T T L N Y R D G E K V S V L C Q E N Y L I Q E G E E I T C K D G R W Q S I P L C V E K I P C S Q P P Q I E H G T

881 I N S S R S S Q E S Y A H G T K L S Y T C E G G F R I S E E N E T T C Y M G K W S S P P Q C E G L P C K S P P E I S H G V V A H M S D S Y Q Y G E E V T Y K C F

961 E G F G I D G P A I A K C L G E K W S H P P S C I K T D C L S L P S F E N A I P M G E K K D V Y K A G E Q V T Y T C A T Y Y K M D G A S N V T C I N S R W T G R

1041 P T C R D T S C V N P P T V Q N A Y I V S R Q M S K Y P S G E R V R Y Q C R S P Y E M F G D E E V M C L N G N W T E P P Q C K D S T G K C G P P P P I D N G D I

1121 T S F P L S V Y A P A S S V E Y Q C Q N L Y Q L E G N K R I T C R N G Q W S E P P K C L H P C V I S R E I M E N Y N I A L R W T A K Q K L Y S R T G E S V E F V

1201 CKRGYRLSSRSHTLRTTCWDGKLEYPTCAKR

5.85 flotillin 1 [Homo sapiens]

Protein Accession gi|5031699
Mean Expression Ratio 1.23
Median Expression Ratio 1.23
Credible Interval (0.95, 1.62)
Associated Peptides 7
Associated Spectra 9
Coverage 0.234

| A | 2.5 | 50 | 97.5 | Sequence |
|---|---|---|---|---|
| 1 | 0.88 | 1.2 | 1.8 | AQQVAVQEQEIAR |
| 1 | 0.88 | 1.3 | 1.8 | DIHDDQDYLHSLGK |
| 1 | 0.85 | 1.2 | 1.8 | LPQVAEEISGPLTSANK |
| 3 | 0.9 | 1.2 | 1.7 | TEAEIAHIALETLEGHQR |
| 1 | 0.87 | 1.3 | 1.8 | VTGEVLDILTR |
| 1 | 0.87 | 1.3 | 1.8 | LTGVSISQVNHKPLR |

(continued)

| A | 2.5 | 50 | 97.5 | Sequence |
|---|-----|-----|------|----------|
| 1 | 0.83 | 1.2 | 1.7 | VFVLPCIQQIQR |

1 M F F T C G P N E A M V V S G F C R S P P V M V A G G R V F V L P C I Q Q I Q R I S L N T L T L N V K S E K V Y T R H G V P I S V T G I A Q V K I Q G Q N K E M

81 L A A A C Q M F L G K T E A E I A H I A L E T L E G H Q R A I M A H M T V E E I Y K D R Q K F S E Q V F K V A S S D L V N M G I S V V S Y T L K D I H D D Q D Y

161 L H S L G K A R T A Q V Q K D A R I G E A E A K R D A G I R E A K A K Q E K V S A Q Y L S E I E M A K A Q R D Y E L K K A A Y D I E V N T R R A Q A D L A Y Q L

241 Q V A K T K Q Q I E E Q R V Q V Q V V E R A Q Q V A V Q E Q E I A R R E K E L E A R V R K P A E A E R Y K L E R L A E A E K S Q L I M Q A E A E A A S V R M R G

321 E A E A F A I G A R A R A E A E Q M A K K A E A F Q L Y Q E A A Q L D M L L E K L P Q V A E E I S G P L T S A N K I T L V S S G S G T M G A A K V T G E V L D I

401 LTRLPESVERLTGVSISQVNHKPLRTA

5.86 hypothetical protein LOC91894 [Homo sapiens]

Protein Accession gi|131889517
Mean Expression Ratio 1.23
Median Expression Ratio 1.23
Credible Interval (0.786, 1.91)
Associated Peptides 1
Associated Spectra 2
Coverage 0.122

| A | 2.5 | 50 | 97.5 | Sequence |
|---|-----|-----|------|----------|
| 2 | 0.83 | 1.3 | 2 | TLKPQPQQLQQNLPK |

1 M G N R V C C G G S W S C P S T F Q K K K K T G S Q T R R T L K P Q P Q Q L Q Q N L P K G H E T T G H T Y E R V L Q Q Q G S Q E R S P G L M S E D S N L H Y A D

81 I Q V C S R P H A R E V K H V H L E N A T E Y A T L R F P Q A T P R Y D S K N G T L V

5.87 ring finger protein 152 [Homo sapiens]

Protein Accession gi|27734873

Mean Expression Ratio 0.815
Median Expression Ratio 0.814
Credible Interval (0.506, 1.31)
Associated Peptides 1
Associated Spectra 1
Coverage 0.143

| A | 2.5 | 50 | 97.5 | Sequence |
|---|-----|-----|------|----------|
| 1 | 0.46 | 0.77 | 1.3 | SVTVVTIPAEQQPLQGGAPQEAVEEEQDR |

1 M E T L S Q D S L L E C Q I C F N Y Y S P R R R P K L L D C K H T C C S V C L Q Q
M R T S Q K D V R C P W C R G V T K L P P G F S V S Q L P D D P E V L A V I A

81 I P H T S E H T P V F I K L P S N G C Y M L P L P I S K E R A L L P G D M G C R L
L P G S Q Q K S V T V V T I P A E Q Q P L Q G G A P Q E A V E E E Q D R R G V

161 V K S S T W S G V C T V I L V A C V L V F L L G I V L H N M S C I S K R F T V I
S C G

5.88 WIRE protein [Homo sapiens]

Protein Accession gi|18959210
Mean Expression Ratio 1.22
Median Expression Ratio 1.23
Credible Interval (0.829, 1.8)
Associated Peptides 2
Associated Spectra 3
Coverage 0.0841

| A | 2.5 | 50 | 97.5 | Sequence |
|---|-----|-----|------|----------|
| 2 | 0.83 | 1.3 | 1.9 | QPPGVPNGPSSPTNESAPELPQR |
| 1 | 0.8 | 1.2 | 1.9 | TPAGPPPPPPPPLR |

1 M P I P P P P P P P G P P P P P T F H Q A N T E Q P K L S R D E Q R G R G A L L Q
D I C K G T K L K K V T N I N D R S A P I L E K P K G S S G G Y G S G G A A

81 L Q P K G G L F Q G G V L K L R P V G A K D G S E N L A G K P A L Q I P S S R A
A A P R P P V S A A S G R P Q D D T D S S R A S L P E L P R M Q R P S L P D L S

161 R P N T T S S T G M K H S S S A P P P P P P G R R A N A P P T P L P M H S S K A
P A Y N R E K P L P P T P G Q R L H P G R E G P P A P P P V K P P P S P V N I R

241 T G P S G Q S L A P P P P P Y R Q P P G V P N G P S S P T N E S A P E L P Q R H N
S L H R K T P G P V R G L A P P P P T S A S P S L L S N R P P P P A R D P P S

321 R G A A P P P P P P V I R N G A R D A P P P P P P Y R M H G S E P P S R G K P P P P P S R T P A G P P P P P P P P P L R N G H R D S I T T V R S F L D D F E S K Y

401 SFHPVEDFPAPEEYKHFQRIYPSKTNRAARGAPPLPPILR

5.89 putative MAPK activating protein PM28 [Homo sapiens]

Protein Accession gi|41281489
Mean Expression Ratio 1.22
Median Expression Ratio 1.22
Credible Interval (0.829, 1.77)
Associated Peptides 3
Associated Spectra 3
Coverage 0.0833

| A | 2.5 | 50 | 97.5 | Sequence |
|---|-----|-----|------|----------|
| 1 | 0.79 | 1.2 | 1.9 | LQSEVAELK |
| 1 | 0.79 | 1.2 | 1.9 | YLIEIAK |
| 1 | 0.81 | 1.3 | 1.9 | KEIADYLAAGKDER |

1 M L G S G F K A E R L R V N L R L V I N R L K L L E K K K T E L A Q K A R K E I A D Y L A A G K D E R A R I R V E H I I R E D Y L V E A M E I L E L Y C D L L L

81 A R F G L I Q S M K E L D S G L A E S V S T L I W A A P R L Q S E V A E L K I V A D Q L C A K Y S K E Y G K L C R T N Q I G T V N D R L M H K L S V E A P P K I

161 L V E R Y L I E I A K N Y N V P Y E P D S V V M A E A P P G V E T D L I D V G F T D D V K K G G P G R G G S G G F T A P V G G P D G T V P M P M P M P M P M P S

241 A N T P F S Y P L P K G P S D F N G L P M G T Y Q A F P N I H P P Q I P A T P P S Y E S M T L M L I R I S L L H R L L V L D P S Q K P L Q S F L P D L Q I T M T

321 TLSYQSCHLCQTHYQLHLLVPAPQHLKTLTLMIFPGGLKS

5.90 flotillin 2 [Homo sapiens]

Protein Accession gi|94538362
Mean Expression Ratio 1.22
Median Expression Ratio 1.22
Credible Interval (0.969, 1.55)
Associated Peptides 8
Associated Spectra 15
Coverage 0.238

| A | 2.5 | 50 | 97.5 | Sequence |
|---|-----|-----|------|----------|
| 1 | 0.87 | 1.2 | 1.7 | ELLAVACEQFLGK |
| 1 | 0.87 | 1.2 | 1.7 | IGEAEAAVIEAMGK |
| 7 | 1.0 | 1.3 | 1.7 | NVVLQTLEGHLR |
| 1 | 0.85 | 1.2 | 1.7 | QIAVEAQEILR |
| 1 | 0.89 | 1.2 | 1.7 | SILGTLTVEQIYQDR |
| 1 | 0.87 | 1.2 | 1.7 | TAEAQLAYELQGAR |
| 2 | 0.88 | 1.2 | 1.7 | MALVLEALPQIAAK |
| 1 | 0.84 | 1.2 | 1.7 | RPAEAEAHR |

1 MGNCHTVGPNEALVVSGGCCGSDYKQYVFGGWAWAWWC
ISDTQRISLEIMTLQPRCEDVETAEGVALTVTGVAQVKIMTE

81 KELLAVACEQFLGKNVQDIKNVVLQTLEGHLRSILGTLTV
EQIYQDRDQFAKLVREVAAPDVGRMGIEILSFTIKDVYDK

161 VDYLSSLGKTQTAVVQRDADIGVAEAERDAGIREAECKK
EMLDVKFMADTKIADSKRAFELQKSAFSEEVNIKTAEAQLA

241 YELQGAREQQKIRQEEIEIEVVQRKKQIAVEAQEILRTDK
ELIATVRRPAEAEAHRIQQIAEGEKVKQVLLAQAEAEKIR

321 KIGEAEAAVIEAMGKAEAERMKLKAEAYQKYGDAAKMA
LVLEALPQIAAKIAAPLTKVDEIVVLSGDNSKVTSEVNRLLA

401 ELPASVHALTGVDLSKIPLIKKATGVQV

5.91 ephrin receptor EphA1 [Homo sapiens]

Protein Accession gi|56119207
Mean Expression Ratio 0.819
Median Expression Ratio 0.821
Credible Interval (0.508, 1.33)
Associated Peptides 1
Associated Spectra 1
Coverage 0.0184

| A | 2.5 | 50 | 97.5 | Sequence |
|---|-----|------|------|----------|
| 1 | 0.46 | 0.78 | 1.3 | APGEGPQVACTGPPSAPR |

1 MERRWPLGLGLVLLLCAPLPPGARAKEVTLMDTSKAQGEL
GWLLDPPKDGWSEQQQILNGTPLYMYQDCPMQGRRDTDHW

81 L R S N W I Y R G E E A S R V H V E L Q F T V R D C K S F P G G A G P L G C K E
T F N L L Y M E S D Q D V G I Q L R R P L F Q K V T T V A A D Q S F T I R D L A

161 S G S V K L N V E R C S L G R L T R R G L Y L A F H N P G A C V A L V S V R V
F Y Q R C P E T L N G L A Q F P D T L P G P A G L V E V A G T C L P H A R A S P R

241 P S G A P R M H C S P D G E W L V P V G R C H C E P G Y E E G G S G E A C V A
C P S G S Y R M D M D T P H C L T C P Q Q S T A E S E G A T I C T C E S G H Y R A

321 P G E G P Q V A C T G P P S A P R N L S F S A S G T Q L S L R W E P P A D T G G
R Q D V R Y S V R C S Q C Q G T A Q D G G P C Q P C G V G V H F S P G A R G L T

401 T P A V H V N G L E P Y A N Y T F N V E A Q N G V S G L G S S G H A S T S V S
I S M G H A E S L S G L S L R L V K K E P R Q L E L T W A G S R P R S P G A N L T

481 Y E L H V L N Q D E E R Y Q M V L E P R V L L T E L Q P D T T Y I V R V R M L
T P L G P G P F S P D H E F R T S P P V S R G L T G G E I V A V I F G L L L G A A

561 L L L G I L V F R S R R A Q R Q R Q Q R Q R D R A T D V D R E D K L W L K P Y
V D L Q A Y E D P A Q G A L D F T R E L D P A W L M V D T V I G E G E F G E V Y R

641 G T L R L P S Q D C K T V A I K T L K D T S P G G Q W W N F L R E A T I M G Q
F S H P H I L H L E G V V T K R K P I M I I T E F M E N G A L D A F L R E R E D Q

721 L V P G Q L V A M L Q G I A S G M N Y L S N H N Y V H R D L A A R N I L V N
Q N L C C K V S D F G L T R L L D D F D G T Y E T Q G G K I P I R W T A P E A I A H

801 R I F T T A S D V W S F G I V M W E V L S F G D K P Y G E M S N Q E V M K S I
E D G Y R L P P P V D C P A P L Y E L M K N C W A Y D R A R R P H F Q K L Q A H
L

881 E Q L L A N P H S L R T I A N F D P R M T L R L P S L S G S D G I P Y R T V S E
W L E S I R M K R Y I L H F H S A G L D T M E C V L E L T A E D L T Q M G I T L

961 PGHQKRILCSIQGFKD

5.92 proteasome beta 3 subunit [Homo sapiens]

Protein Accession gi|22538465
Mean Expression Ratio 1.22
Median Expression Ratio 1.22
Credible Interval (0.756, 1.98)
Associated Peptides 1
Associated Spectra 1
Coverage 0.0878

| A | 2.5 | 50 | 97.5 | Sequence |
|---|-----|-----|------|----------|
| 1 | 0.77 | 1.3 | 2.2 | LYIGLAGLATDVQTVAQR |

1 M S I M S Y N G G A V M A M K G K N C V A I A A D R R F G I Q A Q M V T T D F Q K I F P M G D R L Y I G L A G L A T D V Q T V A Q R L K F R L N L Y E L K E G R

81 Q I K P Y T L M S M V A N L L Y E K R F G P Y Y T E P V I A G L D P K T F K P F I C S L D L I G C P M V T D D F V V S G T C A E Q M Y G M C E S L W E P N M D P

161 D H L F E T I S Q A M L N A V D R D A V S G M G V I V H I I E K D K I T T R T L K A R M D

5.93 cathepsin D preproprotein [Homo sapiens]

Protein Accession gi|4503143
Mean Expression Ratio 0.826
Median Expression Ratio 0.824
Credible Interval (0.69, 0.984)
Associated Peptides 15
Associated Spectra 29
Coverage 0.456

| A | 2.5 | 50 | 97.5 | Sequence |
|---|-----|-----|------|----------|
| 1 | 0.61 | 0.83 | 1.1 | AIGAVPLIQGEYMIPCEK |
| 1 | 0.61 | 0.83 | 1.1 | AYWQVHLDQVEVASGL |
| 4 | 0.63 | 0.82 | 1.1 | DPDAQPGGELMLGGTDSK |
| 5 | 0.6 | 0.78 | 1 | EGCEAIVDTGTSLMVGPVDEVR |
| 4 | 0.63 | 0.83 | 1.1 | ISVNNVLPVFDNLMQQK |
| 1 | 0.62 | 0.85 | 1.2 | LLDIACWIHHK |
| 1 | 0.6 | 0.82 | 1.1 | MVGPVDEVR |
| 1 | 0.61 | 0.84 | 1.2 | QVFGEATK |
| 2 | 0.65 | 0.87 | 1.2 | TVVFDTGSSNLWVPSIHCK |
| 1 | 0.6 | 0.83 | 1.1 | VSTLPAITLK |
| 2 | 0.6 | 0.8 | 1.1 | WILGDVFIGR |
| 3 | 0.6 | 0.8 | 1.1 | FDGILGMAYPR |
| 1 | 0.6 | 0.82 | 1.1 | AYWQVHLDQVEVASGLTLCK |
| 1 | 0.6 | 0.82 | 1.1 | VSQAGK |
| 1 | 0.6 | 0.83 | 1.1 | YSQAVPAVTEGPIPEVLK |

1 M Q P S S L L P L A L C L L A A P A S A L V R I P L H K F T S I R R T M S E V G G S V E D L I A K G P V S K Y S Q A V P A V T E G P I P E V L K N Y M D A Q Y Y

81 GEIGIGTPPQCFTVVFDTGSSNLWVPSIHCKLLDIACWIHH KYNSDKSSTYVKNGTSFDIHYGSGSLSGYLSQDTVSVPC

161 QSASSASALGGVKVERQVFGEATKQPGITFIAAKFDGILG MAYPRISVNNVLPVFDNLMQQKLVDQNIFSFYLSRDPDAQ

241 PGGELMLGGTDSKYYKGSLSYLNVTRKAYWQVHLDQVE VASGLTLCKEGCEAIVDTGTSLMVGPVDEVRELQKAIGAVP L

321 IQGEYMIPCEKVSTLPAITLKLGGKGYKLSPEDYTLKVSQ AGKTLCLSGFMGMDIPPPSGPLWILGDVFIGRYYTVFDRD

401 NNRVGFAEAARL

5.94 hypothetical protein LOC58527 [Homo sapiens]

Protein Accession gi|24308273
Mean Expression Ratio 0.824
Median Expression Ratio 0.827
Credible Interval (0.557, 1.22)
Associated Peptides 2
Associated Spectra 3
Coverage 0.358

| A | 2.5 | 50 | 97.5 | Sequence |
|---|-----|----|------|----------|
| 1 | 0.53 | 0.82 | 1.3 | CANLFEALVGTLK |
| 2 | 0.52 | 0.78 | 1.2 | MNVDHEVNLL VEEIHR |

1 MNVDHEVNLLVEEIHRLGSKNADGKLSVKFGVLFRDDKCA NLFEALVGTLKAAKRRKIVTYPGELLLQGVHDDVDIILLQ

81 D

5.95 annexin 5 [Homo sapiens]

Protein Accession gi|4502107
Mean Expression Ratio 1.21
Median Expression Ratio 1.21
Credible Interval (0.82, 1.80)
Associated Peptides 3
Associated Spectra 3
Coverage 0.141

| A | 2.5 | 50 | 97.5 | Sequence |
|---|-----|-----|------|----------|
| 1 | 0.78 | 1.2 | 1.8 | GTVTDFPGFDER |
| 1 | 0.78 | 1.2 | 1.9 | QEISAAFK |
| 1 | 0.83 | 1.3 | 2 | QVYEEEYGSSLEDDVVGDTSGYYQR |

1 M A Q V L R G T V T D F P G F D E R A D A E T L R K A M K G L G T D E E S I L T L L T S R S N A Q R Q E I S A A F K T L F G R D L L D D L K S E L T G K F E K L

81 I V A L M K P S R L Y D A Y E L K H A L K G A G T N E K V L T E I I A S R T P E E L R A I K Q V Y E E E Y G S S L E D D V V G D T S G Y Y Q R M L V V L L Q A N

161 R D P D A G I D E A Q V E Q D A Q A L F Q A G E L K W G T D E E K F I T I F G T R S V S H L R K V F D K Y M T I S G F Q I E E T I D R E T S G N L E Q L L L A V

241 V K S I R S I P A Y L A E T L Y Y A M K G A G T D D H T L I R V M V S R S E I D L F N I R K E F R K N F A T S L Y S M I K G D T S G D Y K K A L L L L C G E D D

5.96 carboxypeptidase M precursor [Homo sapiens]; carboxypeptidase M precursor [Homo sapiens]; carboxypeptidase M precursor [Homo sapiens]

Protein Accession gi|6631081 gi|53832021 gi|38327526
Mean Expression Ratio 0.83
Median Expression Ratio 0.829
Credible Interval (0.535, 1.28)
Associated Peptides 1
Associated Spectra 2
Coverage NaN

| A | 2.5 | 50 | 97.5 | Sequence |
|---|-----|-----|------|----------|
| 2 | 0.51 | 0.79 | 1.2 | GQVFDQNGNPLPNVIVEVQDR |

5.97 profilin 1 [Homo sapiens]

Protein Accession gi|4826898
Mean Expression Ratio 0.829
Median Expression Ratio 0.83
Credible Interval (0.563, 1.23)
Associated Peptides 3
Associated Spectra 3
Coverage 0.293

| A | 2.5 | 50 | 97.5 | Sequence |
|---|---|---|---|---|
| 1 | 0.52 | 0.8 | 1.2 | DSLLQDGEFSMDLR |
| 1 | 0.52 | 0.83 | 1.3 | EGVHGGLINKK |
| 1 | 0.53 | 0.82 | 1.3 | TFVNITPAEVGVLVGK |

1 MAGWNAYIDNLMADGTCQDAAIVGYKDSPSVWAAVPGKT FVNITPAEVGVLVGKDRSSFYVNGLTLGGQKCSVIRDSLLQ

81 DGEFSMDLRTKSTGGAPTFNVTVTKTDKTLVLLMGKEGV HGGLINKKCYEMASHLRRSQY

5.98 cortactin isoform b [Homo sapiens]; cortactin isoform a [Homo sapiens]

Protein Accession gi|20357556 gi|20357552
Mean Expression Ratio 1.2
Median Expression Ratio 1.20
Credible Interval (0.78, 1.81)
Associated Peptides 2
Associated Spectra 2
Coverage NaN

| A | 2.5 | 50 | 97.5 | Sequence |
|---|---|---|---|---|
| 1 | 0.76 | 1.2 | 1.9 | GPVSGTEPEPVYSMEAADYR |
| 1 | 0.77 | 1.2 | 2.0 | TQTPPVSPAPQPTEER |

5.99 solute carrier family 3, member 1 [Homo sapiens]

Protein Accession gi|187423904
Mean Expression Ratio 0.833
Median Expression Ratio 0.832
Credible Interval (0.642, 1.09)
Associated Peptides 8
Associated Spectra 10
Coverage NaN

| A | 2.5 | 50 | 97.5 | Sequence |
|---|---|---|---|---|
| 1 | 0.56 | 0.8 | 1.1 | CLDWWQEGPMYQIYPR |
| 3 | 0.63 | 0.84 | 1.1 | EVDPIFGTMEDFENLVAAIHDK |
| 1 | 0.6 | 0.85 | 1.2 | FMGTEAYAESIDR |
| 1 | 0.58 | 0.82 | 1.2 | LIIDFIPNHTSDK |
| 1 | 0.59 | 0.84 | 1.2 | LYQDLSLLHANELLLNR |
| 1 | 0.57 | 0.81 | 1.2 | TQIPDTVTQYSELYHDFTTTQVGMHDIVR |

(continued)

| A | 2.5 | 50 | 97.5 | Sequence |
|---|-----|-----|------|----------|
| 1 | 0.58 | 0.82 | 1.2 | WPNWMIGGPDSSR |
| 1 | 0.59 | 0.84 | 1.2 | FLLEAK |

5.100 cell division cycle 42 isoform 1 [Homo sapiens]; cell division cycle 42 isoform 1 [Homo sapiens]

Protein Accession gi|89903012 gi|4757952
Mean Expression Ratio 1.20
Median Expression Ratio 1.20
Credible Interval (0.894, 1.62)
Associated Peptides 5
Associated Spectra 7
Coverage NaN

| A | 2.5 | 50 | 97.5 | Sequence |
|---|-----|-----|------|----------|
| 1 | 0.8 | 1.2 | 1.7 | NVFDEAILAALEPPEPK |
| 3 | 0.95 | 1.3 | 1.8 | QKPITPETAEK |
| 1 | 0.8 | 1.2 | 1.7 | TLGLFDTAGQEDYDR |
| 1 | 0.79 | 1.2 | 1.7 | PYTLGLFDTAGQEDYDR |
| 1 | 0.85 | 1.2 | 1.8 | TPFLLVGTQIDLR |

5.101 CD59 antigen preproprotein [Homo sapiens]; CD59 antigen preproprotein [Homo sapiens]; CD59 antigen preproprotein [Homo sapiens]; CD59 antigen preproprotein [Homo sapiens]; CD59 antigen preproprotein [Homo sapiens]; CD59 antigen preproprotein [Homo sapiens]; CD59 antigen preproprotein [Homo sapiens]; CD59 antigen preproprotein [Homo sapiens]

Protein Accession gi|42761474 gi|42716302 gi|42716299 gi|187829185 gi|187829183 gi|187829070 gi|187828910
Mean Expression Ratio 1.20
Median Expression Ratio 1.2
Credible Interval (0.832, 1.72)
Associated Peptides 1
Associated Spectra 5
Coverage NaN

| A | 2.5 | 50 | 97.5 | Sequence |
|---|-----|-----|------|----------|
| 5 | 0.9 | 1.3 | 1.7 | FEHCNFNDVTTR |

5.102 amnionless protein precursor [Homo sapiens]

Protein Accession gi|110611172
Mean Expression Ratio 1.2
Median Expression Ratio 1.20

Credible Interval (0.807, 1.81)
Associated Peptides 1
Associated Spectra 3
Coverage 0.0353

| A | 2.5 | 50 | 97.5 | Sequence |
|---|-----|----|------|----------|
| 3 | 0.85 | 1.3 | 1.8 | NPVFDVTASEELPLPR |

1 M G V L G R V L L W L Q L C A L T Q A V S K L W V P N T D F D V A A N W S Q N R T P C A G G A V E F P A D K M V S V L V Q E G H A V S D M L L P L D G E L V L A

81 S G A G F G V S D V G S H L D C G A G E P A V F R D S D R F S W H D P H L W R S G D E A P G L F F V D A E R V P C R H D D V F F P P S A S F R V G L G P G A S P

161 V R V R S I S A L G R T F T R D E D L A V F L A S R A G R L R F H G P G A L S V G P E D C A D P S G C V C G N A E A Q P W I C A A L L Q P L G G R C P Q A A C H

241 S A L R P Q G Q C C D L C G A V V L L T H G P A F D L E R Y R A R I L D T F L G L P Q Y H G L Q V A V S K V P R S S R L R E A D T E I Q V V L V E N G P E T G G

321 A G R L A R A L L A D V A E N G E A L G V L E A T M R E S G A H V W G S S A A G L A G G V A A A V L L A L L V L L V A P P L L R R A G R L R W R R H E A A A P A

401 G A P L G F R N P V F D V T A S E E L P L P R R L S L V P K A A A D S T S H S Y F V N P L F A G A E A E A

5.103 von Willebrand factor A domain containing 1 isoform 1 [Homo sapiens]

Protein Accession gi|40068485
Mean Expression Ratio 0.834
Median Expression Ratio 0.836
Credible Interval (0.511, 1.35)
Associated Peptides 1
Associated Spectra 1
Coverage 0.0404

| A | 2.5 | 50 | 97.5 | Sequence |
|---|-----|----|------|----------|
| 1 | 0.48 | 0.8 | 1.3 | EFVGQLVAPLPLGTGALR |

1 M L P W T A L G L A L S L R L A L A R S G A E R G P P A S A P R G D L M F L L D S S A S V S H Y E F S R V R E F V G Q L V A P L P L G T G A L R A S L V H V G S

81 R P Y T E F P F G Q H S S G E A A Q D A V R A S A Q R M G D T H T G L A L V Y A K E Q L F A E A S G A R P G V P K V L V W V T D G G S S D P V G P P M Q E L K D

161 L G V T V F I V S T G R G N F L E L S A A A S A P A E K H L H F V D V D D L H I I V Q E L R G S I L D A M R P Q Q L H A T E I T S S G F R L A W P P L L T A D S

241 G Y Y V L E L V P S A Q P G A A R R Q Q L P G N A T D W I W A G L D P D T D Y D V A L V P E S N V R L L R P Q I L R V R T R P G E A G P G A S G P E S G A G P A

321 P T Q L A A L P A P E E A G P E R I V I S H A R P R S L R V S W A P A L G S A A A L G Y H V Q F G P L R G G E A Q R V E V P A G R N C T T L Q G L A P G T A Y L

401 V T V T A A F R S G R E S A L S A K A C T P D G P R P R P R P V P R A P T P G T A S R E P

5.104 vacuolar protein sorting 28 isoform 1 [Homo sapiens]

Protein Accession gi|7705885
Mean Expression Ratio 1.20
Median Expression Ratio 1.19
Credible Interval (0.815, 1.77)
Associated Peptides 3
Associated Spectra 3
Coverage 0.249

| A | 2.5 | 50 | 97.5 | Sequence |
|---|-----|----|------|----------|
| 1 | 0.79 | 1.2 | 1.9 | QMLFDLESAYNAFNR |
| 1 | 0.82 | 1.3 | 2.0 | QTVSQWLQTLSGMSASDELDDSQVR |
| 1 | 0.76 | 1.2 | 1.8 | QVQGSEISSIDEFCR |

1 M F H G I P A T P G I G A P G N K P E L Y E E V K L Y K N A R E R E K Y D N M A E L F A V V K T M Q A L E K A Y I K D C V S P S E Y T A A C S R L L V Q Y K A A

81 F R Q V Q G S E I S S I D E F C R K F R L D C P L A M E R I K E D R P I T I K D D K G N L N R C I A D V V S L F I T V M D K L R L E I R A M D E I Q P D L R E L

161 M E T M H R M S H L P P D F E G R Q T V S Q W L Q T L S G M S A S D E L D D S Q V R Q M L F D L E S A Y N A F N R F L H A

5.105 mannosidase, alpha, class 2B, member 1 precursor [Homo sapiens]

Protein Accession gi|51873064
Mean Expression Ratio 1.19
Median Expression Ratio 1.19
Credible Interval (0.812, 1.76)
Associated Peptides 3
Associated Spectra 3
Coverage 0.0415

| A | 2.5 | 50 | 97.5 | Sequence |
|---|-----|-----|------|----------|
| 1 | 0.79 | 1.2 | 1.9 | ELVDYFLNVATAQGR |
| 1 | 0.78 | 1.2 | 1.9 | FQVIVYNPLGR |
| 1 | 0.76 | 1.2 | 1.9 | HLVLLDTAQAAAAGHR |

1 MGAYARASGVCARGCLDSAGPWTMSRALRPPLPPLCFFLL LLAAAGARAGGYETCPTVQPNMLNVHLLPHTHDDVGWLKT

81 VDQYFYGIKNDIQHAGVQYILDSVISALLADPTRRFIYVEI AFFSRWWHQQTNATQEVVRDLVRQGRLEFANGGWVMNDE

161 AATHYGAIVDQMTLGLRFLEDTFGNDGRPRVAWHIDPFG HSREQASLFAQMGFDGFFFGRLDYQDKWVRMQKLEMEQVW R

241 ASTSLKPPTADLFTGVLPNGYNPPRNLCWDVLCVDQPLV EDPRSPEYNAKELVDYFLNVATAQGRYYRTNHTVMTMGSD F

321 QYENANMWFKNLDKLIRLVNAQQAKGSSVHVLYSTPAC YLWELNKANLTWSVKHDDFFPYADGPHQFWTGYFSSRPAL KR

401 YERLSYNFLQVCNQLEALVGLAANVGPYGSGDSAPLNEA MAVLQHHDAVSGTSRQHVANDYARQLAAGWGPCEVLLSNA L

481 ARLRGFKDHFTFCQQLNISICPLSQTAARFQVIVYNPLGR KVNWMVRLPVSEGVFVVKDPNGRTVPSDVVIFPSSDSQAH

561 PPELLFSASLPALGFSTYSVAQVPRWKPQARAPQPIPRRS WSPALTIENEHIRATFDPDTGLLMEIMNMNQQLLLPVRQT

641 FFWYNASIGDNESDQASGAYIFRPNQQKPLPVSRWAQIHL VKTPLVQEVHQNFSAWCSQVVRLYPGQRHLELEWSVGPIP

721 V G D T W G K E V I S R F D T P L E T K G R F Y T D S N G R E I L E R R R D Y R P T W K L N Q T E P V A G N Y Y P V N T R I Y I T D G N M Q L T V L T D R S Q G

801 G S S L R D G S L E L M V H R R L L K D D G R G V S E P L M E N G S G A W V R G R H L V L L D T A Q A A A A G H R L L A E Q E V L A P Q V V L A P G G G A A Y N

881 L G A P P R T Q F S G L R R D L P P S V H L L T L A S W G P E M V L L R L E H Q F A V G E D S G R N L S A P V T L N L R D L F S T F T I T R L Q E T T L V A N Q

961 L R E A A S R L K W T T N T G P T P H Q T P Y Q L D P A N I T L E P M E I R T F L A S V Q W K E V D G

5.106 chloride intracellular channel 1 [Homo sapiens]

Protein Accession gi|14251209
Mean Expression Ratio 1.19
Median Expression Ratio 1.19
Credible Interval (0.876, 1.62)
Associated Peptides 4
Associated Spectra 7
Coverage 0.224

| A | 2.5 | 50 | 97.5 | Sequence |
|---|-----|----|------|----------|
| 1 | 0.82 | 1.2 | 1.8 | LAALNPESNTAGLDIFAK |
| 2 | 0.83 | 1.2 | 1.7 | LHIVQVVCK |
| 1 | 0.8 | 1.2 | 1.7 | LTSPLPEEVDETSAEDEGVSQR |
| 3 | 0.9 | 1.3 | 1.8 | VLDNYLTSPLPEEVDETSAEDEGVSQR |

1 M A E E Q P Q V E L F V K A G S D G A K I G N C P F S Q R L F M V L W L K G V T F N V T T V D T K R R T E T V Q K L C P G G Q L P F L L Y G T E V H T D T N K I

81 E E F L E A V L C P P R Y P K L A A L N P E S N T A G L D I F A K F S A Y I K N S N P A L N D N L E K G L L K A L K V L D N Y L T S P L P E E V D E T S A E D E

161 G V S Q R K F L D G N E L T L A D C N L L P K L H I V Q V V C K K Y R G F T I P E A F R G V H R Y L S N A Y A R E E F A S T C P D D E E I E L A Y E Q V A K A L 241 K

5.107 complement factor D preproprotein [Homo sapiens]

Protein Accession gi|42544239
Mean Expression Ratio 0.84

Median Expression Ratio 0.84
Credible Interval (0.568, 1.24)
Associated Peptides 3
Associated Spectra 3
Coverage 0.241

| A | 2.5 | 50 | 97.5 | Sequence |
|---|-----|-----|------|----------|
| 1 | 0.54 | 0.85 | 1.3 | AVPHPDSQPDTIDHDLLLLQLSEK |
| 1 | 0.53 | 0.83 | 1.3 | RPDSLQHVLLPVLDR |
| 1 | 0.51 | 0.81 | 1.3 | GDSGGPLVCGGVLEGVVTSGSR |

1 M H S W E R L A V L V L L G A A A C A A P P R G R I L G G R E A E A H A R P Y M A S V Q L N G A H L C G G V L V A E Q W V L S A A H C L E D A A D G K V Q V L L

81 G A H S L S Q P E P S K R L Y D V L R A V P H P D S Q P D T I D H D L L L L Q L S E K A T L G P A V R P L P W Q R V D R D V A P G T L C D V A G W G I V N H A G

161 R R P D S L Q H V L L P V L D R A T C N R R T H H D G A I T E R L M C A E S N R R D S C K G D S G G P L V C G G V L E G V V T S G S R V C G N R K K P G I Y T R

241 VASYAAWIDSVLA

5.108 scavenger receptor class B, member 2 [Homo sapiens]

Protein Accession gi|5031631

Mean Expression Ratio 0.842

Median Expression Ratio 0.841

Credible Interval (0.677, 1.04)

Associated Peptides 11

Associated Spectra 17

Coverage 0.247

| A | 2.5 | 50 | 97.5 | Sequence |
|---|-----|-----|------|----------|
| 1 | 0.64 | 0.89 | 1.2 | DEVLYVFPSDFCR |
| 1 | 0.59 | 0.83 | 1.2 | EIIEAMLK |
| 1 | 0.58 | 0.81 | 1.1 | FVSAIEGMHPNQEDHETFVDINPLTGIILK |
| 2 | 0.6 | 0.82 | 1.1 | ITFSDYESVQGLPAFR |
| 1 | 0.58 | 0.8 | 1.1 | IVEWNGK |

(continued)

| A | 2.5 | 50 | 97.5 | Sequence |
|---|---|---|---|---|
| 3 | 0.59 | 0.79 | 1.1 | KLDDFVETGDIR |
| 1 | 0.62 | 0.86 | 1.2 | LDDFVETGDIR |
| 1 | 0.6 | 0.83 | 1.2 | SDYESVQGLPAFR |
| 4 | 0.69 | 0.91 | 1.2 | SVYITF SDYESVQGLPAFR |
| 1 | 0.61 | 0.85 | 1.2 | TLNIPVLTVIEWSQVHFLR |
| 1 | 0.61 | 0.84 | 1.2 | TSLDWWITDK |

1 MGRCCFYTAGTLSLLLLVTSVTLLVARVFQKAVDQSIEKKI
VLRNGTEAFDSWEKPPLPVYTQFYFFNVTNPEEILRGET

81 PRVEEVGPYTYRELRNKANIQFGDNGTTISAVSNKAYVFE
RDQSVGDPKIDLIRTLNIPVLTVIEWSQVHFLREIIEAML

161 KAYQQKLFVTHTVDELLWGYKDEILSLIHVFRPDISPYFG
LFYEKNGTNDGDYVFLTGEDSYLNFTKIVEWNGKTSLDWW

241 ITDKCNMINGTDGDSFHPLITKDEVLYVFPSDFCRSVYITF
SDYESVQGLPAFRYKVPAEILANTSDNAGFCIPEGNCLG

321 SGVLNVSICKNGAPIIMSFPHFYQADERFVSAIEGMHPNQ
EDHETFVDINPLTGIILKAAKRFQINIYVKKLDDFVETGD

401 IRTMVFPVMYLNESVHIDKETASRLKSMINTTLIITNIPYII
MALGVFFGLVFTWLACKGQGSMDEGTADERAPLIRT

5.109 plasminogen [Homo sapiens]

Protein Accession gi|4505881

Mean Expression Ratio 0.845

Median Expression Ratio 0.844

Credible Interval (0.519, 1.39)

Associated Peptides 1

Associated Spectra 1

Coverage 0.0272

| A | 2.5 | 50 | 97.5 | Sequence |
|---|-----|-----|------|----------|
| 1 | 0.49 | 0.81 | 1.4 | VILGAHQEVNLEPHVQEIEVSR |

1 M E H K E V V L L L L L F L K S G Q G E P L D D Y V N T Q G A S L F S V T K K Q L G A G S I E E C A A K C E E D E E F T C R A F Q Y H S K E Q Q C V I M A E N R

81 K S S I I I R M R D V V L F E K K V Y L S E C K T G N G K N Y R G T M S K T K N G I T C Q K W S S T S P H R P R F S P A T H P S E G L E E N Y C R N P D N D P Q

161 G P W C Y T T D P E K R Y D Y C D I L E C E E E C M H C S G E N Y D G K I S K T M S G L E C Q A W D S Q S P H A H G Y I P S K F P N K N L K K N Y C R N P D R E

241 L R P W C F T T D P N K R W E L C D I P R C T T P P P S S G P T Y Q C L K G T G E N Y R G N V A V T V S G H T C Q H W S A Q T P H T H N R T P E N F P C K N L D

321 E N Y C R N P D G K R A P W C H T T N S Q V R W E Y C K I P S C D S S P V S T E Q L A P T A P P E L T P V V Q D C Y H G D G Q S Y R G T S S T T T T G K K C Q S

401 W S S M T P H R H Q K T P E N Y P N A G L T M N Y C R N P D A D K G P W C F T T D P S V R W E Y C N L K K C S G T E A S V V A P P P V V L L P D V E T P S E E D

481 C M F G N G K G Y R G K R A T T V T G T P C Q D W A A Q E P H R H S I F T P E T N P R A G L E K N Y C R N P D G D V G G P W C Y T T N P R K L Y D Y C D V P Q C

561 A A P S F D C G K P Q V E P K K C P G R V V G G C V A H P H S W P W Q V S L R T R F G M H F C G G T L I S P E W V L T A A H C L E K S P R P S S Y K V I L G A H

641 Q E V N L E P H V Q E I E V S R L F L E P T R K D I A L L K L S S P A V I T D K V I P A C L P S P N Y V V A D R T E C F I T G W G E T Q G T F G A G L L K E A Q

721 L P V I E N K V C N R Y E F L N G R V Q S T E L C A G H L A G G T D S C Q G D S G G P L V C F E K D K Y I L Q G V T S W G L G C A R P N K P G V Y V R V S R F V

801 TWIEGVMRNN

5.110 ficolin 2 isoform a precursor [Homo sapiens]

Protein Accession gi|61744445
Mean Expression Ratio 0.842
Median Expression Ratio 0.844
Credible Interval (0.539, 1.31)
Associated Peptides 1

Associated Spectra 2
Coverage 0.0671

| A | 2.5 | 50 | 97.5 | Sequence |
|---|---|---|---|---|
| 2 | 0.52 | 0.81 | 1.2 | AGPPGPNGAPGEPQPCLTGPR |

1 M E L D R A V G V L G A A T L L L S F L G M A W A L Q A A D T C P E V K M V G
L E G S D K L T I L R G C P G L P G A P G P K G E A G T N G K R G E R G P P G P P

81 G K A G P P G P N G A P G E P Q P C L T G P R T C K D L L D R G H F L S G W H T
I Y L P D C R P L T V L C D M D T D G G G W T V F Q R R V D G S V D F Y R D W A

161 T Y K Q G F G S R L G E F W L G N D N I H A L T A Q G T S E L R V D L V D F E
D N Y Q F A K Y R S F K V A D E A E K Y N L V L G A F V E G S A G D S L T F H N N

241 Q S F S T K D Q D N D L N T G N C A V M F Q G A W W Y K N C H V S N L N G R
Y L R G T H G S F A N G I N W K S G K G Y N Y S Y K V S E M K V R P A

5.111 glycophorin C isoform 2 [Homo sapiens]; glycophorin C isoform 1 [Homo sapiens]

Protein Accession gi|8051605 gi|4504229
Mean Expression Ratio 1.18
Median Expression Ratio 1.18
Credible Interval (0.727, 1.93)
Associated Peptides 1
Associated Spectra 1
Coverage NaN

| A | 2.5 | 50 | 97.5 | Sequence |
|---|---|---|---|---|
| 1 | 0.72 | 1.2 | 2.1 | GTEFAESADAALQGDPALQDAGDSSR |

5.112 prolylcarboxypeptidase isoform 1 preproprotein [Homo sapiens]; prolylcarboxypeptidase isoform 2 preproprotein [Homo sapiens]

Protein Accession gi|4826940 gi|117306169
Mean Expression Ratio 0.846
Median Expression Ratio 0.847
Credible Interval (0.677, 1.06)
Associated Peptides 9
Associated Spectra 15
Coverage NaN

| A | 2.5 | 50 | 97.5 | Sequence |
|---|-----|-----|------|----------|
| 1 | 0.6 | 0.84 | 1.2 | AMLVFAEHR |
| 1 | 0.59 | 0.83 | 1.2 | DITDTLVAVTISEGAH |
| 1 | 0.59 | 0.83 | 1.2 | DITDTLVAVTISEGAHHL |
| 4 | 0.65 | 0.86 | 1.1 | DITDTLVAVTISEGAHHLDLR |
| 2 | 0.61 | 0.84 | 1.2 | FLTSEQALADFAELIK |
| 3 | 0.63 | 0.84 | 1.1 | HLNFLTSEQALADFAELIK |
| 1 | 0.6 | 0.83 | 1.2 | VDHFGFNTVK |
| 1 | 0.59 | 0.82 | 1.2 | YYGESLPFGDNSFK |
| 1 | 0.62 | 0.87 | 1.2 | NALDPMSVLLAR |

5.113 collagen, type VI, alpha 1 precursor [Homo sapiens]

Protein Accession gi|87196339
Mean Expression Ratio 0.85
Median Expression Ratio 0.848
Credible Interval (0.525, 1.37)
Associated Peptides 1
Associated Spectra 1
Coverage 0.0175

| A | 2.5 | 50 | 97.5 | Sequence |
|---|-----|-----|------|----------|
| 1 | 0.5 | 0.82 | 1.3 | DAEEAISQTIDTIVDMIK |

1 MRAARALLPLLLQACWTAAQDEPETPRAVAFQDCPVDLFF
VLDTSESVALRLKPYGALVDKVKSFTKRFIDNLRDRYYRC

81 DRNLVWNAGALHYSDEVEIIQGLTRMPGGRDALKSSVDA
VKYFGKGTYTDCAIKKGLEQLLVGGSHLKENKYLIVVTDGH

161 PLEGYKEPCGGLEDAVNEAKHLGVKVFSVAITPDHLEPR
LSIIATDHTYRRNFTAADWGQSRDAEEAISQTIDTIVDMIK

241 NNVEQVCCSFECQPARGPPGLRGDPGFEGERGKPGLPGE
KGEAGDPGRPGDLGPVGYQGMKGEKGSRGEKGSRGPKGYK
G

321 EKGKRGIDGVDGVKGEMGYPGLPGCKGSPGFDGIQGPPG
PKGDPGAFGLKGEKGEPGADGEAGRPGSSGPSGDEGQPGEP

401 GPPGEKGEAGDEGNPGPDGAPGERGGPGERGPRGTPGTR
GPRGDPGEAGPQGDQGREGPVGVPGDPGEAGPIGPKGYRGD

481 E G P P G S E G A R G A P G P A G P P G D P G L M G E R G E D G P A G N G T E G F P G F P G Y P G N R G A P G I N G T K G Y P G L K G D E G E A G D P G D D N N

561 D I A P R G V K G A K G Y R G P E G P Q G P P G H Q G P P G P D E C E I L D I I M K M C S C C E C K C G P I D L L F V L D S S E S I G L Q N F E I A K D F V V K

641 V I D R L S R D E L V K F E P G Q S Y A G V V Q Y S H S Q M Q E H V S L R S P S I R N V Q E L K E A I K S L Q W M A G G T F T G E A L Q Y T R D Q L L P P S P N

721 N R I A L V I T D G R S D T Q R D T T P L N V L C S P G I Q V V S V G I K D V F D F I P G S D Q L N V I S C Q G L A P S Q G R P G L S L V K E N Y A E L L E D A

801 F L K N V T A Q I C I D K K C P D Y T C P I T F S S P A D I T I L L D G S A S V G S H N F D T T K R F A K R L A E R F L T A G R T D P A H D V R V A V V Q Y S G

881 T G Q Q R P E R A S L Q F L Q N Y T A L A S A V D A M D F I N D A T D V N D A L G Y V T R F Y R E A S S G A A K K R L L L F S D G N S Q G A T P A A I E K A V Q

961 E A Q R A G I E I F V V V V G R Q V N E P H I R V L V T G K T A E Y D V A Y G E S H L F R V P S Y Q A L L R G V F H Q T V S R K V A L G

5.114 calmodulin 1 [Homo sapiens]; calmodulin 3 [Homo sapiens]; calmodulin 2 [Homo sapiens]

Protein Accession gi|5901912 gi|58218968 gi|4502549
Mean Expression Ratio 1.18
Median Expression Ratio 1.18
Credible Interval (0.728, 1.92)
Associated Peptides 1
Associated Spectra 1
Coverage NaN

| A | 2.5 | 50 | 97.5 | Sequence |
|---|-----|-----|------|----------|
| 1 | 0.74 | 1.2 | 2.1 | VFDKDGNGYISAAELR |

5.115 annexin I [Homo sapiens]

Protein Accession gi|4502101
Mean Expression Ratio 1.17
Median Expression Ratio 1.17
Credible Interval (0.795, 1.74)
Associated Peptides 2
Associated Spectra 3
Coverage 0.078

| A | 2.5 | 50 | 97.5 | Sequence |
|---|-----|-----|------|----------|
| 2 | 0.8 | 1.2 | 1.8 | GLGTDEDTLIEILASR |
| 1 | 0.76 | 1.2 | 1.8 | TPAQFDADELR |

1 MAMVSEFLKQAWFIENEEQEYVQTVKSSKGGPGSAVSPYP
TFNPSSDVAALHKAIMVKGVDEATIIDILTKRNNAQRQQI

81 KAAYLQETGKPLDETLKKALTGHLEEVVLALLKTPAQFDA
DELRAAMKGLGTDEDTLIEILASRTNKEIRDINRVYREEL

161 KRDLAKDITSDTSGDFRNALLSLAKGDRSEDFGVNEDLA
DSDARALYEAGERRKGTDVNVFNTILTTRSYPQLRRVFQKY

241 TKYSKHDMNKVLDLELKGDIEKCLTAIVKCATSKPAFFA
EKLHQAMKGVGTRHKALIRIMVSRSEIDMNDIKAFYQKMYG

321 ISLCQAILDETKGDYEKILVALCGGN

5.116 ras homolog gene family, member G [Homo sapiens]

Protein Accession gi|46249393
Mean Expression Ratio 1.17
Median Expression Ratio 1.17
Credible Interval (0.723, 1.92)
Associated Peptides 1
Associated Spectra 1
Coverage 0.089

| A | 2.5 | 50 | 97.5 | Sequence |
|---|-----|-----|------|----------|
| 1 | 0.73 | 1.2 | 2 | TVNLNLWDTAGQEEYDR |

1 MQSIKCVVVGDGAVGKTCLLICYTTNAFPKEYIPTVFDNYS
AQSAVDGRTVNLNLWDTAGQEEYDRLRTLSYPQTNVFVI

81 CFSIASPPSYENVRHKWHPEVCHHCPDVPILLVGTKKDLR
AQPDTLRRLKEGGQAPITPQQGQALAKQIHAVRYLECSAL

161 QQDGVKEVFAEAVRAVLNPTPIKRGRSCILL

5.117 immunoglobulin superfamily, member 8 [Homo sapiens]

Protein Accession gi|16445029
Mean Expression Ratio 0.849

Median Expression Ratio 0.853
Credible Interval (0.548, 1.29)
Associated Peptides 2
Associated Spectra 2
Coverage 0.0440

| A | 2.5 | 50 | 97.5 | Sequence |
|---|-----|-----|------|----------|
| 1 | 0.52 | 0.84 | 1.3 | VLPDVLQVSAAPPGPR |
| 1 | 0.52 | 0.83 | 1.3 | EVLVPEGPLYR |

1 M G A L R P T L L P P S L P L L L L L M L G M G C W A R E V L V P E G P L Y R V A G T A V S I S C N V T G Y E G P A Q Q N F E W F L Y R P E A P D T A L G I V S

81 T K D T Q F S Y A V F K S R V V A G E V Q V Q R L Q G D A V V L K I A R L Q A Q D A G I Y E C H T P S T D T R Y L G S Y S G K V E L R V L P D V L Q V S A A P P

161 G P R G R Q A P T S P P R M T V H E G Q E L A L G C L A R T S T Q K H T H L A V S F G R S V P E A P V G R S T L Q E V V G I R S D L A V E A G A P Y A E R L A A

241 G E L R L G K E G T D R Y R M V V G G A Q A G D A G T Y H C T A A E W I Q D P D G S W A Q I A E K R A V L A H V D V Q T L S S Q L A V T V G P G E R R I G P G E

321 P L E L L C N V S G A L P P A G R H A A Y S V G W E M A P A G A P G P G R L V A Q L D T E G V G S L G P G Y E G R H I A M E K V A S R T Y R L R L E A A R P G D

401 A G T Y R C L A K A Y V R G S G T R L R E A A S A R S R P L P V H V R E E G V V L E A V A W L A G G T V Y R G E T A S L L C N I S V R G G P P G L R L A A S W W

481 V E R P E D G E L S S V P A Q L V G G V G Q D G V A E L G V R P G G G P V S V E L V G P R S H R L R L H S L G P E D E G V Y H C A P S A W V Q H A D Y S W Y Q A

561 G S A R S G P V T V Y P Y M H A L D T L F V P L L V G T G V A L V T G A T V L G T I T C C F M K R L R K R

5.118 Fc fragment of IgG binding protein [Homo sapiens]

Protein Accession gi|154146262
Mean Expression Ratio 1.17
Median Expression Ratio 1.17
Credible Interval (0.839, 1.62)
Associated Peptides 3

Associated Spectra 6
Coverage 0.00833

| A | 2.5 | 50 | 97.5 | Sequence |
|---|---|---|---|---|
| 4 | 0.84 | 1.2 | 1.6 | VNGVLTALPVSVADGR |
| 1 | 0.8 | 1.2 | 1.8 | YYPLGEVFYPGPECER |
| 1 | 0.8 | 1.2 | 1.8 | APGWDPLCWDECR |

1 M G A L W S W W I L W A G A T L L W G L T Q E A S V D L K N T G R E E F L T A
F L Q N Y Q L A Y S K A Y P R L L I S S L S E S P A S V S I L S Q A D N T S K K V

81 T V R P G E S V M V N I S A K A E M I G S K I F Q H A V V I H S D Y A I S V Q A L
N A K P D T A E L T L L R P I Q A L G T E Y F V L T P P G T S A R N V K E F A

161 V V A G A A G A S V S V T L K G S V T F N G K F Y P A G D V L R V T L Q P Y N
V A Q L Q S S V D L S G S K V T A S S P V A V L S G H S C A Q K H T T C N H V V E

241 Q L L P T S A W G T H Y V V P T L A S Q S R Y D L A F V V A S Q A T K L T Y N
H G G I T G S R G L Q A G D V V E F E V R P S W P L Y L S A N V G I Q V L L F G T

321 G A I R N E V T Y D P Y L V L I P D V A A Y C P A Y V V K S V P G C E G V A L
V V A Q T K A I S G L T I D G H A V G A K L T W E A V P G S E F S Y A E V E L G T

401 A D M I H T A E A T T N L G L L T F G L A K A I G Y A T A A D C G R T V L S P
V E P S C E G M Q C A A G Q R C Q V V G G K A G C V A E S T A V C R A Q G D P H
Y

481 T T F D G R R Y D M M G T C S Y T M V E L C S E D D T L P A F S V E A K N E H
R G S R R V S Y V G L V T V R A Y S H S V S L T R G E V G F V L V D N Q R S R L P

561 V S L S E G R L R V Y Q S G P R A V V E L V F G L V V T Y D W D C Q L A L S L
P A R F Q D Q V C G L C G N Y N G D P A D D F L T P D G A L A P D A V E F A S S W

641 K L D D G D Y L C E D G C Q N N C P A C T P G Q A Q H Y E G D R L C G M L T
K L D G P F A V C H D T L D P R P F L E Q C V Y D L C V V G G E R L S L C R G L S
A

721 Y A Q A C L E L G I S V G D W R S P A N C P L S C P A N S R Y E L C G P A C P T
S C N G A A A P S N C S G R P C V E G C V C L P G F V A S G G A C V P A S S C G

801 C T F Q G L Q L A P G Q E V W A D E L C Q R R C T C N G A T H Q V T C R D K
Q S C P A G E R C S V Q N G L L G C Y P D R F G T C Q G S G D P H Y V S F D G R R
F

881 D F M G T C T Y L L V G S C G Q N A A L P A F R V L V E N E H R G S Q T V S Y T R A V R V E A R G V K V A V R R E Y P G Q V L V D D V L Q Y L P F Q A A D G Q V

961 Q V F R Q G R D A V V R T D F G L T V T Y D W N A R V T A K V P S S Y A E A L C G L C G N F N G D P A D D L A L R G G G Q A A N A L A F G N S W Q E E T R P G C

1041 G A T E P G D C P K L D S L V A Q Q L Q S K N E C G I L A D P K G P F R E C H S K L D P Q G A V R D C V Y D R C L L P G Q S G P L C D A L A T Y A A A C Q A A G

1121 A T V H P W R S E E L C P L S C P P H S H Y E A C S Y G C P L S C G D L P V P G G C G S E C H E G C V C D E G F A L S G E S C L P L A S C G C V H Q G T Y H P P

1201 G Q T F Y P G P G C D S L C H C Q E G G L V S C E S S S C G P H E A C Q P S G G S L G C V A V G S S T C Q A S G D P H Y T T F D G R R F D F M G T C V Y V L A Q

1281 T C G T R P G L H R F A V L Q E N V A W G N G R V S V T R V I T V Q V A N F T L R L E Q R Q W K V T V N G V D M K L P V V L A N G Q I R A S Q H G S D V V I E T

1361 D F G L R V A Y D L V Y Y V R V T V P G N Y Y Q Q M C G L C G N Y N G D P K D D F Q K P N G S Q A G N A N E F G N S W E E V V P D S P C L P P T P C P P G S E D

1441 C I P S H K C P P E L E K K Y Q K E E F C G L L S S P T G P L S S C H K L V D P Q G P L K D C I F D L C L G G G N L S I L C S N I H A Y V S A C Q A A G G H V E

1521 P W R T E T F C P M E C P P N S H Y E L C A D T C S L G C S A L S A P P Q C Q D G C A E G C Q C D S G F L Y N G Q A C V P I Q Q C G C Y H N G V Y Y E P E Q T V

1601 L I D N C R Q Q C T C H A G K G M V C Q E H S C K P G Q V C Q P S G G I L S C V T K D P C H G V T C R P Q E T C K E Q G G Q G V C L P N Y E A T C W L W G D P H

1681 Y H S F D G R K F D F Q G T C N Y V L A T T G C P G V S T Q G L T P F T V T T K N Q N R G N P A V S Y V R V V T V A A L G T N I S I H K D E I G K V R V N G V L

1761 T A L P V S V A D G R I S V T Q G A S K A L L V A D F G L Q V S Y D W N W R V D V T L P S S Y H G A V C G L C G N M D R N P N N D Q V F P N G T L A P S I P I W

1841 GGSWRAPGWDPLCWDECRGSCPTCPEDRLEQYEGPGFC
GPLAPGTGGPFTTCHAHVPPESFFKGCVLDVCMGGGDRDIL
C

1921 KALASYVAACQAAGVVIEDWRAQVGCEITCPENSHYEV
CGSPCPASCPSPAPLTTPAVCEGPCVEGCQCDAGFVLSADRC

2001 VPLNNGCGCWANGTYHEAGSEFWADGTCSQWCRCGPG
GGSLVCTPASCGLGEVCGLLPSGQHGCQPVSTAECQAWGDP
HY

2081 VTLDGHRFNFQGTCEYLLSAPCHGPPLGAENFTVTVANE
HRGSQAVSYTRSVTLQIYNHSLTLSARWPRKLQVDGVFVTL

2161 PFQLDSLLHAHLSGADVVVTTTSGLSLAFDGDSFVRLRV
PAAYAGSLCGLCGNYNQDPADDLKAVGGKPAGWQVGGAQ
GC

2241 GECVSKPCPSPCTPEQQESFGGPDACGVISATDGPLAPCH
GLVPPAQYFQGCLLDACQVQGHPGGLCPAVATYVAACQAA

2321 GAQLREWRRPDFCPFQCPAHSHYELCGDSCPGSCPSLSA
PEGCESACREGCVCDAGFVLSGDTCVPVGQCGCLHDDRYYP

2401 LGQTFYPGPGCDSLCRCREGGEVSCEPSSCGPHETCRPS
GGSLGCVAVGSTTCQASGDPHYTTFDGRRFDFMGTCVYVLA

2481 QTCGTRPGLHRFAVLQENVAWGNGRVSVTRVITVQVAN
FTLRLEQRQWKVTVNGVDMKLPVVLANGQIRASQHGSDVVI
E

2561 TDFGLRVAYDLVYYVRVTVPGNYYQLMCGLCGNYNGD
PKDDFQKPNGSQAGNANEFGNSWEEVVPDSPCLPPPTCPPG
SE

2641 GCIPSEECPPELEKKYQKEEFCGLLSSPTGPLSSCHKLVD
PQGPLKDCIFDLCLGGGNLSILCSNIHAYVSACQAAGGQV
2721 EPWRNETFCPMECPQNSHYELCADTCSLGCSALSAPLQC
PDGCAEGCQCDSGFLYNGQACVPIQQCGCYHNGAYYEPEQT

2801 VLIDNCRQQCTCHVGKVVVCQEHSCKPGQVCQPSGGILS
CVNKDPCHGVTCRPQETCKEQGGQGVCLPNYEATCWLWGD
P

2881 H Y H S F D G R K F D F Q G T C N Y V L A T T G C P G V S T Q G L T P F T V T T K N Q N R G N P A V S Y V R V V T V A A L G T N I S I H K D E I G K V R V N G V

2961 L T A L P V S V A D G R I S V T Q G A S K A L L V A D F G L Q V S Y D W N W R V D V T L P S S Y H G A V C G L C G N M D R N P N N D Q V F P N G T L A P S I P I

3041 W G G S W R A P G W D P L C W D E C R G S C P T C P E D R L E Q Y E G P G F C G P L A P G T G G P F T T C H A H V P P E S F F K G C V L D V C M G G G D R D I L

3121 C K A L A S Y V A A C Q A A G V V I E D W R A Q V G C E I T C P E N S H Y E V C G P P C P A S C P S P A P L T T P A V C E G P C V E G C Q C D A G F V L S A D R

3201 C V P L N N G C G C W A N G T Y H E A G S E F W A D G T C S Q W C R C G P G G G S L V C T P A S C G L G E V C G L L P S G Q H G C Q P V S T A E C Q A W G D P H

3281 Y V T L D G H R F D F Q G T C E Y L L S A P C H G P P L G A E N F T V T V A N E H R G S Q A V S Y T R S V T L Q I Y N H S L T L S A R W P R K L Q V D G V F V T

3361 L P F Q L D S L L H A H L S G A D V V V T T T S G L S L A F D G D S F V R L R V P A A Y A G S L C G L C G N Y N Q D P A D D L K A V G G K P A G W Q V G G A Q G

3441 C G E C V S K P C P S P C T P E Q Q E S F G G P D A C G V I S A T D G P L A P C H G L V P P A Q Y F Q G C L L D A C Q V Q G H P G G L C P A V A T Y V A A C Q A

3521 A G A Q L R E W R R P D F C P F Q C P A H S H Y E L C G D S C P G S C P S L S A P E G C E S A C R E G C V C D A G F V L S G D T C V P V G Q C G C L H D D R Y Y

3601 P L G Q T F Y P G P G C D S L C R C R E G G E V S C E P S S C G P H E T C R P S G G S L G C V A V G S T T C Q A S G D P H Y T T F D G H R F D F M G T C V Y V L

3681 A Q T C G T R P G L H R F A V L Q E N V A W G N G R V S V T R V I T V Q V A N F T L R L E Q R Q W K V T V N G V D M K L P V V L A N G Q I R A S Q H G S D V V I

3761 E T D F G L R V A Y D L V Y Y V R V T V P G N Y Y Q L M C G L C G N Y N G D P K D D F Q K P N G S Q A G N A N E F G N S W E E V V P D S P C L P P P T C P P G S

3841 A G C I P S D K C P P E L E K K Y Q K E E F C G L L S S P T G P L S S C H K L V D P Q G P L K D C I F D L C L G G G N L S I L C S N I H A Y V S A C Q A A G G H

3921 V E P W R N E T F C P M E C P Q N S H Y E L C A D T C S L G C S A L S A P L Q
C P D G C A E G C Q C D S G F L Y N G Q A C V P I Q Q C G C Y H N G V Y Y E P E Q

4001 T V L I D N C R Q Q C T C H V G K V V V C Q E H S C K P G Q V C Q P S G G I L
S C V T K D P C H G V T C R P Q E T C K E Q G G Q G V C L P N Y E A T C W L W G
D

4081 P H Y H S F D G R K F D F Q G T C N Y V L A T T G C P G V S T Q G L T P F T V
T T K N Q N R G N P A V S Y V R V V T V A A L G T N I S I H K D E I G K V R V N G

4161 V L T A L P V S V A D G R I S V A Q G A S K A L L V A D F G L Q V S Y D W N
W R V D V T L P S S Y H G A V C G L C G N M D R N P N N D Q V F P N G T L A P S I
P

4241 I W G G S W R A P G W D P L C W D E C R G S C P T C P E D R L E Q Y E G P G
F C G P L S S G T G G P F T T C H A H V P P E S F F K G C V L D V C M G G G D R D I

4321 L C K A L A S Y V A A C Q A A G V V I E D W R A Q V G C E I T C P E N S H Y
E V C G P P C P A S C P S P A P L T T P A V C E G P C V E G C Q C D A G F V L S A D

4401 R C V P L N N G C G C W A N G T Y H E A G S E F W A D G T C S Q W C R C G
P G G G S L V C T P A S C G L G E V C G L L P S G Q H G C Q P V S T A E C Q A W G
D P

4481 H Y V T L D G H R F D F Q G T C E Y L L S A P C H G P P L G A E N F T V T V A
N E H R G S Q A V S Y T R S V T L Q I Y N H S L T L S A R W P R K L Q V D G V F V

4561 A L P F Q L D S L L H A H L S G A D V V V T T T S G L S L A F D G D S F V R L
R V P A A Y A A S L C G L C G N Y N Q D P A D D L K A V G G K P A G W Q V G G
A Q

4641 G C G E C V S K P C P S P C T P E Q Q E S F G G P D A C G V I S A T D G P L A P
C H G L V P P A Q Y F Q G C L L D A C Q V Q G H P G G L C P A V A T Y V A A C Q

4721 A A G A Q L G E W R R P D F C P L Q C P A H S H Y E L C G D S C P V S C P S L
S A P E G C E S A C R E G C V C D A G F V L S G D T C V P V G Q C G C L H D G R Y

4801 Y P L G E V F Y P G P E C E R R C E C G P G G H V T C Q E G A A C G P H E E C
R L E D G V Q A C H A T G C G R C L A N G G I H Y I T L D G R V Y D L H G S C S Y

4881 V L A Q V C H P K P G D E D F S I V L E K N A A G D L Q R L L V T V A G Q V
V S L A Q G Q Q V T V D G E A V A L P V A V G R V R V T A E G R N M V L Q T T K
G L

4961 R L L F D G D A H L L M S I P S P F R G R L C G L C G N F N G N W S D D F V L P N G S A A S S V E T F G A A W R A P G S S K G C G E G C G P Q G C P V C L A E E

5041 T A P Y E S N E A C G Q L R N P Q G P F A T C Q A V L S P S E Y F R Q C V Y D L C A Q K G D K A F L C R S L A A Y T A A C Q A A G V A V K P W R T D S F C P L H

5121 C P A H S H Y S I C T R T C Q G S C A A L S G L T G C T T R C F E G C E C D D R F L L S Q G V C I P V Q D C G C T H N G R Y L P V N S S L L T S D C S E R C S C

5201 S S S S G L T C Q A A G C P P G R V C E V K A E A R N C W A T R G L C V L S V G A N L T T F D G A R G A T T S P G V Y E L S S R C P G L Q N T I P W Y R V V A E

5281 V Q I C H G K T E A V G Q V H I F F Q D G M V T L T P N K G V W V N G L R V D L P A E K L A S V S V S R T P D G S L L V R Q K A G V Q V W L G A N G K V A V I V

5361 S N D H A G K L C G A C G N F D G D Q T N D W H D S Q E K P A M E K W R A Q D F S P C Y G

5.119 hypothetical protein LOC284422 [Homo sapiens]

    Protein Accession gi|211059425
    Mean Expression Ratio 1.17
    Median Expression Ratio 1.17
    Credible Interval (0.796, 1.74)
    Associated Peptides 2
    Associated Spectra 3
    Coverage NaN

| A | 2.5 | 50 | 97.5 | Sequence |
|---|-----|-----|------|----------|
| 2 | 0.8 | 1.2 | 1.8 | EGESNLGLDLEEKEPGDHER |
| 1 | 0.78 | 1.2 | 1.9 | MESNLYQDQ SEDKR |

5.120 vacuolar protein sorting 37D [Homo sapiens]

    Protein Accession gi|117938318
    Mean Expression Ratio 1.17
    Median Expression Ratio 1.17
    Credible Interval (0.732, 1.91)
    Associated Peptides 1
    Associated Spectra 1
    Coverage 0.0558

| A | 2.5 | 50 | 97.5 | Sequence |
|---|---|---|---|---|
| 1 | 0.73 | 1.2 | 2.0 | SFPAAAVLPTGAAR |

1 MYRARAARAGPEPGSPGRFGILSTGQLRDLLQDEPKLDRIV RLSRKFQGLQLEREACLASNYALAKENLALRPRLEMGRA

81 ALAIKYQELREVAENCADKLQRLEESMHRWSPHCALGWL QAELEEAEQEAEEQMEQLLLGEQSLEAFLPAFQRGRALAHL

161 RRTQAEKLQELLRRRERSAQPAPTSAADPPKSFPAAAVLP TGAARGPPAVPRSLPPLDSRPVPPLKGSPGCPLGPAPLLS

241 PRPSQPEPPHR

5.121 protease, serine, 1 preproprotein [Homo sapiens]

Protein Accession gi|4506145
Mean Expression Ratio 1.17
Median Expression Ratio 1.17
Credible Interval (0.811, 1.68)
Associated Peptides 1
Associated Spectra 5
Coverage 0.0324

| A | 2.5 | 50 | 97.5 | Sequence |
|---|---|---|---|---|
| 5 | 0.88 | 1.2 | 1.7 | NKPGVYTK |

1 MNPLLILTFVAAALAAPFDDDDKIVGGYNCEENSVPYQVS LNSGYHFCGGSLINEQWVVSAGHCYKSRIQVRLGEHNIEV

81 LEGNEQFINAAKIIRHPQYDRKTLNNDIMLIKLSSRAVINA RVSTISLPTAPPATGTKCLISGWGNTASSGADYPDELQC

161 LDAPVLSQAKCEASYPGKITSNMFCVGFLEGGKDSCQGD SGGPVVCNGQLQGVVSWGDGCAQKNKPGVYTKVYNYVKW IK

241 NTIAANS

5.122 prominin 2 [Homo sapiens]

Protein Accession gi|21389623
Mean Expression Ratio 1.17

Median Expression Ratio 1.17
Credible Interval (0.766, 1.82)
Associated Peptides 1
Associated Spectra 2
Coverage 0.03

| A | 2.5 | 50 | 97.5 | Sequence |
|---|-----|----|------|----------|
| 2 | 0.79 | 1.2 | 1.9 | TSMEQLAQELQGLAQAQDNSVLGQR |

1 M K H T L A L L A P L L G L G L G L A L S Q L A A G A T D C K F L G P A E H L T F T P A A R A R W L A P R V R A P G L L D S L Y G T V R R F L S V V Q L N P F P

81 S E L V K A L L N E L A S V K V N E V V R Y E A G Y V V C A V I A G L Y L L L V P T A G L C F C C C R C H R R C G G R V K T E H K A L A C E R A A L M V F L L L

161 T T L L L L I G V V C A F V T N Q R T H E Q M G P S I E A M P E T L L S L W G L V S D V P Q E L Q A V A Q Q F S L P Q E Q V S E E L D G V G V S I G S A I H T Q

241 L R S S V Y P L L A A V G S L G Q V L Q V S V H H L Q T L N A T V V E L Q A G Q Q D L E P A I R E H R D R L L E L L Q E A R C Q G D C A G A L S W A R T L E L G

321 A D F S Q V P S V D H V L H Q L K G V P E A N F S S M V Q E E N S T F N A L P A L A A M Q T S S V V Q E L K K A V A Q Q P E G V R T L A E G F P G L E A A S R W

401 A Q A L Q E V E E S S R P Y L Q E V Q R Y E T Y R W I V G C V L C S V V L F V V L C N L L G L N L G I W G L S A R D D P S H P E A K G E A G A R F L M A G V G L

481 S F L F A A P L I L L V F A T F L V G G N V Q T L V C R S W E N G E L F E F A D T P G N L P P S M N L S Q L L G L R K N I S I H Q A Y Q Q C K E G A A L W T V L

561 Q L N D S Y D L E E H L D I N Q Y T N K L R Q E L Q S L K V D T Q S L D L L S S A A R R D L E A L Q S S G L Q R I H Y P D F L V Q I Q R P V V K T S M E Q L A Q

641 E L Q G L A Q A Q D N S V L G Q R L Q E E A Q G L R N L H Q E K V V P Q Q S L V A K L N L S V R A L E S S A P N L Q L E T S D V L A N V T Y L K G E L P A W A A

721 R I L R N V S E C F L A R E M G Y F S Q Y V A W V R E E V T Q R I A T C Q P L S G A L D N S R V I L C D M M A D P W N A F W F C L A W C T F F L I P S I I F A V

801 KTSKYFRPIRKRLSSTSSEETQLFHIPRVTSLKL

5.123 cathepsin B preproprotein [Homo sapiens]; cathepsin B preproprotein [Homo sapiens]; cathepsin B preproprotein [Homo sapiens]; cathepsin B preproprotein [Homo sapiens]; cathepsin B preproprotein [Homo sapiens]

Protein Accession gi|4503139 gi|22538437 gi|22538435 gi|22538433 gi|22538431 Mean Expression Ratio 0.858
Median Expression Ratio 0.856
Credible Interval (0.556, 1.32)
Associated Peptides 1
Associated Spectra 2
Coverage NaN

| A | 2.5 | 50 | 97.5 | Sequence |
|---|-----|------|-----|--------------------|
| 2 | 0.54 | 0.83 | 1.3 | GQDHCGIESEVVAGIPR |

5.124 radixin [Homo sapiens]

Protein Accession gi|4506467
Mean Expression Ratio 1.16
Median Expression Ratio 1.16
Credible Interval (0.77, 1.75)
Associated Peptides 1
Associated Spectra 3
Coverage 0.0292
A 2.5 50 97.5 Sequence
3 0.82 1.2 1.8 FFPEDVSEELIQEITQR

1 M P K P I N V R V T T M D A E L E F A I Q P N T T G K Q L F D Q V V K T V G L R E V W F F G L Q Y V D S K G Y S T W L K L N K K V T Q Q D V K K E N P L Q F K F

81 R A K F F P E D V S E E L I Q E I T Q R L F F L Q V K E A I L N D E I Y C P P E T A V L L A S Y A V Q A K Y G D Y N K E I H K P G Y L A N D R L L P Q R V L E Q

161 H K L T K E Q W E E R I Q N W H E E H R G M L R E D S M M E Y L K I A Q D L E M Y G V N Y F E I K N K K G T E L W L G V D A L G L N I Y E H D D K L T P K I G F

241 P W S E I R N I S F N D K K F V I K P I D K K A P D F V F Y A P R L R I N K R I L A L C M G N H E L Y M R R R K P D T I E V Q Q M K A Q A R E E K H Q K Q L E R

321 A Q L E N E K K K R E I A E K E K E R I E R E K E E L M E R L K Q I E E Q T I K A Q K E L E E Q T R K A L E L D Q E R K A K E E A E R L E K E R R A A E E A K

401 S A I A K Q A A D Q M K N Q E Q L A A E L A E F T A K I A L L E E A K K K K E E E A T E W Q H K A F A A Q E D L E K T K E E L K T V M S A P P P P P P P P V I P

481 P T E N E H D E H D E N N A E A S A E L S N E G V M N H R S E E E R V T E T Q K N E R V K K Q L Q A L S S E L A Q A R D E T K K T Q N D V L H A E N V K A G R D

561KYKTLRQIRQGNTKQRIDEFEAM

5.125 annexin VI isoform 2 [Homo sapiens]; annexin VI isoform 1 [Homo sapiens]

Protein Accession gi|71773415 gi|71773329
Mean Expression Ratio 1.16
Median Expression Ratio 1.16
Credible Interval (0.832, 1.60)
Associated Peptides 3
Associated Spectra 6
Coverage NaN

| A | 2.5 | 50 | 97.5 | Sequence |
|---|-----|----|----|----------|
| 1 | 0.77 | 1.1 | 1.7 | EAILDIITSR |
| 4 | 0.89 | 1.2 | 1.7 | GLGTDEDTIIDIITHR |
| 1 | 0.78 | 1.2 | 1.7 | GSIHDFPGFDPNQDAEALY |

5.126 cystatin C precursor [Homo sapiens]

Protein Accession gi|4503107
Mean Expression Ratio 0.859
Median Expression Ratio 0.86
Credible Interval (0.716, 1.03)
Associated Peptides 9
Associated Spectra 37
Coverage 0.425

| A | 2.5 | 50 | 97.5 | Sequence |
|---|-----|----|----|----------|
| 1 | 0.63 | 0.88 | 1.2 | ALDFAVGEYNK |
| 3 | 0.63 | 0.83 | 1.1 | KQIVAGVNYFLDVELGR |
| 1 | 0.64 | 0.87 | 1.2 | LDNCPFHDQPHLK |
| 8 | 0.66 | 0.83 | 1.0 | LVGGPMDASVEEEGVR |
| 3 | 0.61 | 0.81 | 1.1 | LVGGPMDASVEEEGVRR |
| 15 | 0.72 | 0.87 | 1.1 | QIVAGVNYFLDVELGR |
| 4 | 0.65 | 0.84 | 1.1 | TQPNLDNCPFHDQPHLK |
| 1 | 0.66 | 0.9 | 1.2 | VGGPMDASVEEEGVR |
| 1 | 0.63 | 0.86 | 1.2 | FLDVELGR |

1 M A G P L R A P L L L L A I L A V A L A V S P A A G S S P G K P P R L V G G P M D A S V E E E G V R R A L D F A V G E Y N K A S N D M Y H S R A L Q V V R A R K

81 Q I V A G V N Y F L D V E L G R T T C T K T Q P N L D N C P F H D Q P H L K R K A F C S F Q I Y A V P W Q G T M T L S K S T C Q D A

5.127 tweety 3 [Homo sapiens]

Protein Accession gi|51100978
Mean Expression Ratio 0.86
Median Expression Ratio 0.86
Credible Interval (0.702, 1.06)
Associated Peptides 8
Associated Spectra 21
Coverage 0.222

| A | 2.5 | 50 | 97.5 | Sequence |
|---|---|---|---|---|
| 1 | 0.62 | 0.86 | 1.2 | AANPFQQK |
| 4 | 0.66 | 0.87 | 1.1 | AAPWWVSLLHR |
| 7 | 0.64 | 0.82 | 1.0 | ALVEMQDVVAELLR |
| 2 | 0.65 | 0.9 | 1.2 | GPDEDGEEEAAPGPR |
| 1 | 0.64 | 0.9 | 1.2 | MVEEYSVLSGDILQYYLACSPR |
| 3 | 0.57 | 0.77 | 1.0 | QLAGRPEPLR |
| 2 | 0.63 | 0.85 | 1.2 | TVSNAPVTEYMSQNANFQNPR |
| 1 | 0.65 | 0.9 | 1.3 | TVPWEQPATKDPLLR |

1 M A G V S Y A A P W W V S L L H R L P H F D L S W E A T S S Q F R P E D T D Y Q Q A L L L L G A A A L A C L A L D L L F L L F Y S F W L C C R R R K S E E H L D

81 A D C C C T A W C V I I A T L V C S A G I A V G F Y G N G E T S D G I H R A T Y S L R H A N R T V A G V Q D R V W D T A V G L N H T A E P S L Q T L E R Q L A G

161 R P E P L R A V Q R L Q G L L E T L L G Y T A A I P F W R N T A V S L E V L A E Q V D L Y D W Y R W L G Y L G L L L L D V I I C L L V L V G L I R S S K G I L V

241 G V C L L G V L A L V I S W G A L G L E L A V S V G S S D F C V D P D A Y V T K M V E E Y S V L S G D I L Q Y Y L A C S P R A A N P F Q Q K L S G S H K A L V E

321 M Q D V V A E L L R T V P W E Q P A T K D P L L R V Q E V L N G T E V N L Q H L T A L V D C R S L H L D Y V Q A L T G F C Y D G V E G L I Y L A L F S F V T A L

401 M F S S I V C S V P H T W Q Q K R G P D E D G E E E A A P G P R Q A H D S L Y R V H M P S L Y S C G S S Y G S E T S I P A A A H T V S N A P V T E Y M S Q N A N

481 F Q N P R C E N T P L I G R E S P P P S Y T S S M R A K Y L A T S Q P R P D S S G S H

5.128 hypothetical protein LOC84418 [Homo sapiens]

Protein Accession gi|14165278
Mean Expression Ratio 1.16
Median Expression Ratio 1.16
Credible Interval (0.761, 1.78)
Associated Peptides 2
Associated Spectra 2
Coverage 0.278

| A | 2.5 | 50 | 97.5 | Sequence |
|---|-----|-----|------|----------|
| 1 | 0.74 | 1.2 | 1.9 | QGYPQYGWQGGPQEPPK |
| 1 | 0.74 | 1.2 | 1.9 | TTVYVVEDQR |

1 M N Q E N P P P Y P G P G P T A P Y P P Y P P Q P M G P G P M G G P Y P P P Q G Y P Y Q G Y P Q Y G W Q G G P Q E P P K T T V Y V V E D Q R R D E L G P S T C L

81 TACWTALCCCCLWDMLT

5.129 CD82 antigen isoform 2 [Homo sapiens]; CD82 antigen isoform 1 [Homo sapiens]

Protein Accession gi|67782354 gi|4504813
Mean Expression Ratio 1.16
Median Expression Ratio 1.16
Credible Interval (0.755, 1.79)
Associated Peptides 2
Associated Spectra 2
Coverage NaN

| A | 2.5 | 50 | 97.5 | Sequence |
|---|-----|-----|------|----------|
| 1 | 0.74 | 1.2 | 1.8 | EDSLQDAWDYVQAQVK |
| 1 | 0.75 | 1.2 | 1.9 | QEMGGIVTELIR |

5.130 napsin A preproprotein [Homo sapiens]

Protein Accession gi|4758754
Mean Expression Ratio 0.862

Median Expression Ratio 0.864
Credible Interval (0.674, 1.09)
Associated Peptides 6
Associated Spectra 13
Coverage 0.207

| A | 2.5 | 50 | 97.5 | Sequence |
|---|-----|-----|------|----------|
| 2 | 0.59 | 0.83 | 1.1 | DPEEPDGGELVLGGSDPAH |
| 5 | 0.57 | 0.76 | 1 | GCAAILDTGTSLITGPTEEIR |
| 2 | 0.61 | 0.85 | 1.2 | ILDTGTSLITGPTEEIR |
| 1 | 0.65 | 0.92 | 1.3 | SPGDKPIFVPLSNYR |
| 1 | 0.6 | 0.85 | 1.2 | TVAFDTGSSNLWVPSR |
| 2 | 0.68 | 0.95 | 1.3 | VDGILSEDKLTIGGIK |

1 M S P P P L L Q P L L L L L L P L L N V E P S G A T L I R I P L H R V Q P G R R I L N
L L R G W R E P A E L P K L G A P S P G D K P I F V P L S N Y R D V Q Y F G

81 E I G L G T P P Q N F T V A F D T G S S N L W V P S R R C H F F S V P C W L H H
R F D P K A S S S F Q A N G T K F A I Q Y G T G R V D G I L S E D K L T I G G I

161 K G A S V I F G E A L W E P S L V F A F A H F D G I L G L G F P I L S V E G V R
P P M D V L V E Q G L L D K P V F S F Y L N R D P E E P D G G E L V L G G S D P

241 A H Y I P P L T F V P V T V P A Y W Q I H M E R V K V G P G L T L C A K G C A
A I L D T G T S L I T G P T E E I R A L H A A I G G I P L L A G E Y I I L C S E I

321 P K L P A V S F L L G G V W F N L T A H D Y V I Q T T R N G V R L C L S G F Q
A L D V P P P A G P F W I L G D V F L G T Y V A V F D R G D M K S S A R V G L A R

401 ARTRGADLGWGETAQAQFPG

5.131 acid phosphatase 2, lysosomal isoform 1 precursor [Homo sapiens]

Protein Accession gi|4557010
Mean Expression Ratio 0.864
Median Expression Ratio 0.864
Credible Interval (0.64, 1.17)
Associated Peptides 4
Associated Spectra 7
Coverage 0.132

| A | 2.5 | 50 | 97.5 | Sequence |
|---|-----|-----|------|----------|
| 4 | 0.6 | 0.83 | 1.1 | EGMLQHWELGQALR |
| 1 | 0.6 | 0.87 | 1.3 | FVTLLYR |

**133**

(continued)

| A | 2.5 | 50 | 97.5 | Sequence |
|---|-----|-----|------|----------|
| 1 | 0.59 | 0.87 | 1.3 | NVYDTLFCEQTHGLR |
| 1 | 0.58 | 0.85 | 1.3 | TLMSAEANLAGLFPPNGMQR |

1 M A G K R S G W S R A A L L Q L L L G V N L V V M P P T R A R S L R F V T L L Y
R H G D R S P V K T Y P K D P Y Q E E W P Q G F G Q L T K E G M L Q H W E L G

81 Q A L R Q R Y H G F L N T S Y H R Q E V Y V R S T D F D R T L M S A E A N L A
G L F P P N G M Q R F N P N I S W Q P I P V H T V P I T E D R L L K F P L G P C P

161 R Y E Q L Q N E T R Q T P E Y Q N E S S R N A Q F L D M V A N E T G L T D L T
L E T V W N V Y D T L F C E Q T H G L R L P P W A S P Q T M Q R L S R L K D F S F

241 R F L F G I Y Q Q A E K A R L Q G G V L L A Q I R K N L T L M A T T S Q L P K L
L V Y S A H D T T L V A L Q M A L D V Y N G E Q A P Y A S C H I F E L Y Q E D S

321 G N F S V E M Y F R N E S D K A P W P L S L P G C P H R C P L Q D F L R L T E P
V V P K D W Q Q E C Q L A S G P A D T E V I V A L A V C G S I L F L L I V L L L

401 TVLFRMQAQPPGYRHVADGEDHA

5.132 apolipoprotein E precursor [Homo sapiens]

Protein Accession gi|4557325
Mean Expression Ratio 0.863
Median Expression Ratio 0.864
Credible Interval (0.56, 1.32)
Associated Peptides 2
Associated Spectra 2
Coverage 0.110

| A | 2.5 | 50 | 97.5 | Sequence |
|---|-----|-----|------|----------|
| 1 | 0.55 | 0.87 | 1.4 | SWFEPLVEDMQR |
| 1 | 0.52 | 0.83 | 1.3 | WVQTLSEQVQEELLSSQVTQELR |

1 M K V L W A A L L V T F L A G C Q A K V E Q A V E T E P E P E L R Q Q T E W Q
S G Q R W E L A L G R F W D Y L R W V Q T L S E Q V Q E E L L S S Q V T Q E L R
A

81 L M D E T M K E L K A Y K S E L E E Q L T P V A E E T R A R L S K E L Q A A Q
A R L G A D M E D V C G R L V Q Y R G E V Q A M L G Q S T E E L R V R L A S H L
R

161 KLRKRLLRDADDLQKRLAVYQAGAREGAERGLSAIRERL GPLVEQGRVRAATVGSLAGQPLQERAQAWGERLRARMEEM G

241 SRTRDRLDEVKEQVAEVRAKLEEQAQQIRLQAEAFQARL KSWFEPLVEDMQRQWAGLVEKVQAAVGTSAAPVPSDNH

5.133 transmembrane protein 176B isoform b [Homo sapiens]; transmembrane protein 176B isoform a [Homo sapiens] ; transmembrane protein 176B isoform a [Homo sapiens]; transmembrane protein 176B isoform a [Homo sapiens]; transmembrane protein 176B isoform a [Homo sapiens]

Protein Accession gi|156416022 gi|156416020 gi|156416018 gi|156416016 gi|156416014
Mean Expression Ratio 0.864
Median Expression Ratio 0.865
Credible Interval (0.64, 1.17)
Associated Peptides 3
Associated Spectra 8
Coverage NaN

| A | 2.5 | 50 | 97.5 | Sequence |
|---|-----|-----|------|----------|
| 5 | 0.6 | 0.82 | 1.1 | FLFHPGDTVPSTAR |
| 1 | 0.58 | 0.85 | 1.2 | LFHPGDTVPSTAR |
| 2 | 0.62 | 0.89 | 1.3 | LLGENSVPPSPSR |

5.134 GM2 ganglioside activator precursor [Homo sapiens]

Protein Accession gi|39995109
Mean Expression Ratio 1.15
Median Expression Ratio 1.16
Credible Interval (0.703, 1.88)
Associated Peptides 1
Associated Spectra 1
Coverage 0.104

| A | 2.5 | 50 | 97.5 | Sequence |
|---|-----|-----|------|----------|
| 1 | 0.71 | 1.2 | 2 | SEFVVPDLELPSWLTTGNYR |

1 MQSLMQAPLLIALGLLLAAPAQAHLKKPSQLSSFSWDNCD EGKDPAVIRSLTLEPDPIIVPGNVTLSVMGSTSVPLSSPL

81 KVDLVLEKEVAGLWIKIPCTDYIGSCTFEHFCDVLDMLIPT GEPCPEPLRTYGLPCHCPFKEGTYSLPKSEFVVPDLELP

161 SWLTTGNYRIESVLSSSGKRLGCIKIAASLKGI

5.135 mercaptopyruvate sulfurtransferase isoform 2 [Homo sapiens]; mercaptopyruvate sulfurtransferase isoform 2 [Homo sapiens]; mercaptopyruvate sulfurtransferase isoform 1 [Homo sapiens]

Protein Accession gi|61835204 gi|194473681 gi|194473668
Mean Expression Ratio 0.865
Median Expression Ratio 0.866
Credible Interval (0.564, 1.33)
Associated Peptides 2
Associated Spectra 2
Coverage NaN

| A | 2.5 | 50 | 97.5 | Sequence |
|---|-----|----|------|----------|
| 1 | 0.53 | 0.85 | 1.4 | ALVSAQWVAEALR |
| 1 | 0.53 | 0.85 | 1.4 | HIPGAAFFDIDQCSDR |

5.136 mannosidase, alpha, class 1A, member 1 [Homo sapiens]

Protein Accession gi|24497519
Mean Expression Ratio 0.866
Median Expression Ratio 0.867
Credible Interval (0.556, 1.32)
Associated Peptides 2
Associated Spectra 2
Coverage 0.0475

| A | 2.5 | 50 | 97.5 | Sequence |
|---|-----|----|------|----------|
| 1 | 0.53 | 0.86 | 1.4 | GLPPVDFVPPIGVESR |
| 1 | 0.53 | 0.85 | 1.3 | MYFDAVQAIETHLIR |

```
  1 M P V G G L L P L F S S P A G G V L G G G L G G G G G R K G S G P A A L R L T E
    K F V L L L V F S A F I T L C F G A I F F L P D S S K L L S G V L F H S S P A L

 81 Q P A A D H K P G P G A R A E D A A E G R A R R R E E G A P G D P E A A L E D
    N L A R I R E N H E R A L R E A K E T L Q K L P E E I Q R D I L L E K K K V A Q D

161 Q L R D K A P F R G L P P V D F V P P I G V E S R E P A D A A I R E K R A K I K
    E M M K H A W N N Y K G Y A W G L N E L K P I S K G G H S S S L F G N I K G A T

241 I V D A L D T L F I M E M K H E F E E A K S W V E E N L D F N V N A E I S V F E
    V N I R F V G G L L S A Y Y L S G E E I F R K K A V E L G V K L L P A F H T P S

321 G I P W A L L N M K S G I G R N W P W A S G G S S I L A E F G T L H L E F M H
    L S H L S G N P I F A E K V M N I R T V L N K L E K P Q G L Y P N Y L N P S S G Q
```

401 W G Q H H V S V G G L G D S F Y E Y L L K A W L M S D K T D L E A K K M Y F D A V Q A I E T H L I R K S S S G L T Y I A E W K G G L L E H K M G H L T C F A G G

481 M F A L G A D A A P E G M A Q H Y L E L G A E I A R T C H E S Y N R T F M K L G P E A F R F D G G V E A I A T R Q N E K Y Y I L R P E V M E T Y M Y M W R L T H

561 D P K Y R K W A W E A V E A L E N H C R V N G G Y S G L R D V Y L L H E S Y D D V Q Q S F F L A E T L K Y L Y L I F S D D D L L P L E H W I F N S E A H L L P I

641 LPKDKKEVEIREE

5.137 integral membrane protein 2B [Homo sapiens]

Protein Accession gi|11527402
Mean Expression Ratio 0.87
Median Expression Ratio 0.868
Credible Interval (0.571, 1.35)
Associated Peptides 2
Associated Spectra 2
Coverage 0.109

| A | 2.5 | 50 | 97.5 | Sequence |
|---|-----|----|------|----------|
| 1 | 0.53 | 0.85 | 1.4 | SGEEALIIPPDAVAVDCK |
| 1 | 0.54 | 0.86 | 1.4 | DPDDVVPVGQR |

1 M V K V T F N S A L A Q K E A K K D E P K S G E E A L I I P P D A V A V D C K D P D D V V P V G Q R R A W C W C M C F G L A F M L A G V I L G G A Y L Y K Y F A

81 L Q P D D V Y Y C G I K Y I K D D V I L N E P S A D A P A A L Y Q T I E E N I K I F E E E E V E F I S V P V P E F A D S D P A N I V H D F N K K L T A Y L D L N

161 L D K C Y V I P L N T S I V M P P R N L L E L L I N I K A G T Y L P Q S Y L I H E H M V I T D R I E N I D H L G F F I Y R L C H D K E T Y K L Q R R E T I K G I

241 QKREASNCFAIRHFENKFAVETLICS

5.138 tetraspan 1 [Homo sapiens]

Protein Accession gi|21264578

Mean Expression Ratio 1.16

Median Expression Ratio 1.15

Credible Interval (0.857, 1.57)

Associated Peptides 4

Associated Spectra 8

Coverage 0.112

| A | 2.5 | 50 | 97.5 | Sequence |
|---|-----|-----|------|----------|
| 1 | 0.8 | 1.2 | 1.7 | AHDQKVEGCFNQLLYDIR |
| 1 | 0.77 | 1.1 | 1.7 | QKAHDQK |
| 5 | 0.92 | 1.2 | 1.7 | VEGCFNQLLYDIR |
| 1 | 0.77 | 1.1 | 1.7 | LVVPAIK |

1 MQCFSFIKTMMILFNLLIFLCGAALLAVGIWVSIDGASFLKI FGPLSSSAMQFVNVGYFLIAAGVVVFALGFLGCYGAKT

81 ESKCALVTFFFILLLIFIAEVAAAVVALVYTTMAEHFLTLL VVPAIKKDYGSQEDFTQVWNTTMKGLKCCGFTNYTDFED

161 SPYFKENSAFPPFCCNDNVTNTANETCTKQKAHDQKVEG CFNQLLYDIRTNAVTVGGVAAGIGGLELAAMIVSMYLYCNL

241 Q

5.139 apolipoprotein D precursor [Homo sapiens]

Protein Accession gi|4502163

Mean Expression Ratio 0.869

Median Expression Ratio 0.868

Credible Interval (0.709, 1.06)

Associated Peptides 9

Associated Spectra 23

Coverage 0.344

| A | 2.5 | 50 | 97.5 | Sequence |
|---|-----|------|------|----------|
| 5 | 0.65 | 0.85 | 1.1 | CPNPPVQENFDVNK |
| 1 | 0.61 | 0.84 | 1.2 | IPTTFENGR |
| 4 | 0.73 | 0.97 | 1.3 | KMTVTDQVNCPK |
| 2 | 0.63 | 0.86 | 1.2 | MTVTDQVNCPK |

(continued)

| A | 2.5 | 50 | 97.5 | Sequence |
|---|-----|-----|------|----------|
| 3 | 0.65 | 0.87 | 1.2 | NILT SNNID VK |
| 2 | 0.62 | 0.83 | 1.1 | NPNLPPETVDSLK |
| 1 | 0.63 | 0.87 | 1.2 | NPPVQENFDVNK |
| 3 | 0.64 | 0.85 | 1.1 | WYEIEK |
| 2 | 0.64 | 0.86 | 1.2 | WYEIEKIPTTFENGR |

1 MVMLLLLLSALAGLFGAAEGQAFHLGKCPNPPVQENFDVN KYLGRWYEIEKIPTTFENGRCIQANYSLMENGKIKVLNQE

81 LRADGTVNQIEGEATPVNLTEPAKLEVKFSWFMPSAPYWI LATDYENYALVYSCTCIIQLFHVDFAWILARNPNLPPETV

161 DSLKNILTSNNIDVKKMTVTDQVNCPKLS

5.140 stomatin isoform a [Homo sapiens]

Protein Accession gi|38016911
Mean Expression Ratio 0.87
Median Expression Ratio 0.869
Credible Interval (0.7, 1.07)
Associated Peptides 9
Associated Spectra 18
Coverage 0.417

| A | 2.5 | 50 | 97.5 | Sequence |
|---|-----|-----|------|----------|
| 2 | 0.67 | 0.92 | 1.3 | EASMVITESPAALQLR |
| 4 | 0.66 | 0.87 | 1.2 | EEIAHNMQSTLDDATDAWGIK |
| 1 | 0.62 | 0.87 | 1.2 | FILPCTDSFIK |
| 2 | 0.6 | 0.83 | 1.1 | TISFDIPPQEILTK |
| 1 | 0.63 | 0.88 | 1.2 | VIAAEGEMNASR |
| 4 | 0.6 | 0.8 | 1.1 | VQNATLAVANITNADSATR |
| 1 | 0.62 | 0.88 | 1.2 | YLQTLTTIAAEK |
| 2 | 0.65 | 0.89 | 1.2 | LPVQLQR |
| 1 | 0.61 | 0.87 | 1.2 | LLAQTTLR |

1 MAEKRHTRDSEAQRLPDSFKDSPSKGLGPCGWILVAFSFLF TVITFPISIWMCIKIIKEYERAIIFRLGRILQGGAKGPG

81 LFFILPCTDSFIKVDMRTISFDIPPQEILTKDSVTISVDGVV YYRVQNATLAVANITNADSATRLLAQTTLRNVLGTKNL

161 S Q I L S D R E E I A H N M Q S T L D D A T D A W G I K V E R V E I K D V K L P
V Q L Q R A M A A E A E A S R E A R A K V I A A E G E M N A S R A L K E A S M V

241 I T E S P A A L Q L R Y L Q T L T T I A A E K N S T I V F P L P I D M L Q G I I G
A K H S H L G

5.141 eukaryotic translation initiation factor 6 isoform c [Homo sapiens]

Protein Accession gi|31563374
Mean Expression Ratio 0.867
Median Expression Ratio 0.869
Credible Interval (0.534, 1.41)
Associated Peptides 1
Associated Spectra 1
Coverage 0.106

| A | 2.5 | 50 | 97.5 | Sequence |
|---|-----|-----|------|----------|
| 1 | 0.5 | 0.84 | 1.4 | TSIEDQDELSSLLQVPLVAGTVNR |

1 M A V R A S F E N N C E I G C F A K L T N T Y C L V A I G G S E N F Y R C G G S P
G A Y G G G E A C A G V K S S G S G R V P A P L P R H H R V H V P T V C S R A

81 S S P I P S P W C T R L S P A A A S S G A C V W E T E E I L A D V L K V E V F R Q
T V A D Q V L V G S Y C V F S N Q G G L V H P K T S I E D Q D E L S S L L Q V

161 P L V A G T V N R G S E V I A A G M V V N D W C A F C G L D T T S T E L S V V
E S V F K L N E A Q P S T I A T S M R D S L I D S L T

5.142 kininogen 1 isoform 2 [Homo sapiens]

Protein Accession gi|4504893
Mean Expression Ratio 0.876
Median Expression Ratio 0.869
Credible Interval (0.603, 1.30)
Associated Peptides 3
Associated Spectra 3
Coverage 0.0937

| A | 2.5 | 50 | 97.5 | Sequence |
|---|------|-----|------|----------|
| 1 | 0.55 | 0.84 | 1.3 | DIPTNSPELEETLTHTITK |
| 1 | 0.55 | 0.86 | 1.3 | YFIDFVAR |
| 1 | 0.58 | 0.89 | 1.4 | IGEIKEETTSHLR |

1 M K L I T I L F L C S R L L L S L T Q E S Q S E E I D C N D K D L F K A V D A A L K K Y N S Q N Q S N N Q F V L Y R I T E A T K T V G S D T F Y S F K Y E I K E

81 G D C P V Q S G K T W Q D C E Y K D A A K A A T G E C T A T V G K R S S T K F S V A T Q T C Q I T P A E G P V V T A Q Y D C L G C V H P I S T Q S P D L E P I L

161 R H G I Q Y F N N N T Q H S S L F M L N E V K R A Q R Q V V A G L N F R I T Y S I V Q T N C S K E N F L F L T P D C K S L W N G D T G E C T D N A Y I D I Q L R

241 I A S F S Q N C D I Y P G K D F V Q P P T K I C V G C P R D I P T N S P E L E E T L T H T I T K L N A E N N A T F Y F K I D N V K K A R V Q V V A G K K Y F I D

321 F V A R E T T C S K E S N E E L T E S C E T K K L G Q S L D C N A E V Y V V P W E K K I Y P T V N C Q P L G M I S L M K R P P G F S P F R S S R I G E I K E E T

401 TSHLRSCEYKGRPPKAGAEPASEREVS

5.143 syntenin isoform 3 [Homo sapiens]; syntenin isoform 3 [Homo sapiens]

Protein Accession gi|55749523 gi|55749515
Mean Expression Ratio 1.15
Median Expression Ratio 1.15
Credible Interval (0.747, 1.77)
Associated Peptides 1
Associated Spectra 2
Coverage NaN

| A | 2.5 | 50 | 97.5 | Sequence |
|---|-----|-----|------|----------|
| 2 | 0.77 | 1.2 | 1.8 | ANVAVVSGAPLQGLVAR |

5.144 guanine nucleotide binding protein (G protein), alpha inhibiting activity polypeptide 2 [Homo sapiens]

Protein Accession gi|4504041
Mean Expression Ratio 1.15
Median Expression Ratio 1.15
Credible Interval (0.8, 1.64)
Associated Peptides 3
Associated Spectra 4
Coverage 0.101

| A | 2.5 | 50 | 97.5 | Sequence |
|---|-----|-----|------|----------|
| 1 | 0.77 | 1.2 | 1.8 | ALSCTAEEQGVLPDDLSGVIR |
| 1 | 0.78 | 1.2 | 1.8 | IAQSDYIPTQQDVLR |
| 2 | 0.77 | 1.1 | 1.7 | SCTAEEQGVLPDDLSGVIR |

MGCTVSAEDKAAAERSKMIDKNLREDGEKAAREVKLLLLG AGESGKSTIVKQMKIIHEDGYSEECRQYRAVVYSNTIQS

IMAIVKAMGNLQIDFADPSRADDARQLFALSCTAEEQGVL PDDLSGVIRRLWADHGVQACFGRSREYQLNDSAAYYLNDL

ERIAQSDYIPTQQDVLRTRVKTTGIVETHFTFKDLHFKMF DVGGQRSERKKWIHCFEGVTAIIFCVALSAYDLVLAEDEE

MNRMHESMKLFDSICNNKWFTDTSIILFLNKKDLFEEKIT HSPLTICFPEYTGANKYDEAASYIQSKFEDLNKRKDTKEI

321 YTHFTCATDTKNVQFVFDAVTDVIIKNNLKDCGLF

5.145 guanine nucleotide binding protein, alpha z polypeptide [Homo sapiens]

Protein Accession gi|4504051
Mean Expression Ratio 1.15
Median Expression Ratio 1.15
Credible Interval (0.708, 1.89)
Associated Peptides 1
Associated Spectra 1
Coverage 0.0423

| A | 2.5 | 50 | 97.5 | Sequence |
|---|-----|-----|------|----------|
| 1 | 0.72 | 1.2 | 2 | IAAADYIPTVEDILR |

MGCRQSSEEKEAARRSRRIDRHLRSESQRQRREIKLLLLGT SNSGKSTIVKQMKIIHSGGFNLEACKEYKPLIIYNAIDS

LTRIIRALAALRIDFHNPDRAYDAVQLFALTGPAESKGEIT PELLGVMRRLWADPGAQACFSRSSEYHLEDNAAYYLNDL

ERIAAADYIPTVEDILRSRDMTTGIVENKFTFKELTFKMV DVGGQRSERKKWIHCFEGVTAIIFCVELSGYDLKLYEDNQ

TSRMAESLRLFDSICNNNWFINTSLILFLNKKDLLAEKIRR IPLTICFPEYKGQNTYEEAAVYIQRQFEDLNRNKETKEI

321 YSHFTCATDTSNIQFVFDAVTDVIIQNNLKYIGLC

5.146 cytochrome P450, family 4, subfamily A, polypeptide 11 [Homo sapiens]

Protein Accession gi|158937242
Mean Expression Ratio 0.875
Median Expression Ratio 0.873
Credible Interval (0.54, 1.41)
Associated Peptides 1
Associated Spectra 1
Coverage 0.0231

| A | 2.5 | 50 | 97.5 | Sequence |
|---|-----|-----|------|----------|
| 1 | 0.51 | 0.84 | 1.4 | VWPNPEVFDPFR |

1 MSVSVLSPSRLLGDVSGILQAASLLILLLLIKAVQLYLHRQ WLLKALQQFPCPPSHWLFGHIQELQQDQELQRIQKWVE

81 TFPSACPHWLWGGKVRVQLYDPDYMKVILGRSDPKSHGS YRFLAPWIGYGLLLLNGQTWFQHRRMLTPAFHYDILKPYVG

161 LMADSVRVMLDKWEELLGQDSPLEVFQHVSLMTLDTIM KCAFSHQGSIQVDRNSQSYIQAISDLNNLVFSRVRNAFHQND

241 TIYSLTSAGRWTHRACQLAHQHTDQVIQLRKAQLQKEGE LEKIKRKRHLDFLDILLLAKMENGSILSDKDLRAEVDTFMF

321 EGHDTTASGISWILYALATHPKHQERCREEIHSLLGDGAS ITWNHLDQMPYTTMCIKEALRLYPPVPGIGRELSTPVTFP

401 DGRSLPKGIMVLLSIYGLHHNPKVWPNPEVFDPFRFAPGS AQHSHAFLPFSGGSRNCIGKQFAMNELKVATALTLLRFEL

481 LPDPTRIPIPIARLVLKSKNGIHLRLRRLPNPCEDKDQL

5.147 olfactomedin 4 precursor [Homo sapiens]

Protein Accession gi|32313593
Mean Expression Ratio 0.876
Median Expression Ratio 0.876
Credible Interval (0.784, 0.983)
Associated Peptides 39
Associated Spectra 112
Coverage 0.657

| A | 2.5 | 50 | 97.5 | Sequence |
|---|-----|-----|------|----------|
| 1 | 0.63 | 0.84 | 1.1 | DLGDVGPPIPSPGFSSFPGVDSSSSFSSSSR |
| 1 | 0.64 | 0.86 | 1.1 | DLSVLQKPQ |
| 1 | 0.66 | 0.88 | 1.2 | DQNTPVVHPPPTPGSC |

(continued)

| A | 2.5 | 50 | 97.5 | Sequence |
|---|-----|-----|------|----------|
| 1 | 0.67 | 0.89 | 1.2 | DQNTPVVHPPPTPGSCGH |
| 3 | 0.68 | 0.9 | 1.2 | DTISYTELDFELIK |
| 3 | 0.67 | 0.87 | 1.1 | DYSPQHPNK |
| 40 | 0.7 | 0.8 | 0.91 | ESFGGSSEIVDQLEVEIR |
| 1 | 0.65 | 0.87 | 1.2 | EYVQLISVYEK |
| 1 | 0.69 | 0.92 | 1.2 | GFSYLYGAWGR |
| 1 | 0.68 | 0.9 | 1.2 | GLYWVAPLNTDGR |
| 2 | 0.61 | 0.8 | 1.1 | GTCQCSVSLPDTTFPVDR |
| 1 | 0.65 | 0.86 | 1.1 | ITYGQGSGTAVYNNNMYVNMYNTGNIAR |
| 6 | 0.7 | 0.88 | 1.1 | LDIVMHK |
| 1 | 0.64 | 0.85 | 1.1 | LEFTAHVL |
| 4 | 0.72 | 0.92 | 1.2 | LEFTAHVLSQK |
| 1 | 0.67 | 0.89 | 1.2 | LEFTAHVLSQKFEK |
| 4 | 0.68 | 0.88 | 1.1 | LETLDKNNVLAIR |
| 2 | 0.67 | 0.88 | 1.1 | LLEYYR |
| 4 | 0.72 | 0.94 | 1.2 | LYNTLDDLLLYINAR |
| 3 | 0.68 | 0.9 | 1.2 | LYVYNDGYLLNYDLSVLQKPQ |
| 1 | 0.66 | 0.88 | 1.2 | QCSVSLPDTTFPVDR |
| 1 | 0.66 | 0.87 | 1.2 | QYKPSASNAF |
| 4 | 0.65 | 0.83 | 1.1 | QYKPSASNAFMVCGVLYATR |
| 2 | 0.67 | 0.89 | 1.2 | SVSLPDTTFPVDR |
| 1 | 0.64 | 0.85 | 1.1 | TIAVTQTLPNAAYNNR |
| 3 | 0.67 | 0.87 | 1.1 | WVAPLNTDGR |
| 2 | 0.66 | 0.87 | 1.1 | TEEIFYYYDTNTGK |
| 5 | 0.72 | 0.91 | 1.2 | VQSINYNPFDQK |
| 1 | 0.64 | 0.86 | 1.1 | YYDTNTGK |
| 1 | 0.67 | 0.9 | 1.2 | LLNYDLSVLQKPQ |
| 1 | 0.66 | 0.88 | 1.2 | YYYDTNTGK |
| 2 | 0.66 | 0.87 | 1.1 | LKECEASK |
| 1 | 0.67 | 0.9 | 1.2 | EIVALK |
| 1 | 0.66 | 0.89 | 1.2 | IDIMEK |
| 1 | 0.67 | 0.9 | 1.2 | LETLDK |
| 1 | 0.67 | 0.9 | 1.2 | VYNDGYLLNYDLSVLQKPQ |
| 1 | 0.67 | 0.9 | 1.2 | YNPFDQK |
| 1 | 0.66 | 0.87 | 1.2 | LYNTLDDLLLY |
| 1 | 0.63 | 0.83 | 1.1 | VNMYNTGNIARVNLTTNTIAVTQTLPNAAYNNR |

MRPGLSFLLALLFFLGQAAGDLGDVGPPIPSPGFSSFPGVDS
SSSFSSSSRSGSSSSRSLGSGGSVSQLFSNFTGSVDDR

GTCQCSVSLPDTTFPVDRVERLEFTAHVLSQKFEKELSKV
REYVQLISVYEKKLLNLTVRIDIMEKDTISYTELDFELIK

VEVKEMEKLVIQLKESFGGSSEIVDQLEVEIRNMTLLVEK
LETLDKNNVLAIRREIVALKTKLKECEASKDQNTPVVHPP

PTPGSCGHGGVVNISKPSVVQLNWRGFSYLYGAWGRDYS
PQHPNKGLYWVAPLNTDGRLLEYYRLYNTLDDLLLYINARE

LRITYGQGSGTAVYNNNMYVNMYNTGNIARVNLTTNTIA
VTQTLPNAAYNNRFSYANVAWQDIDFAVDENGLWVIYSTEA

STGNMVISKLNDTTLQVLNTWYTKQYKPSASNAFMVCG
VLYATRTMNTRTEEIFYYYDTNTGKEGKLDIVMHKMQEKV
QS

INYNPFDQKLYVYNDGYLLNYDLSVLQKPQ

5.148 peptidoglycan recognition protein 2 precursor [Homo sapiens]

Protein Accession gi|156616294
Mean Expression Ratio 0.877
Median Expression Ratio 0.876
Credible Interval (0.542, 1.41)
Associated Peptides 1
Associated Spectra 1
Coverage 0.0226

| A | 2.5 | 50 | 97.5 | Sequence |
|---|-----|-----|------|----------|
| 1 | 0.52 | 0.85 | 1.4 | TDCPGDALFDLLR |

MAQGVLWILLGLLLWSDPGTASLPLLMDSVIQALAELEQK
VPAAKTRHTASAWLMSAPNSGPHNRLYHFLLGAWSLNATE

LDPCPLSPELLGLTKEVARHDVREGKEYGVVLAPDGSTVA
VEPLLAGLEAGLQGRRVINLPLDSMAAPWETGDTFPDVVA

IAPDVRATSSPGLRDGSPDVTTADIGANTPDATKGCPDVQ
ASLPDAKAKSPPTMVDSLLAVTLAGNLGLTFLRGSQTQSH

241 P D L G T E G C W D Q L S A P R T F T L L D P K A S L L T M A F L N G A L D G V I L G D Y L S R T P E P R P S L S H L L S Q Y Y G A G V A R D P G F R S N F R R

321 Q N G A A L T S A S I L A Q Q V W G T L V L L Q R L E P V H L Q L Q C M S Q E Q L A Q V A A N A T K E F T E A F L G C P A I H P R C R W G A A P Y R G R P K L L

401 Q L P L G F L Y V H H T Y V P A P P C T D F T R C A A N M R S M Q R Y H Q D T Q G W G D I G Y S F V V G S D G Y V Y E G R G W H W V G A H T L G H N S R G F G V

481 A I V G N Y T A A L P T E A A L R T V R D T L P S C A V R A G L L R P D Y A L L G H R Q L V R T D C P G D A L F D L L R T W P H F T A T V K P R P A R S V S K R

561 SRREPPPRTLPATDLQ

5.149 SMAD family member 9 isoform b [Homo sapiens]; SMAD family member 9 isoform a [Homo sapiens]

Protein Accession gi|5174519 gil187828357
Mean Expression Ratio 1.14
Median Expression Ratio 1.14
Credible Interval (0.696, 1.84)
Associated Peptides 1
Associated Spectra 1
Coverage NaN

| A | 2.5 | 50 | 97.5 | Sequence |
|---|-----|-----|------|----------|
| 1 | 0.71 | 1.2 | 1.9 | VETPVLPPVLVPR |

5.150 brain creatine kinase [Homo sapiens]

Protein Accession gi|21536286
Mean Expression Ratio 1.14
Median Expression Ratio 1.14
Credible Interval (0.776, 1.69)
Associated Peptides 3
Associated Spectra 3
Coverage 0.150

| A | 2.5 | 50 | 97.5 | Sequence |
|---|-----|-----|------|----------|
| 1 | 0.75 | 1.2 | 1.8 | GTGGVDTAAVGGVFDVSNADR |
| 1 | 0.76 | 1.2 | 1.8 | TDLNPDNLQGGDDLDPNYVLSSR |
| 1 | 0.74 | 1.1 | 1.8 | TFLVWVNEEDHLR |

1 M P F S N S H N A L K L R F P A E D E F P D L S A H N N H M A K V L T P E L Y A E L R A K S T P S G F T L D D V I Q T G V D N P G H P Y I M T V G C V A G D E E

81 S Y E V F K D L F D P I I E D R H G G Y K P S D E H K T D L N P D N L Q G G D D L D P N Y V L S S R V R T G R S I R G F C L P P H C S R G E R R A I E K L A V E

161 A L S S L D G D L A G R Y Y A L K S M T E A E Q Q Q L I D D H F L F D K P V S P L L L A S G M A R D W P D A R G I W H N D N K T F L V W V N E E D H L R V I S M

241 Q K G G N M K E V F T R F C T G L T Q I E T L F K S K D Y E F M W N P H L G Y I L T C P S N L G T G L R A G V H I K L P N L G K H E K F S E V L K R L R L Q K R

321 G T G G V D T A A V G G V F D V S N A D R L G F S E V E L V Q M V V D G V K L L I E M E Q R L E Q G Q A I D D L M P A Q K

5.151 chromatin modifying protein 2A [Homo sapiens]; chromatin modifying protein 2A [Homo sapiens]

Protein Accession gi|7656922 gi|38372933
Mean Expression Ratio 1.14
Median Expression Ratio 1.14
Credible Interval (0.798, 1.62)
Associated Peptides 3
Associated Spectra 4
Coverage NaN

| A | 2.5 | 50 | 97.5 | Sequence |
|---|-----|-----|------|----------|
| 1 | 0.76 | 1.2 | 1.8 | AEAAASALADADADLEER |
| 2 | 0.8 | 1.2 | 1.8 | KAEAAASALADADADLEER |
| 1 | 0.73 | 1.1 | 1.6 | KIIADIK |

5.152 solute carrier family 4, sodium bicarbonate cotransporter, member 4 isoform 2 [Homo sapiens]

Protein Accession gi|4507025
Mean Expression Ratio 0.88
Median Expression Ratio 0.878
Credible Interval (0.573, 1.34)
Associated Peptides 2
Associated Spectra 2
Coverage 0.0329

| A | 2.5 | 50 | 97.5 | Sequence |
|---|-----|------|------|----------|
| 1 | 0.53 | 0.84 | 1.3 | LQQAVMLGALTEVPVPTR |

(continued)

| A | 2.5 | 50 | 97.5 | Sequence |
|---|-----|-----|------|----------|
| 1 | 0.55 | 0.9 | 1.4 | ILVATDASFLVQYFTR |

1 M S T E N V E G K P S N L G E R G R A R S S T F L R V V Q P M F N H S I F T S A V S P A A E R I R F I L G E E D D S P A P P Q L F T E L D E L L A V D G Q E M E

81 W K E T A R W I K F E E K V E Q G G E R W S K P H V A T L S L H S L F E L R T C M E K G S I M L D R E A S S L P Q L V E M I V D H Q I E T G L L K P E L K D K V

161 T Y T L L R K H R H Q T K K S N L R S L A D I G K T V S S A S R M F T N P D N G S P A M T H R N L T S S S L N D I S D K P E K D Q L K N K F M K K L P R D A E A

241 S N V L V G E V D F L D T P F I A F V R L Q Q A V M L G A L T E V P V P T R F L F I L L G P K G K A K S Y H E I G R A I A T L M S D E V F H D I A Y K A K D R H

321 D L I A G I D E F L D E V I V L P P G E W D P A I R I E P P K S L P S S D K R K N M Y S G G E N V Q M N G D T P H D G G H G G G G H G D C E E L Q R T G R F C G

401 G L I K D I K R K A P F F A S D F Y D A L N I Q A L S A I L F I Y L A T V T N A I T F G G L L G D A T D N M Q G V L E S F L G T A V S G A I F C L F A G Q P L T

481 I L S S T G P V L V F E R L L F N F S K D N N F D Y L E F R L W I G L W S A F L C L I L V A T D A S F L V Q Y F T R F T E E G F S S L I S F I F I Y D A F K K M

561 I K L A D Y Y P I N S N F K V G Y N T L F S C T C V P P D P A N I S I S N D T T L A P E Y L P T M S S T D M Y H N T T F D W A F L S K K E C S K Y G G N L V G N

641 N C N F V P D I T L M S F I L F L G T Y T S S M A L K K F K T S P Y F P T T A R K L I S D F A I I L S I L I F C V I D A L V G V D T P K L I V P S E F K P T S P

721 N R G W F V P P F G E N P W W V C L A A A I P A L L V T I L I F M D Q Q I T A V I V N R K E H K L K K G A G Y H L D L F W V A I L M V I C S L M A L P W Y V A A

801 T V I S I A H I D S L K M E T E T S A P G E Q P K F L G V R E Q R V T G T L V F I L T G L S V F M A P I L K F I P M P V L Y G V F L Y M G V A S L N G V Q F M D

881 R L K L L L M P L K H Q P D F I Y L R H V P L R R V H L F T F L Q V L C L A L L W I L K S T V A A I I F P V M I L A L V A V R K G M D Y L F S Q H D L S F L D D

961 V I P E K D K K K K E D E K K K K K K K G S L D S D N D D S D C P Y S E K V P S I K I P M D I M E Q Q P F L S D S K P S D R E R S P T F L E R H T S C

5.153 phospholipid transfer protein isoform a precursor [Homo sapiens]

Protein Accession gi|5453914
Mean Expression Ratio 0.875
Median Expression Ratio 0.878
Credible Interval (0.563, 1.35)
Associated Peptides 1
Associated Spectra 2
Coverage 0.0304

| A | 2.5 | 50 | 97.5 | Sequence |
|---|-----|----|------|----------|
| 2 | 0.54 | 0.84 | 1.3 | FLEQELETITIPDLR |

1 MALFGALFLALLAGAHAEFPGCKIRVTSKALELVKQEGLR
FLEQELETITIPDLRGKEGHFYYNISEVKVTELQLTSSEL

81 DFPQQELMLQITNASLGLRFRRQLLYWFFYDGGYINASA
EGVSIRTGLELSRDPAGRMKVSNVSCQASVSRMHAAFGGT

161 FKKVYDFLSTFITSGMRFLLNQQICPVLYHAGTVLLNSLL
DTVPVRSSVDELVGIDYSLMKDPVASTSNLDMDFRGAFFP

241 LTERNWSLPNRAVEPQLQEEERMVYVAFSEFFFDSAMES
YFRAGALQLLLVGDKVPHDLDMLLRATYFGSIVLLSPAVID

321 SPLKLELRVLAPPRCTIKPSGTTISVTASVTIALVPPDQPE
VQLSSMTMDARLSAKMALRGKALRTQLDLRRFRIYSNHS

401 ALESLALIPLQAPLKTMLQIGVMPMLNERTWRGVQIPLPE
GINFVHEVVTNHAGFLTIGADLHFAKGLREVIEKNRPADV

481 RASTAPTPSTAAV

5.154 ceruloplasmin precursor [Homo sapiens]

Protein Accession gi|4557485
Mean Expression Ratio 0.879
Median Expression Ratio 0.878
Credible Interval (0.737, 1.05)
Associated Peptides 20
Associated Spectra 27
Coverage 0.232

| A | 2.5 | 50 | 97.5 | Sequence |
|---|---|---|---|---|
| 2 | 0.65 | 0.86 | 1.2 | AEEEHLGILGPQLHADVGDK |
| 2 | 0.6 | 0.82 | 1.1 | ALYLQYTDETFR |
| 1 | 0.65 | 0.9 | 1.2 | DIFTGLIGPMK |
| 2 | 0.65 | 0.87 | 1.2 | DLYSGLIGPLIVCR |
| 1 | 0.63 | 0.86 | 1.2 | DVDKEFYLFPTVFDENESLLLEDNIR |
| 2 | 0.66 | 0.89 | 1.2 | EFYLFPTVFDENESLLLEDNIR |
| 1 | 0.63 | 0.87 | 1.2 | GAYPLSIEPIGVR |
| 2 | 0.65 | 0.86 | 1.2 | GPEEEHLGILGPVIWAEVGDTIR |
| 1 | 0.62 | 0.86 | 1.2 | GVYSSDVFDIFPGTYQTLEMFPR |
| 1 | 0.63 | 0.86 | 1.2 | IYHSHIDAPK |
| 1 | 0.65 | 0.88 | 1.2 | KAEEEHLGILGPQLHADVGDK |
| 1 | 0.65 | 0.88 | 1.2 | LFPTVFDENESLLLEDNIR |
| 1 | 0.63 | 0.86 | 1.2 | MFTTAPDQVDKEDEDFQESNK |
| 1 | 0.67 | 0.9 | 1.2 | MLLATEEQSPGEGDGNCVTR |
| 1 | 0.64 | 0.87 | 1.2 | SVPPSASHVAPTETFTY |
| 1 | 0.66 | 0.9 | 1.2 | TTIEKPVWLGFLGPIIK |
| 3 | 0.69 | 0.9 | 1.2 | VNKDDEEFIESNK |
| 1 | 0.64 | 0.88 | 1.2 | YLFPTVFDENESLLLEDNIR |
| 1 | 0.64 | 0.88 | 1.2 | VTFHNK |
| 1 | 0.69 | 0.93 | 1.3 | GPEEEHLGILGPVIW |

MKILILGIFLFLCSTPAWAKEKHYYIGIIETTWDYASDHGEK
KLISVDTEHSNIYLQNGPDRIGRLYKKALYLQYTDETF

RTTIEKPVWLGFLGPIIKAETGDKVYVHLKNLASRPYTFHS
HGITYYKEHEGAIYPDNTTDFQRADDKVYPGEQYTYMLL

ATEEQSPGEGDGNCVTRIYHSHIDAPKDIASGLIGPLIICK
KDSLDKEKEKHIDREFVVMFSVVDENFSWYLEDNIKTYC

SEPEKVDKDNEDFQESNRMYSVNGYTFGSLPGLSMCAED
RVKWYLFGMGNEVDVHAAFFHGQALTNKNYRIDTINLFPAT

LFDAYMVAQNPGEWMLSCQNLNHLKAGLQAFFQVQECN
KSSSKDNIRGKHVRHYYIAAEEIIWNYAPSGIDIFTKENLTA

PGSDSAVFFEQGTTRIGGSYKKLVYREYTDASFTNRKERG
PEEEHLGILGPVIWAEVGDTIRVTFHNKGAYPLSIEPIGV

481 R F K N N E G T Y Y S P N Y N P Q S R S V P P S A S H V A P T E T F T Y E W T V P K E V G P T N A D P V C L A K M Y Y S A V D P T K D I F T G L I G P M K I C

561 K K G S L H A N G R Q K D V D K E F Y L F P T V F D E N E S L L L E D N I R M F T T A P D Q V D K E D E D F Q E S N K M H S M N G F M Y G N Q P G L T M C K G D

641 S V V W Y L F S A G N E A D V H G I Y F S G N T Y L W R G E R R D T A N L F P Q T S L T L H M W P D T E G T F N V E C L T T D H Y T G G M K Q K Y T V N Q C R R

721 Q S E D S T F Y L G E R T Y Y I A A V E V E W D Y S P Q R E W E K E L H H L Q E Q N V S N A F L D K G E F Y I G S K Y K K V V Y R Q Y T D S T F R V P V E R K A

801 E E E H L G I L G P Q L H A D V G D K V K I I F K N M A T R P Y S I H A H G V Q T E S S T V T P T L P G E T L T Y V W K I P E R S G A G T E D S A C I P W A Y Y

881 S T V D Q V K D L Y S G L I G P L I V C R R P Y L K V F N P R R K L E F A L L F L V F D E N E S W Y L D D N I K T Y S D H P E K V N K D D E E F I E S N K M H A

961 I N G R M F G N L Q G L T M H V G D E V N W Y L M G M G N E I D L H T V H F H G H S F Q Y K H R G V Y S S D V F D I F P G T Y Q T L E M F P R T P G I W L L H C

1041 H V T D H I H A G M E T T Y T V L Q N E D T K S G

5.155 glycerophosphodiester phosphodiesterase domain containing 3 [Homo sapiens]

Protein Accession gi|146198640
Mean Expression Ratio 0.88
Median Expression Ratio 0.88
Credible Interval (0.617, 1.25)
Associated Peptides 4
Associated Spectra 4
Coverage 0.186

| A | 2.5 | 50 | 97.5 | Sequence |
|---|-----|-----|------|----------|
| 1 | 0.56 | 0.85 | 1.3 | GGSGELLENTMEAMENSMAQR |
| 1 | 0.58 | 0.88 | 1.3 | SDLLELDCQLTR |
| 1 | 0.57 | 0.87 | 1.3 | DVGSLDFEDLPLYK |
| 1 | 0.58 | 0.9 | 1.4 | VVVVSHDENLCR |

1 M S L L L Y Y A L P A L G S Y A M L S I F F L R R P H L L H T P R A P T F R I R L G A H R G G S G E L L E N T M E A M E N S M A Q R S D L L E L D C Q L T R D R

81 V V V V S H D E N L C R Q S G L N R D V G S L D F E D L P L Y K E K L E V Y F S P G H F A H G S D R R M V R L E D L F Q R F P R T P M S V E I K G K N E E L I R

161 E I A G L V R R Y D R N E I T I W A S E K S S V M K K C K A A N P E M P L S F T I S R G F W V L L S Y Y L G L L P F I P I P E K F F F C F L P N I I N R T Y F P

241 F S C S C L N Q L L A V V S K W L I M R K S L I R H L E E R G V Q V V F W C L N E E S D F E A A F S V G A T G V I T D Y P T A L R H Y L D N H G P A A R T S

5.156 glucuronidase, beta [Homo sapiens]

Protein Accession gi|4504223
Mean Expression Ratio 1.14
Median Expression Ratio 1.14
Credible Interval (0.797, 1.61)
Associated Peptides 4
Associated Spectra 4
Coverage 0.0707

| A | 2.5 | 50 | 97.5 | Sequence |
|---|-----|----|------|----------|
| 1 | 0.74 | 1.1 | 1.7 | GFDWPLLVK |
| 1 | 0.76 | 1.1 | 1.7 | NFADFMTEQSPTR |
| 1 | 0.76 | 1.2 | 1.7 | YVVGELIWNFADFMTEQSPTR |
| 1 | 0.75 | 1.1 | 1.7 | TSHYPYAEEVMQMCDR |

1 M A R G S A V A W A A L G P L L W G C A L G L Q G G M L Y P Q E S P S R E C K E L D G L W S F R A D F S D N R R R G F E E Q W Y R R P L W E S G P T V D M P V P

81 S S F N D I S Q D W R L R H F V G W V W Y E R E V I L P E R W T Q D L R T R V V L R I G S A H S Y A I V W V N G V D T L E H E G G Y L P F E A D I S N L V Q V G

161 P L P S R L R I T I A I N N T L T P T T L P P G T I Q Y L T D T S K Y P K G Y F V Q N T Y F D F F N Y A G L Q R S V L L Y T T P T T Y I D D I T V T T S V E Q D

241 S G L V N Y Q I S V K G S N L F K L E V R L L D A E N K V V A N G T G T Q G Q L K V P G V S L W W P Y L M H E R P A Y L Y S L E V Q L T A Q T S L G P V S D F Y

321 T L P V G I R T V A V T K S Q F L I N G K P F Y F H G V N K H E D A D I R G K G F D W P L L V K D F N L L R W L G A N A F R T S H Y P Y A E E V M Q M C D R Y G

401 I V V I D E C P G V G L A L P Q F F N N V S L H H H M Q V M E E V V R R D K N H P A V V M W S V A N E P A S H L E S A G Y Y L K M V I A H T K S L D P S R P V T

481 F V S N S N Y A A D K G A P Y V D V I C L N S Y Y S W Y H D Y G H L E L I Q L
Q L A T Q F E N W Y K K Y Q K P I I Q S E Y G A E T I A G F H Q D P P L M F T E E

561 Y Q K S L L E Q Y H L G L D Q K R R K Y V V G E L I W N F A D F M T E Q S P T
R V L G N K K G I F T R Q R Q P K S A A F L L R E R Y W K I A N E T R Y P H S V A

641 K S Q C L E N S P F T

5.157 solute carrier family 4, anion exchanger, member 1 [Homo sapiens]

Protein Accession gi|4507021
Mean Expression Ratio 1.13
Median Expression Ratio 1.14
Credible Interval (0.734, 1.73)
Associated Peptides 2
Associated Spectra 2
Coverage 0.0329

| A | 2.5 | 50 | 97.5 | Sequence |
|---|-----|----|------|----------|
| 1 | 0.72 | 1.1 | 1.8 | ADFLEQPVLGFVR |
| 1 | 0.73 | 1.2 | 1.8 | LQEAAELEAVELPVPIR |

1 M E E L Q D D Y E D M M E E N L E Q E E Y E D P D I P E S Q M E E P A A H D T E
A T A T D Y H T T S H P G T H K V Y V E L Q E L V M D E K N Q E L R W M E A A R

81 W V Q L E E N L G E N G A W G R P H L S H L T F W S L L E L R R V F T K G T V
L L D L Q E T S L A G V A N Q L L D R F I F E D Q I R P Q D R E E L L R A L L L K

161 H S H A G E L E A L G G V K P A V L T R S G D P S Q P L L P Q H S S L E T Q L F
C E Q G D G G T E G H S P S G I L E K I P P D S E A T L V L V G R A D F L E Q P

241 V L G F V R L Q E A A E L E A V E L P V P I R F L F V L L G P E A P H I D Y T Q
L G R A A A T L M S E R V F R I D A Y M A Q S R G E L L H S L E G F L D C S L V

321 L P P T D A P S E Q A L L S L V P V Q R E L L R R R Y Q S S P A K P D S S F Y K
G L D L N G G P D D P L Q Q T G Q L F G G L V R D I R R R Y P Y Y L S D I T D A

401 F S P Q V L A A V I F I Y F A A L S P A I T F G G L L G E K T R N Q M G V S E L
L I S T A V Q G I L F A L L G A Q P L L V V G F S G P L L V F E E A F F S F C E

481 T N G L E Y I V G R V W I G F W L I L L V V L V V A F E G S F L V R F I S R Y T
Q E I F S F L I S L I F I Y E T F S K L I K I F Q D H P L Q K T Y N Y N V L M V

561 P K P Q G P L P N T A L L S L V L M A G T F F F A M M L R K F K N S S Y F P G K L R R V I G D F G V P I S I L I M V L V D F F I Q D T Y T Q K L S V P D G F K V

641 S N S S A R G W V I H P L G L R S E F P I W M M F A S A L P A L L V F I L I F L E S Q I T T L I V S K P E R K M V K G S G F H L D L L L V V G M G G V A A L F G

721 M P W L S A T T V R S V T H A N A L T V M G K A S T P G A A A Q I Q E V K E Q R I S G L L V A V L V G L S I L M E P I L S R I P L A V L F G I F L Y M G V T S L

801 S G I Q L F D R I L L L F K P P K Y H P D V P Y V K R V K T W R M H L F T G I Q I I C L A V L W V V K S T P A S L A L P F V L I L T V P L R R V L L P L I F R N

881 V E L Q C L D A D D A K A T F D E E G R D E Y D E V A M P V

5.158 arylsulfatase A isoform a precursor [Homo sapiens]; arylsulfatase A isoform a precursor [Homo sapiens]; arylsulfatase A isoform a precursor [Homo sapiens]; arylsulfatase A isoform b [Homo sapiens]; arylsulfatase A isoform a precursor [Homo sapiens]

Protein Accession gi|6005990 gi|146229331 gi|146229329 gi|146229327 gi|146229324
Mean Expression Ratio 1.14
Median Expression Ratio 1.14
Credible Interval (0.749, 1.77)
Associated Peptides 2
Associated Spectra 2
Coverage NaN

| A | 2.5 | 50 | 97.5 | Sequence |
|---|-----|----|------|----------|
| 1 | 0.72 | 1.1 | 1.8 | GGLPLEEVTVAEVLAAR |
| 1 | 0.73 | 1.2 | 1.9 | YMAFAHDLMADAQR |

5.159 guanine nucleotide binding protein (G protein) alpha 12 [Homo sapiens]

Protein Accession gi|42476111
Mean Expression Ratio 1.14
Median Expression Ratio 1.14
Credible Interval (0.701, 1.86)
Associated Peptides 1
Associated Spectra 1
Coverage 0.0341

| A | 2.5 | 50 | 97.5 | Sequence |
|---|-----|----|------|----------|
| 1 | 0.7 | 1.2 | 2 | ATKGIVEHDFVIK |

MSGVVRTLSRCLLPAEAGGARERRAGSGARDAEREARRRS
RDIDALLARERRAVRRLVKILLLGAGESGKSTFLKQMRII

HGREFDQKALLEFRDTIFDNILKGSRVLVDARDKLGIPWQ
YSENEKHGMFLMAFENKAGLPVEPATFQLYVPALSALWRD

SGIREAFSRRSEFQLGESVKYFLDNLDRIGQLNYFPSKQDI
LLARKATKGIVEHDFVIKKIPFKMVDVGGQRSQRQKWFQ

CFDGITSILFMVSSSEYDQVLMEDRRTNRLVESMNIFETI
VNNKLFFNVSIILFLNKMDLLVEKVKTVSIKKHFPDFRGD

PHRLEDVQRYLVQCFDRKRRNRSKPLFHHFTTAIDTENV
RFVFHAVKDTILQENLKDIMLQ

5.160 solute carrier family 1, member 1 [Homo sapiens]

Protein Accession gi|66773030
Mean Expression Ratio 0.882
Median Expression Ratio 0.881
Credible Interval (0.541, 1.44)
Associated Peptides 1
Associated Spectra 1
Coverage 0.0344

| A | 2.5 | 50 | 97.5 | Sequence |
|---|-----|-----|------|----------|
| 1 | 0.51 | 0.86 | 1.4 | TGSTPEVSTVDAMLDLIR |

MGKPARKGCEWKRFLKNNWVLLSTVAAVVLGITTGVLVR
EHSNLSTLEKFYFAFPGEILMRMLKLIILPLIISSMITGVA

ALDSNVSGKIGLRAVVYYFCTTLIAVILGIVLVVSIKPGVT
QKVGEIARTGSTPEVSTVDAMLDLIRNMFPENLVQACFQ

QYKTKREEVKPPSDPEMNMTEESFTAVMTTAISKNKTKE
YKIVGMYSDGINVLGLIVFCLVFGLVIGKMGEKGQILVDFF

NALSDATMKIVQIIMCYMPLGILFLIAGKIIEVEDWEIFRK
LGLYMATVLTGLAIHSIVILPLIYFIVVRKNPFRFAMGM

AQALLTALMISSSSATLPVTFRCAEENNQVDKRITRFVLP
VGATINMDGTALYEAVAAVFIAQLNDLDLGIGQIITISIT

401 A T S A S I G A A G V P Q A G L V T M V I V L S A V G L P A E D V T L I I A V D W L L D R F R T M V N V L G D A F G T G I V E K L S K K E L E Q M D V S S E V N

481 I V N P F A L E S T I L D N E D S D T K K S Y V N G G F A V D K S D T I S F T Q T S Q F

5.161 keratin 9 [Homo sapiens]

Protein Accession gi|55956899
Mean Expression Ratio 0.881
Median Expression Ratio 0.882
Credible Interval (0.578, 1.34)
Associated Peptides 2
Associated Spectra 2
Coverage 0.0642

| A | 2.5 | 50 | 97.5 | Sequence |
|---|-----|----|------|----------|
| 1 | 0.54 | 0.86 | 1.4 | HGVQELEIELQSQLSK |
| 1 | 0.55 | 0.87 | 1.4 | SYGGGSGGGFSASSLGGGFGGGSR |

1 M S C R Q F S S S Y L S R S G G G G G G G G G L G S G G S I R S S Y S R F S S S G G G G G G G R F S S S S G Y G G G S S R V C G R G G G G G S F G Y S Y G G G S G G G

81 F S A S S L G G G F G G G S R G F G G A S G G G Y S S S G G F G G G F G G G S G G G F G G G Y G S G F G G F G G F G G G A G G G D G G I L T A N E K S T M Q E L

161 N S R L A S Y L D K V Q A L E E A N N D L E N K I Q D W Y D K K G P A A I Q K N Y S P Y Y N T I D D L K D Q I V D L T V G N N K T L L D I D N T R M T L D D F R

241 I K F E M E Q N L R Q G V D A D I N G L R Q V L D N L T M E K S D L E M Q Y E T L Q E E L M A L K K N H K E E M S Q L T G Q N S G D V N V E I N V A P G K D L T

321 K T L N D M R Q E Y E Q L I A K N R K D I E N Q Y E T Q I T Q I E H E V S S S G Q E V Q S S A K E V T Q L R H G V Q E L E I E L Q S Q L S K K A A L E K S L E D

401 T K N R Y C G Q L Q M I Q E Q I S N L E A Q I T D V R Q E I E C Q N Q E Y S L L L S I K M R L E K E I E T Y H N L L E G G Q E D F E S S G A G K I G L G G R G G

481 S G G S Y G R G S R G G S G G S Y G G G G S G G G Y G G G S G S R G G S G G S Y G G G S G S G G G S G G G Y G G G S G G G H S G G S G G G H S G G S G G N Y G G

561 G S G S G G G S G G G Y G G G S G S R G G S G G S H G G G S G F G G E S G G S Y G G G E E A S G S G G G Y G G G G S G K S S H S

5.162 pyruvate kinase, muscle isoform M2 [Homo sapiens]

Protein Accession gi|33286418
Mean Expression Ratio 1.13
Median Expression Ratio 1.13
Credible Interval (0.83, 1.57)
Associated Peptides 4
Associated Spectra 6
Coverage 0.0923

| A | 2.5 | 50 | 97.5 | Sequence |
|---|---|---|---|---|
| 1 | 0.78 | 1.1 | 1.7 | DPVQEAWAEDVDLR |
| 1 | 0.78 | 1.2 | 1.7 | FDEILEASDGIMVAR |
| 3 | 0.8 | 1.1 | 1.6 | HLQLFEELR |
| 1 | 0.76 | 1.1 | 1.7 | LDIDSPPITAR |

1 M S K P H S E A G T A F I Q T Q Q L H A A M A D T F L E H M C R L D I D S P P I T A R N T G I I C T I G P A S R S V E T L K E M I K S G M N V A R L N F S H G T

81 H E Y H A E T I K N V R T A T E S F A S D P I L Y R P V A V A L D T K G P E I R T G L I K G S G T A E V E L K K G A T L K I T L D N A Y M E K C D E N I L W L D

161 Y K N I C K V V E V G S K I Y V D D G L I S L Q V K Q K G A D F L V T E V E N G G S L G S K K G V N L P G A A V D L P A V S E K D I Q D L K F G V E Q D V D M V

241 F A S F I R K A S D V H E V R K V L G E K G K N I K I I S K I E N H E G V R R F D E I L E A S D G I M V A R G D L G I E I P A E K V F L A Q K M M I G R C N R A

321 G K P V I C A T Q M L E S M I K K P R P T R A E G S D V A N A V L D G A D C I M L S G E T A K G D Y P L E A V R M Q H L I A R E A E A A I Y H L Q L F E E L R R

401 L A P I T S D P T E A T A V G A V E A S F K C C S G A I I V L T K S G R S A H Q V A R Y R P R A P I I A V T R N P Q T A R Q A H L Y R G I F P V L C K D P V Q E

481 A W A E D V D L R V N F A M N V G K A R G F F K K G D V V I V L T G W R P G S G F T N T M R V V P V P

5.163 solute carrier family 13 member 3 isoform b [Homo sapiens]; solute carrier family 13 member 3 isoform a [Homo sapiens]

Protein Accession gi|58761541 gi|31377715
Mean Expression Ratio 0.884
Median Expression Ratio 0.883
Credible Interval (0.572, 1.37)
Associated Peptides 1
Associated Spectra 2
Coverage NaN

| A | 2.5 | 50 | 97.5 | Sequence |
|---|-----|------|------|-----------------|
| 2 | 0.55 | 0.86 | 1.3 | DHPGETEVPLDLPADSR |

5.164 GTPase Rab14 [Homo sapiens]

Protein Accession gi|19923483
Mean Expression Ratio 0.887
Median Expression Ratio 0.883
Credible Interval (0.541, 1.46)
Associated Peptides 1
Associated Spectra 1
Coverage 0.140

| A | 2.5 | 50 | 97.5 | Sequence |
|---|-----|------|------|--------------------------------|
| 1 | 0.51 | 0.86 | 1.5 | IYQNIQDGSLDLNAAESGVQHKPSAPQGGR |

1 M A T A P Y N Y S Y I F K Y I I I G D M G V G K S C L L H Q F T E K K F M A D C P H T I G V E F G T R I I E V S G Q K I K L Q I W D T A G Q E R F R A V T R S Y

81 Y R G A A G A L M V Y D I T R R S T Y N H L S S W L T D A R N L T N P N T V I I L I G N K A D L E A Q R D V T Y E E A K Q F A E E N G L L F L E A S A K T G E N

161 V E D A F L E A A K K I Y Q N I Q D G S L D L N A A E S G V Q H K P S A P Q G G R L T S E P Q P Q R E G C G C

5.165 coagulation factor II preproprotein [Homo sapiens]

Protein Accession gi|4503635
Mean Expression Ratio 0.887
Median Expression Ratio 0.885
Credible Interval (0.613, 1.30)
Associated Peptides 3
Associated Spectra 3

Coverage 0.0836

| A | 2.5 | 50 | 97.5 | Sequence |
|---|-----|-----|------|----------|
| 1 | 0.57 | 0.88 | 1.4 | HQDFNSAVQLVENFCR |
| 1 | 0.57 | 0.87 | 1.3 | SEGSSVNLSPPLEQCVPDR |
| 1 | 0.58 | 0.88 | 1.4 | TFGSGEADCGLRPLFEK |

1 M A H V R G L Q L P G C L A L A A L C S L V H S Q H V F L A P Q Q A R S L L Q R V R R A N T F L E E V R K G N L E R E C V E E T C S Y E E A F E A L E S S T A T

81 D V F W A K Y T A C E T A R T P R D K L A A C L E G N C A E G L G T N Y R G H V N I T R S G I E C Q L W R S R Y P H K P E I N S T T H P G A D L Q E N F C R N P

161 D S S T T G P W C Y T T D P T V R R Q E C S I P V C G Q D Q V T V A M T P R S E G S S V N L S P P L E Q C V P D R G Q Q Y Q G R L A V T T H G L P C L A W A S A

241 Q A K A L S K H Q D F N S A V Q L V E N F C R N P D G D E E G V W C Y V A G K P G D F G Y C D L N Y C E E A V E E T G D G L D E D S D R A I E G R T A T S E Y

321 Q T F F N P R T F G S G E A D C G L R P L F E K K S L E D K T E R E L L E S Y I D G R I V E G S D A E I G M S P W Q V M L F R K S P Q E L L C G A S L I S D R W

401 V L T A A H C L L Y P P W D K N F T E N D L L V R I G K H S R T R Y E R N I E K I S M L E K I Y I H P R Y N W R E N L D R D I A L M K L K K P V A F S D Y I H P

481 V C L P D R E T A A S L L Q A G Y K G R V T G W G N L K E T W T A N V G K G Q P S V L Q V V N L P I V E R P V C K D S T R I R I T D N M F C A G Y K P D E G K R

561 G D A C E G D S G G P F V M K S P F N N R W Y Q M G I V S W G E G C D R D G K Y G F Y T H V F R L K K W I Q K V I D Q F G E

5.166 proteasome alpha 6 subunit [Homo sapiens]

Protein Accession gi|23110944
Mean Expression Ratio 1.13
Median Expression Ratio 1.13
Credible Interval (0.738, 1.76)
Associated Peptides 2
Associated Spectra 2
Coverage 0.122

| A | 2.5 | 50 | 97.5 | Sequence |
|---|-----|-----|------|----------|
| 1 | 0.72 | 1.2 | 1.9 | AINQGGLTSVAVR |
| 1 | 0.71 | 1.1 | 1.8 | ILTEAEIDAHLVALAER |

1 MSRGSSAGFDRHITIFSPEGRLYQVEYAFKAINQGGLTSVA VRGKDCAVIVTQKKVPDKLLDSSTVTHLFKITENIGCVM

81 TGMTADSRSQVQRARYEAANWKYKYGYEIPVDMLCKRIA DISQVYTQNAEMRPLGCCMILIGIDEEQGPQVYKCDPAGYY

161 CGFKATAAGVKQTESTSFLEKKVKKKFDWTFEQTVETAI TCLSTVLSIDFKPSEIEVGVVTVENPKFRILTEAEIDAHLV

241 ALAERD

5.167 tubulin, beta, 2 [Homo sapiens]

Protein Accession gi|5174735

Mean Expression Ratio 1.13

Median Expression Ratio 1.13

Credible Interval (0.767, 1.67)

Associated Peptides 2

Associated Spectra 3

Coverage 0.0742

| A | 2.5 | 50 | 97.5 | Sequence |
|---|-----|-----|------|----------|
| 2 | 0.78 | 1.2 | 1.8 | GHYTEGAELVDSVLDVVR |
| 1 | 0.72 | 1.1 | 1.7 | AVLVDLEPGTMDSVR |

1 MREIVHLQAGQCGNQIGAKFWEVISDEHGIDPTGTYHGDS DLQLERINVYYNEATGGKYVPRAVLVDLEPGTMDSVRSGP

81 FGQIFRPDNFVFGQSGAGNNWAKGHYTEGAELVDSVLDV VRKEAESCDCLQGFQLTHSLGGGTGSGMGTLLISKIREEYP

161 DRIMNTFSVVPSPKVSDTVVEPYNATLSVHQLVENTDET YCIDNEALYDICFRTLKLTTPTYGDLNHLVSATMSGVTTCL

241 R F P G Q L N A D L R K L A V N M V P F P R L H F F M P G F A P L T S R G S Q Q Y R A L T V P E L T Q Q M F D A K N M M A A C D P R H G R Y L T V A A V F R G R

321 M S M K E V D E Q M L N V Q N K N S S Y F V E W I P N N V K T A V C D I P P R G L K M S A T F I G N S T A I Q E L F K R I S E Q F T A M F R R K A F L H W Y T G

401 E G M D E M E F T E A E S N M N D L V S E Y Q Q Y Q D A T A E E E G E F E E E A E E E V A

5.168 solute carrier family 6, member 19 [Homo sapiens]

Protein Accession gi|51468073

Mean Expression Ratio 0.887

Median Expression Ratio 0.887

Credible Interval (0.638, 1.24)

Associated Peptides 4

Associated Spectra 5

Coverage 0.0489

| A | 2.5 | 50 | 97.5 | Sequence |
|---|-----|-----|------|----------|
| 1 | 0.58 | 0.87 | 1.3 | IPSLAELETIEQEEAS SR |
| 2 | 0.62 | 0.9 | 1.3 | IPSLAELETIEQEEAS SRPK |
| 1 | 0.6 | 0.9 | 1.4 | LVLPNPGLDAR |
| 1 | 0.57 | 0.85 | 1.3 | VLPNPGLDAR |

1 M V R L V L P N P G L D A R I P S L A E L E T I E Q E E A S S R P K W D N K A Q Y M L T C L G F C V G L G N V W R F P Y L C Q S H G G G A F M I P F L I L L V L

81 E G I P L L Y L E F A I G Q R L R R G S L G V W S S I H P A L K G L G L A S M L T S F M V G L Y Y N T I I S W I M W Y L F N S F Q E P L P W S D C P L N E N Q T

161 G Y V D E C A R S S P V D Y F W Y R E T L N I S T S I S D S G S I Q W W M L L C L A C A W S V L Y M C T I R G I E T T G K A V Y I T S T L P Y V V L T I F L I R

241 G L T L K G A T N G I V F L F T P N V T E L A Q P D T W L D A G A Q V F F S F S L A F G G L I S F S S Y N S V H N N C E K D S V I V S I I N G F T S V Y V A I V

321 V Y S V I G F R A T Q R Y D D C F S T N I L T L I N G F D L P E G N V T Q E N F V D M Q Q R C N A S D P A A Y A Q L V F Q T C D I N A F L S E A V E G T G L A F

401 I V F T E A I T K M P L S P L W S V L F F I M L F C L G L S S M F G N M E G V V V P L Q D L R V I P P K W P K E V L T G L I C L G T F L I G F I F T L N S G Q Y

481 W L S L L D S Y A G S I P L L I I A F C E M F S V V Y V Y G V D R F N K D I E F M I G H K P N I F W Q V T W R V V S P L L M L I I F L F F F V V E V S Q E L T Y

561 S I W D P G Y E E F P K S Q K I S Y P N W V Y V V V V I V A G V P S L T I P G Y A I Y K L I R N H C Q K P G D H Q G L V S T L S T A S M N G D L K Y

5.169 decay accelerating factor for complement isoform 2 precursor [Homo sapiens]

Protein Accession gi|168693643
Mean Expression Ratio 1.13
Median Expression Ratio 1.13
Credible Interval (0.736, 1.71)
Associated Peptides 2
Associated Spectra 2
Coverage NaN

| A | 2.5 | 50 | 97.5 | Sequence |
|---|---|---|---|---|
| 1 | 0.7 | 1.2 | 1.8 | EIYCPAPPQIDNGIIQGER |
| 1 | 0.72 | 1.1 | 1.8 | DCGLPPDVPNAQPALEGR |

5.170 membrane alanine aminopeptidase precursor [Homo sapiens]

Protein Accession gi|157266300
Mean Expression Ratio 1.13
Median Expression Ratio 1.13
Credible Interval (1.02, 1.25)
Associated Peptides 54
Associated Spectra 111
Coverage 0.483

| A | 2.5 | 50 | 97.5 | Sequence |
|---|---|---|---|---|
| 1 | 0.87 | 1.1 | 1.5 | AIAQGGEEEWDFAWEQFR |
| 8 | 1.0 | 1.3 | 1.6 | AQIINDAFNLASAHK |
| 1 | 0.81 | 1.1 | 1.4 | ASSHPLSTPASEINTPAQISELFDAISY |
| 5 | 0.88 | 1.1 | 1.4 | CNAIAQGGEEEWDFAWEQFR |
| 1 | 0.85 | 1.1 | 1.5 | DFAWEQFR |
| 1 | 0.82 | 1.1 | 1.4 | DFVQSNWK |

(continued)

| A | 2.5 | 50 | 97.5 | Sequence |
|---|---|---|---|---|
| 3 | 0.87 | 1.1 | 1.5 | DHSAIPVINR |
| 2 | 0.87 | 1.2 | 1.5 | DLMVLNDVYR |
| 1 | 0.86 | 1.1 | 1.5 | DLTALSNMLPK |
| 1 | 0.82 | 1.1 | 1.5 | ELQQLEQFK |
| 1 | 0.83 | 1.1 | 1.5 | ELWILNR |
| 1 | 0.86 | 1.1 | 1.5 | ENSLLFDPLSSSSSNK |
| 2 | 0.88 | 1.2 | 1.5 | EVVLQWFTENSK |
| 2 | 0.82 | 1.1 | 1.4 | FFAGHYDTPYPLPK |
| 1 | 0.86 | 1.1 | 1.5 | FSTEYELQQLEQFK |
| 2 | 0.84 | 1.1 | 1.5 | FSTEYELQQLEQFKK |
| 1 | 0.84 | 1.1 | 1.5 | GVGGSQPPDIDKTELVEPTEY |
| 1 | 0.88 | 1.2 | 1.6 | GVGGSQPPDIDKTELVEPTEYLVVHLK |
| 1 | 0.84 | 1.1 | 1.5 | IVSEFDYVEK |
| 1 | 0.86 | 1.2 | 1.6 | KIQTQLQR |
| 1 | 0.86 | 1.2 | 1.5 | KLFNDYGGGSFSFSNLIQAVTR |
| 1 | 0.84 | 1.1 | 1.5 | KQVTPLFIHFR |
| 5 | 0.9 | 1.1 | 1.4 | KVVATTQMQAADAR |
| 4 | 0.87 | 1.1 | 1.4 | LFNDYGGGSFSFSNLIQAVTR |
| 1 | 0.86 | 1.1 | 1.5 | LLAFIVSEFDYVEK |
| 1 | 0.85 | 1.1 | 1.5 | LPNTLKPDSYR |
| 2 | 0.86 | 1.1 | 1.5 | MLSSFLSEDVFK |
| 1 | 0.85 | 1.1 | 1.5 | NAIAQGGEEEWDFAWEQFR |
| 2 | 0.84 | 1.1 | 1.5 | NYVWIVPITSIR |
| 2 | 0.88 | 1.2 | 1.5 | PSEFNYVWIVPITSIR |
| 2 | 0.89 | 1.2 | 1.5 | QQQDYWLIDVR |
| 2 | 0.79 | 1.0 | 1.4 | QVTPLFIHFR |
| 1 | 0.83 | 1.1 | 1.5 | QWMENPNNNPIHPN |
| 10 | 0.82 | 1 | 1.2 | QWMENPNNNPIHPNLR |
| 4 | 0.96 | 1.2 | 1.6 | QYMPWEAALSSLSYFK |
| 2 | 0.85 | 1.1 | 1.5 | RFSTEYELQQLEQFK |
| 4 | 1.0 | 1.3 | 1.7 | SDQIGLPDFNAGAMENWGLVTYR |
| 1 | 0.86 | 1.1 | 1.5 | SFPCFDEPAMK |
| 2 | 0.81 | 1.1 | 1.4 | SFSNLIQAVTR |
| 1 | 0.84 | 1.1 | 1.5 | SIQLPTTVR |
| 3 | 0.82 | 1.1 | 1.4 | STVYCNAIAQGGEEEWDFAWEQFR |
| 1 | 0.85 | 1.1 | 1.5 | VKDSQYEMDSEFEGELADDLAGFYR |
| 1 | 0.85 | 1.1 | 1.5 | VNYDEENWR |
| 1 | 0.85 | 1.1 | 1.5 | VVATTQMQAADAR |

(continued)

| A | 2.5 | 50 | 97.5 | Sequence |
|---|-----|----|------|----------|
| 1 | 0.81 | 1.1 | 1.4 | VWIVPITSIR |
| 1 | 0.85 | 1.1 | 1.5 | YLSYTLNPDLIR |
| 8 | 0.91 | 1.1 | 1.4 | DSQYEMDSEFEGELADDLAGFYR |
| 1 | 0.85 | 1.1 | 1.5 | AGHYDTPYPLPK |
| 1 | 0.84 | 1.1 | 1.5 | EATDVIIIHSK |
| 3 | 0.87 | 1.1 | 1.5 | LVVHLK |
| 1 | 0.86 | 1.1 | 1.5 | RF STEYELQQLEQFKK |
| 1 | 0.84 | 1.1 | 1.5 | YVWIVPITSIR |
| 1 | 0.85 | 1.1 | 1.5 | GFYISK |
| 1 | 0.87 | 1.2 | 1.5 | KDNEETGFGSGTR |

1 MAKGFYISKSLGILGILLGVAAVCTIIALSVVYSQEKNKNA NSSPVASTTPSASATTNPASATTLDQSKAWNRYRLPNTL

81 KPDSYRVTLRPYLTPNDRGLYVFKGSSTVRFTCKEATDVII IHSKKLNYTLSQGHRVVLRGVGGSQPPDIDKTELVEPTE

161 YLVVHLKGSLVKDSQYEMDSEFEGELADDLAGFYRSEYM EGNVRKVVATTQMQAADARKSFPCFDEPAMKAEFNITLIHP

241 KDLTALSNMLPKGPSTPLPEDPNWNVTEFHTTPKMSTYL LAFIVSEFDYVEKQASNGVLIRIWARPSAIAAGHGDYALNV

321 TGPILNFFAGHYDTPYPLPKSDQIGLPDFNAGAMENWGL VTYRENSLLFDPLSSSSSNKERVVTVIAHELAHQWFGNLVT

401 IEWWNDLWLNEGFASYVEYLGADYAEPTWNLKDLMVLN DVYRVMAVDALASSHPLSTPASEINTPAQISELFDAISYSKG

481 ASVLRMLSSFLSEDVFKQGLASYLHTFAYQNTIYLNLWD HLQEAVNNRSIQLPTTVRDIMNRWTLQMGFPVITVDTSTGT

561 LSQEHFLLDPDSNVTRPSEFNYVWIVPITSIRDGRQQQDY WLIDVRAQNDLFSTSGNEWVLLNLNVTGYYRVNYDEENWR

641 KIQTQLQRDHSAIPVINRAQIINDAFNLASAHKVPVTLAL NNTLFLIEERQYMPWEAALSSLSYFKLMFDRSEVYGPMKN

721 YLKKQVTPLFIHFRNNTNNWREIPENLMDQYSEVNAISTA CSNGVPECEEMVSGLFKQWMENPNNNPIHPNLRSTVYCNA

801 I A Q G G E E E W D F A W E Q F R N A T L V N E A D K L R A A L A C S K E L W I L N R Y L S Y T L N P D L I R K Q D A T S T I I S I T N N V I G Q G L V W D F V

881 Q S N W K K L F N D Y G G G S F S F S N L I Q A V T R R F S T E Y E L Q Q L E Q F K K D N E E T G F G S G T R A L E Q A L E K T K A N I K W V K E N K E V V L Q

961 WF TEN SK

5.171 transmembrane protein 9 [Homo sapiens]

Protein Accession gi|7705999
Mean Expression Ratio 0.888
Median Expression Ratio 0.888
Credible Interval (0.541, 1.42)
Associated Peptides 1
Associated Spectra 1
Coverage 0.104

| A | 2.5 | 50 | 97.5 | Sequence |
|---|-----|-----|------|----------|
| 1 | 0.52 | 0.87 | 1.4 | KPDAYTEQLHNEEENEDAR |

1 M K L L S L V A V V G C L L V P P A E A N K S S E D I R C K C I C P P Y R N I S G H I Y N Q N V S Q K D C N C L H V V E P M P V P G H D V E A Y C L L C E C R Y

81 E E R S T T T I K V I I V I Y L S V V G A L L L Y M A F L M L V D P L I R K P D A Y T E Q L H N E E E N E D A R S M A A A A A S L G G P R A N T V L E R V E G A

161 QQRWKLQVQEQRKTVFDRHKML S

5.172 toll interacting protein [Homo sapiens]

Protein Accession gi|21361619
Mean Expression Ratio 1.13
Median Expression Ratio 1.12
Credible Interval (0.772, 1.68)
Associated Peptides 3
Associated Spectra 3
Coverage 0.106

| A | 2.5 | 50 | 97.5 | Sequence |
|---|-----|-----|------|----------|
| 1 | 0.72 | 1.1 | 1.7 | AIQDMFPNMDQEVIR |
| 1 | 0.78 | 1.2 | 1.9 | GPVYIGELPQDFLR |
| 1 | 0.72 | 1.1 | 1.7 | IGELPQDFLR |

MATTVSTQRGPVYIGELPQDFLRITPTQQQRQVQLDAQAAQQLQYGGAVGTVGRLNITVVQAKLAKNYGMTRMDPYCRLR

LGYAVYETPTAHNGAKNPRWNKVIHCTVPPGVDSFYLEIFDERAFSMDDRIAWTHITIPESLRQGKVEDKWYSLSGRQGD

DKEGMINLVMSYALLPAAMVMPPQPVVLMPTVYQQGVGYVPITGMPAVCSPGMVPVALPPAAVNAQPRCSEEDLKAIQDM

FPNMDQEVIRSVLEAQRGNKDAAINSLLQMGEEP

5.173 arylsulfatase F precursor [Homo sapiens]

Protein Accession gi|31742482
Mean Expression Ratio 0.893
Median Expression Ratio 0.89
Credible Interval (0.584, 1.38)
Associated Peptides 2
Associated Spectra 2
Coverage 0.0593

| A | 2.5 | 50 | 97.5 | Sequence |
|---|-----|------|------|----------|
| 1 | 0.57 | 0.9 | 1.5 | ILDAIDDFGLR |
| 1 | 0.53 | 0.85 | 1.4 | EPTSLMDILPTVASVSGGSLPQDR |

MRPRRPLVFMSLVCALLNTCQAHRVHDDKPNIVLIMVDDLGIGDLGCYGNDTMRTPHIDRLAREGVRLTQHISAASLCSP

SRSAFLTGRYPIRSGMVSSGNRRVIQNLAVPAGLPLNETTLAALLKKQGYSTGLIGKWHQGLNCDSRSDQCHHPYNYGFD

YYYGMPFTLVDSCWPDPSRNTELAFESQLWLCVQLVAIAILTLTFGKLSGWVSVPWLLIFSMILFIFLLGYAWFSSHTSP

LYWDCLLMRGHEITEQPMKAERAGSIMVKEAISFLERHSKETFLLFFSFLHVHTPLPTTDDFTGTSKHGLYGDNVEEMDS

MVGKILDAIDDFGLRNNTLVYFTSDHGGHLEARRGHAQLGGWNGIYKGGKGMGGWEGGIRVPGIVRWPGKVPAGRLIKEP

TSLMDILPTVASVSGGSLPQDRVIDGRDLMPLLQGNVRHSEHEFLFHYCGSYLHAVRWIPKDDSGSVWKAHYVTPVFQPP

481 A S G G C Y V T S L C R C F G E Q V T Y H N P P L L F D L S R D P S E S T P L T P A T E P L H D F V I K K V A N A L K E H Q E T I V P V T Y Q L S E L N Q G R T

561 W L K P C C G V F P F C L C D K E E E V S Q P R G P N E K R

5.174 calcium binding protein 39 [Homo sapiens]; calcium binding protein 39 [Homo sapiens]; calcium binding protein 39 [Homo sapiens]

Protein Accession gi|7706481 gi|195927022 gi|195927020
Mean Expression Ratio 1.12
Median Expression Ratio 1.12
Credible Interval (0.693, 1.81)
Associated Peptides 1
Associated Spectra 1
Coverage NaN

| A | 2.5 | 50 | 97.5 | Sequence |
|---|-----|-----|------|----------|
| 1 | 0.69 | 1.2 | 1.9 | LLSAEFLEQHYDR |

5.175 hemicentin 1 [Homo sapiens]

Protein Accession gi|118572606
Mean Expression Ratio 0.889
Median Expression Ratio 0.89
Credible Interval (0.544, 1.45)
Associated Peptides 1
Associated Spectra 1
Coverage 0.00284

| A | 2.5 | 50 | 97.5 | Sequence |
|---|-----|------|------|----------|
| 1 | 0.51 | 0.86 | 1.4 | LVSLPFGIATNQDLIR |

1 M I S W E V V H T V F L F A L L Y S S L A Q D A S P Q S E I R A E E I P E G A S T L A F V F D V T G S M Y D D L V Q V I E G A S K I L E T S L K R P K R P L F N

81 F A L V P F H D P E I G P V T I T T D P K K F Q Y E L R E L Y V Q G G G D C P E M S I G A I K I A L E I S L P G S F I Y V F T D A R S K D Y R L T H E V L Q L I

161 Q Q K Q S Q V V F V L T G D C D D R T H I G Y K V Y E E I A S T S S G Q V F H L D K K Q V N E V L K W V E E A V Q A S K V H L L S T D H L E Q A V N T W R I P F

241 D P S L K E V T V S L S G P S P M I E I R N P L G K L I K K G F G L H E L L N I H N S A K V V N V K E P E A G M W T V K T S S S G R H S V R I T G L S T I D F R

321 A G F S R K P T L D F K K T V S R P V Q G I P T Y V L L N T S G I S T P A R I D L
L E L L S I S G S S L K T I P V K Y Y P H R K P Y G I W N I S D F V P P N E A

401 F F L K V T G Y D K D D Y L F Q R V S S V S F S S I V P D A P K V T M P E K T P
G Y Y L Q P G Q I P C S V D S L L P F T L S F V R N G V T L G V D Q Y L K E S A

481 S V N L D I A K V T L S D E G F Y E C I A V S S A G T G R A Q T F F D V S E P P
P V I Q V P N N V T V T P G E R A V L T C L I I S A V D Y N L T W Q R N D R D V

561 R L A E P A R I R T L A N L S L E L K S V K F N D A G E Y H C M V S S E G G S S
A A S V F L T V Q E P P K V T V M P K N Q S F T G G S E V S I M C S A T G Y P K

641 P K I A W T V N D M F I V G S H R Y R M T S D G T L F I K N A A P K D A G I Y
G C L A S N S A G T D K Q N S T L R Y I E A P K L M V V Q S E L L V A L G D I T V
721 M E C K T S G I P P P Q V K W F K G D L E L R P S T F L I I D P L L G L L K I Q E
T Q D L D A G D Y T C V A I N E A G R A T G K I T L D V G S P P V F I Q E P A

801 D V S M E I G S N V T L P C Y V Q G Y P E P T I K W R R L D N M P I F S R P F S
V S S I S Q L R T G A L F I L N L W A S D K G T Y I C E A E N Q F G K I Q S E T

881 T V T V T G L V A P L I G I S P S V A N V I E G Q Q L T L P C T L L A G N P I P E
R R W I K N S A M L L Q N P Y I T V R S D G S L H I E R V Q L Q D G G E Y T C

961 V A S N V A G T N N K T T S V V V H V L P T I Q H G Q Q I L S T I E G I P V T L
P C K A S G N P K P S V I W S K K G E L I S T S S A K F S A G A D G S L Y V V S

1041 P G G E E S G E Y V C T A T N T A G Y A K R K V Q L T V Y V R P R V F G D Q
R G L S Q D K P V E I S V L A G E E V T L P C E V K S L P P P I I T W A K E T Q L I

1121 S P F S P R H T F L P S G S M K I T E T R T S D S G M Y L C V A T N I A G N V T
Q A V K L N V H V P P K I Q R G P K H L K V Q V G Q R V D I P C N A Q G T P L P

1201 V I T W S K G G S T M L V D G E H H V S N P D G T L S I D Q A T P S D A G I Y
T C V A T N I A G T D E T E I T L H V Q E P P T V E D L E P P Y N T T F Q E R V A

1281 N Q R I E F P C P A K G T P K P T I K W L H N G R E L T G R E P G I S I L E D G
T L L V I A S V T P Y D N G E Y I C V A V N E A G T T E R K Y N L K V H V P P V

1361 I K D K E Q V T N V S V L L N Q L T N L F C E V E G T P S P I I M W Y K D N V
Q V T E S S T I Q T V N N G K I L K L F R A T P E D A G R Y S C K A I N I A G T S

1441 Q K Y F N I D V L V P P T I I G T N F P N E V S V V L N R D V A L E C Q V K G
T P F P D I H W F K D G K P L F L G D P N V E L L D R G Q V L H L K N A R R N D K

1521 GRYQCTVSNAAGKQAKDIKLTIYIPPSIKGGNVTTDISVL
INSLIKLECETRGLPMPAITWYKDGQPIMSSSQALYIDKG

1601 QYLHIPRAQVSDSATYTCHVANVAGTAEKSFHVDVYVP
PMIEGNLATPLNKQVVIAHSLTLECKAAGNPSPILTWLKDGV

1681 PVKANDNIRIEAGGKKLEIMSAQEIDRGQYICVATSVAG
EKEIKYEVDVLVPPAIEGGDETSYFIVMVNNLLELDCHVTG

1761 SPPPTIMWLKDGQLIDERDGFKILLNGRKLVIAQAQVSN
TGLYRCMAANTAGDHKKEFEVTVHVPPTIKSSGLSERVVVK

1841 YKPVALQCIANGIPNPSITWLKDDQPVNTAQGNLKIQSS
GRVLQIAKTLLEDAGRYTCVATNAAGETQQHIQLHVHEPPS

1921 LEDAGKMLNETVLVSNPVQLECKAAGNPVPVITWYKDN
RLLSGSTSMTFLNRGQIIDIESAQISDAGIYKCVAINSAGAT

2001 ELFYSLQVHVAPSISGSNNMVAVVVNNPVRLECEARGIP
APSLTWLKDGSPVSSFSNGLQVLSGGRILALTSAQISDTGR

2081 YTCVAVNAAGEKQRDIDLRVYVPPNIMGEEQNVSVLISQ
AVELLCQSDAIPPPTLTWLKDGHPLLKKPGLSISENRSVLK

2161 IEDAQVQDTGRYTCEATNVAGKTEKNYNVNIWVPPNIG
GSDELTQLTVIEGNLISLLCESSGIPPPNLIWKKKGSPVLTD

2241 SMGRVRILSGGRQLQISIAEKSDAALYSCVASNVAGTAK
KEYNLQVYIRPTITNSGSHPTEIIVTRGKSISLECEVQGIP

2321 PPTVTWMKDGHPLIKAKGVEILDEGHILQLKNIHVSDTG
RYVCVAVNVAGMTDKKYDLSVHAPPSIIGNHRSPENISVVE

2401 KNSVSLTCEASGIPLPSITWFKDGWPVSLSNSVRILSGGR
MLRLMQTTMEDAGQYTCVVRNAAGEERKIFGLSVLVPPHI

2481 VGENTLEDVKVKEKQSVTLTCEVTGNPVPEITWHKDGQ
PLQEDEAHHIISGGRFLQITNVQVPHTGRYTCLASSPAGHKS

2561 RSFSLNVFVSPTIAGVGSDGNPEDVTVILNSPTSLVCEAY
SYPPATITWFKDGTPLESNRNIRILPGGRTLQILNAQEDN

2641 A G R Y S C V A T N E A G E M I K H Y E V K V Y I P P I I N K G D L W G P G L S P K E V K I K V N N T L T L E C E A Y A I P S A S L S W Y K D G Q P L K S D D H

2721 V N I A A N G H T L Q I K E A Q I S D T G R Y T C V A S N I A G E D E L D F D V N I Q V P P S F Q K L W E I G N M L D T G R N G E A K D V I I N N P I S L Y C E

2801 T N A A P P P T L T W Y K D G H P L T S S D K V L I L P G G R V L Q I P R A K V E D A G R Y T C V A V N E A G E D S L Q Y D V R V L V P P I I K G A N S D L P E

2881 E V T V L V N K S A L I E C L S S G S P A P R N S W Q K D G Q P L L E D D H H K F L S N G R I L Q I L N T Q I T D I G R Y V C V A E N T A G S A K K Y F N L N V

2961 H V P P S V I G P K S E N L T V V V N N F I S L T C E V S G F P P P D L S W L K N E Q P I K L N T N T L I V P G G R T L Q I I R A K V S D G G E Y T C I A I N Q

3041 A G E S K K K F S L T V Y V P P S I K D H D S E S L S V V N V R E G T S V S L E C E S N A V P P P V I T W Y K N G R M I T E S T H V E I L A D G Q M L H I K K A

3121 E V S D T G Q Y V C R A I N V A G R D D K N F H L N V Y V P P S I E G P E R E V I V E T I S N P V T L T C D A T G I P P P T I A W L K N H K R I E N S D S L E V

3201 R I L S G G S K L Q I A R S Q H S D S G N Y T C I A S N M E G K A Q K Y Y F L S I Q V P P S V A G A E I P S D V S V L L G E N V E L V C N A N G I P T P L I Q W

3281 L K D G K P I A S G E T E R I R V S A N G S T L N I Y G A L T S D T G K Y T C V A T N P A G E E D R I F N L N V Y V T P T I R G N K D E A E K L M T L V D T S I

3361 N I E C R A T G T P P P Q I N W L K N G L P L P L S S H I R L L A A G Q V I R I V R A Q V S D V A V Y T C V A S N R A G V D N K H Y N L Q V F A P P N M D N S M

3441 G T E E I T V L K G S S T S M A C I T D G T P A P S M A W L R D G Q P L G L D A H L T V S T H G M V L Q L L K A E T E D S G K Y T C I A S N E A G E V S K H F I

3521 L K V L E P P H I N G S E E H E E I S V I V N N P L E L T C I A S G I P A P K M T W M K D G R P L P Q T D Q V Q T L G G G E V L R I S T A Q V E D T G R Y T C L

3601 A S S P A G D D D K E Y L V R V H V P P N I A G T D E P R D I T V L R N R Q V T L E C K S D A V P P P V I T W L R N G E R L Q A T P R V R I L S G G R Y L Q I N

3681 N A D L G D T A N Y T C V A S N I A G K T T R E F I L T V N V P P N I K G G P Q S L V I L L N K S T V L E C I A E G V P T P R I T W R K D G A V L A G N H A R Y

3761 S I L E N G F L H I Q S A H V T D T G R Y L C M A T N A A G T D R R R I D L Q
V H V P P S I A P G P T N M T V I V N V Q T T L A C E A T G I P K P S I N W R K N

3841 G H L L N V D Q N Q N S Y R L L S S G S L V I I S P S V D D T A T Y E C T V T
N G A G D D K R T V D L T V Q V P P S I A D E P T D F L V T K H A P A V I T C T A

3921 S G V P F P S I H W T K N G I R L L P R G D G Y R I L S S G A I E I L A T Q L N
H A G R Y T C V A R N A A G S A H R H V T L H V H E P P V I Q P Q P S E L H V I

4001 L N N P I L L P C E A T G T P S P F I T W Q K E G I N V N T S G R N H A V L P S
G G L Q I S R A V R E D A G T Y M C V A Q N P A G T A L G K I K L N V Q V P P V

4081 I S P H L K E Y V I A V D K P I T L S C E A D G L P P P D I T W H K D G R A I V
E S I R Q R V L S S G S L Q I A F V Q P G D A G H Y T C M A A N V A G S S S T S

4161 T K L T V H V P P R I R S T E G H Y T V N E N S Q A I L P C V A D G I P T P A I
N W K K D N V L L A N L L G K Y T A E P Y G E L I L E N V V L E D S G F Y T C V

4241 A N N A A G E D T H T V S L T V H V L P T F T E L P G D V S L N K G E Q L R L
S C K A T G I P L P K L T W T F N N N I I P A H F D S V N G H S E L V I E R V S K

4321 E D S G T Y V C T A E N S V G F V K A I G F V Y V K E P P V F K G D Y P S N
W I E P L G G N A I L N C E V K G D P T P T I Q W N R K G V D I E I S H R I R Q L G

4401 N G S L A I Y G T V N E D A G D Y T C V A T N E A G V V E R S M S L T L Q S
P P I I T L E P V E T V I N A G G K I I L N C Q A T G E P Q P T I T W S R Q G H S I

4481 S W D D R V N V L S N N S L Y I A D A Q K E D T S E F E C V A R N L M G S V
L V R V P V I V Q V H G G F S Q W S A W R A C S V T C G K G I Q K R S R L C N Q P
L

4561 P A N G G K P C Q G S D L E M R N C Q N K P C P V D G S W S E W S L W E E C
T R S C G R G N Q T R T R T C N N P S V Q H G G R P C E G N A V E I I M C N I R P C

4641 P V H G A W S A W Q P W G T C S E S C G K G T Q T R A R L C N N P P P A F G
G S Y C D G A E T Q M Q V C N E R N C P I H G K W A T W A S W S A C S V S C G G
G A

4721 R Q R T R G C S D P V P Q Y G G R K C E G S D V Q S D F C N S D P C P T H G
N W S P W S G W G T C S R T C N G G Q M R R Y R T C D N P P P S N G G R A C G G
P D

SQIQRCNTDMCPVDGSWGSWHSWSQCSASCGGGEKTRK RLCDHPVPVKGGRPCPGDTTQVTRCNVQACPGGPQRARGSV I

GNINDVEFGIAFLNATITDSPNSDTRIIRAKITNVPRSLGS AMRKIVSILNPIYWTTAKEIGEAVNGFTLTNAVFKRETQ

VEFATGEILQMSHIARGLDSDGSLLLDIVVSGYVLQLQSP AEVTVKDYTEDYIQTGPGQLYAYSTRLFTIDGISIPYTWN

HTVFYDQAQGRMPFLVETLHASSVESDYNQIEETLGFKI HASISKGDRSNQCPSGFTLDSVGPFCADEDECAAGNPCSHS

CHNAMGTYYCSCPKGLTIAADGRTCQDIDECALGRHTC HAGQDCDNTIGSYRCVVRCGSGFRRTSDGLSCQDINECQESS

PCHQRCFNAIGSFHCGCEPGYQLKGRKCMDVNECRQNV CRPDQHCKNTRGGYKCIDLCPNGMTKAENGTCIDIDECKDG T

HQCRYNQICENTRGSYRCVCPRGYRSQGVGRPCMDINE CEQVPKPCAHQCSNTPGSFKCICPPGQHLLGDGKSCAGLERL

PNYGTQYSSYNLARFSPVRNNYQPQQHYRQYSHLYSSYS EYRNSRTSLSRTRRTIRKTCPEGSEASHDTCVDIDECENTD

ACQHECKNTFGSYQCICPPGYQLTHNGKTCQDIDECLEQ NVHCGPNRMCFNMRGSYQCIDTPCPPNYQRDPVSGFCLKNC

PPNDLECALSPYALEYKLVSLPFGIATNQDLIRLVAYTQD GVMHPRTTFLMVDEEQTVPFALRDENLKGVVYTTRPLREA

ETYRMRVRASSYSANGTIEYQTTFIVYIAVSAYPY

5.176 prostate stem cell antigen preproprotein [Homo sapiens]

Protein Accession gi|5031995
Mean Expression Ratio 1.12
Median Expression Ratio 1.12
Credible Interval (0.692, 1.81)
Associated Peptides 1
Associated Spectra 1
Coverage 0.0813

| A | 2.5 | 50 | 97.5 | Sequence |
|---|-----|----|------|----------|
| 1 | 0.7 | 1.2 | 1.9 | AVGLLTVISK |

1 M K A V L L A L L M A G L A L Q P G T A L L C Y S C K A Q V S N E D C L Q V E N C T Q L G E Q C W T A R I R A V G L L T V I S K G C S L N C V D D S Q D Y Y V G

81 K K N I T C C D T D L C N A S G A H A L Q P A A A I L A L L P A L G L L L W G P G Q L

5.177 solute carrier family 47, member 1 [Homo sapiens]

Protein Accession gi|22907060
Mean Expression Ratio 1.12
Median Expression Ratio 1.12
Credible Interval (0.725, 1.74)
Associated Peptides 1
Associated Spectra 2
Coverage 0.0246

| A | 2.5 | 50 | 97.5 | Sequence |
|---|-----|----|------|----------|
| 2 | 0.74 | 1.2 | 1.8 | QEEPLPEHPQDGAK |

1 M E A P E E P A P V R G G P E A T L E V R G S R C L R L S A F R E E L R A L L V L A G P A F L V Q L M V F L I S F I S S V F C G H L G K L E L D A V T L A I A V

81 I N V T G V S V G F G L S S A C D T L I S Q T Y G S Q N L K H V G V I L Q R S A L V L L L C C F P C W A L F L N T Q H I L L L F R Q D P D V S R L T Q T Y V T I

161 F I P A L P A T F L Y M L Q V K Y L L N Q G I V L P Q I V T G V A A N L V N A L A N Y L F L H Q L H L G V I G S A L A N L I S Q Y T L A L L L F L Y I L G K K L

241 H Q A T W G G W S L E C L Q D W A S F L R L A I P S M L M L C M E W W A Y E V G S F L S G I L G M V E L G A Q S I V Y E L A I I V Y M V P A G F S V A A S V R V

321 G N A L G A G D M E Q A R K S S T V S L L I T V L F A V A F S V L L L S C K D H V G Y I F T T D R D I I N L V A Q V V P I Y A V S H L F E A L A C T S G G V L R

401 G S G N Q K V G A I V N T I G Y Y V V G L P I G I A L M F A T T L G V M G L W S G I I I C T V F Q A V C F L G F I I Q L N W K K A C Q Q A Q V H A N L K V N N V

481 P R S G N S A L P Q D P L H P G C P E N L E G I L T N D V G K T G E P Q S D Q Q M R Q E E P L P E H P Q D G A K L S R K Q L V L R R G L L L L L G V F L I L L V G

561 I L V R F Y V R I Q

5.178 polymeric immunoglobulin receptor [Homo sapiens]

Protein Accession gi|31377806

Mean Expression Ratio 1.12

Median Expression Ratio 1.12

Credible Interval (0.926, 1.35)

Associated Peptides 16

Associated Spectra 25

Coverage 0.234

| A | 2.5 | 50 | 97.5 | Sequence |
|---|---|---|---|---|
| 1 | 0.83 | 1.1 | 1.6 | DGSFSVVITGLR |
| 2 | 0.78 | 1.0 | 1.4 | ILLNPQDKDGSFSVVITGLR |
| 1 | 0.83 | 1.1 | 1.6 | LDIQGTGQLLF S VVINQLR |
| 1 | 0.8 | 1.1 | 1.5 | LVSLTLNLVTR |
| 9 | 0.9 | 1.1 | 1.4 | NADLQVLKPEPELVYEDLR |
| 1 | 0.83 | 1.2 | 1.6 | QLFVNEESTIPR |
| 1 | 0.8 | 1.1 | 1.5 | QSSGENCDVVVNTLGK |
| 1 | 0.85 | 1.2 | 1.6 | TVTINCPFK |
| 1 | 0.81 | 1.1 | 1.5 | VVNIAQLSQDDSGR |
| 1 | 0.78 | 1.1 | 1.5 | YLCGAHSDGQLQEGSPIQAW |
| 1 | 0.8 | 1.1 | 1.5 | YWCLWEGAQNGR |
| 1 | 0.82 | 1.1 | 1.6 | SVVINQLR |
| 1 | 0.83 | 1.1 | 1.6 | KTENAQK |
| 1 | 0.79 | 1.1 | 1.5 | HCALGPEVANVAK |
| 1 | 0.82 | 1.1 | 1.5 | ILLNPQDK |
| 1 | 0.8 | 1.1 | 1.5 | ADAAPDEK |

1 M L L F V L T C L L A V F P A I S T K S P I F G P E E V N S V E G N S V S I T C Y Y
P P T S V N R H T R K Y W C R Q G A R G G C I T L I S S E G Y V S S K Y A G

81 R A N L T N F P E N G T F V V N I A Q L S Q D D S G R Y K C G L G I N S R G L S
F D V S L E V S Q G P G L L N D T K V Y T V D L G R T V T I N C P F K T E N A Q

161 K R K S L Y K Q I G L Y P V L V I D S S G Y V N P N Y T G R I R L D I Q G T G Q
L L F S V V I N Q L R L S D A G Q Y L C Q A G D D S N S N K K N A D L Q V L K P

EPELVYEDLRGSVTFHCALGPEVANVAKFLCRQSSGENCDVVVNTLGKRAPAFEGRILLNPQDKDGSFSVVITGLRKEDA

GRYLCGAHSDGQLQEGSPIQAWQLFVNEESTIPRSPTVVKGVAGGSVAVLCPYNRKESKSIKYWCLWEGAQNGRCPLLVD

SEGWVKAQYEGRLSLLEEPGNGTFTVILNQLTSRDAGFYWCLTNGDTLWRTTVEIKIIEGEPNLKVPGNVTAVLGETLKV

PCHFPCKFSSYEKYWCKWNNTGCQALPSQDEGPSKAFVNCDENSRLVSLTLNLVTRADEGWYWCGVKQGHFYGETAAVYV

AVEERKAAGSRDVSLAKADAAPDEKVLDSGFREIENKAIQDPRLFAEEKAVADTRDQADGSRASVDSGSSEEQGGSSRAL

VSTLVPLGLVLAVGAVAVGVARARHRKNVDRVSIRSYRTDISMSDFENSREFGANDNMGASSITQETSLGGKEEFVATTE

STTETKEPKKAKRSSKEEAEMAYKDFLLQSSTVAAEAQDGPQEA

5.179 cathepsin C isoform a preproprotein [Homo sapiens]

Protein Accession gi|189083844
Mean Expression Ratio 1.12
Median Expression Ratio 1.12
Credible Interval (0.874, 1.43)
Associated Peptides 6
Associated Spectra 13
Coverage NaN

| A | 2.5 | 50 | 97.5 | Sequence |
|---|-----|----|------|----------|
| 4 | 0.83 | 1.1 | 1.5 | AVAFEVYDDFLHYK |
| 2 | 0.78 | 1.1 | 1.5 | EVYDDFLHYK |
| 1 | 0.79 | 1.1 | 1.6 | GTDECAIESIAVAATPIPK |
| 3 | 0.83 | 1.1 | 1.6 | NVHGINFVSPVR |
| 1 | 0.8 | 1.1 | 1.6 | WFAFFK |
| 2 | 0.84 | 1.1 | 1.6 | LELVHHGPMAVAF |

5.180 biliverdin reductase B (flavin reductase (NADPH)) [Homo sapiens]

Protein Accession gi|4502419
Mean Expression Ratio 0.897
Median Expression Ratio 0.896
Credible Interval (0.551, 1.46)
Associated Peptides 1
Associated Spectra 1
Coverage 0.0728

| A | 2.5 | 50 | 97.5 | Sequence |
|---|-----|-----|------|----------|
| 1 | 0.52 | 0.87 | 1.5 | TVAGQDAVIVLLGTR |

1 MAVKKIAIFGATGQTGLTTLAQAVQAGYEVTVLVRDSSRL
PSEGPRPAHVVVGDVLQAADVDKTVAGQDAVIVLLGTRND

81 LSPTTVMSEGARNIVAAMKAHGVDKVVACTSAFLLWDPT
KVPPRLQAVTDDHIRMHKVLRESGLKYVAVMPPHIGDQPLT

161 GAYTVTLDGRGPSRVISKHDLGHFMLRCLTTDEYDGHST
YPSHQYQ

5.181 prostatic binding protein [Homo sapiens]

Protein Accession gi|4505621
Mean Expression Ratio 1.12
Median Expression Ratio 1.12
Credible Interval (0.79, 1.59)
Associated Peptides 2
Associated Spectra 5
Coverage 0.144

| A | 2.5 | 50 | 97.5 | Sequence |
|---|-----|-----|------|----------|
| 2 | 0.78 | 1.1 | 1.7 | LYTLVLTDPDAPSR |
| 3 | 0.78 | 1.1 | 1.6 | YVWLVYEQDRPLK |

1 MPVDLSKWSGPLSLQEVDEQPQHPLHVTYAGAAVDELGKV
LTPTQVKNRPTSISWDGLDSGKLYTLVLTDPDAPSRKDPK

81 YREWHHFLVVNMKGNDISSGTVLSDYVGSGPPKGTGLHR
YVWLVYEQDRPLKCDEPILSNRSGDHRGKFKVASFRKKYEL

161 RAPVAGTCYQAEWDDYVPKLYEQLSGK

5.182 proteasome alpha 4 subunit isoform 1 [Homo sapiens]; proteasome alpha 4 subunit isoform 1 [Homo sapiens]

Protein Accession gi|4506185 gi|156713442
Mean Expression Ratio 1.12
Median Expression Ratio 1.12
Credible Interval (0.696, 1.82)
Associated Peptides 1
Associated Spectra 1
Coverage NaN

| A | 2.5 | 50 | 97.5 | Sequence |
|---|---|---|---|---|
| 1 | 0.69 | 1.2 | 1.9 | LLDEVFFSEK |

5.183 palate, lung and nasal epithelium associated precursor [Homo sapiens]; palate, lung and nasal epithelium associated precursor [Homo sapiens]

Protein Accession gi|7706119 gi|18765705
Mean Expression Ratio 0.896
Median Expression Ratio 0.897
Credible Interval (0.552, 1.44)
Associated Peptides 1
Associated Spectra 1
Coverage NaN

| A | 2.5 | 50 | 97.5 | Sequence |
|---|---|---|---|---|
| 1 | 0.52 | 0.87 | 1.5 | LQVNTPLVGASLLR |

5.184 vacuolar protein sorting factor 4A [Homo sapiens]

Protein Accession gi|7019569
Mean Expression Ratio 1.12
Median Expression Ratio 1.12
Credible Interval (0.773, 1.59)
Associated Peptides 4
Associated Spectra 4
Coverage 0.144

| A | 2.5 | 50 | 97.5 | Sequence |
|---|---|---|---|---|
| 1 | 0.74 | 1.1 | 1.7 | IYIPLPEEAAR |
| 1 | 0.74 | 1.1 | 1.7 | LHLGSTPHNLTDANIHELAR |
| 1 | 0.75 | 1.1 | 1.7 | QHKPSIIFIDEVDSLCGSR |
| 1 | 0.73 | 1.1 | 1.7 | LLEPVVCMSDMLR |

1 M T T S T L Q K A I D L V T K A T E E D K A K N Y E E A L R L Y Q H A V E Y F L H A I K Y E A H S D K A K E S I R A K C V Q Y L D R A E K L K D Y L R S K E K H

GKKPVKENQSEGKGSDSDSEGDNPEKKKLQEQLMGAVVM
EKPNIRWNDVAGLEGAKEALKEAVILPIKFPHLFTGKRTPW

RGILLFGPPGTGKSYLAKAVATEANNSTFFSVSSSDLMSK
WLGESEKLVKNLFELARQHKPSIIFIDEVDSLCGSRNENE

SEAARRIKTEFLVQMQGVGNNNDGTLVLGATNIPWVLDS
AIRRRFEKRIYIPLPEEAARAQMFRLHLGSTPHNLTDANIH

ELARKTEGYSGADISIIVRDSLMQPVRKVQSATHFKKVCG
PSRTNPSMMIDDLLTPCSPGDPGAMEMTWMDVPGDKLLEP

VVCMSDMLRSLATTRPTVNADDLLKVKKFSEDFGQES

5.185 transmembrane protein 192 [Homo sapiens]

Protein Accession gi|154240704
Mean Expression Ratio 0.899
Median Expression Ratio 0.899
Credible Interval (0.586, 1.35)
Associated Peptides 2
Associated Spectra 2
Coverage 0.0996

| A | 2.5 | 50 | 97.5 | Sequence |
|---|-----|------|------|-----------------|
| 1 | 0.57 | 0.9 | 1.4 | IYAYPSNITSETGFR |
| 1 | 0.55 | 0.88 | 1.4 | AKPEPDILEEEK |

MAAGGRMEDGSLDITQSIEDDPLLDAQLLPHHSLQAHFRPR
FHPLPTVIIVNLLWFIHLVFVVLAFLTGVLCSYPNPNED

KCPGNYTNPLKVQTVIILGKVILWILHLLLECYIQYHHSKI
RNRGYNLIYRSTRHLKRLALMIQSSGNTVLLLILCMQHS

FPEPGRLYLDLILAILALELICSLICLLIYTVKIRRFNKAKP
EPDILEEEKIYAYPSNITSETGFRTISSLEEIVEKQGD

TIEYLKRHNALLSKRLLALTSSDLGCQPSRT

5.186 vitronectin precursor [Homo sapiens]

Protein Accession gi|88853069
Mean Expression Ratio 0.901
Median Expression Ratio 0.9

Credible Interval (0.619, 1.31)
Associated Peptides 3
Associated Spectra 3
Coverage 0.09

| A | 2.5 | 50 | 97.5 | Sequence |
|---|-----|----|------|----------|
| 1 | 0.57 | 0.87 | 1.3 | DVWGIEGPIDAAFTR |
| 1 | 0.58 | 0.9 | 1.4 | DWHGVPGQVDAAMAGR |
| 1 | 0.59 | 0.91 | 1.4 | FEDGVLDPDYPR |

1 M A P L R P L L I L A L L A W V A L A D Q E S C K G R C T E G F N V D K K C Q C D E L C S Y Y Q S C C T D Y T A E C K P Q V T R G D V F T M P E D E Y T V Y D D

81 G E E K N N A T V H E Q V G G P S L T S D L Q A Q S K G N P E Q T P V L K P E E E A P A P E V G A S K P E G I D S R P E T L H P G R P Q P P A E E E L C S G K P

161 F D A F T D L K N G S L F A F R G Q Y C Y E L D E K A V R P G Y P K L I R D V W G I E G P I D A A F T R I N C Q G K T Y L F K G S Q Y W R F E D G V L D P D Y P

241 R N I S D G F D G I P D N V D A A L A L P A H S Y S G R E R V Y F F K G K Q Y W E Y Q F Q H Q P S Q E E C E G S S L S A V F E H F A M M Q R D S W E D I F E L L

321 F W G R T S A G T R Q P Q F I S R D W H G V P G Q V D A A M A G R I Y I S G M A P R P S L A K K Q R F R H R N R K G Y R S Q R G H S R G R N Q N S R R P S R A T

401 W L S L F S S E E S N L G A N N Y D D Y R M D W L V P A T C E P I Q S V F F F S G D K Y Y R V N L R T R R V D T V D P P Y P R S I A Q Y W L G C P A P G H L

5.187 von Willebrand factor preproprotein [Homo sapiens]

Protein Accession gi|89191868

Mean Expression Ratio 1.11

Median Expression Ratio 1.11

Credible Interval (0.866, 1.41)

Associated Peptides 7

Associated Spectra 13

Coverage 0.0387

| A | 2.5 | 50 | 97.5 | Sequence |
|---|---|---|---|---|
| 1 | 0.81 | 1.1 | 1.6 | AFVLSSVDELEQQR |
| 2 | 0.8 | 1.1 | 1.5 | APILIQDFETLPR |
| 4 | 0.8 | 1.1 | 1.4 | AVVILVTDVSVDSVDAAADAAR |
| 1 | 0.78 | 1.1 | 1.6 | LLDLVFLLDGS SR |
| 3 | 0.81 | 1.1 | 1.5 | LPGDIQVVPIGVGPNANVQELER |
| 1 | 0.81 | 1.1 | 1.6 | VIVIPVGIGPHANLK |
| 1 | 0.81 | 1.1 | 1.6 | EAPDLVLQR |

1 MIPARFAGVLLALALILPGTLCAEGTRGRSSTARCSLFGSD
FVNTFDGSMYSFAGYCSYLLAGGCQKRSFSIIGDFQNGK

81 RVSLSVYLGEFFDIHLFVNGTVTQGDQRVSMPYASKGLYL
ETEAGYYKLSGEAYGFVARIDGSGNFQVLLSDRYFNKTCG

161 LCGNFNIFAEDDFMTQEGTLTSDPYDFANSWALSSGEQW
CERASPPSSSCNISSGEMQKGLWEQCQLLKSTSVFARCHPL

241 VDPEPFVALCEKTLCECAGGLECACPALLEYARTCAQEG
MVLYGWTDHSACSPVCPAGMEYRQCVSPCARTCQSLHINE
M

321 CQERCVDGCSCPEGQLLDEGLCVESTECPCVHSGKRYPP
GTSLSRDCNTCICRNSQWICSNEECPGECLVTGQSHFKSFD

401 NRYFTFSGICQYLLARDCQDHSFSIVIETVQCADDRDAVC
TRSVTVRLPGLHNSLVKLKHGAGVAMDGQDVQLPLLKGDL

481 RIQHTVTASVRLSYGEDLQMDWDGRGRLLVKLSPVYAG
KTCGLCGNYNGNQGDDFLTPSGLAEPRVEDFGNAWKLHGD
CQ

561 DLQKQHSDPCALNPRMTRFSEEACAVLTSPTFEACHRAV
SPLPYLRNCRYDVCSCSDGRECLCGALASYAAACAGRGVRV

641 AWREPGRCELNCPKGQVYLQCGTPCNLTCRSLSYPDEEC
NEACLEGCFCPPGLYMDERGDCVPKAQCPCYYDGEIFQPED

721 IFSDHHTMCYCEDGFMHCTMSGVPGSLLPDAVLSSPLSH
RSKRSLSCRPPMVKLVCPADNLRAEGLECTKTCQNYDLECM

801 SMGCVSGCLCPPGMVRHENRCVALERCPCFHQGKEYAPG
ETVKIGCNTCVCRDRKWNCTDHVCDATCSTIGMAHYLTFDG

881 L K Y L F P G E C Q Y V L V Q D Y C G S N P G T F R I L V G N K G C S H P S V K C K K R V T I L V E G G E I E L F D G E V N V K R P M K D E T H F E V V E S G R

961 Y I I L L L G K A L S V V W D R H L S I S V V L K Q T Y Q E K V C G L C G N F D G I Q N N D L T S S N L Q V E E D P V D F G N S W K V S S Q C A D T R K V P L D

1041 S S P A T C H N N I M K Q T M V D S S C R I L T S D V F Q D C N K L V D P E P Y L D V C I Y D T C S C E S I G D C A C F C D T I A A Y A H V C A Q H G K V V T W

1121 R T A T L C P Q S C E E R N L R E N G Y E C E W R Y N S C A P A C Q V T C Q H P E P L A C P V Q C V E G C H A H C P P G K I L D E L L Q T C V D P E D C P V C E

1201 V A G R R F A S G K K V T L N P S D P E H C Q I C H C D V V N L T C E A C Q E P G G L V V P P T D A P V S P T T L Y V E D I S E P P L H D F Y C S R L L D L V F

1281 L L D G S S R L S E A E F E V L K A F V V D M M E R L R I S Q K W V R V A V V E Y H D G S H A Y I G L K D R K R P S E L R R I A S Q V K Y A G S Q V A S T S E V

1361 L K Y T L F Q I F S K I D R P E A S R I T L L L M A S Q E P Q R M S R N F V R Y V Q G L K K K K V I V I P V G I G P H A N L K Q I R L I E K Q A P E N K A F V L

1441 S S V D E L E Q Q R D E I V S Y L C D L A P E A P P P T L P P D M A Q V T V G P G L L G V S T L G P K R N S M V L D V A F V L E G S D K I G E A D F N R S K E F

1521 M E E V I Q R M D V G Q D S I H V T V L Q Y S Y M V T V E Y P F S E A Q S K G D I L Q R V R E I R Y Q G G N R T N T G L A L R Y L S D H S F L V S Q G D R E Q A

1601 P N L V Y M V T G N P A S D E I K R L P G D I Q V V P I G V G P N A N V Q E L E R I G W P N A P I L I Q D F E T L P R E A P D L V L Q R C C S G E G L Q I P T L

1681 S P A P D C S Q P L D V I L L L D G S S S F P A S Y F D E M K S F A K A F I S K A N I G P R L T Q V S V L Q Y G S I T T I D V P W N V V P E K A H L L S L V D V

1761 M Q R E G G P S Q I G D A L G F A V R Y L T S E M H G A R P G A S K A V V I L V T D V S V D S V D A A A D A A R S N R V T V F P I G I G D R Y D A A Q L R I L A

1841 G P A G D S N V V K L Q R I E D L P T M V T L G N S F L H K L C S G F V R I C M D E D G N E K R P G D V W T L P D Q C H T V T C Q P D G Q T L L K S H R V N C D

1921 R G L R P S C P N S Q S P V K V E E T C G C R W T C P C V C T G S S T R H I V T F D G Q N F K L T G S C S Y V L F Q N K E Q D L E V I L H N G A C S P G A R Q G

2001 C M K S I E V K H S A L S V E L H S D M E V T V N G R L V S V P Y V G G N M E V N V Y G A I M H E V R F N H L G H I F T F T P Q N N E F Q L Q L S P K T F A S K

2081 T Y G L C G I C D E N G A N D F M L R D G T V T T D W K T L V Q E W T V Q R P G Q T C Q P I L E E Q C L V P D S S H C Q V L L L P L F A E C H K V L A P A T F Y

2161 A I C Q Q D S C H Q E Q V C E V I A S Y A H L C R T N G V C V D W R T P D F C A M S C P P S L V Y N H C E H G C P R H C D G N V S S C G D H P S E G C F C P P D

2241 K V M L E G S C V P E E A C T Q C I G E D G V Q H Q F L E A W V P D H Q P C Q I C T C L S G R K V N C T T Q P C P T A K A P T C G L C E V A R L R Q N A D Q C C

2321 P E Y E C V C D P V S C D L P P V P H C E R G L Q P T L T N P G E C R P N F T C A C R K E E C K R V S P P S C P P H R L P T L R K T Q C C D E Y E C A C N C V N

2401 S T V S C P L G Y L A S T A T N D C G C T T T T C L P D K V C V H R S T I Y P V G Q F W E E G C D V C T C T D M E D A V M G L R V A Q C S Q K P C E D S C R S G

2481 F T Y V L H E G E C C G R C L P S A C E V V T G S P R G D S Q S S W K S V G S Q W A S P E N P C L I N E C V R V K E E V F I Q Q R N V S C P Q L E V P V C P S G

2561 F Q L S C K T S A C C P S C R C E R M E A C M L N G T V I G P G K T V M I D V C T T C R C M V Q V G V I S G F K L E C R K T T C N P C P L G Y K E E N N T G E C

2641 C G R C L P T A C T I Q L R G G Q I M T L K R D E T L Q D G C D T H F C K V N E R G E Y F W E K R V T G C P P F D E H K C L A E G G K I M K I P G T C C D T C E

2721 E P E C N D I T A R L Q Y V K V G S C K S E V E V D I H Y C Q G K C A S K A M Y S I D I N D V Q D Q C S C C S P T R T E P M Q V A L H C T N G S V V Y H E V L N

2801 A M E C K C S P R K C S K

5.188 tripartite motif protein TRIM14 isoform alpha [Homo sapiens]; tripartite motif protein TRIM14 isoform alpha [Homo sapiens]; tripartite motif protein TRIM14 isoform alpha [Homo sapiens]

Protein Accession gi|15208667 gi|15208665 gi|15208663
Mean Expression Ratio 0.9
Median Expression Ratio 0.9

Credible Interval (0.61, 1.35)
Associated Peptides 2
Associated Spectra 3
Coverage NaN

| A | 2.5 | 50 | 97.5 | Sequence |
|---|-----|-----|------|----------|
| 2 | 0.61 | 0.9 | 1.4 | GLVEAVESTLQTPLDIR |
| 1 | 0.57 | 0.88 | 1.4 | ATFQEPLYPALR |

5.189 PREDICTED: similar to hCG1642538 [Homo sapiens]; PREDICTED: similar to hCG1642538 [Homo sapiens]

Protein Accession gi|169216113 gi|169215489
Mean Expression Ratio 1.11
Median Expression Ratio 1.11
Credible Interval (0.773, 1.61)
Associated Peptides 1
Associated Spectra 5
Coverage NaN

| A | 2.5 | 50 | 97.5 | Sequence |
|---|-----|-----|------|----------|
| 5 | 0.82 | 1.1 | 1.6 | DIQMTQSPSSLSASVGDR |

5.190 actin, alpha 1, skeletal muscle [Homo sapiens]

Protein Accession gi|4501881
Mean Expression Ratio 1.11
Median Expression Ratio 1.11
Credible Interval (0.691, 1.82)
Associated Peptides 1
Associated Spectra 1
Coverage 0.0424

| A | 2.5 | 50 | 97.5 | Sequence |
|---|-----|-----|------|----------|
| 1 | 0.69 | 1.1 | 1.9 | YPIEHGIITNWDDMEK |

1 M C D E D E T T A L V C D N G S G L V K A G F A G D D A P R A V F P S I V G R P
R H Q G V M V G M G Q K D S Y V G D E A Q S K R G I L T L K Y P I E H G I I T N

81 W D D M E K I W H H T F Y N E L R V A P E E H P T L L T E A P L N P K A N R E
K M T Q I M F E T F N V P A M Y V A I Q A V L S L Y A S G R T T G I V L D S G D G

161 V T H N V P I Y E G Y A L P H A I M R L D L A G R D L T D Y L M K I L T E R G
Y S F V T T A E R E I V R D I K E K L C Y V A L D F E N E M A T A A S S S S L E K

241 S Y E L P D G Q V I T I G N E R F R C P E T L F Q P S F I G M E S A G I H E T T Y N S I M K C D I D I R K D L Y A N N V M S G G T T M Y P G I A D R M Q K E I T

321 A L A P S T M K I K I I A P P E R K Y S V W I G G S I L A S L S T F Q Q M W I T K Q E Y D E A G P S I V H R K C F

5.191 RAP2B, member of RAS oncogene family [Homo sapiens]

Protein Accession gi|38201690
Mean Expression Ratio 1.11
Median Expression Ratio 1.11
Credible Interval (0.688, 1.78)
Associated Peptides 1
Associated Spectra 1
Coverage 0.0656

| A | 2.5 | 50 | 97.5 | Sequence |
|---|---|---|---|---|
| 1 | 0.68 | 1.1 | 1.9 | ASVDELFAEIVR |

1 M R E Y K V V V L G S G G V G K S A L T V Q F V T G S F I E K Y D P T I E D F Y R K E I E V D S S P S V L E I L D T A G T E Q F A S M R D L Y I K N G Q G F I L

81 V Y S L V N Q Q S F Q D I K P M R D Q I I R V K R Y E R V P M I L V G N K V D L E G E R E V S Y G E G K A L A E E W S C P F M E T S A K N K A S V D E L F A E I

161 V R Q M N Y A A Q P N G D E G C C S A C V I L

5.192 ectonucleotide pyrophosphatase/phosphodiesterase 4 (putative function) [Homo sapiens]

Protein Accession gi|7662358
Mean Expression Ratio 0.907
Median Expression Ratio 0.905
Credible Interval (0.622, 1.33)
Associated Peptides 3
Associated Spectra 3
Coverage 0.0486

| A | 2.5 | 50 | 97.5 | Sequence |
|---|---|---|---|---|
| 1 | 0.58 | 0.9 | 1.4 | IDDLIGDLVQR |
| 1 | 0.58 | 0.9 | 1.4 | LLLVSFDGFR |
| 1 | 0.58 | 0.9 | 1.4 | KIDDLIGDLVQR |

1 M K L L V I L L F S G L I T G F R S D S S S S L P P K L L L V S F D G F R A D Y L K N Y E F P H L Q N F I K E G V L V E H V K N V F I T K T F P N H Y S I V T G

81 L Y E E S H G I V A N S M Y D A V T K K H F S D S N D K D P F W W N E A V P I W V T N Q L Q E N R S S A A A M W P G T D V P I H D T I S S Y F M N Y N S S V S F

161 E E R L N N I T M W L N N S N P P V T F A T L Y W E E P D A S G H K Y G P E D K E N M S R V L K K I D D L I G D L V Q R L K M L G L W E N L N V I I T S D H G M

241 T Q C S Q D R L I N L D S C I D H S Y Y T L I D L S P V A A I L P K I N R T E V Y N K L K N C S P H M N V Y L K E D I P N R F Y Y Q H N D R I Q P I I L V A D E

321 G W T I V L N E S S Q K L G D H G Y D N S L P S M H P F L A A H G P A F H K G Y K H S T I N I V D I Y P M M C H I L G L K P H P N N G T F G H T K C L L V D Q W

401 C I N L P E A I A I V I G S L L V L T M L T C L I I I M Q N R L S V P R P F S R L Q L Q E D D D D P L I G

5.193 transmembrane BAX inhibitor motif containing 1 [Homo sapiens]

Protein Accession gi|50593008
Mean Expression Ratio 0.905
Median Expression Ratio 0.906
Credible Interval (0.645, 1.27)
Associated Peptides 1
Associated Spectra 8
Coverage 0.045

| A | 2.5 | 50 | 97.5 | Sequence |
|---|-----|-----|------|----------|
| 8 | 0.67 | 0.88 | 1.2 | A VSDSFGPGEWDDR |

1 M S N P S A P P P Y E D R N P L Y P G P P P P G G Y G Q P S V L P G G Y P A Y P G Y P Q P G Y G H P A G Y P Q P M P P T H P M P M N Y G P G H G Y D G E E R A V

81 S D S F G P G E W D D R K V R H T F I R K V Y S I I S V Q L L I T V A I I A I F T F V E P V S A F V R R N V A V Y Y V S Y A V F V V T Y L I L A C C Q G P R R R

161 F P W N I I L L T L F T F A M G F M T G T I S S M Y Q T K A V I I A M I I T A V V S I S V T I F C F Q T K V D F T S C T G L F C V L G I V L L V T G I V T S I V

241 L Y F Q Y V V Y W L H M L Y A A L G A I C F T L F L A Y D T Q L V L G N R K H T I S P E D Y I T G A L Q I Y T D I I Y I F T F V L Q L M G D R N

5.194 mucolipin 1 [Homo sapiens]

Protein Accession gi|10092597
Mean Expression Ratio 1.1
Median Expression Ratio 1.10
Credible Interval (0.676, 1.79)
Associated Peptides 1
Associated Spectra 1
Coverage 0.0483

| A | 2.5 | 50 | 97.5 | Sequence |
|---|-----|----|------|----------|
| 1 | 0.67 | 1.1 | 1.9 | LLTPNPGYGTQAGPSPAPPTPPEEEDLR |

```
  1 M T A P A G P R G S E T E R L L T P N P G Y G T Q A G P S P A P P T P P E E E D L
    R R R L K Y F F M S P C D K F R A K G R K P C K L M L Q V V K I L V V T V Q L

 81 I L F G L S N Q L A V T F R E E N T I A F R H L F L L G Y S D G A D D T F A A Y T
    R E Q L Y Q A I F H A V D Q Y L A L P D V S L G R Y A Y V R G G G D P W T N G

161 S G L A L C Q R Y Y H R G H V D P A N D T F D I D P M V V T D C I Q V D P P E
    R P P P P P S D D L T L L E S S S S Y K N L T L K F H K L V N V T I H F R L K T I

241 N L Q S L I N N E I P D C Y T F S V L I T F D N K A H S G R I P I S L E T Q A H I Q
    E C K H P S V F Q H G D N S F R L L F D V V V I L T C S L S F L L C A R S L

321 L R G F L L Q N E F V G F M W R Q R G R V I S L W E R L E F V N G W Y I L L V
    T S D V L T I S G T I M K I G I E A K N L A S Y D V C S I L L G T S T L L V W V G

401 V I R Y L T F F H N Y N I L I A T L R V A L P S V M R F C C C V A V I Y L G Y C
    F C G W I V L G P Y H V K F R S L S M V S E C L F S L I N G D D M F V T F A A M

481 Q A Q Q G R S S L V W L F S Q L Y L Y S F I S L F I Y M V L S L F I A L I T G A Y
    D T I K H P G G A G A E E S E L Q A Y I A Q C Q D S P T S G K F R R G S G S A

561 C S L L C C C G R D P S E E H S L L V N
```

5.195 annexin IV [Homo sapiens]

Protein Accession gi|4502105
Mean Expression Ratio 1.10
Median Expression Ratio 1.10
Credible Interval (0.674, 1.81)
Associated Peptides 1
Associated Spectra 1

Coverage 0.0343

| A | 2.5 | 50 | 97.5 | Sequence |
|---|-----|----|------|----------|
| 1 | 0.67 | 1.1 | 1.9 | NHLLHVFDEYK |

1 M A M A T K G G T V K A A S G F N A M E D A Q T L R K A M K G L G T D E D A I I S V L A Y R N T A Q R Q E I R T A Y K S T I G R D L I D D L K S E L S G N F E Q

81 V I V G M M T P T V L Y D V Q E L R R A M K G A G T D E G C L I E I L A S R T P E E I R R I S Q T Y Q Q Q Y G R S L E D D I R S D T S F M F Q R V L V S L S A G

161 G R D E G N Y L D D A L V R Q D A Q D L Y E A G E K K W G T D E V K F L T V L C S R N R N H L L H V F D E Y K R I S Q K D I E Q S I K S E T S G S F E D A L L A

241 I V K C M R N K S A Y F A E K L Y K S M K G L G T D D N T L I R V M V S R A E I D M L D I R A H F K R L Y G K S L Y S F I K G D T S G D Y R K V L L V L C G G D

321 D

5.196 PREDICTED: similar to kappa immunoglobulin (subgroup V kappa I) [Homo sapiens]

Protein Accession gi|169218204
Mean Expression Ratio 0.907
Median Expression Ratio 0.908
Credible Interval (0.615, 1.35)
Associated Peptides 2
Associated Spectra 3
Coverage NaN

| A | 2.5 | 50 | 97.5 | Sequence |
|---|-----|----|------|----------|
| 1 | 0.57 | 0.89 | 1.4 | DIQLTQSPSFLSASVGDR |
| 2 | 0.6 | 0.9 | 1.3 | LLIYAASTLQSGVPSR |

5.197 diazepam binding inhibitor isoform 2 [Homo sapiens]; diazepam binding inhibitor isoform 3 [Homo sapiens]; diazepam binding inhibitor isoform 1 [Homo sapiens]

Protein Accession gi|120433593 gi|120433590 gi|10140853
Mean Expression Ratio 1.1
Median Expression Ratio 1.10
Credible Interval (0.677, 1.80)
Associated Peptides 1
Associated Spectra 1
Coverage NaN

| A | 2.5 | 50 | 97.5 | Sequence |
|---|---|---|---|---|
| 1 | 0.67 | 1.1 | 1.9 | QATVGDINTERPGMLDFTGK |

5.198 CD63 antigen isoform B [Homo sapiens]; CD63 antigen isoform A [Homo sapiens]

Protein Accession gi|91199546 gi|4502679
Mean Expression Ratio 1.10
Median Expression Ratio 1.10
Credible Interval (0.871, 1.40)
Associated Peptides 8
Associated Spectra 15
Coverage NaN

| A | 2.5 | 50 | 97.5 | Sequence |
|---|---|---|---|---|
| 1 | 0.78 | 1.1 | 1.6 | DKVMSEFNNNFR |
| 7 | 0.83 | 1.1 | 1.4 | VMSEFNNNFR |
| 2 | 0.84 | 1.2 | 1.6 | QQMENYPK |
| 1 | 0.8 | 1.1 | 1.6 | MQADFK |
| 1 | 0.78 | 1.1 | 1.5 | GINFNEK |
| 1 | 0.78 | 1.1 | 1.5 | EGCVEK |
| 1 | 0.78 | 1.1 | 1.6 | IGGWLR |
| 1 | 0.77 | 1.1 | 1.5 | AIHKEGCVEK |

5.199 tubulin, alpha 4a [Homo sapiens]

Protein Accession gi|17921989
Mean Expression Ratio 1.10
Median Expression Ratio 1.1
Credible Interval (0.715, 1.69)
Associated Peptides 1
Associated Spectra 2
Coverage 0.0335

| A | 2.5 | 50 | 97.5 | Sequence |
|---|---|---|---|---|
| 2 | 0.71 | 1.1 | 1.7 | AVFVDLEPTVIDEIR |

1 M R E C I S V H V G Q A G V Q M G N A C W E L Y C L E H G I Q P D G Q M P S D
K T I G G G D D S F T T F F C E T G A G K H V P R A V F V D L E P T V I D E I R N

81 G P Y R Q L F H P E Q L I T G K E D A A N N Y A R G H Y T I G K E I I D P V L D R
I R K L S D Q C T G L Q G F L V F H S F G G G T G S G F T S L L M E R L S V D

161 Y G K K S K L E F S I Y P A P Q V S T A V V E P Y N S I L T T H T T L E H S D C A F M V D N E A I Y D I C R R N L D I E R P T Y T N L N R L I S Q I V S S I T A

241 S L R F D G A L N V D L T E F Q T N L V P Y P R I H F P L A T Y A P V I S A E K A Y H E Q L S V A E I T N A C F E P A N Q M V K C D P R H G K Y M A C C L L Y R

321 G D V V P K D V N A A I A A I K T K R S I Q F V D W C P T G F K V G I N Y Q P P T V V P G G D L A K V Q R A V C M L S N T T A I A E A W A R L D H K F D L M Y A

401 K R A F V H W Y V G E G M E E G E F S E A R E D M A A L E K D Y E E V G I D S Y E D E D E G E E

5.200 MARVEL domain containing 3 isoform 1 [Homo sapiens]

Protein Accession gi|65301122
Mean Expression Ratio 1.10
Median Expression Ratio 1.1
Credible Interval (0.737, 1.62)
Associated Peptides 2
Associated Spectra 3
Coverage 0.061

| A | 2.5 | 50 | 97.5 | Sequence |
|---|-----|----|------|----------|
| 1 | 0.69 | 1.1 | 1.7 | EAPEPPQPQR |
| 2 | 0.75 | 1.1 | 1.7 | GLTWDAAAPPGPAPWEAPEPPQPQR |

1 M E D P S G A R E P R A R P R E R D P G R R P H P D Q G R T H D R P R D R P G D P R R K R S S D G N R R R D G D R D P E R D Q E R D G N R D R N R D R E R E R E

81 R E R D P D R G P R R D T H R D A G P R A G E H G V W E K P R Q S R T R D G A R G L T W D A A A P P G P A P W E A P E P P Q P Q R K G D P G R R R P E S E P P S

161 E R Y L P S T P R P G R E E V E Y Y Q S E A E G L L E C H K C K Y L C T G R G V V Q I V E V V L N G M V L I C I V A S Y F V L A G F S A S F S S G G G F G N N Y

241 Y S P F E G T E L E Q V R Q L D Q Q Y T I L R S P L I Y G G V A V S L G L G V L T M G V L L Q G A K S R T M L S G K W L L T E A A F S L L A A V G Y C T G I G V

321 Y L H V A L Q I N S T D T C K T R E R L Y A R K G L T W M D C Q L A G T D G A A A T F A C L L V I M Y G A S V V L A L R S Y R E Q K R Y K G S R E Q P G S Y S D

401 A P E Y L W S G T L

5.201 sialin [Homo sapiens]

Protein Accession gi|6912666
Mean Expression Ratio 0.91
Median Expression Ratio 0.91
Credible Interval (0.65, 1.25)
Associated Peptides 4
Associated Spectra 5
Coverage 0.0848

| A | 2.5 | 50 | 97.5 | Sequence |
|---|-----|-----|------|----------|
| 2 | 0.64 | 0.94 | 1.4 | ACPEHSAPIK |
| 1 | 0.6 | 0.91 | 1.4 | ALEGLGEGVTFPAMH |
| 1 | 0.58 | 0.87 | 1.3 | EYILSSLR |
| 1 | 0.6 | 0.9 | 1.4 | TPLLPGAPR |

1 M R S P V R D L A R N D G E E S T D R T P L L P G A P R A E A A P V C C S A R Y
N L A I L A F F G F F I V Y A L R V N L S V A L V D M V D S N T T L E D N R T S

81 K A C P E H S A P I K V H H N Q T G K K Y Q W D A E T Q G W I L G S F F Y G Y I
I T Q I P G G Y V A S K I G G K M L L G F G I L G T A V L T L F T P I A A D L G

161 V G P L I V L R A L E G L G E G V T F P A M H A M W S S W A P P L E R S K L L
S I S Y A G A Q L G T V I S L P L S G I I C Y Y M N W T Y V F Y F F G T I G I F W

241 F L L W I W L V S D T P Q K H K R I S H Y E K E Y I L S S L R N Q L S S Q K S V
P W V P I L K S L P L W A I V V A H F S Y N W T F Y T L L T L L P T Y M K E I L

321 R F N V Q E N G F L S S L P Y L G S W L C M I L S G Q A A D N L R A K W N F S
T L C V R R I F S L I G M I G P A V F L V A A G F I G C D Y S L A V A F L T I S T

401 T L G G F C S S G F S I N H L D I A P S Y A G I L L G I T N T F A T I P G M V G P
V I A K S L T P D N T V G E W Q T V F Y I A A A I N V F G A I F F T L F A K G

481 E V Q N W A L N D H H G H R H

5.202 solute carrier family 1 (neutral amino acid transporter), member 5 [Homo sapiens]

Protein Accession gi|5032093
Mean Expression Ratio 0.912
Median Expression Ratio 0.91
Credible Interval (0.561, 1.48)
Associated Peptides 1
Associated Spectra 1

Coverage 0.0203

| A | 2.5 | 50 | 97.5 | Sequence |
|---|---|---|---|---|
| 1 | 0.54 | 0.89 | 1.5 | EVLDSFLDLAR |

1 M V A D P P R D S K G L A A A E P T A N G G L A L A S I E D Q G A A A G G Y C G S R D Q V R R C L R A N L L V L L T V V A V V A G V A L G L G V S G A G G A L A

81 L G P E R L S A F V F P G E L L L R L L R M I I L P L V V C S L I G G A A S L D P G A L G R L G A W A L L F F L V T T L L A S A L G V G L A L A L Q P G A A S A

161 A I N A S V G A A G S A E N A P S K E V L D S F L D L A R N I F P S N L V S A A F R S Y S T T Y E E R N I T G T R V K V P V G Q E V E G M N I L G L V V F A I V

241 F G V A L R K L G P E G E L L I R F F N S F N E A T M V L V S W I M W Y A P V G I M F L V A G K I V E M E D V G L L F A R L G K Y I L C C L L G H A I H G L L V

321 L P L I Y F L F T R K N P Y R F L W G I V T P L A T A F G T S S S S A T L P L M M K C V E E N N G V A K H I S R F I L P I G A T V N M D G A A L F Q C V A A V F

401 I A Q L S Q Q S L D F V K I I T I L V T A T A S S V G A A G I P A G G V L T L A I I L E A V N L P V D H I S L I L A V D W L V D R S C T V L N V E G D A L G A G

481 L L Q N Y V D R T E S R S T E P E L I Q V K S E L P L D P L P V P T E E G N P L L K H Y R G P A G D A T V A S E K E S V M

5.203 claudin 2 [Homo sapiens]

Protein Accession gi|9966781
Mean Expression Ratio 1.10
Median Expression Ratio 1.10
Credible Interval (0.686, 1.79)
Associated Peptides 1
Associated Spectra 1
Coverage 0.0652

| A | 2.5 | 50 | 97.5 | Sequence |
|---|---|---|---|---|
| 1 | 0.68 | 1.1 | 1.9 | SNYYDAYQAQPLATR |

1 M A S L G L Q L V G Y I L G L L G L L G T L V A M L L P S W K T S S Y V G A S I V T A V G F S K G L W M E C A T H S T G I T Q C D I Y S T L L G L P A D I Q A A

81 Q A M M V T S S A I S S L A C I I S V V G M R C T V F C Q E S R A K D R V A V A G G V F F I L G G L L G F I P V A W N L H G I L R D F Y S P L V P D S M K F E I

161 G E A L Y L G I I S S L F S L I A G I I L C F S C S S Q R N R S N Y Y D A Y Q A Q P L A T R S S P R P G Q P P K V K S E F N S Y S L T G Y V

5.204 myoferlin isoform a [Homo sapiens]; myoferlin isoform b [Homo sapiens]

    Protein Accession gi|7305053 gi|19718759
    Mean Expression Ratio 1.10
    Median Expression Ratio 1.10
    Credible Interval (0.677, 1.78)
    Associated Peptides 1
    Associated Spectra 1
    Coverage NaN

| A | 2.5 | 50 | 97.5 | Sequence |
|---|---|---|---|---|
| 1 | 0.66 | 1.1 | 1.9 | IYPLPDDPSVPAPPR |

5.205 tubulin, alpha 1a [Homo sapiens]

    Protein Accession gi|17986283
    Mean Expression Ratio 1.10
    Median Expression Ratio 1.10
    Credible Interval (0.708, 1.68)
    Associated Peptides 2
    Associated Spectra 2
    Coverage 0.0532

| A | 2.5 | 50 | 97.5 | Sequence |
|---|---|---|---|---|
| 1 | 0.7 | 1.1 | 1.8 | AVFVDLEPTVIDEVR |
| 1 | 0.67 | 1.1 | 1.7 | EIIDLVLDR |

1 M R E C I S I H V G Q A G V Q I G N A C W E L Y C L E H G I Q P D G Q M P S D K T I G G G D D S F N T F F S E T G A G K H V P R A V F V D L E P T V I D E V R T

81 G T Y R Q L F H P E Q L I T G K E D A A N N Y A R G H Y T I G K E I I D L V L D R I R K L A D Q C T G L Q G F L V F H S F G G G T G S G F T S L L M E R L S V D

161 Y G K K S K L E F S I Y P A P Q V S T A V V E P Y N S I L T T H T T L E H S D C A F M V D N E A I Y D I C R R N L D I E R P T Y T N L N R L I G Q I V S S I T A

241 S L R F D G A L N V D L T E F Q T N L V P Y P R I H F P L A T Y A P V I S A E K A Y H E Q L S V A E I T N A C F E P A N Q M V K C D P R H G K Y M A C C L L Y R

321 G D V V P K D V N A A I A T I K T K R T I Q F V D W C P T G F K V G I N Y Q P P T V V P G G D L A K V Q R A V C M L S N T T A I A E A W A R L D H K F D L M Y A

401 K R A F V H W Y V G E G M E E G E F S E A R E D M A A L E K D Y E E V G V D S V E G E G E E E G E E Y

5.206 PDZ domain containing 1 [Homo sapiens]

Protein Accession gi|21361142
Mean Expression Ratio 0.913
Median Expression Ratio 0.915
Credible Interval (0.722, 1.15)
Associated Peptides 8
Associated Spectra 14
Coverage 0.212

| A | 2.5 | 50 | 97.5 | Sequence |
|---|-----|------|------|----------|
| 2 | 0.66 | 0.9 | 1.2 | AGVLADDHLIEVNGENVEDASHEEVVEK |
| 2 | 0.63 | 0.87 | 1.2 | EAPAPTPTSLEVSSPPDTTEEVDHKPK |
| 5 | 0.66 | 0.87 | 1.2 | INGVFVDKEEHMQVVDLVR |
| 1 | 0.66 | 0.93 | 1.3 | GVYMTDITPQGVAMR |
| 1 | 0.66 | 0.93 | 1.3 | MTDITPQGVAMR |
| 1 | 0.63 | 0.89 | 1.3 | VMFLLVDK |
| 1 | 0.66 | 0.94 | 1.3 | VMFLLVDKETDKR |
| 1 | 0.67 | 0.95 | 1.3 | AYDYFQAK |

1 M T S T F N P R E C K L S K Q E G Q N Y G F F L R I E K D T E G H L V R V V E K C S P A E K A G L Q D G D R V L R I N G V F V D K E E H M Q V V D L V R K S G N

81 S V T L L V L D G D S Y E K A V K T R V D L K E L G Q S Q K E Q G L S D N I L S P V M N G G V Q T W T Q P R L C Y L V K E G G S Y G F S L K T V Q G K K G V Y M

161 T D I T P Q G V A M R A G V L A D D H L I E V N G E N V E D A S H E E V V E K V K K S G S R V M F L L V D K E T D K R H V E Q K I Q F K R E T A S L K L L P H Q

241 P R I V E M K K G S N G Y G F Y L R A G S E Q K G Q I I K D I D S G S P A E E A G L K N N D L V V A V N G E S V E T L D H D S V V E M I R K G G D Q T S L L V V

321 D K E T D N M Y R L A H F S P F L Y Y Q S Q E L P N G S V K E A P A P T P T S L E V S S P P D T T E E V D H K P K L C R L A K G E N G Y G F H L N A I R G L P G

401 S F I K E V Q K G G P A D L A G L E D E D V I I E V N G V N V L D E P Y E K V V D R I Q S S G K N V T L L V C G K K A Y D Y F Q A K K I P I V S S L A D P L D T

481 P P D S K E G I V V E S N H D S H M A K E R A H S T A S H S S S N S E D T E M

5.207 deleted in malignant brain tumors 1 isoform c precursor [Homo sapiens]; deleted in malignant brain tumors 1 isoform b precursor [Homo sapiens]

Protein Accession gi|148539844 gi|148539842
Mean Expression Ratio 1.09
Median Expression Ratio 1.09
Credible Interval (0.668, 1.80)
Associated Peptides 1
Associated Spectra 1
Coverage NaN

| A | 2.5 | 50 | 97.5 | Sequence |
|---|-----|-----|------|----------|
| 1 | 0.67 | 1.1 | 1.9 | FGQGSGPIVLDDVR |

5.208 galactosylceramidase isoform a precursor [Homo sapiens]

Protein Accession gi|83281450
Mean Expression Ratio 0.915
Median Expression Ratio 0.916
Credible Interval (0.556, 1.50)
Associated Peptides 1
Associated Spectra 1
Coverage 0.0234

| A | 2.5 | 50 | 97.5 | Sequence |
|---|-----|-----|------|----------|
| 1 | 0.53 | 0.9 | 1.5 | EFDGIGAVSGGGATSR |

1 M A E W L L S A S W Q R R A K A M T A A A G S A G R A A V P L L L C A L L A P G G A Y V L D D S D G L G R E F D G I G A V S G G G A T S R L L V N Y P E P Y R S

81 Q I L D Y L F K P N F G A S L H I L K V E I G G D G Q T T D G T E P S H M H Y A L D E N Y F R G Y E W W L M K E A K K R N P N I T L I G L P W S F P G W L G K G

161 F D W P Y V N L Q L T A Y Y V V T W I V G A K R Y H D L D I D Y I G I W N E R S Y N A N Y I K I L R K M L N Y Q G L Q R V K I I A S D N L W E S I S A S M L L D

241 A E L F K V V D V I G A H Y P G T H S A K D A K L T G K K L W S S E D F S T L N S D M G A G C W G R I L N Q N Y I N G Y M T S T I A W N L V A S Y Y E Q L P Y G

321 R C G L M T A Q E P W S G H Y V V E S P V W V S A H T T Q F T Q P G W Y Y L K T V G H L E K G G S Y V A L T D G L G N L T I I E T M S H K H S K C I R P F L P

401 Y F N V S Q Q F A T F V L K G S F S E I P E L Q V W Y T K L G K T S E R F L F K Q L D S L W L L D S D G S F T L S L H E D E L F T L T T L T T G R K G S Y P L P

481 P K S Q P F P S T Y K D D F N V D Y P F F S E A P N F A D Q T G V F E Y F T N I E D P G E H H F T L R Q V L N Q R P I T W A A D A S N T I S I I G D Y N W T N L

561 T I K C D V Y I E T P D T G G V F I A G R V N K G G I L I R S A R G I F F W I F A N G S Y R V T G D L A G W I I Y A L G R V E V T A K K W Y T L T L T I K G H F

641 T S G M L N D K S L W T D I P V N F P K N G W A A I G T H S F E F A Q F D N F L V E A T R

5.209 target of myb1 isoform 1 [Homo sapiens]; target of myb1 isoform 2 [Homo sapiens]

Protein Accession gi|4885637 gi|209180457
Mean Expression Ratio 0.916
Median Expression Ratio 0.917
Credible Interval (0.615, 1.34)
Associated Peptides 2
Associated Spectra 3
Coverage NaN

| A | 2.5 | 50 | 97.5 | Sequence |
|---|-----|-----|------|----------|
| 2 | 0.58 | 0.89 | 1.3 | LPNLSSPSAEGPPGPPSGPAPR |
| 1 | 0.6 | 0.93 | 1.5 | SSPDLTGVVTIYEDLR |

5.210 hypothetical protein LOC55194 [Homo sapiens]; PREDICTED: hypothetical protein [Homo sapiens]

Protein Accession gi|8922567 gi|169160626
Mean Expression Ratio 1.09
Median Expression Ratio 1.09
Credible Interval (0.666, 1.81)
Associated Peptides 1
Associated Spectra 1
Coverage NaN

| A | 2.5 | 50 | 97.5 | Sequence |
|---|-----|----|------|----------|
| 1 | 0.67 | 1.1 | 1.9 | TGQPDLLGTGTLGPSPTATGTLGR |

5.211 cystin 1 [Homo sapiens]

Protein Accession gi|81158080
Mean Expression Ratio 1.09
Median Expression Ratio 1.09
Credible Interval (0.737, 1.62)
Associated Peptides 2
Associated Spectra 3
Coverage 0.241

| A | 2.5 | 50 | 97.5 | Sequence |
|---|-----|----|------|----------|
| 1 | 0.71 | 1.1 | 1.7 | SPESLPAGPGAAALEGGTR |
| 2 | 0.73 | 1.1 | 1.7 | VPVAAAEVPGAAAEEAPGR |

1 M G S G S S R S S R T L R R R S P E S L P A G P G A A A L E G G T R R R V P V A
A A E V P G A A A E E A P G R D P S P V A P P D G R D E T L R L L D E L L A E

81 S A A W G P P E P A P R R P A R L R P T A V A G S A V C A E Q S T E G H P G S G
N V S E A P G S G R K K P E R P A A I S Y D H S E E G L M A S I E R E Y C R

5.212 CD151 antigen [Homo sapiens]; CD151 antigen [Homo sapiens]; CD151 antigen [Homo sapiens]; CD151 antigen [Homo sapiens]

Protein Accession gi|87159824 gi|87159822 gi|21237751 gi|21237748
Mean Expression Ratio 1.09
Median Expression Ratio 1.09
Credible Interval (0.733, 1.62)
Associated Peptides 1
Associated Spectra 3
Coverage NaN

| A | 2.5 | 50 | 97.5 | Sequence |
|---|-----|----|------|----------|
| 3 | 0.76 | 1.1 | 1.6 | LETFIQEHLR |

5.213 RAB2A, member RAS oncogene family [Homo sapiens]

Protein Accession gi|4506365
Mean Expression Ratio 0.921
Median Expression Ratio 0.921

Credible Interval (0.596, 1.43)
Associated Peptides 1
Associated Spectra 2
Coverage 0.066

| A | 2.5 | 50 | 97.5 | Sequence |
|---|-----|-----|------|----------|
| 2 | 0.58 | 0.9 | 1.4 | DTFNHLTTWLEDAR |

1 M A Y A Y L F K Y I I I G D T G V G K S C L L L Q F T D K R F Q P V H D L T I G V E F G A R M I T I D G K Q I K L Q I W D T A G Q E S F R S I T R S Y Y R G A A

81 G A L L V Y D I T R R D T F N H L T T W L E D A R Q H S N S N M V I M L I G N K S D L E S R R E V K K E E G E A F A R E H G L I F M E T S A K T A S N V E E A F

161 I N T A K E I Y E K I Q E G V F D I N N E A N G I K I G P Q H A A T N A T H A G N Q G G Q Q A G G G C C

5.214 CNDP dipeptidase 2 [Homo sapiens]

Protein Accession gi|8922699
Mean Expression Ratio 1.08
Median Expression Ratio 1.09
Credible Interval (0.811, 1.46)
Associated Peptides 5
Associated Spectra 8
Coverage 0.135

| A | 2.5 | 50 | 97.5 | Sequence |
|---|-----|-----|------|----------|
| 1 | 0.75 | 1.1 | 1.6 | GNILIPGINEAVAAVTEEEHK |
| 3 | 0.77 | 1.1 | 1.5 | LPDGSEIPLPPILLGR |
| 1 | 0.74 | 1.1 | 1.6 | LYDDIDFDIEEFAK |
| 2 | 0.77 | 1.1 | 1.6 | QKLPDGSEIPLPPILLGR |
| 1 | 0.76 | 1.1 | 1.6 | TVFGVEPDLTR |

1 M A A L T T L F K Y I D E N Q D R Y I K K L A K W V A I Q S V S A W P E K R G E I R R M M E V A A A D V K Q L G G S V E L V D I G K Q K L P D G S E I P L P P I

81 L L G R L G S D P Q K K T V C I Y G H L D V Q P A A L E D G W D S E P F T L V E R D G K L Y G G G S T D D K G P V A G W I N A L E A Y Q K T G Q E I P V N V R F

161 C L E G M E E S G S E G L D E L I F A R K D T F F K D V D Y V C I S D N Y W L G K K K P C I T Y G L R G I C Y F F I E V E C S N K D L H S G V Y G G S V H E A M

241 T D L I L L M G S L V D K R G N I L I P G I N E A V A A V T E E E H K L Y D D I D F D I E E F A K D V G A Q I L L H S H K K D I L M H R W R Y P S L S L H G I E

321 G A F S G S G A K T V I P R K V V G K F S I R L V P N M T P E V V G E Q V T S Y L T K K F A E L R S P N E F K V Y M G H G G K P W V S D F S H P H Y L A G R R A

401 M K T V F G V E P D L T R E G G S I P V T L T F Q E A T G K N V M L L P V G S A D D G A H S Q N E K L N R Y N Y I E G T K M L A A Y L Y E V S Q L K D

5.215 syntaxin binding protein 2 isoform b [Homo sapiens]; syntaxin binding protein 2 isoform a [Homo sapiens]

Protein Accession gi|188528901 gi|188528689
Mean Expression Ratio 1.09
Median Expression Ratio 1.09
Credible Interval (0.709, 1.67)
Associated Peptides 2
Associated Spectra 2
Coverage NaN

| A | 2.5 | 50 | 97.5 | Sequence |
|---|-----|----|------|----------|
| 1 | 0.7 | 1.1 | 1.8 | NLEQLGGTVTNPGGSGTSSR |
| 1 | 0.67 | 1.1 | 1.7 | VLLLYILLR |

5.216 actinin, alpha 4 [Homo sapiens]

Protein Accession gi|12025678
Mean Expression Ratio 1.09
Median Expression Ratio 1.08
Credible Interval (0.672, 1.74)
Associated Peptides 1
Associated Spectra 1
Coverage 0.0132

| A | 2.5 | 50 | 97.5 | Sequence |
|---|-----|----|------|----------|
| 1 | 0.65 | 1.1 | 1.8 | VGWEQLLTTIAR |

1 M V D Y H A A N Q S Y Q Y G P S S A G N G A G G G G S M G D Y M A Q E D D W D R D L L L D P A W E K Q Q R K T F T A W C N S H L R K A G T Q I E N I D E D F R D

81 G L K L M L L L E V I S G E R L P K P E R G K M R V H K I N N V N K A L D F I A S K G V K L V S I G A E E I V D G N A K M T L G M I W T I I L R F A I Q D I S V

161 E E T S A K E G L L L W C Q R K T A P Y K N V N V Q N F H I S W K D G L A F N A L I H R H R P E L I E Y D K L R K D D P V T N L N N A F E V A E K Y L D I P K M

241 L D A E D I V N T A R P D E K A I M T Y V S S F Y H A F S G A Q K A E T A A N R I C K V L A V N Q E N E H L M E D Y E K L A S D L L E W I R R T I P W L E D R V

321 P Q K T I Q E M Q Q K L E D F R D Y R R V H K P P K V Q E K C Q L E I N F N T L Q T K L R L S N R P A F M P S E G K M V S D I N N G W Q H L E Q A E K G Y E E W

401 L L N E I R R L E R L D H L A E K F R Q K A S I H E A W T D G K E A M L K H R D Y E T A T L S D I K A L I R K H E A F E S D L A A H Q D R V E Q I A A I A Q E L

481 N E L D Y Y D S H N V N T R C Q K I C D Q W D A L G S L T H S R R E A L E K T E K Q L E A I D Q L H L E Y A K R A A P F N N W M E S A M E D L Q D M F I V H T I

561 E E I E G L I S A H D Q F K S T L P D A D R E R E A I L A I H K E A Q R I A E S N H I K L S G S N P Y T T V T P Q I I N S K W E K V Q Q L V P K R D H A L L E E

641 Q S K Q Q S N E H L R R Q F A S Q A N V V G P W I Q T K M E E I G R I S I E M N G T L E D Q L S H L K Q Y E R S I V D Y K P N L D L L E Q Q H Q L I Q E A L I F

721 D N K H T N Y T M E H I R V G W E Q L L T T I A R T I N E V E N Q I L T R D A K G I S Q E Q M Q E F R A S F N H F D K D H G G A L G P E E F K A C L I S L G Y D

801 V E N D R Q G E A E F N R I M S L V D P N H S G L V T F Q A F I D F M S R E T T D T D T A D Q V I A S F K V L A G D K N F I T A E E L R R E L P P D Q A E Y C I

881 A R M A P Y Q G P D A V P G A L D Y K S F S T A L Y G E S D L

5.217 matrix metalloproteinase 7 preproprotein [Homo sapiens]

Protein Accession gi|4505219
Mean Expression Ratio 0.919
Median Expression Ratio 0.923
Credible Interval (0.603, 1.41)
Associated Peptides 2
Associated Spectra 2
Coverage 0.120

| A | 2.5 | 50 | 97.5 | Sequence |
|---|---|---|---|---|
| 1 | 0.57 | 0.9 | 1.4 | AFAPGTGLGGDAHFDEDER |
| 1 | 0.57 | 0.91 | 1.5 | FFGLPITGMLNSR |

MRLTVLCAVCLLPGSLALPLPQEAGGMSELQWEQAQDYLKRFYLYDSETKNANSLEAKLKEMQKFFGLPITGMLNSRVIE

IMQKPRCGVPDVAEYSLFPNSPKWTSKVVTYRIVSYTRDLPHITVDRLVSKALNMWGKEIPLHFRKVVWGTADIMIGFAR

GAHGDSYPFDGPGNTLAHAFAPGTGLGGDAHFDEDERWTDGSSLGINFLYAATHELGHSLGMGHSSDPNAVMYPTYGNGD

PQNFKLSQDDIKGIQKLYGKRSNSRKK

5.218 keratin 1 [Homo sapiens]

Protein Accession gi|119395750
Mean Expression Ratio 0.923
Median Expression Ratio 0.923
Credible Interval (0.668, 1.28)
Associated Peptides 3
Associated Spectra 6
Coverage 0.0637

| A | 2.5 | 50 | 97.5 | Sequence |
|---|-----|-----|------|----------|
| 1 | 0.6 | 0.9 | 1.3 | QISNLQQSISDAEQR |
| 4 | 0.68 | 0.94 | 1.3 | THNLEPYFESFINNLR |
| 1 | 0.62 | 0.91 | 1.4 | NKYEDEINKR |

MSRQFSSRSGYRSGGGFSSGSAGIINYQRRTTSSSTRRSGGGGGRFSSCGGGGGGSFGAGGGFGSRSLVNLGGSKSISISV

ARGGGRGSGFGGGYGGGGFGGGGFGGGGFGGGGIGGGGFGGFGSGGGGFGGGGFGGGGYGGGYGPVCPPGGIQEVTINQS

LLQPLNVEIDPEIQKVKSREREQIKSLNNQFASFIDKVRFLEQQNQVLQTKWELLQQVDTSTRTHNLEPYFESFINNLRR

RVDQLKSDQSRLDSELKNMQDMVEDYRNKYEDEINKRTNAENEFVTIKKDVDGAYMTKVDLQAKLDNLQQEIDFLTALYQ

AELSQMQTQISETNVILSMDNNRSLDLDSIIAEVKAQYEDIAQKSKAEAESLYQSKYEELQITAGRHGDSVRNSKIEISE

401 L N R V I Q R L R S E I D N V K K Q I S N L Q Q S I S D A E Q R G E N A L K D A K N K L N D L E D A L Q Q A K E D L A R L L R D Y Q E L M N T K L A L D L E I A

481 T Y R T L L E G E E S R M S G E C A P N V S V S V S T S H T T I S G G G S R G G G G G G Y G S G G S S Y G S G G G S Y G S G G G G G G G R G S Y G S G G S S Y G

561 S G G G S Y G S G G G G G G H G S Y G S G S S S G G Y R G G S G G G G G G G S S G G R G S G G G G S S G G S I G G R G S S S S G G V K S S G G G S S S V K F V S T T Y S

641 GVTR

5.219 lactotransferrin precursor [Homo sapiens]

Protein Accession gi|54607120
Mean Expression Ratio 0.925
Median Expression Ratio 0.924
Credible Interval (0.744, 1.15)
Associated Peptides 12
Associated Spectra 16
Coverage 0.217

| A | 2.5 | 50 | 97.5 | Sequence |
|---|-----|-----|------|----------|
| 2 | 0.66 | 0.9 | 1.2 | CSTSPLLEACEFLR |
| 2 | 0.69 | 0.94 | 1.3 | DEYELLCPDNTR |
| 1 | 0.67 | 0.94 | 1.3 | DSPIQCIQAIAENR |
| 1 | 0.65 | 0.92 | 1.3 | DVTVLQNTDGNNNEAWAK |
| 1 | 0.68 | 0.95 | 1.3 | ESTVFEDLSDEAER |
| 2 | 0.63 | 0.85 | 1.2 | ESTVFEDLSDEAERDEYELLCPDNTR |
| 1 | 0.66 | 0.93 | 1.3 | FDEYFSQSCAPGSDPR |
| 1 | 0.68 | 0.95 | 1.3 | FQLFGSPSGQK |
| 1 | 0.66 | 0.93 | 1.3 | GGSFQLNELQGLK |
| 1 | 0.65 | 0.91 | 1.3 | IDSGLYLGSGYFTAIQNLR |
| 2 | 0.69 | 0.94 | 1.3 | QVLLHQQAK |
| 1 | 0.67 | 0.93 | 1.3 | YLGPQYVAGITNLK |

1 M K L V F L V L L F L G A L G L C L A G R R R S V Q W C A V S Q P E A T K C F Q W Q R N M R K V R G P P V S C I K R D S P I Q C I Q A I A E N R A D A V T L D G

81 G F I Y E A G L A P Y K L R P V A A E V Y G T E R Q P R T H Y Y A V A V V K K G G S F Q L N E L Q G L K S C H T G L R R T A G W N V P I G T L R P F L N W T G P

161 P E P I E A A V A R F F S A S C V P G A D K G Q F P N L C R L C A G T G E N K C A F S S Q E P Y F S Y S G A F K C L R D G A G D V A F I R E S T V F E D L S D E

241 A E R D E Y E L L C P D N T R K P V D K F K D C H L A R V P S H A V V A R S V N G K E D A I W N L L R Q A Q E K F G K D K S P K F Q L F G S P S G Q K D L L F K

321 D S A I G F S R V P P R I D S G L Y L G S G Y F T A I Q N L R K S E E E V A A R R A R V V W C A V G E Q E L R K C N Q W S G L S E G S V T C S S A S T T E D C I

401 A L V L K G E A D A M S L D G G Y V Y T A G K C G L V P V L A E N Y K S Q Q S S D P D P N C V D R P V E G Y L A V A V V R R S D T S L T W N S V K G K K S C H T

481 A V D R T A G W N I P M G L L F N Q T G S C K F D E Y F S Q S C A P G S D P R S N L C A L C I G D E Q G E N K C V P N S N E R Y Y G Y T G A F R C L A E N A G D

561 V A F V K D V T V L Q N T D G N N N E A W A K D L K L A D F A L L C L D G K R K P V T E A R S C H L A M A P N H A V V S R M D K V E R L K Q V L L H Q Q A K F G

641 R N G S D C P D K F C L F Q S E T K N L L F N D N T E C L A R L H G K T T Y E K Y L G P Q Y V A G I T N L K K C S T S P L L E A C E F L R K

5.220 tumor differentially expressed protein 1 [Homo sapiens]; tumor differentially expressed protein 1 [Homo sapiens]

Protein Accession gi|5803193 gi|39812106
Mean Expression Ratio 0.926
Median Expression Ratio 0.926
Credible Interval (0.567, 1.51)
Associated Peptides 1
Associated Spectra 1
Coverage NaN

| A | 2.5 | 50 | 97.5 | Sequence |
|---|-----|-----|------|----------|
| 1 | 0.54 | 0.9 | 1.5 | LTLSGSDSVILGDTTTSGASDEEDGQPR |

5.221 phosphodiesterase 8A isoform 2 [Homo sapiens]; phosphodiesterase 8A isoform 1 [Homo sapiens]

Protein Accession gi|47132537 gi|27734721
Mean Expression Ratio 1.08
Median Expression Ratio 1.08
Credible Interval (0.667, 1.74)
Associated Peptides 1
Associated Spectra 1

Coverage NaN

| A | 2.5 | 50 | 97.5 | Sequence |
|---|-----|-----|------|----------|
| 1 | 0.66 | 1.1 | 1.8 | LVAEDAPSPAAPPLSSGGPR |

5.222 SH3 domain and tetratricopeptide repeats 1 [Homo sapiens]

Protein Accession gi|145386551
Mean Expression Ratio 0.928
Median Expression Ratio 0.927
Credible Interval (0.572, 1.50)
Associated Peptides 1
Associated Spectra 1
Coverage 0.0112

| A | 2.5 | 50 | 97.5 | Sequence |
|---|-----|-----|------|----------|
| 1 | 0.54 | 0.9 | 1.5 | QALASLTPGTGQALR |

```
  1 MENLPAVTTEEPTPMGRGPVGPSGGGSTRDQVRTVVMRPS
    VSWEKAGPEEAKAPVRGDEAPPARVAGPAAGTPPCQMGVY

 81 PTDLTLQLLAVRRKSRLRDPGLQQTLRGQLRLLENDSREM
    ARVLGELSARLLSIHSDQDRIVVTFKTFEEIWKFSTYHAL

161 GFTHHCLANLLMDQAFWLLLPSEEEETAIQVHVDENALR
    LTHESLLIQEGPFFVLCPDHHVRVMTGPRDAGNGPQALRQA

241 SGAPQGEAAPETDSSPPSPSVSSEEVAVAAAPEPLIPFHQ
    WALRIPQDPIDDAMGGPVMPGNPLMAVGLASALADFQGSG

321 PEEMTFRGGDLIEILGAQVPSLPWCVGRHAASGRVGFVRS
    SLISMQGPVSELESAIFLNEEEKSFFSEGCFSEEDARQLL

401 RRMSGTDVCSVYSLDSVEEAETEQPQEKEIPPPCLSPEPQ
    ETLQKVKNVLEQCKTCPGCPQEPASWGLCAASSDVSLQDP

481 EEPSFCLEAEDDWEDPEALSSLLLFLNAPGYKASFRGLYD
    VALPWLSSVFRSFSDEEELTGRLAQARGAAKKAGLLMALA

561 RLCFLLGRLCSRRLKLSQARVYFEEALGALEGSFGDLFLV
    VAVYANLASIYRKQKNREKCAQVVPKAMALLLGTPDHICS
```

641 TEAEGELLQLALRRAVGGQSLQAEARACFLLARHHVHLK QPEEALPFLERLLLLHRDSGAPEAAWLSDCYLLLADIYSRK

721 CLPHLVLSCVKVASLRTRGSLAGSLRSVNLVLQNAPQPHS LPAQTSHYLRQALASLTPGTGQALRGPLYTSLAQLYSHHG

801 CHGPAITFMTQAVEASAIAGVRAIVDHLVALAWLHVLHG QSPVALDILQSVRDAVVASEDQEGVIANMVAVALKRTGRTR

881 QAAESYYRALRVARDLGQQRNQAVGLANFGALCLHAGA SRLAQHYLLEAVRLFSRLPLGECGRDFTHVLLQLGHLCTRQ G

961 PAQQGKGYYEWALLVAVEMGHVESQLRAVQRLCHFYSA VMPSEAQCVIYHELQLSLACKVADKVLEGQLLETISQLYLSL

1041 GTERAYKSALDYTKRSLGIFIDLQKKEKEAHAWLQAGKI YYILRQSELVDLYIQVAQNVALYTGDPNLGLELFEAAGDIF

1121 FDGAWEREKAVSFYRDRALPLAVTTGNRKAELRLCNKL VALLATLEEPQEGLEFAHMALALSITLGDRLNERVAYHRLA A

1201 LQHRLGHGELAEHFYLKALSLCNSPLEFDEETLYYVKVY LVLGDIIFYDLKDPFDAAGYYQLALAAAVDLGNKKAQLKIY

1281 TRLATIYHNFLLDREKSLFFYQKARTFATELNVRRVNLP PLPLCGWAPWLAPSHPR

5.223 aminoacylase 1 [Homo sapiens]

Protein Accession gi|4501901
Mean Expression Ratio 0.926
Median Expression Ratio 0.927
Credible Interval (0.649, 1.32)
Associated Peptides 3
Associated Spectra 4
Coverage 0.125

| A | 2.5 | 50 | 97.5 | Sequence |
|---|-----|-----|------|----------|
| 1 | 0.59 | 0.91 | 1.4 | DMNLTLEPEIMPAATDNR |
| 1 | 0.61 | 0.92 | 1.4 | GPEEEHPSVTLFR |
| 2 | 0.63 | 0.93 | 1.4 | TVQPKPDYGAAVAFFEETAR |

1 MTSKGPEEHPSVTLFRQYLRIRTVQPKPDYGAAVAFFEET ARQLGLGCQKVEVAPGYVVTVLTWPGTNPTLSSILLNSH

81 TDVVPVFKEHWSHDPFEAFKDSEGYIYARGAQDMKCVSIQ YLEAVRRLKVEGHRFPRTIHMTFVPDEEVGGHQGMELFVQ

161 RPEFHALRAGFALDEGIANPTDAFTVFYSERSPWWVRVT STGRPGHASRFMEDTAAEKLHKVVNSILAFREKEWQRLQSN

241 PHLKEGSVTSVNLTKLEGGVAYNVIPATMSASFDFRVAP DVDFKAFEEQLQSWCQAAGEGVTLEFAQKWMHPQVTPTDD S

321 NPWWAAFSRVCKDMNLTLEPEIMPAATDNRYIRAVGVPA LGFSPMNRTPVLLHDHDERLHEAVFLRGVDIYTRLLPALAS

401 VPALPSDS

5.224 cathepsin A isoform a precursor [Homo sapiens]

Protein Accession gi|119395729
Mean Expression Ratio 0.928
Median Expression Ratio 0.928
Credible Interval (0.708, 1.21)
Associated Peptides 9
Associated Spectra 9
Coverage 0.207

| A | 2.5 | 50 | 97.5 | Sequence |
|---|---|---|---|---|
| 1 | 0.66 | 0.94 | 1.3 | CNFYDNK |
| 1 | 0.65 | 0.94 | 1.3 | DLECVTNLQEVAR |
| 1 | 0.67 | 0.96 | 1.4 | HLHYWFVESQK |
| 1 | 0.64 | 0.91 | 1.3 | NFLVNLQYR |
| 1 | 0.66 | 0.93 | 1.3 | QYSGYLK |
| 1 | 0.65 | 0.94 | 1.3 | YGDSGEQIAGFVK |
| 1 | 0.61 | 0.88 | 1.3 | YATNDTEVAQSNFEALQDFFR |
| 1 | 0.64 | 0.91 | 1.3 | ALNIPEQLPQWDMCNFLVNLQYR |
| 1 | 0.63 | 0.9 | 1.3 | EFSHIAFL |

1 MTSSPRAPPGEQGRGGAEMIRAAPPPLFLLLLLLLLLVSWA SRGEAAPDQDEIQRLPGLAKQPSFRQYSGYLKGSGSKHL

81 HYWFVESQKDPENSPVVLWLNGGPGCSSLDGLLTEHGPFL VQPDGVTLEYNPYSWNLIANVLYLESPAGVGFSYSDDKFY

161 ATNDTEVAQSNFEALQDFFRLFPEYKNNKLFLTGESYAGI YIPTLAVLVMQDPSMNLQGLAVGNGLSSYEQNDNSLVYFA

241 YYHGLLGNRLWSSLQTHCCSQNKCNFYDNKDLECVTNL QEVARIVGNSGLNIYNLYAPCAGGVPSHFRYEKDTVVVQDL G

321 NIFTRLPLKRMWHQALLRSGDKVRMDPPCTNTTAASTYL NNPYVRKALNIPEQLPQWDMCNFLVNLQYRRLYRSMNSQY L

401 KLLSSQKYQILLYNGDVDMACNFMGDEWFVDSLNQKME VQRRPWLVKYGDSGEQIAGFVKEFSHIAFLTIKGAGHMVPT D

481 KPLAAFTMFSRFLNKQPY

5.225 pleckstrin homology domain containing, family B (evectins) member 2 isoform 3 [Homo sapiens]; pleckstrin homology domain containing, family B (evectins) member 2 isoform 2 [Homo sapiens]

Protein Accession gi|154800478 gi|154800476
Mean Expression Ratio 0.93
Median Expression Ratio 0.929
Credible Interval (0.652, 1.32)
Associated Peptides 4
Associated Spectra 4
Coverage NaN

| A | 2.5 | 50 | 97.5 | Sequence |
|---|-----|-----|------|----------|
| 1 | 0.62 | 0.93 | 1.4 | AGLYGQQPANQVIIR |
| 1 | 0.61 | 0.93 | 1.4 | GQQPANQVIIR |
| 1 | 0.6 | 0.91 | 1.4 | NWFDLWSDGHLIYYDDQTR |
| 1 | 0.61 | 0.93 | 1.4 | VHMPMDCINIR |

5.226 BH3 interacting domain death agonist isoform 2 [Homo sapiens]; BH3 interacting domain death agonist isoform 3 [Homo sapiens]; BH3 interacting domain death agonist isoform 1 [Homo sapiens]

Protein Accession gi|4557361 gi|37574728 gi|37574726
Mean Expression Ratio 0.929
Median Expression Ratio 0.93
Credible Interval (0.572, 1.52)
Associated Peptides 1

Associated Spectra 1
Coverage NaN

| A | 2.5 | 50 | 97.5 | Sequence |
|---|-----|----|------|----------|
| 1 | 0.54 | 0.9 | 1.5 | DLATALEQLLQAYPR |

5.227 enolase 1 [Homo sapiens]

Protein Accession gi|4503571
Mean Expression Ratio 1.08
Median Expression Ratio 1.08
Credible Interval (0.799, 1.44)
Associated Peptides 5
Associated Spectra 7
Coverage 0.184

| A | 2.5 | 50 | 97.5 | Sequence |
|---|-----|----|------|----------|
| 2 | 0.75 | 1.1 | 1.5 | AAVPSGASTGIYEALELR |
| 2 | 0.75 | 1.1 | 1.5 | HIADLAGNSEVILPVPAFNVINGGSHAGNK |
| 1 | 0.76 | 1.1 | 1.6 | YISPDQLADLYK |
| 1 | 0.73 | 1.1 | 1.5 | VVIGMDVAASEFFR |
| 1 | 0.74 | 1.1 | 1.6 | YDLDFK |

1 MSILKIHAREIFDSRGNPTVEVDLFTSKGLFRAAVPSGASTG
IYEALELRDNDKTRYMGKGVSKAVEHINKTIAPALVSK

81 KLNVTEQEKIDKLMIEMDGTENKSKFGANAILGVSLAVCK
AGAVEKGVPLYRHIADLAGNSEVILPVPAFNVINGGSHAG

161 NKLAMQEFMILPVGAANFREAMRIGAEVYHNLKNVIKEK
YGKDATNVGDEGGFAPNILENKEGLELLKTAIGKAGYTDKV

241 VIGMDVAASEFFRSGKYDLDFKSPDDPSRYISPDQLADLY
KSFIKDYPVVSIEDPFDQDDWGAWQKFTASAGIQVVGDDL

321 TVTNPKRIAKAVNEKSCNCLLLKVNQIGSVTESLQACKLA
QANGWGVMVSHRSGETEDTFIADLVVGLCTGQIKTGAPCR

401 SERLAKYNQLLRIEEELGSKAKFAGRNFRNPLAK

5.228 hypothetical protein LOC64855 isoform 2 [Homo sapiens]; hypothetical protein LOC64855 isoform 1 [Homo sapiens]

Protein Accession gi|79750824 gi|51093863
Mean Expression Ratio 1.08
Median Expression Ratio 1.08
Credible Interval (0.708, 1.64)
Associated Peptides 2
Associated Spectra 2
Coverage NaN

| A | 2.5 | 50 | 97.5 | Sequence |
|---|---|---|---|---|
| 1 | 0.68 | 1.1 | 1.7 | FQELIFEDFAR |
| 1 | 0.68 | 1.1 | 1.7 | EASPESPPPASPDGVTEIR |

5.229 ribophorin II isoform 2 precursor [Homo sapiens]

Protein Accession gi|209413738

Mean Expression Ratio 0.93

Median Expression Ratio 0.93

Credible Interval (0.573, 1.5)

Associated Peptides 1

Associated Spectra 1

Coverage NaN

| A | 2.5 | 50 | 97.5 | Sequence |
|---|---|---|---|---|
| 1 | 0.56 | 0.91 | 1.5 | SIVEEIEDLVAR |

5.230 urate anion exchanger 1 isoform a [Homo sapiens]

Protein Accession gi|24497485

Mean Expression Ratio 0.93

Median Expression Ratio 0.932

Credible Interval (0.608, 1.41)

Associated Peptides 2

Associated Spectra 2

Coverage 0.0561

| A | 2.5 | 50 | 97.5 | Sequence |
|---|-----|-----|------|----------|
| 1 | 0.57 | 0.92 | 1.5 | AF SELLDLVGGLGR |
| 1 | 0.59 | 0.93 | 1.5 | GAVQDTLTPEVLLSAMR |

MAFSELLDLVGGLGRFQVLQTMALMVSIMWLCTQSMLENFSAAVPSHRCWAPLLDNSTAQASILGSLSPEALLAISIPPG

81 PNQRPHQCRRFRQPQWQLLDPNATATSWSEADTEPCVDGWVYDRSIFTSTIVAKWNLVCDSHALKPMAQSIYLAGILVGA

161 AACGPASDRFGRRLVLTWSYLQMAVMGTAAAFAPAFPVYCLFRFLLAFAVAGVMMNTGTLLMEWTAARARPLVMTLNSLG

241 FSFGHGLTAAVAYGVRDWTLLQLVVSVPFFLCFLYSWWLAESARWLLTTGRLDWGLQELWRVAAINGKGAVQDTLTPEVL

321 LSAMREELSMGQPPASLGTLLRMPGLRFRTCISTLCWFAFGFTFFGLALDLQALGSNIFLLQMFIGVVDIPAKMGALLLL

401 SHLGRRPTLAASLLLAGLCILANTLVPHEMGALRSALAVLGLGGVGAAFTCITIYSSELFPTVLRMTAVGLGQMAARGGA

481 ILGPLVRLLGVHGPWLPLLVYGTVPVLSGLAALLLPETQSLPLPDTIQDVQNQAVKKATHGTLGNSVLKSTQF

5.231 chromatin modifying protein 4C [Homo sapiens]

Protein Accession gi|22748643
Mean Expression Ratio 1.07
Median Expression Ratio 1.07
Credible Interval (0.65, 1.75)
Associated Peptides 1
Associated Spectra 1
Coverage 0.0687

| A | 2.5 | 50 | 97.5 | Sequence |
|---|-----|-----|------|----------|
| 1 | 0.66 | 1.1 | 1.8 | QLTQIDGTLSTIEFQR |

MSKLGKFFKGGGSSKSRAAPSPQEALVRLRETEEMLGKKQEYLENRIQREIALAKKHGTQNKRAALQALKRKKRFEKQLT

81 Q I D G T L S T I E F Q R E A L E N S H T N T E V L R N M G F A A K A M K S V H E N M D L N K I D D L M Q E I T E Q Q D I A Q E I S E A F S Q R V G F G D D F D

161 E D E L M A E L E E L E Q E E L N K K M T N I R L P N V P S S S L P A Q P N R K P G M S S T A R R S R A A S S Q R A E E E D D D I K Q L A A W A T

5.232 sacsin [Homo sapiens]

Protein Accession gi|163659918
Mean Expression Ratio 0.932
Median Expression Ratio 0.932
Credible Interval (0.577, 1.52)
Associated Peptides 1
Associated Spectra 1
Coverage NaN

| A | 2.5 | 50 | 97.5 | Sequence |
|---|-----|----|------|----------|
| 1 | 0.55 | 0.91 | 1.5 | NHPSVSWLK |

5.233 lumican precursor [Homo sapiens]

Protein Accession gi|4505047
Mean Expression Ratio 0.936
Median Expression Ratio 0.932
Credible Interval (0.577, 1.53)
Associated Peptides 1
Associated Spectra 1
Coverage 0.0444
A 2.5 50 97.5 Sequence
1 0.55 0.92 1.5 ISETSLPPDMYECLR

1 M S L S A F T L F L A L I G G T S G Q Y Y D Y D F P L S I Y G Q S S P N C A P E C N C P E S Y P S A M Y C D E L K L K S V P M V P P G I K Y L Y L R N N Q I D H

81 I D E K A F E N V T D L Q W L I L D H N L L E N S K I K G R V F S K L K Q L K K L H I N H N N L T E S V G P L P K S L E D L Q L T H N K I T K L G S F E G L V N

161 L T F I H L Q H N R L K E D A V S A A F K G L K S L E Y L D L S F N Q I A R L P S G L P V S L L T L Y L D N N K I S N I P D E Y F K R F N A L Q Y L R L S H N E

241 L A D S G I P G N S F N V S S L V E L D L S Y N K L K N I P T V N E N L E N Y Y L E V N Q L E K F D I K S F C K I L G P L S Y S K I K H L R L D G N R I S E T S

321 LPPDMYECLRVANEVTLN

5.234 chromatin modifying protein 1B [Homo sapiens]

Protein Accession gi|31542306
Mean Expression Ratio 1.07
Median Expression Ratio 1.07
Credible Interval (0.662, 1.73)
Associated Peptides 1
Associated Spectra 1
Coverage 0.0302

| A | 2.5 | 50 | 97.5 | Sequence |
|---|-----|----|------|----------|
| 1 | 0.65 | 1.1 | 1.8 | HLFNLK |

1 MSNMEKHLFNLKFAAKELSRSAKKCDKEEKAEKAKIKKAI
QKGNMEVARIHAENAIRQKNQAVNFLRMSARVDAVAARVQ

81 TAVTMGKVTKSMAGVVKSMDATLKTMNLEKISALMDKFE
HQFETLDVQTQQMEDTMSSTTTLTTPQNQVDMLLQEMADE
A

161 GLDLNMELPQGQTGSVGTSVASAEQDELSQRLARLRDQV

5.235 SHC (Src homology 2 domain containing) transforming protein 1 isoform 2 [Homo sapiens]; SHC (Src homology 2 domain containing) transforming protein 1 isoform 3 [Homo sapiens]

Protein Accession gi|32261324 gi|194239664
Mean Expression Ratio 0.933
Median Expression Ratio 0.936
Credible Interval (0.571, 1.50)
Associated Peptides 1
Associated Spectra 1
Coverage NaN

| A | 2.5 | 50 | 97.5 | Sequence |
|---|-----|----|------|----------|
| 1 | 0.55 | 0.92 | 1.5 | QAVGGAGPPNPAINGSAPR |

5.236 hypothetical protein LOC28970 [Homo sapiens]

Protein Accession gi|21361495
Mean Expression Ratio 1.07
Median Expression Ratio 1.07
Credible Interval (0.755, 1.53)
Associated Peptides 3
Associated Spectra 4
Coverage 0.109

| A | 2.5 | 50 | 97.5 | Sequence |
|---|-----|----|------|----------|
| 2 | 0.7 | 1.0 | 1.5 | APLVCLPVFVSR |
| 1 | 0.73 | 1.1 | 1.7 | FLPAEFLYR |
| 1 | 0.71 | 1.1 | 1.6 | WLHFYEMK |

1 M A C A E F S F H V P S L E E L A G V M Q K G L K D N F A D V Q V S V V D C P D L T K E P F T F P V K G I C G K T R I A E V G G V P Y L L P L V N Q K K V Y D L

81 N K I A K E I K L P G A F I L G A G A G P F Q T L G F N S E F M P V I Q T E S E H K P P V N G S Y F A H V N P A D G G C L L E K Y S E K C H D F Q C A L L A N L

161 F A S E G Q P G K P A E F S S C P L N S D E E V N K W L H F Y E M K A P L V C L P V F V S R D P G F D L R L E H T H F F S R H G E G G H Y H Y D T T P D I V E Y

241 LGYFLPAEFLYRIDQPKETHSIGRD

5.237 ATPase, H+ transporting, lysosomal V0 subunit a4 [Homo sapiens]; ATPase, H+ transporting, lysosomal V0 subunit a4 [Homo sapiens]; ATPase, H+ transporting, lysosomal V0 subunit a4 [Homo sapiens]

Protein Accession gi|85386547 gi|85386056 gi|85386053
Mean Expression Ratio 1.07
Median Expression Ratio 1.07
Credible Interval (0.659, 1.76)
Associated Peptides 1
Associated Spectra 1
Coverage NaN

| A | 2.5 | 50 | 97.5 | Sequence |
|---|-----|----|------|----------|
| 1 | 0.65 | 1.1 | 1.8 | LEDLITVITQTESHR |

5.238 chloride channel 7 isoform b [Homo sapiens]; chloride channel 7 isoform a [Homo sapiens]

Protein Accession gi|167466160 gi|14149607
Mean Expression Ratio 0.934
Median Expression Ratio 0.937
Credible Interval (0.616, 1.43)
Associated Peptides 2
Associated Spectra 2
Coverage NaN

| A | 2.5 | 50 | 97.5 | Sequence |
|---|-----|----|------|----------|
| 1 | 0.59 | 0.93 | 1.5 | IFEYFR |
| 1 | 0.58 | 0.92 | 1.4 | YESLDYDNSENQLFLEEER |

5.239 solute carrier family 34 (sodium phosphate), member 2 [Homo sapiens]

Protein Accession gi|110611906
Mean Expression Ratio 1.07
Median Expression Ratio 1.07
Credible Interval (0.785, 1.44)
Associated Peptides 5
Associated Spectra 7
Coverage 0.058

| A | 2.5 | 50 | 97.5 | Sequence |
|---|-----|-----|------|----------|
| 1 | 0.72 | 1.1 | 1.5 | APETFDNITISR |
| 3 | 0.86 | 1.2 | 1.7 | KLQNWNFLPLWMR |
| 1 | 0.71 | 1.0 | 1.5 | YLEGAAGQQPTAPDK |
| 1 | 0.72 | 1.1 | 1.5 | LQNWNFLPLWMR |
| 1 | 0.7 | 1 | 1.5 | NFLPLWMR |

1 MAPWPELGDAQPNPDKYLEGAAGQQPTAPDKSKETNKTD
NTEAPVTKIELLPSYSTATLIDEPTEVDDPWNLPTLQDSGI

81 KWSERDTKGKILCFFQGIGRLILLLGFLYFFVCSLDILSSAF
QLVGGKMAGQFFSNSSIMSNPLLGLVIGVLVTVLVQSS

161 STSTSIVVSMVSSSLLTVRAAIPIIMGANIGTSITNTIVALM
QVGDRSEFRRAFAGATVHDFFNWLSVLVLLPVEVATHY

241 LEIITQLIVESFHFKNGEDAPDLLKVITKPFTKLIVQLDKK
VISQIAMNDEKAKNKSLVKIWCKTFTNKTQINVTVPSTA

321 NCTSPSLCWTDGIQNWTMKNVTYKENIAKCQHIFVNFHL
PDLAVGTILLILSLLVLCGCLIMIVKILGSVLKGQVATVIK

401 KTINTDFPFPFAWLTGYLAILVGAGMTFIVQSSSVFTSAL
TPLIGIGVITIERAYPLTLGSNIGTTTTAILAALASPGNA

481 LRSSLQIALCHFFFNISGILLWYPIPFTRLPIRMAKGLGNIS
AKYRWFAVFYLIIFFFLIPLTVFGLSLAGWRVLVGVGV

561 PVVFIIILVLCLRLLQSRCPRVLPKKLQNWNFLPLWMRSL
KPWDAVVSKFTGCFQMRCCCCCRVCCRACCLLCGCPKCCR

641 CSKCCEDLEEAQEGQDVPVKAPETFDNITISREAQGEVPA
SDSKTECTAL

5.240 UBX domain protein 6 [Homo sapiens]

Protein Accession gi|13376854
Mean Expression Ratio 1.07
Median Expression Ratio 1.07
Credible Interval (0.666, 1.74)
Associated Peptides 1
Associated Spectra 1
Coverage 0.0544

| A | 2.5 | 50 | 97.5 | Sequence |
|---|-----|-----|------|----------|
| 1 | 0.66 | 1.1 | 1.8 | ELQAEATVSGSPEAPGTNVVSEPR |

1 M K K F F Q E F K A D I K F K S A G P G Q K L K E S V G E K A H K E K P N Q P A
P R P P R Q G P T N E A Q M A A A A A L A R L E Q K Q S R A W G P T S Q D T I R

81 N Q V R K E L Q A E A T V S G S P E A P G T N V V S E P R E E G S A H L A V P G
V Y F T C P L T G A T L R K D Q R D A C I K E A I L L H F S T D P V A A S I M K

161 I Y T F N K D Q D R V K L G V D T I A K Y L D N I H L H P E E E K Y R K I K L Q
N K V F Q E R I N C L E G T H E F F E A I G F Q K V L L P A Q D Q E D P E E F Y

241 V L S E T T L A Q P Q S L E R H K E Q L L A A E P V R A K L D R Q R R V F Q P S
P L A S Q F E L P G D F F N L T A E E I K R E Q R L R S E A V E R L S V L R T K

321 A M R E K E E Q R G L R K Y N Y T L L R V R L P D G C L L Q G T F Y A R E R L
G A V Y G F V R E A L Q S D W L P F E L L A S G G Q K L S E D E N L A L N E C G L

401 V P S A L L T F S W D M A V L E D I K A A G A E P D S I L K P E L L S A I E K L
L

5.241 tetratricopeptide repeat domain 38 [Homo sapiens]

Protein Accession gi|116812608
Mean Expression Ratio 0.935
Median Expression Ratio 0.938
Credible Interval (0.577, 1.50)
Associated Peptides 1
Associated Spectra 1
Coverage 0.0192

| A | 2.5 | 50 | 97.5 | Sequence |
|---|-----|-----|------|----------|
| 1 | 0.55 | 0.92 | 1.5 | VLELLLPIR |

MAAASPLRDCQAWKDARLPLSTTSNEACKLFDATLTQYVK
WTNDKSLGGIEGCLSKLKAADPTFVMGHAMATGLVLIGTG

81 SSVKLDKELDLAVKTMVEISRTQPLTRREQLHVSAVETFA
NGNFPKACELWEQILQDHPTDMLALKFSHDAYFYLGYQEQ

161 MRDSVARIYPFWTPDIPLSSYVKGIYSFGLMETNFYDQAE
KLAKEALSINPTDAWSVHTVAHIHEMKAEIKDGLEFMQHS

241 ETLWKDSDMLACHNYWHWALYLIEKGEYEAALTIYDTHI
LPSLQANDAMLDVVDSCSMLYRLQMEGVSVGQRWQDVLPV
A

321 RKHSRDHILLFNDAHFLMASLGAHDPQTTQELLTTLRDA
SESPGENCQHLLARDVGLPLCQALVEAEDGNPDRVLELLLP

401 IRYRIVQLGGSNAQRDVFNQLLIHAALNCTSSVHKNVARS
LLMERDALKPNSPLTERLIRKAATVHLMQ

5.242 crystallin, alpha B [Homo sapiens]

Protein Accession gi|4503057
Mean Expression Ratio 1.06
Median Expression Ratio 1.07
Credible Interval (0.686, 1.63)
Associated Peptides 1
Associated Spectra 2
Coverage 0.0629

| A | 2.5 | 50 | 97.5 | Sequence |
|---|-----|-----|------|----------|
| 2 | 0.69 | 1.1 | 1.7 | VLGDVIEVHGK |

MDIAIHHPWIRRPFFPFHSPSRLFDQFFGEHLLESDLFPTST
SLSPFYLRPPSFLRAPSWFDTGLSEMRLEKDRFSVNLD

81 VKHFSPEELKVKVLGDVIEVHGKHEERQDEHGFISREFHR
KYRIPADVDPLTITSSLSSDGVLTVNGPRKQVSGPERTIP

161 ITREEKPAVTAAPKK

5.243 dual oxidase 2 precursor [Homo sapiens]

Protein Accession gi|132566532

Mean Expression Ratio 1.07
Median Expression Ratio 1.07
Credible Interval (0.658, 1.73)
Associated Peptides 1
Associated Spectra 1
Coverage 0.00775

| A | 2.5 | 50 | 97.5 | Sequence |
|---|---|---|---|---|
| 1 | 0.65 | 1.1 | 1.8 | AQLAEVVESMFR |

```
1   M L R A R P E A L M L L G A L L T G S L G P S G N Q D A L S L P W E V Q R Y D G
    W F N N L R H H E R G A V G C R L Q R R V P A N Y A D G V Y Q A L E E P Q L P N

81  P R R L S N A A T R G I A G L P S L H N R T V L G V F F G Y H V L S D V V S V E
    T P G C P A E F L N I R I P P G D P V F D P D Q R G D V V L P F Q R S R W D P E

161 T G R S P S N P R D L A N Q V T G W L D G S A I Y G S S H S W S D A L R S F S
    G G Q L A S G P D P A F P R D S Q N P L L M W A A P D P A T G Q N G P R G L Y A F

241 G A E R G N R E P F L Q A L G L L W F R Y H N L W A Q R L A R Q H P D W E D
    E E L F Q H A R K R V I A T Y Q N I A V Y E W L P S F L Q K T L P E Y T G Y R P F L

321 D P S I S P E F V V A S E Q F F S T M V P P G V Y M R N A S C H F R K V L N K G
    F Q S S Q A L R V C N N Y W I R E N P N L N S T Q E V N E L L L G M A S Q I S E

401 L E D N I V V E D L R D Y W P G P G K F S R T D Y V A S S I Q R G R D M G L P
    S Y S Q A L L A F G L D I P R N W S D L N P N V D P Q V L E A T A A L Y N Q D L S

481 Q L E L L L G G L L E S H G D P G P L F S A I V L D Q F V R L R D G D R Y W F E
    N T R N G L F S K K E I E D I R N T T L R D V L V A V I N I D P S A L Q P N V F

561 V W H K G A P C P Q P K Q L T T D G L P Q C A P L T V L D F F E G S S P G F A I
    T I I A L C C L P L V S L L L S G V V A Y F R G R E H K K L Q K K L K E S V K K

641 E A A K D G V P A M E W P G P K E R S S P I I I Q L L S D R C L Q V L N R H L T
    V L R V V Q L Q P L Q Q V N L I L S N N R G C R T L L L K I P K E Y D L V L L F

721 S S E E E R G A F V Q Q L W D F C V R W A L G L H V A E M S E K E L F R K A
    V T K Q Q R E R I L E I F F R H L F A Q V L D I N Q A D A G T L P L D S S Q K V R E

801 A L T C E L S R A E F A E S L G L K P Q D M F V E S M F S L A D K D G N G Y L
    S F R E F L D I L V V F M K G S P E D K S R L M F T M Y D L D E N G F L S K D E F
```

881 FTMMRSFIEISNNCLSKAQLAEVVESMFRESGFQDKEELT
WEDFHFMLRDHDSELRFTQLCVKGGGGGGNGIRDIFKQNI

961 SCRVSFITRTPGERSHPQGLGPPAPEAPELGGPGLKKRFG
KKAAVPTPRLYTEALQEKMQRGFLAQKLQQYKRFVENYRR

1041 HIVCVAIFSAICVGVFADRAYYYGFASPPSDIAQTTLVGI
ILSRGTAASVSFMFSYILLTMCRNLITFLRETFLNRYVPF

1121 DAAVDFHRWIAMAAVVLAILHSAGHAVNVYIFSVSPLSL
LACIFPNVFVNDGSKLPQKFYWWFFQTVPGMTGVLLLLVLA

1201 IMYVFASHHFRRRSFRGFWLTHHLYILLYALLIIHGSYAL
IQLPTFHIYFLVPAIIYGGDKLVSLSRKKVEISVVKAELL

1281 PSGVTYLQFQRPQGFEYKSGQWVRIACLALGTTEYHPFT
LTSAPHEDTLSLHIRAVGPWTTRLREIYSSPKGNGCAGYPK

1361 LYLDGPFGEGHQEWHKFEVSVLVGGGIGVTPFASILKDL
VFKSSLGSQMLCKKIYFIWVTRTQRQFEWLADIIQEVEEND

1441 HQDLVSVHIYVTQLAEKFDLRTTMLYICERHFQKVLNRS
LFTGLRSITHFGRPPFEPFFNSLQEVHPQVRKIGVFSCGPP

1521 GMTKNVEKAC QLVNRQDRAHFMHHYENF

5.244 DnaJ (Hsp40) homolog, subfamily C, member 5 [Homo sapiens]

Protein Accession gi|45504382
Mean Expression Ratio 0.938
Median Expression Ratio 0.938
Credible Interval (0.626, 1.41)
Associated Peptides 1
Associated Spectra 3
Coverage 0.126

| A | 2.5 | 50 | 97.5 | Sequence |
|---|---|---|---|---|
| 3 | 0.62 | 0.92 | 1.4 | APEGEETEFYVSPEDLEAQLQSDER |

1 MADQRQRSLSTSGESLYHVLGLDKNATSDDIKKSYRKLAL
KYHPDKNPDNPEAADKFKEINNAHAILTDATKRNIYDKYG

81 S L G L Y V A E Q F G E E N V N T Y F V L S S W W A K A L F V F C G L L T C C
Y C C C C L C C C F N C C C G K C K P K A P E G E E T E F Y V S P E D L E A Q L Q

161 SDEREATDTPIVIQPASATETTQLTADSHPSYHTDGFN

5.245 vacuolar H+ATPase B2 [Homo sapiens]

Protein Accession gi|19913428
Mean Expression Ratio 0.939
Median Expression Ratio 0.939
Credible Interval (0.652, 1.36)
Associated Peptides 3
Associated Spectra 4
Coverage 0.110

| A | 2.5 | 50 | 97.5 | Sequence |
|---|-----|-----|------|----------|
| 2 | 0.62 | 0.9 | 1.3 | GIVNGAAPELPVPTGGPAVGAR |
| 1 | 0.63 | 0.95 | 1.5 | IPIFSAAGLPHNEIAAQICR |
| 1 | 0.61 | 0.94 | 1.4 | QIYPPINVLPSLSR |

1 M A L R A M R G I V N G A A P E L P V P T G G P A V G A R E Q A L A V S R N Y L
S Q P R L T Y K T V S G V N G P L V I L D H V K F P R Y A E I V H L T L P D G T

81 K R S G Q V L E V S G S K A V V Q V F E G T S G I D A K K T S C E F T G D I L R
T P V S E D M L G R V F N G S G K P I D R G P V V L A E D F L D I M G Q P I N P

161 Q C R I Y P E E M I Q T G I S A I D G M N S I A R G Q K I P I F S A A G L P H N E
I A A Q I C R Q A G L V K K S K D V V D Y S E E N F A I V F A A M G V N M E T

241 A R F F K S D F E E N G S M D N V C L F L N L A N D P T I E R I I T P R L A L T T
A E F L A Y Q C E K H V L V I L T D M S S Y A E A L R E V S A A R E E V P G R

321 R G F P G Y M Y T D L A T I Y E R A G R V E G R N G S I T Q I P I L T M P N D D
I T H P I P D L T G Y I T E G Q I Y V D R Q L H N R Q I Y P P I N V L P S L S R

401 L M K S A I G E G M T R K D H A D V S N Q L Y A C Y A I G K D V Q A M K A V
V G E E A L T S D D L L Y L E F L Q K F E R N F I A Q G P Y E N R T V F E T L D I G

481 WQLLRIFPKEMLKRIPQSTLSEFYPRDSAKH

5.246 angiotensin I converting enzyme 2 precursor [Homo sapiens]

Protein Accession gi|11225609

Mean Expression Ratio 0.939
Median Expression Ratio 0.939
Credible Interval (0.697, 1.27)
Associated Peptides 5
Associated Spectra 7
Coverage 0.0745

| A | 2.5 | 50 | 97.5 | Sequence |
|---|-----|-----|------|----------|
| 1 | 0.63 | 0.92 | 1.3 | GQKPNIDVTDAMVDQAWDAQR |
| 2 | 0.66 | 0.93 | 1.3 | LWAWESWR |
| 1 | 0.66 | 0.97 | 1.4 | NQMILFGEEDVR |
| 2 | 0.68 | 0.98 | 1.4 | QLRPLYEEYVVLK |
| 1 | 0.6 | 0.88 | 1.3 | WSAFLK |

1 MSSSSWLLLSLVAVTAAQSTIEEQAKTFLDKFNHEAEDLFY
QSSLASWNYNTNITEENVQNMNNAGDKWSAFLKEQSTLA

81 QMYPLQEIQNLTVKLQLQALQQNGSSVLSEDKSKRLNTIL
NTMSTIYSTGKVCNPDNPQECLLLEPGLNEIMANSLDYNE

161 RLWAWESWRSEVGKQLRPLYEEYVVLKNEMARANHYED
YGDYWRGDYEVNGVDGYDYSRGQLIEDVEHTFEEIKPLYEH
L

241 HAYVRAKLMNAYPSYISPIGCLPAHLLGDMWGRFWTNLY
SLTVPFGQKPNIDVTDAMVDQAWDAQRIFKEAEKFFVSVGL

321 PNMTQGFWENSMLTDPGNVQKAVCHPTAWDLGKGDFRI
LMCTKVTMDDFLTAHHEMGHIQYDMAYAAQPFLLRNGANE
GF

401 HEAVGEIMSLSAATPKHLKSIGLLSPDFQEDNETEINFLLK
QALTIVGTLPFTYMLEKWRWMVFKGEIPKDQWMKKWWEM

481 KREIVGVVEPVPHDETYCDPASLFHVSNDYSFIRYYTRTL
YQFQFQEALCQAAKHEGPLHKCDISNSTEAGQKLFNMLRL

561 GKSEPWTLALENVVGAKNMNVRPLLNYFEPLFTWLKDQ
NKNSFVGWSTDWSPYADQSIKVRISLKSALGDKAYEWNDNE
M

641 YLFRSSVAYAMRQYFLKVKNQMILFGEEDVRVANLKPRI
SFNFFVTAPKNVSDIIPRTEVEKAIRMSRSRINDAFRLNDN

721 S L E F L G I Q P T L G P P N Q P P V S I W L I V F G V V M G V I V V G I V I L I F
T G I R D R K K K N K A R S G E N P Y A S I D I S K G E N N P G F Q N T D D

801 VQTSF

5.247 aquaporin 1 [Homo sapiens]

Protein Accession gi|37694062
Mean Expression Ratio 1.07
Median Expression Ratio 1.07
Credible Interval (0.791, 1.44)
Associated Peptides 3
Associated Spectra 10
Coverage 0.145

| A | 2.5 | 50 | 97.5 | Sequence |
|---|-----|-----|------|----------|
| 1 | 0.75 | 1.1 | 1.6 | DLGGSAPLAIGLSVALGHL |
| 1 | 0.71 | 1.1 | 1.5 | TSGQVEEYDLDADDINSR |
| 8 | 0.83 | 1.1 | 1.4 | VWTSGQVEEYDLDADDINSR |

1 M A S E F K K K L F W R A V V A E F L A T T L F V F I S I G S A L G F K Y P V G N
N Q T A V Q D N V K V S L A F G L S I A T L A Q S V G H I S G A H L N P A V T

81 L G L L L S C Q I S I F R A L M Y I I A Q C V G A I V A T A I L S G I T S S L T G N
S L G R N D L A D G V N S G Q G L G I E I I G T L Q L V L C V L A T T D R R

161 R R D L G G S A P L A I G L S V A L G H L L A I D Y T G C G I N P A R S F G S A
V I T H N F S N H W I F W V G P F I G G A L A V L I Y D F I L A P R S S D L T D

241 RVKVWTSGQVEEYDLDADDINSRVEMKPK

5.248 secretoglobin, family 1A, member 1 (uteroglobin) [Homo sapiens]

Protein Accession gi|4507809
Mean Expression Ratio 0.938
Median Expression Ratio 0.939
Credible Interval (0.586, 1.51)
Associated Peptides 1
Associated Spectra 1
Coverage 0.11

| A | 2.5 | 50 | 97.5 | Sequence |
|---|-----|-----|------|----------|
| 1 | 0.56 | 0.92 | 1.5 | LVDTLPQKPR |

1 M K L A V T L T L V T L A L C C S S A S A E I C P S F Q R V I E T L L M D T P S S Y E A A M E L F S P D Q D M R E A G A Q L K K L V D T L P Q K P R E S I I K L

81 MEKIAQ S SLCN

5.249 structural maintenance of chromosomes 2 [Homo sapiens]; structural maintenance of chromosomes 2 [Homo sapiens]; structural maintenance of chromosomes 2 [Homo sapiens]

Protein Accession gi|110347425 gi|110347420 gi|110347418
Mean Expression Ratio 1.06
Median Expression Ratio 1.06
Credible Interval (0.654, 1.72)
Associated Peptides 1
Associated Spectra 1
Coverage NaN

| A | 2.5 | 50 | 97.5 | Sequence |
|---|-----|-----|------|----------|
| 1 | 0.64 | 1.1 | 1.8 | LGRNVNMRAMNVLTEAEER |

5.250 superoxide dismutase 1, soluble [Homo sapiens]

Protein Accession gi|4507149
Mean Expression Ratio 1.07
Median Expression Ratio 1.06
Credible Interval (0.661, 1.73)
Associated Peptides 1
Associated Spectra 1
Coverage 0.091

| A | 2.5 | 50 | 97.5 | Sequence |
|---|-----|-----|------|----------|
| 1 | 0.65 | 1.1 | 1.8 | GDGPVQGIINFEQK |

1 M A T K A V C V L K G D G P V Q G I I N F E Q K E S N G P V K V W G S I K G L T E G L H G F H V H E F G D N T A G C T S A G P H F N P L S R K H G G P K D E E R

81 H V G D L G N V T A D K D G V A D V S I E D S V I S L S G D H C I I G R T L V V H E K A D D L G K G G N E E S T K T G N A G S R L A C G V I G I A Q

5.251 phospholipid scramblase 1 [Homo sapiens]

Protein Accession gi|10863877
Mean Expression Ratio 1.06
Median Expression Ratio 1.06
Credible Interval (0.774, 1.46)

Associated Peptides 4
Associated Spectra 6
Coverage 0.170

| A | 2.5 | 50 | 97.5 | Sequence |
|---|-----|-----|------|----------|
| 1 | 0.72 | 1.1 | 1.6 | EAFTDADNFGIQFPLDLDVK |
| 3 | 0.76 | 1.1 | 1.5 | IIDNMGQEVITLER |
| 1 | 0.71 | 1.0 | 1.5 | SGYPGPQVSYPPPPAGH |
| 1 | 0.74 | 1.1 | 1.6 | IIDNMGQEVITLERPLR |

1 MDKQNSQMNASHPETNLPVGYPPQYPPTAFQGPPGYSGYP
GPQVSYPPPPAGHSGPGPAGFPVPNQPVYNQPVYNQPVGA

81 AGVPWMPAPQPPLNCPPGLEYLSQIDQILIHQQIELLEVLT
GFETNNKYEIKNSFGQRVYFAAEDTDCCTRNCCGPSRPF

161 TLRIIDNMGQEVITLERPLRCSSCCCPCCLQEIEIQAPPGV
PIGYVIQTWHPCLPKFTIQNEKREDVLKISGPCVVCSCC

241 GDVDFEIKSLDEQCVVGKISKHWTGILREAFTDADNFGIQ
FPLDLDVKMKAVMIGACFLIDFMFFESTGSQEQKSGVW

5.252 ATPase, H+ transporting, lysosomal V0 subunit a1 isoform c [Homo sapiens]; ATPase, H+ transporting, lysosomal V0 subunit a1 isoform b [Homo sapiens]; ATPase, H+ transporting, lysosomal V0 subunit a1 isoform a [Homo sapiens]

Protein Accession gi|19913418 gi|194097403 gi|194097401
Mean Expression Ratio 0.94
Median Expression Ratio 0.94
Credible Interval (0.663, 1.34)
Associated Peptides 4
Associated Spectra 4
Coverage NaN

| A | 2.5 | 50 | 97.5 | Sequence |
|---|-----|------|------|----------|
| 1 | 0.62 | 0.94 | 1.4 | ANIPIMDTGENPEVPFPR |
| 1 | 0.62 | 0.94 | 1.4 | ASLYPCPETPQER |
| 1 | 0.61 | 0.92 | 1.4 | IDDLQMVLNQTEDHR |
| 1 | 0.63 | 0.95 | 1.5 | KANIPIMDTGENPEVPFPR |

5.253 solute carrier family 44, member 2 [Homo sapiens]

Protein Accession gi|31377727

Mean Expression Ratio 1.06
Median Expression Ratio 1.06
Credible Interval (0.646, 1.73)
Associated Peptides 1
Associated Spectra 1
Coverage 0.0227

| A | 2.5 | 50 | 97.5 | Sequence |
|---|-----|----|------|----------|
| 1 | 0.65 | 1.1 | 1.8 | DGDCPAVLIPSKPLAR |

1 M G D E R P H Y Y G K H G T P Q K Y D P T F K G P I Y N R G C T D I I C C V F L L
L A I V G Y V A V G I I A W T H G D P R K V I Y P T D S R G E F C G Q K G T K

81 N E N K P Y L F Y F N I V K C A S P L V L L E F Q C P T P Q I C V E K C P D R Y L
T Y L N A R S S R D F E Y Y K Q F C V P G F K N N K G V A E V L R D G D C P A

161 V L I P S K P L A R R C F P A I H A Y K G V L M V G N E T T Y E D G H G S R K
N I T D L V E G A K K A N G V L E A R Q L A M R I F E D Y T V S W Y W I I I G L V

241 I A M A M S L L F I I L L R F L A G I M V W V M I I M V I L V L G Y G I F H C Y
M E Y S R L R G E A G S D V S L V D L G F Q T D F R V Y L H L R Q T W L A F M I

321 I L S I L E V I I I L L L I F L R K R I L I A I A L I K E A S R A V G Y V M C S L L Y
P L V T F F L L C L C I A Y W A S T A V F L S T S N E A V Y K I F D D S P

401 C P F T A K T C N P E T F P S S N E S R Q C P N A R C Q F A F Y G G E S G Y H R
A L L G L Q I F N A F M F F W L A N F V L A L G Q V T L A G A F A S Y Y W A L R

481 K P D D L P A F P L F S A F G R A L R Y H T G S L A F G A L I L A I V Q I I R V I
L E Y L D Q R L K A A E N K F A K C L M T C L K C C F W C L E K F I K F L N R

561 N A Y I M I A I Y G T N F C T S A R N A F F L L M R N I I R V A V L D K V T D F
L F L L G K L L I V G S V G I L A F F F F T H R I R I V Q D T A P P L N Y Y W V

641 P I L T V I V G S Y L I A H G F F S V Y G M C V D T L F L C F L E D L E R N D G
S A E R P Y F M S S T L K K P L N K T N K K A A E S

5.254 guanine nucleotide binding protein (G protein), alpha inhibiting activity polypeptide 3 [Homo sapiens]

Protein Accession gi|5729850
Mean Expression Ratio 1.07
Median Expression Ratio 1.06
Credible Interval (0.702, 1.63)
Associated Peptides 2

Associated Spectra 2
Coverage 0.0763

| A | 2.5 | 50 | 97.5 | Sequence |
|---|-----|-----|------|----------|
| 1 | 0.66 | 1.1 | 1.7 | ISQSNYIPTQQDVLR |
| 1 | 0.68 | 1.1 | 1.7 | SNTIQSIIAIIR |

1 M G C T L S A E D K A A V E R S K M I D R N L R E D G E K A A K E V K L L L L G
A G E S G K S T I V K Q M K I I H E D G Y S E D E C K Q Y K V V V Y S N T I Q S

81 I I A I I R A M G R L K I D F G E A A R A D D A R Q L F V L A G S A E E G V M T P
E L A G V I K R L W R D G G V Q A C F S R S R E Y Q L N D S A S Y Y L N D L D

161 R I S Q S N Y I P T Q Q D V L R T R V K T T G I V E T H F T F K D L Y F K M F D
V G G Q R S E R K K W I H C F E G V T A I I F C V A L S D Y D L V L A E D E E M

241 N R M H E S M K L F D S I C N N K W F T E T S I I L F L N K K D L F E E K I K R
S P L T I C Y P E Y T G S N T Y E E A A A Y I Q C Q F E D L N R R K D T K E I Y

321 THFTCATDTKNVQFVFDAVTDVIIKNNLKECGLY

5.255 PREDICTED: hypothetical protein [Homo sapiens]

Protein Accession gi|169217813
Mean Expression Ratio 1.06
Median Expression Ratio 1.06
Credible Interval (0.685, 1.62)
Associated Peptides 1
Associated Spectra 2
Coverage NaN

| A | 2.5 | 50 | 97.5 | Sequence |
|---|-----|-----|------|----------|
| 2 | 0.69 | 1.1 | 1.7 | YNRITLNNDIMLIK |

5.256 dipeptidyl peptidase 7 preproprotein [Homo sapiens]

Protein Accession gi|62420888
Mean Expression Ratio 0.94
Median Expression Ratio 0.943
Credible Interval (0.655, 1.34)
Associated Peptides 2
Associated Spectra 4
Coverage 0.065

| A | 2.5 | 50 | 97.5 | Sequence |
|---|-----|-----|------|----------|
| 3 | 0.63 | 0.92 | 1.3 | GHTELLTVEQALADFAELLR |
| 1 | 0.62 | 0.95 | 1.4 | DLFLQGAYDTVR |

1 MGSAPWAPVLLLALGLRGLQAGARRAPDPGFQERFFQQRL
DHFNFERFGNKTFPQRFLVSDRFWVRGEGPIFFYTGNEGD

81 VWAFANNSAFVAELAAERGALLVFAEHRYYGKSLPFGAQ
STQRGHTELLTVEQALADFAELLRALRRDLGAQDAPAIAFG

161 GSYGGMLSAYLRMKYPHLVAGALAASAPVLAVAGLGDS
NQFFRDVTADFEGQSPKCTQGVREAFRQIKDLFLQGAYDTV
R

241 WEFGTCQPLSDEKDLTQLFMFARNAFTVLAMMDYPYPTD
FLGPLPANPVKVGCDRLLSEAQRITGLRALAGLVYNASGSE

321 HCYDIYRLYHSCADPTGCGTGPDARAWDYQACTEINLTF
ASNNVTDMFPDLPFTDELRQRYCLDTWGVWPRPDWLLTSF
W

401 GGDLRAASNIIFSNGNLDPWAGGGIRRNLSASVIAVTIQG
GAHHLDLRASHPEDPASVVEARKLEATIIGEWVKAARREQ

481 QPALRGGPRLSL

5.257 tropomyosin 4 [Homo sapiens]

Protein Accession gi|4507651
Mean Expression Ratio 1.06
Median Expression Ratio 1.06
Credible Interval (0.66, 1.71)
Associated Peptides 1
Associated Spectra 1
Coverage 0.0403

| A | 2.5 | 50 | 97.5 | Sequence |
|---|-----|-----|------|----------|
| 1 | 0.65 | 1.1 | 1.8 | IQLVEEELDR |

1 MAGLNSLEAVKRKIQALQQQADEAEDRAQGLQRELDGERE
RREKAEGDVAALNRRIQLVEEELDRAQERLATALQKLEEA

81 E K A A D E S E R G M K V I E N R A M K D E E K M E I Q E M Q L K E A K H I A E E A D R K Y E E V A R K L V I L E G E L E R A E E R A E V S E L K C G D L E E E

161 L K N V T N N L K S L E A A S E K Y S E K E D K Y E E E I K L L S D K L K E A E T R A E F A E R T V A K L E K T I D D L E E K L A Q A K E E N V G L H Q T L D Q

241 TLNELNCI

5.258 keratin 10 [Homo sapiens]

Protein Accession gi|195972866
Mean Expression Ratio 0.945
Median Expression Ratio 0.944
Credible Interval (0.663, 1.35)
Associated Peptides 3
Associated Spectra 4
Coverage NaN

| A | 2.5 | 50 | 97.5 | Sequence |
|---|-----|----|----|----------|
| 2 | 0.65 | 0.95 | 1.4 | NQILNLTTDNANILLQIDNAR |
| 1 | 0.61 | 0.92 | 1.4 | SQYEQLAEQNRK |
| 1 | 0.63 | 0.95 | 1.4 | SQYEQLAEQNR |

5.259 myosin VI [Homo sapiens]

Protein Accession gi|92859701
Mean Expression Ratio 1.05
Median Expression Ratio 1.06
Credible Interval (0.647, 1.72)
Associated Peptides 1
Associated Spectra 1
Coverage 0.0101

| A | 2.5 | 50 | 97.5 | Sequence |
|---|-----|----|----|----------|
| 1 | 0.64 | 1.1 | 1.8 | LVGILDILDEENR |

1 M E D G K P V W A P H P T D G F Q M G N I V D I G P D S L T I E P L N Q K G K T F L A L I N Q V F P A E E D S K K D V E D N C S L M Y L N E A T L L H N I K V R

81 Y S K D R I Y T Y V A N I L I A V N P Y F D I P K I Y S S E A I K S Y Q G K S L G T R P P H V F A I A D K A F R D M K V L K M S Q S I I V S G E S G A G K T E N

161 TKFVLRYLTESYGTGQDIDDRIVEANPLLEAFGNAKTVRN
NNSSRFGKFVEIHFNEKSSVVGGFVSHYLLEKSRICVQGK

241 EERNYHIFYRLCAGASEDIREKLHLSSPDNFRYLNRGCTR
YFANKETDKQILQNRKSPEYLKAGSMKDPLLDDHGDFIRM

321 CTAMKKIGLDDEEKLDLFRVVAGVLHLGNIDFEEAGSTS
GGCNLKNKSAQSLEYCAELLGLDQDDLRVSLTTRVMLTTAG

401 GTKGTVIKVPLKVEQANNARDALAKTVYSHLFDHVVNR
VNQCFPFETSSYFIGVLDIAGFEYFEHNSFEQFCINYCNEKL

481 QQFFNERILKEEQELYQKEGLGVNEVHYVDNQDCIDLIEA
KLVGILDILDEENRLPQPSDQHFTSAVHQKHKDHFRLTIP

561 RKSKLAVHRNIRDDEGFIIRHFAGAVCYETTQFVEKNNDA
LHMSLESLICESRDKFIRELFESSTNNNKDTKQKAGKLSF

641 ISVGNKFKTQLNLLLDKLRSTGASFIRCIKPNLKMTSHHF
EGAQILSQLQCSGMVSVLDLMQGGYPSRASFHELYNMYKK

721 YMPDKLARLDPRLFCKALFKALGLNENDYKFGLTKVFFR
PGKFAEFDQIMKSDPDHLAELVKRVNHWLTCSRWKKVQWC
S

801 LSVIKLKNKIKYRAEACIKMQKTIRMWLCKRRHKPRIDGL
VKVGTLKKRLDKFNEVVSVLKDGKPEMNKQIKNLEISIDT

881 LMAKIKSTMMTQEQIQKEYDALVKSSEELLSALQKKKQQ
EEEAERLRRIQEEMEKERKRREEDEKRRRKEEEERRMKLEM

961 EAKRKQEEEERKKREDDEKRIQAEVEAQLARQKEEESQQ
QAVLEQERRDRELALRIAQSEAELISDEAQADLALRRNDGT

1041 RPKMTPEQMAKEMSEFLSRGPAVLATKAAAGTKKYDLS
KWKYAELRDTINTSCDIELLAACREEFHRRLKVYHAWKSKN
K

1121 KRNTETEQRAPKSVTDYDFAPFLNNSPQQNPAAQIPARQ
REIEMNRQQRFFRIPFIRPADQYKDPQSKKKGWWYAHFDGP

1201 WIARQMELHPDKPPILLVAGKDDMEMCELNLEETGLTR
KRGAEILPRQFEEIWERCGGIQYLQNAIESRQARPTYATAML

1281 QSLLK

5.260 heat shock protein 90kDa alpha (cytosolic), class A member 1 isoform 1 [Homo sapiens]

Protein Accession gi|153792590
Mean Expression Ratio 1.06
Median Expression Ratio 1.06
Credible Interval (0.65, 1.72)
Associated Peptides 1
Associated Spectra 1
Coverage 0.0176

| A | 2.5 | 50 | 97.5 | Sequence |
|---|-----|-----|------|----------|
| 1 | 0.65 | 1.1 | 1.8 | HLEINPDHSIIETLR |

1 M P P C S G G D G S T P P G P S L R D R D C P A Q S A E Y P R D R L D P R P G S P
S E A S S P P F L R S R A P V N W Y Q E K A Q V F L W H L M V S G S T T L L C

81 L W K Q P F H V S A F P V T A S L A F R Q S Q G A G Q H L Y K D L Q P F I L L R
L L M P E E T Q T Q D Q P M E E E E V E T F A F Q A E I A Q L M S L I I N T F Y

161 S N K E I F L R E L I S N S S D A L D K I R Y E S L T D P S K L D S G K E L H I N
L I P N K Q D R T L T I V D T G I G M T K A D L I N N L G T I A K S G T K A F

241 M E A L Q A G A D I S M I G Q F G V G F Y S A Y L V A E K V T V I T K H N D D
E Q Y A W E S S A G G S F T V R T D T G E P M G R G T K V I L H L K E D Q T E Y L

321 E E R R I K E I V K K H S Q F I G Y P I T L F V E K E R D K E V S D D E A E E K E
D K E E E K E K E E K E S E D K P E I E D V G S D E E E K K D G D K K K K

401 K I K E K Y I D Q E E L N K T K P I W T R N P D D I T N E E Y G E F Y K S L T N
D W E D H L A V K H F S V E G Q L E F R A L L F V P R R A P F D L F E N R K K K

481 N N I K L Y V R R V F I M D N C E E L I P E Y L N F I R G V V D S E D L P L N I S
R E M L Q Q S K I L K V I R K N L V K K C L E L F T E L A E D K E N Y K K F Y

561 E Q F S K N I K L G I H E D S Q N R K K L S E L L R Y Y T S A S G D E M V S L K
D Y C T R M K E N Q K H I Y Y I T G E T K D Q V A N S A F V E R L R K H G L E V

641 I Y M I E P I D E Y C V Q Q L K E F E G K T L V S V T K E G L E L P E D E E E K
K K Q E E K K T K F E N L C K I M K D I L E K K V E K V V V S N R L V T S P C C

721 I V T S T Y G W T A N M E R I M K A Q A L R D N S T M G Y M A A K K H L E I N P D H S I I E T L R Q K A E A D K N D K S V K D L V I L L Y E T A L L S S G F S L

801 E D P Q T H A N R I Y R M I K L G L G I D E D D P T A D D T S A A V T E E M P P L E G D D D T S R M E E V D

5.261 RAB1A, member RAS oncogene family isoform 1 [Homo sapiens]; RAB1B, member RAS oncogene family [Homo sapiens]

Protein Accession gi|4758988 gi|13569962
Mean Expression Ratio 1.06
Median Expression Ratio 1.06
Credible Interval (0.65, 1.70)
Associated Peptides 1
Associated Spectra 1
Coverage NaN

| A | 2.5 | 50 | 97.5 | Sequence |
|---|-----|----|------|----------|
| 1 | 0.64 | 1.1 | 1.8 | EFADSLGIPFLETSAK |

5.262 villin 1 [Homo sapiens]

Protein Accession gi|194394237
Mean Expression Ratio 1.05
Median Expression Ratio 1.05
Credible Interval (0.685, 1.62)
Associated Peptides 2
Associated Spectra 2
Coverage NaN

| A | 2.5 | 50 | 97.5 | Sequence |
|---|-----|----|------|----------|
| 1 | 0.65 | 1.0 | 1.7 | LMEVMNHVLGK |
| 1 | 0.66 | 1.1 | 1.7 | LQEENLVITPR |

5.263 PREDICTED: similar to hCG1992647, partial [Homo sapiens]

Protein Accession gi|169217452
Mean Expression Ratio 0.95
Median Expression Ratio 0.95
Credible Interval (0.604, 1.47)
Associated Peptides 1
Associated Spectra 2
Coverage NaN

| A | 2.5 | 50 | 97.5 | Sequence |
|---|-----|-----|------|----------|
| 2 | 0.6 | 0.94 | 1.5 | AVTELGRPDAEYWNSQK |

5.264 aldehyde dehydrogenase 1A1 [Homo sapiens]

Protein Accession gi|21361176
Mean Expression Ratio 1.05
Median Expression Ratio 1.05
Credible Interval (0.723, 1.55)
Associated Peptides 2
Associated Spectra 3
Coverage 0.0439

| A | 2.5 | 50 | 97.5 | Sequence |
|---|-----|-----|------|----------|
| 2 | 0.7 | 1.0 | 1.6 | IFVEESIYDEFVR |
| 1 | 0.69 | 1.1 | 1.7 | ILDLIESGK |

1 MSSSGTPDLPVLLTDLKIQYTKIFINNEWHDSVSGKKFPVF
NPATEEELCQVEEGDKEDVDKAVKAARQAFQIGSPWRTM

81 DASERGRLLYKLADLIERDRLLLATMESMNGGKLYSNAY
LNDLAGCIKTLRYCAGWADKIQGRTIPIDGNFFTYTRHEPI

161 GVCGQIIPWNFPLVMLIWKIGPALSCGNTVVVKPAEQTPL
TALHVASLIKEAGFPPGVVNIVPGYGPTAGAAISSHMDID

241 KVAFTGSTEVGKLIKEAAGKSNLKRVTLELGGKSPCIVLA
DADLDNAVEFAHHGVFYHQGQCCIAASRIFVEESIYDEFV

321 RRSVERAKKYILGNPLTPGVTQGPQIDKEQYDKILDLIES
GKKEGAKLECGGGPWGNKGYFVQPTVFSNVTDEMRIAKEE

401 IFGPVQQIMKFKSLDDVIKRANNTFYGLSAGVFTKDIDKA
ITISSALQAGTVWVNCYGVVSAQCPFGGFKMSGNGRELGE

481 YGFHEYTEVKTVTVKISQKNS

5.265 phosphoglycerate kinase 1 [Homo sapiens]

Protein Accession gi|4505763
Mean Expression Ratio 1.05
Median Expression Ratio 1.05
Credible Interval (0.758, 1.47)

Associated Peptides 4
Associated Spectra 5
Coverage 0.161

| A | 2.5 | 50 | 97.5 | Sequence |
|---|-----|-----|------|----------|
| 2 | 0.72 | 1.0 | 1.5 | ACANPAAGSVILLENLR |
| 1 | 0.72 | 1.1 | 1.6 | ALESPERPFLAILGGAK |
| 1 | 0.74 | 1.1 | 1.6 | ITLPVDFVTADKFDENAK |
| 1 | 0.68 | 1.0 | 1.5 | LGDVYVNDAFGTAHR |

1 M S L S N K L T L D K L D V K G K R V V M R V D F N V P M K N N Q I T N N Q R I K A A V P S I K F C L D N G A K S V V L M S H L G R P D G V P M P D K Y S L E P

81 V A V E L K S L L G K D V L F L K D C V G P E V E K A C A N P A A G S V I L L E N L R F H V E E E G K G K D A S G N K V K A E P A K I E A F R A S L S K L G D V

161 Y V N D A F G T A H R A H S S M V G V N L P Q K A G G F L M K K E L N Y F A K A L E S P E R P F L A I L G G A K V A D K I Q L I N N M L D K V N E M I I G G G M

241 A F T F L K V L N N M E I G T S L F D E E G A K I V K D L M S K A E K N G V K I T L P V D F V T A D K F D E N A K T G Q A T V A S G I P A G W M G L D C G P E S

321 S K K Y A E A V T R A K Q I V W N G P V G V F E W E A F A R G T K A L M D E V V K A T S R G C I T I I G G G D T A T C C A K W N T E D K V S H V S T G G G A S L

401 ELLEGKVLPGVDALSNI

5.266 eukaryotic translation initiation factor 5A isoform A [Homo sapiens]

Protein Accession gi|219555707
Mean Expression Ratio 1.05
Median Expression Ratio 1.05
Credible Interval (0.636, 1.73)
Associated Peptides 1
Associated Spectra 1
Coverage NaN

| A | 2.5 | 50 | 97.5 | Sequence |
|---|-----|-----|------|----------|
| 1 | 0.65 | 1.1 | 1.8 | NDFQLIGIQDGYLSLLQDSGEVR |

5.267 heat shock 70kDa protein 1A [Homo sapiens]; heat shock 70kDa protein 1B [Homo sapiens]

Protein Accession gi|194248072 gi|167466173
Mean Expression Ratio 1.05
Median Expression Ratio 1.05
Credible Interval (0.861, 1.29)
Associated Peptides 12
Associated Spectra 21
Coverage NaN

| A | 2.5 | 50 | 97.5 | Sequence |
|---|-----|-----|------|----------|
| 2 | 0.74 | 1.0 | 1.4 | AQIHDLVLVGGSTR |
| 2 | 0.77 | 1.1 | 1.4 | ATAGDTHLGGEDFDNR |
| 1 | 0.74 | 1.0 | 1.4 | AVITVPAYFNDSQR |
| 2 | 0.79 | 1.1 | 1.4 | EIAEAYLGYPVTNAVITVPAYFNDSQR |
| 3 | 0.78 | 1.1 | 1.4 | FEELCSDLFR |
| 1 | 0.76 | 1.1 | 1.5 | FELSGIPPAPR |
| 1 | 0.77 | 1.1 | 1.5 | HWPFQVINDGDKPK |
| 3 | 0.77 | 1.0 | 1.4 | LLQDFFNGR |
| 3 | 0.78 | 1.0 | 1.4 | LVNHFVEEFK |
| 1 | 0.79 | 1.1 | 1.5 | LVNHFVEEFKR |
| 1 | 0.75 | 1.0 | 1.4 | NQVALNPQNTVFDAK |
| 1 | 0.77 | 1.1 | 1.5 | QTQIFTTYSDNQPGVLIQVYEGER |

5.268 hypothetical protein LOC126321 isoform b [Homo sapiens]; hypothetical protein LOC126321 isoform c [Homo sapiens]; hypothetical protein LOC126321 isoform a [Homo sapiens]

Protein Accession gi|111378395 gi|111378391 gi|111378386
Mean Expression Ratio 0.954
Median Expression Ratio 0.952
Credible Interval (0.647, 1.41)
Associated Peptides 2
Associated Spectra 3
Coverage NaN

| A | 2.5 | 50 | 97.5 | Sequence |
|---|-----|------|------|----------|
| 1 | 0.62 | 0.96 | 1.5 | GPGPPAAGAAPSPR |
| 2 | 0.63 | 0.94 | 1.4 | VEPTQDISISDQLGGQDVPVFR |

5.269 PDZK1 interacting protein 1 [Homo sapiens]

Protein Accession gi|5031657
Mean Expression Ratio 0.954
Median Expression Ratio 0.954
Credible Interval (0.637, 1.43)
Associated Peptides 1

Associated Spectra 3
Coverage 0.149

| A | 2.5 | 50 | 97.5 | Sequence |
|---|-----|----|------|----------|
| 3 | 0.64 | 0.94 | 1.4 | SSEHENAYENVPEEEGK |

M S A L S L L I L G L L T A V P P A S C Q Q G L G N L Q P W M Q G L I A V A V F L V L V A I A F A V N H F W C Q E E P E P A H M I L T V G N K A D G V L V G T D

81 GRYSSMAASFRSSEHENAYENVPEEEGKVRSTPM

5.270 hexosaminidase A preproprotein [Homo sapiens]

Protein Accession gi|189181666
Mean Expression Ratio 0.954
Median Expression Ratio 0.954
Credible Interval (0.589, 1.53)
Associated Peptides 1
Associated Spectra 1
Coverage NaN

| A | 2.5 | 50 | 97.5 | Sequence |
|---|-----|----|------|----------|
| 1 | 0.57 | 0.95 | 1.6 | IQPDTIIQVWR |

5.271 paralemmin isoform 1 [Homo sapiens]

Protein Accession gi|93141031
Mean Expression Ratio 1.05
Median Expression Ratio 1.05
Credible Interval (0.637, 1.73)
Associated Peptides 1
Associated Spectra 1
Coverage 0.0491

| A | 2.5 | 50 | 97.5 | Sequence |
|---|-----|----|------|----------|
| 1 | 0.63 | 1.1 | 1.8 | EPAPPNGSAAEPPTEAASR |

M E V L A A E T T S Q Q E R L Q A I A E K R K R Q A E I E N K R R Q L E D E R R Q L Q H L K S K A L R E R W L L E G T P S S A S E G D E D L R R Q M D D E Q K

81 T R L L E D S V S R L E K E I E V L E R G D S A P A T A K E N A A A P S P V R A P A P S P A K E E R K T E V V M N S Q Q T P V G T P K D K R V S N T P L R T V D

161 G S P M M K A A M Y S V E I T V E K D K V T G E T R V L S S T T L L P R Q P L P L G I K V Y E D E T K V V H A V D G T A E N G I H P L S S S E V D E L I H K A D

241 E V T L S E A G S T A G A A E T R G A V E G A A R T T P S R R E I T G V Q A Q P G E A T S G P P G I Q P G Q E P P V T M I F M G Y Q N V E D E A E T K K V L G L

321 Q D T I T A E L V V I E D A A E P K E P A P P N G S A A E P P T E A A S R E E N Q A G P E A T T S D P Q D L D M K K H R C K C C S I M

5.272 mannosidase, alpha, class 2B, member 2 [Homo sapiens]

Protein Accession gi|50659093
Mean Expression Ratio 1.04
Median Expression Ratio 1.05
Credible Interval (0.676, 1.62)
Associated Peptides 2
Associated Spectra 2
Coverage 0.0238

| A | 2.5 | 50 | 97.5 | Sequence |
|---|-----|-----|------|----------|
| 1 | 0.66 | 1.1 | 1.7 | FIAVEQEFFR |
| 1 | 0.65 | 1.0 | 1.7 | IEQEYQAGPLELNR |

1 M G Q L C W L P L L A P L L L L R P P G V Q S A G P I R A F V V P H S H M D V G W V Y T V Q E S M R A Y A A N V Y T S V V E E L A R G Q Q R R F I A V E Q E F F

81 R L W W D G V A S D Q Q K Y Q V R Q L L E E G R L E F V I G G Q V M H D E A V T H L D D Q I L Q L T E G H G F L Y E T F G I R P Q F S W H V D P F G A S A T T P

161 T L F A L A G F N A H L G S R I D Y D L K A A M Q E A R G L Q F V W R G S P S L S E R Q E I F T H I M D Q Y S Y C T P S H I P F S N R S G F Y W N G V A V F P K

241 P P Q D G V Y P N M S E P V T P A N I N L Y A E A L V A N V K Q R A A W F R T P H V L W P W G C D K Q F F N A S V Q F A N M D P L L D H I N S H A A E L G V S V

321 Q Y A T L G D Y F R A L H A L N V T W R V R D H H D F L P Y S T E P F Q A W T G F Y T S R S S L K G L A R R A S A L L Y A G E S M F T R Y L W P A P R G H L D P

401 T W A L Q Q L Q Q L R W A V S E V Q H H D A I T G T E S P K V R D M Y A T H L A S G M L G M R K L M A S I V L D E L Q P Q A P M A A S S D A G P A G H F A S V Y

234

481 NPLAWTVTTIVTLTVGFPGVRVTDEAGHPVPSQIQNSTET PSAYDLLILTTIPGLSYRHYNIRPTAGAQEGTQEPAATVA

561 STLQFGRRLRRRTSHAGRYLVPVANDCYIVLLDQDTNLM HSIWERQSNRTVRVTQEFLEYHVNGDVKQGPISDNYLFTPG

641 KAAVPAWEAVEMEIVAGQLVTEIRQYFYRNMTAQNYTY AIRSRLTHVPQGHDGELLCHRIEQEYQAGPLELNREAVLRTS

721 TNLNSQQVIYSDNNGYQMQRRPYVSYVNNSIARNYYPMV QSAFMEDGKSRLVLLSERAHGISSQGNGQVEVMLHRRLWN N

801 FDWDLGYNLTLNDTSVVHPVLWLLLGSWSLTTALRQRSA LALQHRPVVLFGDLAGTAPKLPGPQQQEAVTLPPNLHLQIL

881 SIPGWRYSSNHTEHSQNLRKGHRGEAQADLRRVLLRLYH LYEVGEDPVLSQPVTVNLEAVLQALGSVVAVEERSLTGTWD

961 LSMLHRWSWRTGPGRHRGDTTSPSRPPGGPIITVHPKEIR TFFIHFQQQ

5.273 CD74 antigen isoform a [Homo sapiens]; CD74 antigen isoform c [Homo sapiens]; CD74 antigen isoform b [Homo sapiens]

Protein Accession gi|68448544 gi|68448537 gi|10835071
Mean Expression Ratio 1.04
Median Expression Ratio 1.04
Credible Interval (0.672, 1.58)
Associated Peptides 2
Associated Spectra 2
Coverage NaN

| A | 2.5 | 50 | 97.5 | Sequence |
|---|-----|-----|------|----------|
| 1 | 0.65 | 1.1 | 1.7 | DLISNNEQLPMLGR |
| 1 | 0.66 | 1.1 | 1.7 | RPGAPESK |

5.274 dipeptidylpeptidase IV [Homo sapiens]

Protein Accession gi|18765694
Mean Expression Ratio 0.958
Median Expression Ratio 0.957
Credible Interval (0.749, 1.22)
Associated Peptides 9

Associated Spectra 12
Coverage 0.141

| A | 2.5 | 50 | 97.5 | Sequence |
|---|---|---|---|---|
| 1 | 0.68 | 0.96 | 1.4 | CGIAVAPVSR |
| 1 | 0.71 | 0.98 | 1.4 | FRPSEPHFTLDGNSFYK |
| 1 | 0.66 | 0.94 | 1.3 | ISLQWLR |
| 2 | 0.63 | 0.87 | 1.2 | LGTFEVEDQIEAAR |
| 1 | 0.67 | 0.95 | 1.4 | SFYSDESLQYPK |
| 1 | 0.7 | 0.98 | 1.4 | VWNNDIYVK |
| 1 | 0.68 | 0.97 | 1.4 | WEYYDSVYTER |
| 2 | 0.71 | 0.99 | 1.4 | YMGLPTPEDNLDHYR |
| 2 | 0.7 | 0.97 | 1.3 | FWYQMILPPHFDK |

1 MKTPWKVLLGLLGAAALVTIITVPVVLLNKGTDDATADSR
KTYTLTDYLKNTYRLKLYSLRWISDHEYLYKQENNILVFN

81 AEYGNSSVFLENSTFDEFGHSINDYSISPDGQFILLEYNYV
KQWRHSYTASYDIYDLNKRQLITEERIPNNTQWVTWSPV

161 GHKLAYVWNNDIYVKIEPNLPSYRITWTGKEDIIYNGITD
WVYEEEVFSAYSALWWSPNGTFLAYAQFNDTEVPLIEYSF

241 YSDESLQYPKTVRVPYPKAGAVNPTVKFFVVNTDSLSSV
TNATSIQITAPASMLIGDHYLCDVTWATQERISLQWLRRIQ

321 NYSVMDICDYDESSGRWNCLVARQHIEMSTTGWVGRFRP
SEPHFTLDGNSFYKIISNEEGYRHICYFQIDKKDCTFITKG

401 TWEVIGIEALTSDYLYYISNEYKGMPGGRNLYKIQLSDYT
KVTCLSCELNPERCQYYSVSFSKEAKYYQLRCSGPGLPLY

481 TLHSSVNDKGLRVLEDNSALDKMLQNVQMPSKKLDFIIL
NETKFWYQMILPPHFDKSKKYPLLLDVYAGPCSQKADTVFR

561 LNWATYLASTENIIVASFDGRGSGYQGDKIMHAINRRLGT
FEVEDQIEAARQFSKMGFVDNKRIAIWGWSYGGYVTSMVL

641 GSGSGVFKCGIAVAPVSRWEYYDSVYTERYMGLPTPEDN
LDHYRNSTVMSRAENFKQVEYLLIHGTADDNVHFQQSAQIS

721 K A L V D V G V D F Q A M W Y T D E D H G I A S S T A H Q H I Y T H M S H F I K Q C F S L P

5.275 lipocalin 2 [Homo sapiens]

Protein Accession gi|38455402
Mean Expression Ratio 0.955
Median Expression Ratio 0.959
Credible Interval (0.658, 1.37)
Associated Peptides 3
Associated Spectra 4
Coverage 0.247

| A | 2.5 | 50 | 97.5 | Sequence |
|---|-----|-----|------|----------|
| 2 | 0.63 | 0.93 | 1.4 | SLGLPENHIVFPVPIDQCIDG |
| 1 | 0.64 | 0.96 | 1.5 | VPLQQNFQDNQFQGK |
| 1 | 0.63 | 0.97 | 1.5 | WYVVGLAGNAILR |

1 M P L G L L W L G L A L L G A L H A Q A Q D S T S D L I P A P P L S K V P L Q Q N F Q D N Q F Q G K W Y V V G L A G N A I L R E D K D P Q K M Y A T I Y E L K E

81 D K S Y N V T S V L F R K K K C D Y W I R T F V P G C Q P G E F T L G N I K S Y P G L T S Y L V R V V S T N Y N Q H A M V F F K K V S Q N R E Y F K I T L Y G R

161 TKELTSELKENFIRFSKSLGLPENHIVFPVPIDQCIDG

5.276 zinc finger protein 262 [Homo sapiens]

Protein Accession gi|44890068
Mean Expression Ratio 0.956
Median Expression Ratio 0.959
Credible Interval (0.585, 1.54)
Associated Peptides 1
Associated Spectra 1
Coverage 0.00904

| A | 2.5 | 50 | 97.5 | Sequence |
|---|-----|-----|------|----------|
| 1 | 0.57 | 0.95 | 1.6 | SSAEMIENTNSLGK |

1 M A E R E V E S G P R K R F E Q K S G A V F D E I V E N C G G I M D T E M S E D I D H N L T P T L D S M S Y G M P N Q T G S E N S L L D E D D Y F L N S G D L A

81 G I P V V G S D N E D E Q D F S S K D N L V S S I H T D D S L E V E R R V T Q H
E S D N E N E I Q I Q N K L K K D F P K Q F D Q V S V F K S I R K D F S L V R E

161 N S K E T F S G K E K N R D L T Y E R E K R L D K P H K D L D S R L K S S F F D
K A A N Q V E E T L H T H L P Q T P E T N F R D S S Y P F A N K E S I G S E L G

241 N S F A S N I R I K E E P L D D E Y D K A M A P Q Q G L L D K I K D E P D N A Q
E Y S H G Q Q Q K T Q E G E L K I S A V F S V S G S P L A P Q L T T G F Q P S L

321 A S S G M N K M L P S V P A T A V R V S C S G C K K I L Q K G Q T A Y Q R K G
S T Q L F C S T L C L T G Y T V P P A R P P P P L T K K T C S S C S K D I L N P K

401 D V I S A Q F E N T T T S K D F C S Q S C L S T Y E L K K K P I V T I N T N S I S
T K C S M C Q K N A V I R H E V N Y Q N V V H K L C S D A C F S K F R S A N N

481 L T M N C C E N C G G Y C Y S G S G Q C H M L Q I E G Q S K K F C S S S C I T
A Y K Q K S A K I T P C A L C K S L R S S A E M I E N T N S L G K T E L F C S V N

561 C L S A Y R V K M V T S A G V Q V Q C N S C K T S A I P Q Y H L A M S D G S I
R N F C S Y S C V V A F Q N L F N K P T G M N S S V V P L S Q G Q V I V S I P T G

641 S T V S A G G G S T S A V S P T S I S S S A A A G L Q R L A A Q S Q H V G F A R
S V V K L K C Q H C N R L F A T K P E L L D Y K G K M F Q F C G K N C S D E Y K

721 K I N N V M A M C E Y C K I E K I V K E T V R F S G A D K S F C S E G C K L L
Y K H D L A K R W G N H C K M C S Y C L Q T S P K L V Q N N L G G K V E E F C C
E

801 E C M S K Y T V L F Y Q M A K C D A C K R Q G K L S E S L K W R G E M K H F
C N L L C I L M F C N Q Q S V C D P P S Q N N A A N I S M V Q A A S A G P P S L R
K

881 D S T P V I A N V V S L A S A P A A Q P T V N S N S V L Q G A V P T V T A K I I
G D A S T Q T D A L K L P P S Q P P R L L K N K A L L C K P I T Q T K A T S C K

961 P H T Q N K E C Q T E D T P S Q P Q I I V V P V P V P V F V P I P L H L Y T Q Y A
P V P F G I P V P M P V P M L I P S S M D S E D K V T E S I E D I K E K L P T

1041 H P F E A D L L E M A E M I A E D E E K K T L S Q G E S Q T S E H E L F L D T
K I F E K D Q G S T Y S G D L E S E A V S T P H S W E E L N H Y A L K S N A V Q

1121 E A D S E L K Q F S K G E T E Q D L E A D F P S D S F D P L N K G Q G I Q A R
S R T R R R H R D G F P Q P R R R G R K K S I V A V E P R S L I Q G A F Q G C S V

1201 SGMTLKYMYGVNAWKNWVQWKNAKEEQGDLKCGGVE QASSSPRSDPLGSTQDHALSQESSEPGCRVRSIKLKEDILSCT F

1281 AELSLGLCQFIQEVRRPNGEKYDPDSILYLCLGIQQYLFE NGRIDNIFTEPYSRFMIELTKLLKIWEPTILPNGYMFSRI

1361 EEEHLWECKQLGAYSPIVLLNTLLFFNTKYFQLKNVTEH LKLSFAHVMRRTRTLKYSTKMTYLRFFPPLQKQESEPDKLT

1441 VGKRKRNEDDEVPVGVEMAENTDNPLRCPVRLYEFYLS KCSESVKQRNDVFYLQPERSCVPNSPMWYSTFPIDPGTLDT M

1521 LTRILMVREVHEELAKAKSEDSDVELSD

5.277 angiotensin I converting enzyme isoform 1 precursor [Homo sapiens]

Protein Accession gi|4503273
Mean Expression Ratio 1.04
Median Expression Ratio 1.04
Credible Interval (0.681, 1.59)
Associated Peptides 2
Associated Spectra 2
Coverage 0.0253

| A | 2.5 | 50 | 97.5 | Sequence |
|---|-----|----|------|----------|
| 1 | 0.65 | 1.0 | 1.7 | SMYETPSLEQDLER |
| 1 | 0.65 | 1.1 | 1.7 | RQEEAALLSQEFAEAWGQK |

1 MGAASGRRGPGLLLPLPLLLLLPPQPALALDPGLQPGNFSA DEAGAQLFAQSYNSSAEQVLFQSVAASWAHDTNITAENA

81 RRQEEAALLSQEFAEAWGQKAKELYEPIWQNFTDPQLRRI IGAVRTLGSANLPLAKRQQYNALLSNMSRIYSTAKVCLPN

161 KTATCWSLDPDLTNILASSRSYAMLLFAWEGWHNAAGIP LKPLYEDFTALSNEAYKQDGFTDTGAYWRSWYNSPTFEDDL

241 EHLYQQLEPLYLNLHAFVRRALHRRYGDRYINLRGPIPAH LLGDMWAQSWENIYDMVVPFPDKPNLDVTSTMLQQGWNAT

321 H M F R V A E E F F T S L E L S P M P P E F W E G S M L E K P A D G R E V V C H A S A W D F Y N R K D F R I K Q C T R V T M D Q L S T V H H E M G H I Q Y Y L Q

401 Y K D L P V S L R R G A N P G F H E A I G D V L A L S V S T P E H L H K I G L L D R V T N D T E S D I N Y L L K M A L E K I A F L P F G Y L V D Q W R W G V F S

481 G R T P P S R Y N F D W W Y L R T K Y Q G I C P P V T R N E T H F D A G A K F H V P N V T P Y I R Y F V S F V L Q F Q F H E A L C K E A G Y E G P L H Q C D I Y

561 R S T K A G A K L R K V L Q A G S S R P W Q E V L K D M V G L D A L D A Q P L L K Y F Q P V T Q W L Q E Q N Q Q N G E V L G W P E Y Q W H P P L P D N Y P E G I

641 D L V T D E A E A S K F V E E Y D R T S Q V V W N E Y A E A N W N Y N T N I T T E T S K I L L Q K N M Q I A N H T L K Y G T Q A R K F D V N Q L Q N T T I K R I

721 I K K V Q D L E R A A L P A Q E L E E Y N K I L L D M E T T Y S V A T V C H P N G S C L Q L E P D L T N V M A T S R K Y E D L L W A W E G W R D K A G R A I L Q

801 F Y P K Y V E L I N Q A A R L N G Y V D A G D S W R S M Y E T P S L E Q D L E R L F Q E L Q P L Y L N L H A Y V R R A L H R H Y G A Q H I N L E G P I P A H L L

881 G N M W A Q T W S N I Y D L V V P F P S A P S M D T T E A M L K Q G W T P R R M F K E A D D F F T S L G L L P V P P E F W N K S M L E K P T D G R E V V C H A S

961 A W D F Y N G K D F R I K Q C T T V N L E D L V V A H H E M G H I Q Y F M Q Y K D L P V A L R E G A N P G F H E A I G D V L A L S V S T P K H L H S L N L L S S

1041 E G G S D E H D I N F L M K M A L D K I A F I P F S Y L V D Q W R W R V F D G S I T K E N Y N Q E W W S L R L K Y Q G L C P P V P R T Q G D F D P G A K F H I P

1121 S S V P Y I R Y F V S F I I Q F Q F H E A L C Q A A G H T G P L H K C D I Y Q S K E A G Q R L A T A M K L G F S R P W P E A M Q L I T G Q P N M S A S A M L S Y

1201 F K P L L D W L R T E N E L H G E K L G W P Q Y N W T P N S A R S E G P L P D S G R V S F L G L D L D A Q Q A R V G Q W L L L F L G I A L L V A T L G L S Q R L

1281 FSIRHRSLHRHSHGPQFGSEVELRHS

5.278 skeletal muscle receptor tyrosine kinase [Homo sapiens]

Protein Accession gi|5031927
Mean Expression Ratio 0.956
Median Expression Ratio 0.96
Credible Interval (0.579, 1.54)
Associated Peptides 1
Associated Spectra 1
Coverage 0.0196

| A | 2.5 | 50 | 97.5 | Sequence |
|---|-----|----|------|----------|
| 1 | 0.56 | 0.94 | 1.6 | CIARQVAAGMAYLSERK |

1 M R E L V N I P L V H I L T L V A F S G T E K L P K A P V I T T P L E T V D A L V E
E V A T F M C A V E S Y P Q P E I S W T R N K I L I K L F D T R Y S I R E N

81 G Q L L T I L S V E D S D D G I Y C C T A N N G V G G A V E S C G A L Q V K M
K P K I T R P P I N V K I I E G L K A V L P C T T M G N P K P S V S W I K G D S P

161 L R E N S R I A V L E S G S L R I H N V Q K E D A G Q Y R C V A K N S L G T A
Y S K V V K L E V E V F A R I L R A P E S H N V T F G S F V T L H C T A T G I P V

241 P T I T W I E N G N A V S S G S I Q E S V K D R V I D S R L Q L F I T K P G L Y T
C I A T N K H G E K F S T A K A A A T I S I A E W S K P Q K D N K G Y C A Q Y

321 R G E V C N A V L A K D A L V F L N T S Y A D P E E A Q E L L V H T A W N E L
K V V S P V C R P A A E A L L C N H I F Q E C S P G V V P T P I P I C R E Y C L A

401 V K E L F C A K E W L V M E E K T H R G L Y R S E M H L L S V P E C S K L P S
M H W D P T A C A R L P H L D Y N K E N L K T F P P M T S S K P S V D I P N L P S

481 S S S S S F S V S P T Y S M T V I I S I M S S F A I F V L L T I T T L Y C C R R R K
Q W K N K K R E S A A V T L T T L P S E L L L D R L H P N P M Y Q R M P L L

561 L N P K L L S L E Y P R N N I E Y V R D I G E G A F G R V F Q A R A P G L L P Y
E P F T M V A V K M L K E E A S A D M Q A D F Q R E A A L M A E F D N P N I V K

641 L L G V C A V G K P M C L L F E Y M A Y G D L N E F L R S M S P H T V C S L S
H S D L S M R A Q V S S P G P P P L S C A E Q L C I A R Q V A A G M A Y L S E R K

721 F V H R D L A T R N C L V G E N M V V K I A D F G L S R N I Y S A D Y Y K A N
E N D A I P I R W M P P E S I F Y N R Y T T E S D V W A Y G V V L W E I F S Y G L

801 Q P Y Y G M A H E E V I Y Y V R D G N I L S C P E N C P V E L Y N L M R L C W S K L P A D R P S F T S I H R I L E R M C E R A E G T V S V

5.279 spastin isoform 2 [Homo sapiens]; spastin isoform 1 [Homo sapiens]

Protein Accession gi|40806170 gi|11875211
Mean Expression Ratio 1.04
Median Expression Ratio 1.04
Credible Interval (0.638, 1.69)
Associated Peptides 1
Associated Spectra 1
Coverage NaN

| A | 2.5 | 50 | 97.5 | Sequence |
|---|-----|-----|------|----------|
| 1 | 0.64 | 1.0 | 1.8 | SSGAAPAPASASAPAPVPGGEAER |

5.280 allograft inflammatory factor 1 isoform 3 [Homo sapiens]

Protein Accession gi|14574568
Mean Expression Ratio 1.05
Median Expression Ratio 1.04
Credible Interval (0.698, 1.57)
Associated Peptides 1
Associated Spectra 3
Coverage 0.0748

| A | 2.5 | 50 | 97.5 | Sequence |
|---|-----|-----|------|----------|
| 3 | 0.71 | 1.1 | 1.6 | MSQTRDLQGGK |

1 M S Q T R D L Q G G K A F G L L K A Q Q E E R L D E I N K Q F L D D P K Y S S D E D L P S K L E G F K E K Y M E F D L N G N G D I D I M S L K R M L E K L G V P

81 K T H L E L K K L I G E V S S G S G E T F S Y P D F L R M M L G K R S A I L K M I L M Y E E K A R E K E K P T G P P A K K A I S E L P

5.281 carbonyl reductase 1 [Homo sapiens]

Protein Accession gi|4502599
Mean Expression Ratio 1.05
Median Expression Ratio 1.04
Credible Interval (0.726, 1.51)
Associated Peptides 2
Associated Spectra 4
Coverage 0.105

| A | 2.5 | 50 | 97.5 | Sequence |
|---|-----|-----|------|----------|
| 3 | 0.73 | 1.0 | 1.5 | FHQLDIDDLQSIR |
| 1 | 0.68 | 1.1 | 1.6 | GQAAVQQLQAEGLSPR |

1 M S S G I H V A L V T G G N K G I G L A I V R D L C R L F S G D V V L T A R D V T R G Q A A V Q Q L Q A E G L S P R F H Q L D I D D L Q S I R A L R D F L R K E

81 Y G G L D V L V N N A G I A F K V A D P T P F H I Q A E V T M K T N F F G T R D V C T E L L P L I K P Q G R V V N V S S I M S V R A L K S C S P E L Q Q K F R S

161 E T I T E E E L V G L M N K F V E D T K K G V H Q K E G W P S S A Y G V T K I G V T V L S R I H A R K L S E Q R K G D K I L L N A C C P G W V R T D M A G P K A

241 TKSPEEGAETPVYLALLPPDAEGPHGQFVSEKRVEQW

5.282 sodium/hydrogen exchanger regulatory factor 1 [Homo sapiens]

Protein Accession gi|4759140
Mean Expression Ratio 0.961
Median Expression Ratio 0.96
Credible Interval (0.78, 1.19)
Associated Peptides 10
Associated Spectra 19
Coverage 0.26

| A | 2.5 | 50 | 97.5 | Sequence | |
|---|-----|-----|------|----------|---|
| 6 | 0.75 | 0.97 | 1.2 | AQEAPGQAEPPAAAEVQGAGNENEPR | |
| 1 | 0.72 | 1 | 1.4 | EADKSHPEQR | |
| 1 | 0.69 | 0.96 | 1.4 | EALAEAALESPR | |
| 1 | 0.65 | 0.9 | 1.2 | EAPGQAEPPAAAEVQGAGNENEPR | |
| 3 | 0.75 | 1 | 1.4 | EPPAAAEVQGAGNENEPR | |
| 1 | 0.68 | 0.93 | 1.3 | QEAPGQAEPPAAAEVQGAGNENEPR | |
| 1 | 0.73 | 1.0 | 1.4 | SKPGQFIR | |
| 3 | 0.68 | 0.9 | 1.2 | VIPSQEHLNGPLPVPF | |
| 1 | 0.7 | 0.97 | 1.3 | ETDEFFK | |
| 1 | 0.69 | 0.97 | 1.3 | SVDPDSPAEASGLR | |

1 M S A D A A A G A P L P R L C C L E K G P N G Y G F H L H G E K G K L G Q Y I R L V E P G S P A E K A G L L A G D R L V E V N G E N V E K E T H Q Q V V S R I R

81 A A L N A V R L L V V D P E T D E Q L Q K L G V Q V R E E L L R A Q E A P G Q A E P P A A A E V Q G A G N E N E P R E A D K S H P E Q R E L R P R L C T M K K G

161 P S G Y G F N L H S D K S K P G Q F I R S V D P D S P A E A S G L R A Q D R I V E V N G V C M E G K Q H G D V V S A I R A G G D E T K L L V V D R E T D E F F K

241 K C R V I P S Q E H L N G P L P V P F T N G E I Q K E N S R E A L A E A A L E S P R P A L V R S A S S D T S E E L N S Q D S P P K Q D S T A P S S T S S S D P I

321 LDFNISLAMAKERAHQKRSSKRAPQMDWSKKNELFSNL

5.283 argininosuccinate synthetase 1 [Homo sapiens]; argininosuccinate synthetase 1 [Homo sapiens]

Protein Accession gi|53759107 gi|16950633
Mean Expression Ratio 1.04
Median Expression Ratio 1.04
Credible Interval (0.682, 1.58)
Associated Peptides 2
Associated Spectra 2
Coverage NaN

| A | 2.5 | 50 | 97.5 | Sequence |
|---|-----|-----|------|----------|
| 1 | 0.65 | 1.1 | 1.7 | EFVEEFIWPAIQSSALYEDR |
| 1 | 0.64 | 1.0 | 1.7 | APNTPDILEIEFK |

5.284 spinster homolog 1 isoform 1 [Homo sapiens]; spinster homolog 1 isoform 1 [Homo sapiens]

Protein Accession gi|215490096 gi|14042968
Mean Expression Ratio 1.04
Median Expression Ratio 1.04
Credible Interval (0.707, 1.55)
Associated Peptides 2
Associated Spectra 3
Coverage NaN

| A | 2.5 | 50 | 97.5 | Sequence |
|---|-----|-----|------|----------|
| 1 | 0.66 | 1.0 | 1.6 | AQLHVQGLLHEAGSTDDR |
| 2 | 0.7 | 1.1 | 1.6 | SEEPEVPDQEGLQR |

5.285 quinolinate phosphoribosyltransferase [Homo sapiens]

Protein Accession gi|45269149
Mean Expression Ratio 1.04
Median Expression Ratio 1.04
Credible Interval (0.64, 1.67)
Associated Peptides 1
Associated Spectra 1

Coverage 0.0539

| A | 2.5 | 50 | 97.5 | Sequence |
|---|-----|-----|------|----------|
| 1 | 0.63 | 1.0 | 1.7 | CSGIASAAAAVEAAR |

1 M D A E G L A L L L P P V T L A A L V D S W L R E D C P G L N Y A A L V S G A G
P S Q A A L W A K S P G V L A G Q P F F D A I F T Q L N C Q V S W F L P E G S K

81 L V P V A R V A E V R G P A H C L L L G E R V A L N T L A R C S G I A S A A A A
A V E A A R G A G W T G H V A G T R K T T P G F R L V E K Y G L L V G G A A S H

161 R Y D L G G L V M V K D N H V V A A G G V E K A V R A A R Q A A D F A L K
V E V E C S S L Q E A V Q A A E A G A D L V L L D N F K P E E L H P T A T V L K A
Q F

241 P S V A V E A S G G I T L D N L P Q F C G P H I D V I S M G M L T Q A A P A L D
F S L K L F A K E V A P V P K I H

5.286 transmembrane protein 106A [Homo sapiens]; PREDICTED: similar to Transmembrane protein 106A [Homo sapiens]; PREDICTED: similar to Transmembrane protein 106A isoform 3 [Homo sapiens]

Protein Accession gi|21450796 gi|169211133 gi|13427131
Mean Expression Ratio 0.97
Median Expression Ratio 0.965
Credible Interval (0.601, 1.58)
Associated Peptides 1
Associated Spectra 1
Coverage NaN

| A | 2.5 | 50 | 97.5 | Sequence |
|---|-----|------|------|----------|
| 1 | 0.58 | 0.96 | 1.6 | QLVALIPYGDQR |

5.287 uroplakin 1A [Homo sapiens]

Protein Accession gi|5902148
Mean Expression Ratio 1.04
Median Expression Ratio 1.04
Credible Interval (0.64, 1.70)
Associated Peptides 1
Associated Spectra 1
Coverage 0.062

| A | 2.5 | 50 | 97.5 | Sequence |
|---|-----|-----|------|----------|
| 1 | 0.63 | 1.1 | 1.8 | AATPEVVFPWPPLCCR |

1 M A S A A A A E A E K G S P V V V G L L V V G N I I I L L S G L S L F A E T I W V T A D Q Y R V Y P L M G V S G K D D V F A G A W I A I F C G F S F F M V A S F

81 G V G A A L C R R R S M V L T Y L V L M L I V Y I F E C A S C I T S Y T H R D Y M V S N P S L I T K Q M L T F Y S A D T D Q G Q E L T R L W D R V M I E Q E C C

161 G T S G P M D W V N F T S A F R A A T P E V V F P W P P L C C R R T G N F I P L N E E G C R L G H M D Y L F T K G C F E H I G H A I D S Y T W G I S W F G F A I

241 LMWTLPVMLIAMYFYTML

5.288 secretory carrier membrane protein 3 isoform 1 [Homo sapiens]

Protein Accession gi|16445419
Mean Expression Ratio 0.964
Median Expression Ratio 0.965
Credible Interval (0.593, 1.56)
Associated Peptides 1
Associated Spectra 1
Coverage 0.075

| A | 2.5 | 50 | 97.5 | Sequence |
|---|-----|-----|------|----------|
| 1 | 0.57 | 0.95 | 1.6 | EPPPAYEPPAPAPLPPPSAPSLQPSR |

1 M A Q S R D G G N P F A E P S E L D N P F Q D P A V I Q H R P S R Q Y A T L D V Y N P F E T R E P P P A Y E P P A P A P L P P P S A P S L Q P S R K L S P T E P

81 K N Y G S Y S T Q A S A A A A T A E L L K K Q E E L N R K A E E L D R R E R E L Q H A A L G G T A T R Q N N W P P L P S F C P V Q P C F F Q D I S M E I P Q E F

161 Q K T V S T M Y Y L W M C S T L A L L L N F L A C L A S F C V E T N N G A G F G L S I L W V L L F T P C S F V C W Y R P M Y K A F R S D S S F N F F V F F F I F

241 F V Q D V L F V L Q A I G I P G W G F S G W I S A L V V P K G N T A V S V L M L L V A L L F T G I A V L G I V M L K R I H S L Y R R T G A S F Q K A Q Q E F A A

321 GVFSNPAVRTAAANAAAGAAENAFRAP

5.289 guanine nucleotide binding protein (G protein), gamma 2 [Homo sapiens]

Protein Accession gi|54114974
Mean Expression Ratio 1.04
Median Expression Ratio 1.04
Credible Interval (0.635, 1.69)
Associated Peptides 1
Associated Spectra 1
Coverage 0.225

| A | 2.5 | 50 | 97.5 | Sequence |
|---|---|---|---|---|
| 1 | 0.62 | 1.0 | 1.8 | EDPLLTPVPASENPFR |

1 MASNNTASIAQARKLVEQLKMEANIDRIKVSKAAADLMAY
CEAHAKEDPLLTPVPASENPFREKKFFCAIL

5.290 sorbitol dehydrogenase [Homo sapiens]

Protein Accession gi|156627571
Mean Expression Ratio 0.967
Median Expression Ratio 0.967
Credible Interval (0.601, 1.58)
Associated Peptides 1
Associated Spectra 1
Coverage 0.0476

| A | 2.5 | 50 | 97.5 | Sequence |
|---|---|---|---|---|
| 1 | 0.58 | 0.95 | 1.6 | LENYPIPEPGPNEVLLR |

1 MAAAAKPNNLSLVVHGPGDLRLENYPIPEPGPNEVLLRMH
SVGICGSDVHYWEYGRIGNFIVKKPMVLGHEASGTVEKVG

81 SSVKHLKPGDRVAIEPGAPRENDEFCKMGRYNLSPSIFFCA
TPPDDGNLCRFYKHNAAFCYKLPDNVTFEEGALIEPLSV

161 GIHACRRGGVTLGHKVLVCGAGPIGMVTLLVAKAMGAA
QVVVTDLSATRLSKAKEIGADLVLQISKESPQEIARKVEGQL

241 GCKPEVTIECTGAEASIQAGIYATRSGGNLVLVGLGSEMT
TVPLLHAAIREVDIKGVFRYCNTWPVAISMLASKSVNVKP

321 LVTHRFPLEKALEAFETFKKGLGLKIMLKCDPSDQNP

5.291 major facilitator superfamily domain containing 1 [Homo sapiens]

Protein Accession gi|12232393

Mean Expression Ratio 0.97
Median Expression Ratio 0.967
Credible Interval (0.601, 1.6)
Associated Peptides 1
Associated Spectra 1
Coverage 0.0258

| A | 2.5 | 50 | 97.5 | Sequence |
|---|-----|----|----|----|
| 1 | 0.56 | 0.96 | 1.6 | ALLAGGPDEADR |

1 M E E E D E E A R A L L A G G P D E A D R G A P A A P G A L P A L C D P S R L A
H R L L V L L L M C F L G F G S Y F C Y D N P A A L Q T Q V K R D M Q V N T T K

81 F M L L Y A W Y S W P N V V L C F F G G F L I D R V F G I R W G T I I F S C F V
C I G Q V V F A L G G I F N A F W L M E F G R F V F G I G G E S L A V A Q N T Y

161 A V S W F K G K E L N L V F G L Q L S M A R I G S T V N M N L M G W L Y S K
I E A L L G S A G H T T L G I T L M I G G V T C I L S L I C A L A L A Y L D Q R A E

241 R I L H K E Q G K T G E V I K L T D V K D F S L P L W L I F I I C V C Y Y V A V
F P F I G L G K V F F T E K F G F S S Q A A S A I N S V V Y V I S A P M S P V F

321 G L L V D K T G K N I I W V L C A V A A T L V S H M M L A F T M W N P W I A
M C L L G L S Y S L L A C A L W P M V A F V V P E H Q L G T A Y G F M Q S I Q N L
G

401 L A I I S I I A G M I L D S R G Y L F L E V F F I A C V S L S L L S V V L L Y L V N
R A Q G G N L N Y S A R Q R E E I K F S H T E

5.292 superoxide dismutase 3, extracellular precursor [Homo sapiens]

Protein Accession gi|118582275
Mean Expression Ratio 1.03
Median Expression Ratio 1.03
Credible Interval (0.669, 1.6)
Associated Peptides 2
Associated Spectra 2
Coverage 0.1

| A | 2.5 | 50 | 97.5 | Sequence |
|---|-----|----|----|----|
| 1 | 0.65 | 1.1 | 1.7 | VTEIWQEVMQR |
| 1 | 0.64 | 1.0 | 1.7 | AVVVHAGEDDLGR |

MLALLCSCLLLAAGASDAWTGEDSAEPNSDSAEWIRDMYA
KVTEIWQEVMQRRDDDGALHAACQVQPSATLDAAQPRVTG

VVLFRQLAPRAKLDAFFALEGFPTEPNSSSRAIHVHQFGDL
SQGCESTGPHYNPLAVPHPQHPGDFGNFAVRDGSLWRYR

AGLAASLAGPHSIVGRAVVVHAGEDDLGRGGNQASVEN
GNAGRRLACCVVGVCGPGLWERQAREHSERKKRRRESECK
AA

5.293 nuclear transport factor 2 [Homo sapiens]

Protein Accession gi|5031985
Mean Expression Ratio 0.967
Median Expression Ratio 0.967
Credible Interval (0.599, 1.58)
Associated Peptides 1
Associated Spectra 1
Coverage 0.110

| A | 2.5 | 50 | 97.5 | Sequence |
|---|-----|-----|------|----------|
| 1 | 0.57 | 0.96 | 1.6 | NINDAWVCTNDMFR |

MGDKPIWEQIGSSFIQHYYQLFDNDRTQLGAIYIDASCLTW
EGQQFQGKAAIVEKLSSLPFQKIQHSITAQDHQPTPDSC

IISMVVGQLKADEDPIMGFHQMFLLKNINDAWVCTNDMF
RLALHNFG

5.294 hexosaminidase B preproprotein [Homo sapiens]

Protein Accession gi|4504373
Mean Expression Ratio 0.967
Median Expression Ratio 0.967
Credible Interval (0.686, 1.36)
Associated Peptides 4
Associated Spectra 4
Coverage 0.0971

| A | 2.5 | 50 | 97.5 | Sequence |
|---|-----|-----|------|----------|
| 1 | 0.64 | 0.97 | 1.4 | EISEVFPDQFIHLGGDEVEFK |
| 1 | 0.63 | 0.96 | 1.5 | LAPGTIVEVWK |
| 1 | 0.64 | 0.97 | 1.5 | VLDIIATINK |

(continued)

| A | 2.5 | 50 | 97.5 | Sequence |
|---|---|---|---|---|
| 1 | 0.63 | 0.96 | 1.4 | YLDLISYGQDWR |

1 M E L C G L G L P R P P M L L A L L L A T L L A A M L A L L T Q V A L V V Q V A E A A R A P S V S A K P G P A L W P L P L S V K M T P N L L H L A P E N F Y I S

81 H S P N S T A G P S C T L L E E A F R R Y H G Y I F G F Y K W H H E P A E F Q A K T Q V Q Q L L V S I T L Q S E C D A F P N I S S D E S Y T L L V K E P V A V L

161 K A N R V W G A L R G L E T F S Q L V Y Q D S Y G T F T I N E S T I I D S P R F S H R G I L I D T S R H Y L P V K I I L K T L D A M A F N K F N V L H W H I V D

241 D Q S F P Y Q S I T F P E L S N K G S Y S L S H V Y T P N D V R M V I E Y A R L R G I R V L P E F D T P G H T L S W G K G Q K D L L T P C Y S R Q N K L D S F G

321 P I N P T L N T T Y S F L T T F F K E I S E V F P D Q F I H L G G D E V E F K C W E S N P K I Q D F M R Q K G F G T D F K K L E S F Y I Q K V L D I I A T I N K

401 G S I V W Q E V F D D K A K L A P G T I V E V W K D S A Y P E E L S R V T A S G F P V I L S A P W Y L D L I S Y G Q D W R K Y Y K V E P L D F G G T Q K Q K Q L

481 F I G G E A C L W G E Y V D A T N L T P R L W P R A S A V G E R L W S S K D V R D M D D A Y D R L T R H C R M V E R G I A A Q P L Y A G Y C N H E N M

5.295 nucleoporin 188kDa [Homo sapiens]

Protein Accession gi|62955803
Mean Expression Ratio 0.967
Median Expression Ratio 0.967
Credible Interval (0.665, 1.41)
Associated Peptides 1
Associated Spectra 4
Coverage 0.00515

| A | 2.5 | 50 | 97.5 | Sequence |
|---|---|---|---|---|
| 4 | 0.67 | 0.96 | 1.4 | MLQHYLQNK |

1 M A A A A G G P C V R S S R E L W T I L L G R S A L R E L S Q I E A E L N K H W R R L L E G L S Y Y K P P S P S S A E K V K A N K D V A S P L K E L G L R I S K

81 F L G L D E E Q S V Q L L Q C Y L Q E D Y R G T R D S V K T V L Q D E R Q S Q A L I L K I A D Y Y Y E E R T C I L R C V L H L L T Y F Q D E R H P Y R V E Y A D

161 C V D K L E K E L V S K Y R Q Q F E E L Y K T E A P T W E T H G N L M T E R Q
V S R W F V Q C L R E Q S M L L E I I F L Y Y A Y F E M A P S D L L V L T K M F K

241 E Q G F G S R Q T N R H L V D E T M D P F V D R I G Y F S A L I L V E G M D I E
S L H K C A L D D R R E L H Q F A Q D G L I C Q D M D C L M L T F G D I P H H A

321 P V L L A W A L L R H T L N P E E T S S V V R K I G G T A I Q L N V F Q Y L T R
L L Q S L A S G G N D C T T S T A C M C V Y G L L S F V L T S L E L H T L G N Q

401 Q D I I D T A C E V L A D P S L P E L F W G T E P T S G L G I I L D S V C G M F P
H L L S P L L Q L L R A L V S G K S T A K K V Y S F L D K M S F Y N E L Y K H

481 K P H D V I S H E D G T L W R R Q T P K L L Y P L G G Q T N L R I P Q G T V G
Q V M L D D R A Y L V R W E Y S Y S S W T L F T C E I E M L L H V V S T A D V I Q

561 H C Q R V K P I I D L V H K V I S T D L S I A D C L L P I T S R I Y M L L Q R L T
T V I S P P V D V I A S C V N C L T V L A A R N P A K V W T D L R H T G F L P

641 F V A H P V S S L S Q M I S A E G M N A G G Y G N L L M N S E Q P Q G E Y G V
T I A F L R L I T T L V K G Q L G S T Q S Q G L V P C V M F V L K E M L P S Y H K

721 W R Y N S H G V R E Q I G C L I L E L I H A I L N L C H E T D L H S S H T P S L
Q F L C I C S L A Y T E A G Q T V I N I M G I G V D T I D M V M A A Q P R S D G

801 A E G Q G Q G Q L L I K T V K L A F S V T N N V I R L K P P S N V V S P L E Q A
L S Q H G A H G N N L I A V L A K Y I Y H K H D P A L P R L A I Q L L K R L A T

881 V A P M S V Y A C L G N D A A A I R D A F L T R L Q S K I E D M R I K V M I L
E F L T V A V E T Q P G L I E L F L N L E V K D G S D G S K E F S L G M W S C L H

961 A V L E L I D S Q Q Q D R Y W C P P L L H R A A I A F L H A L W Q D R R D S A
M L V L R T K P K F W E N L T S P L F G T L S P P S E T S E P S I L E T C A L I M

1041 K I I C L E I Y Y V V K G S L D Q S L K D T L K K F S I E K R F A Y W S G Y V
K S L A V H V A E T E G S S C T S L L E Y Q M L V S A W R M L L I I A T T H A D I

1121 M H L T D S V V R R Q L F L D V L D G T K A L L L V P A S V N C L R L G S M
K C T L L L I L L R Q W K R E L G S V D E I L G P L T E I L E G V L Q A D Q Q L M E

1201 K T K A K V F S A F I T V L Q M K E M K V S D I P Q Y S Q L V L N V C E T L Q
E E V I A L F D Q T R H S L A L G S A T E D K D S M E T D D C S R S R H R D Q R D

1281 G V C V L G L H L A K E L C E V D E D G D S W L Q V T R R L P I L P T L L T T L E V S L R M K Q N L H F T E A T L H L L L T L A R T Q Q G A T A V A G A G I T Q

1361 S I C L P L L S V Y Q L S T N G T A Q T P S A S R K S L D A P S W P G V Y R L S M S L M E Q L L K T L R Y N F L P E A L D F V G V H Q E R T L Q C L N A V R T V

1441 Q S L A C L E E A D H T V G F I L Q L S N F M K E W H F H L P Q L M R D I Q V N L G Y L C Q A C T S L L H S R K M L Q H Y L Q N K N G D G L P S A V A Q R V Q R

1521 P P S A A S A A P S S S K Q P A A D T E A S E Q Q A L H T V Q Y G L L K I L S K T L A A L R H F T P D V C Q I L L D Q S L D L A E Y N F L F A L S F T T P T F D

1601 S E V A P S F G T L L A T V N V A L N M L G E L D K K K E P L T Q A V G L S T Q A E G T R T L K S L L M F T M E N C F Y L L I S Q A M R Y L R D P A V H P R D K

1681 Q R M K Q E L S S E L S T L L S S L S R Y F R R G A P S S P A T G V L P S P Q G K S T S L S K A S P E S Q E P L I Q L V Q A F V R H M Q R

5.296 hypothetical protein LOC54978 [Homo sapiens]

Protein Accession gi|31542711
Mean Expression Ratio 0.965
Median Expression Ratio 0.967
Credible Interval (0.589, 1.58)
Associated Peptides 1
Associated Spectra 1
Coverage 0.0323

| A | 2.5 | 50 | 97.5 | Sequence |
|---|-----|----|------|----------|
| 1 | 0.57 | 0.95 | 1.6 | GRPLAEESEQER |

1 M A W T K Y Q L F L A G L M L V T G S I N T L S A K W A D N F M A E G C G G S K E H S F Q H P F L Q A V G M F L G E F S C L A A F Y L L R C R A A G Q S D S S V

81 D P Q Q P F N P L L F L P P A L C D M T G T S L M Y V A L N M T S A S S F Q M L R G A V I I F T G L F S V A F L G R R L V L S Q W L G I L A T I A G L V V V G L

161 A D L L S K H D S Q H K L S E V I T G D L L I I M A Q I I V A I Q M V L E E K F V Y K H N V H P L R A V G T E G L F G F V I L S L L L V P M Y Y I P A G S F S G

241 N P R G T L E D A L D A F C Q V G Q Q P L I A V A L L G N I S S I A F F N F A G I S V T K E L S A T T R M V L D S L R T V V I W A L S L A L G W E A F H A L Q I

321 L G F L I L L I G T A L Y N G L H R P L L G R L S R G R P L A E E S E Q E R L L G G T R T P I N D A S

5.297 disrupted in renal carcinoma 2 [Homo sapiens]

Protein Accession gi|14249552
Mean Expression Ratio 1.03
Median Expression Ratio 1.03
Credible Interval (0.705, 1.52)
Associated Peptides 2
Associated Spectra 3
Coverage 0.069

| A | 2.5 | 50 | 97.5 | Sequence |
|---|-----|-----|------|----------|
| 2 | 0.7 | 1.0 | 1.6 | EAAAAALPAAVPGPGR |
| 1 | 0.66 | 1.0 | 1.6 | QPLLGPGLGPGLGASWR |

1 M G S R W S S E E E R Q P L L G P G L G P G L G A S W R S R E A A A A A L P A A V P G P G R V Y G R R W L V L L L F S L L A F V Q G L V W N T W G P I Q N S A R

81 Q A Y G F S S W D I A L L V L W G P I G F L P C F A F M W L L D K R G L R I T V L L T S F L M V L G T G L R C I P I S D L I L K R R L I H G G Q M L N G L A G P

161 T V M N A A P F L S T T W F S A D E R A T A T A I A S M L S Y L G G A C A F L V G P L V V P A P N G T S P L L A A E S S R A H I K D R I E A V L Y A E F G V V C

241 L I F S A T L A Y F P P R P P L P P S V A A A S Q R L S Y R R S V C R L L S N F R F L M I A L A Y A I P L G V F A G W S G V L D L I L T P A H V S Q V D A G W I

321 G F W S I V G G C V V G I A M A R F A D F I R G M L K L I L L L L F S G A T L S S T W F T L T C L N S I T H L P L T T V T L Y A S C I L L G V F L N S S V P I F

401 F E L F V E T V Y P V P E G I T C G V V T F L S N M F M G V L L F F L T F Y H T E L S W F N W C L P G S C L L S L L L I L C F R E S Y D R L Y L D V V V S V

5.298 adenine phosphoribosyltransferase isoform b [Homo sapiens]

Protein Accession gi|71773201
Mean Expression Ratio 1.03
Median Expression Ratio 1.03
Credible Interval (0.636, 1.69)
Associated Peptides 1
Associated Spectra 1
Coverage 0.097

| A | 2.5 | 50 | 97.5 | Sequence |
|---|-----|-----|------|----------|
| 1 | 0.62 | 1.0 | 1.7 | SFPDFPTPGVVFR |

1 M A D S E L Q L V E Q R I R S F P D F P T P G V V F R D I S P V L K D P A S F R A A I G L L A R H L K A T H G G R I D Y I A G L D S R G F L F G P S L A Q E L G

81 L G C V L I R K R G K L P G P T L W A S Y S L E Y G K A E L E I Q K D A L E P G Q R V V V V D D L L A T G V

5.299 GNAS complex locus XLas [Homo sapiens]

Protein Accession gi|117938759
Mean Expression Ratio 1.03
Median Expression Ratio 1.03
Credible Interval (0.755, 1.40)
Associated Peptides 4
Associated Spectra 7
Coverage 0.0501

| A | 2.5 | 50 | 97.5 | Sequence |
|---|-----|------|------|----------|
| 3 | 0.7 | 0.98 | 1.4 | IEDYFPEFAR |
| 1 | 0.69 | 1.0 | 1.5 | SNLVPPVELANPENQFR |
| 2 | 0.8 | 1.1 | 1.6 | YTTPEDATPEPGEDPR |
| 1 | 0.7 | 1.0 | 1.5 | LQEALNLFK |

1 M G V R N C L Y G N N M S G Q R D I P P E I G E Q P E Q P P L E A P G A A A P G A G P S P A E E M E T E P P H N E P I P V E N D G E A C G P P E V S R P N F Q V

81 L N P A F R E A G A H G S Y S P P P E E A M P F E A E Q P S L G G F W P T L E Q P G F P S G V H A G L E A F G P A L M E P G A F S G A R P G L G G Y S P P P E E

161 A M P F E F D Q P A Q R G C S Q L L L Q V P D L A P G G P G A A G V P G A P P E E P Q A L R P A K A G S R G G Y S P P P E E T M P F E L D G E G F G D D S P P P

241 G L S R V I A Q V D G S S Q F A A V A A S S A V R L T P A A N A P P L W V P G A I G S P S Q E A V R P P S N F T G S S P W M E I S G P P F E I G S A P A G V D D

321 T P V N M D S P P I A L D G P P I K V S G A P D K R E R A E R P P V E E E A A E M E G A A D A A E G G K V P S P G Y G S P A A G A A S A D T A A R A A P A A P A

401 D P D S G A T P E D P D S G T A P A D P D S G A F A A D P D S G A A P A A P A D P D S G A A P D A P A D P D S G A A P D A P A D P D A G A A P E A P A A P A A A

481 E T R A A H V A P A A P D A G A P T A P A A S A T R A A Q V R R A A S A A P A S G A R R K I H L R P P S P E I Q A A D P P T P R P T R A S A W R G K S E S S R G

561 R R V Y Y D E G V A S S D D D S S G D E S D D G T S G C L R W F Q H R R N R R R R K P Q R N L L R N F L V Q A F G G C F G R S E S P Q P K A S R S L K V K K V P

641 L A E K R R Q M R K E A L E K R A Q K R A E K K R S K L I D K Q L Q D E K M G Y M C T H R L L L L G A G E S G K S T I V K Q M R I L H V N G F N G E G G E E D P

721 Q A A R S N S D G E K A T K V Q D I K N N L K E A I E T I V A A M S N L V P P V E L A N P E N Q F R V D Y I L S V M N V P D F D F P P E F Y E H A K A L W E D E

801 G V R A C Y E R S N E Y Q L I D C A Q Y F L D K I D V I K Q A D Y V P S D Q D L L R C R V L T S G I F E T K F Q V D K V N F H M F D V G G Q R D E R R K W I Q C

881 F N D V T A I I F V V A S S S Y N M V I R E D N Q T N R L Q E A L N L F K S I W N N R W L R T I S V I L F L N K Q D L L A E K V L A G K S K I E D Y F P E F A R

961 Y T T P E D A T P E P G E D P R V T R A K Y F I R D E F L R I S T A S G D G R H Y C Y P H F T C A V D T E N I R R V F N D C R D I I Q R M H L R Q Y E L L

5.300 A kinase (PRKA) anchor protein 12 isoform 2 [Homo sapiens]

Protein Accession gi|21493024
Mean Expression Ratio 1.03
Median Expression Ratio 1.03
Credible Interval (0.673, 1.58)
Associated Peptides 2
Associated Spectra 2
Coverage 0.0232

| A | 2.5 | 50 | 97.5 | Sequence |
|---|-----|-----|------|----------|
| 1 | 0.64 | 1.0 | 1.6 | EESQLPGTGGPEDVLQPVQR |
| 1 | 0.65 | 1.0 | 1.7 | EVIAEEEPPTVTEPLPENR |

1 M L G T I T I T V G Q R D S E D V S K R D S D K E M A T K S A V V H D I T D D G Q E E T P E I I E Q I P S S E S N L E E L T Q P T E S Q A N D I G F K K V F K F

81 V G F K F T V K K D K T E K P D T V Q L L T V K K D E G E G A A G A G D H K D P S L G A G E A A S K E S E P K Q S T E K P E E T L K R E Q S H A E I S P P A E S

161 G Q A V E E C K E E G E E K Q E K E P S K S A E S P T S P V T S E T G S T F K K
F F T Q G W A G W R K K T S F R K P K E D E V E A S E K K K E Q E P E K V D T E

241 E D G K A E V A S E K L T A S E Q A H P Q E P A E S A H E P R L S A E Y E K V
E L P S E E Q V S G S Q G P S E E K P A P L A T E V F D E K I E V H Q E E V V A E

321 V H V S T V E E R T E E Q K T E V E E T A G S V P A E E L V E M D A E P Q E A
E P A K E L V K L K E T C V S G E D P T Q G A D L S P D E K V L S K P P E G V V S

401 E V E M L S S Q E R M K V Q G S P L K K L F T S T G L K K L S G K K Q K G K R
G G G D E E S G E H T Q V P A D S P D S Q E E Q K G E S S A S S P E E P E E I T C

481 L E K G L A E V Q Q D G E A E E G A T S D G E K K R E G V T P W A S F K K M
V T P K K R V R R P S E S D K E D E L D K V K S A T L S S T E S T A S E M Q E E M
K

561 G S V E E P K P E E P K R K V D T S V S W E A L I C V G S S K K R A R R G S S S
D E E G G P K A M G G D H Q K A D E A G K D K E T G T D G I L A G S Q E H D P G

641 Q G S S S P E Q A G S P T E G E G V S T W E S F K R L V T P R K K S K S K L E E
K S E D S I A G S G V E H S T P D T E P G K E E S W V S I K K F I P G R R K K R

721 P D G K Q E Q A P V E D A G P T G A N E D D S D V P A V V P L S E Y D A V E R
E K M E A Q Q A Q K S A E Q P E Q K A A T E V S K E L S E S Q V H M M A A A V A
D

801 G T R A A T I I E E R S P S W I S A S V T E P L E Q V E A E A A L L T E E V L E R
E V I A E E E P P T V T E P L P E N R E A R G D T V V S E A E L T P E A V T A

881 A E T A G P L G A E E G T E A S A A E E T T E M V S A V S Q L T D S P D T T E E
A T P V Q E V E G G V P D I E E Q E R R T Q E V L Q A V A E K V K E E S Q L P G

961 T G G P E D V L Q P V Q R A E A E R P E E Q A E A S G L K K E T D V V L K V D
A Q E A K T E P F T Q G K V V G Q T T P E S F E K A P Q V T E S I E S S E L V T T

1041 C Q A E T L A G V K S Q E M V M E Q A I P P D S V E T P T D S E T D G S T P V
A D F D A P G T T Q K D E I V E I H E E N E V A S G T Q S G G T E A E A V P A Q K

1121 E R P P A P S S F V F Q E E T K E Q S K M E D T L E H T D K E V S V E T V S I L
S K T E G T Q E A D Q Y A D E K T K D V P F F E G L E G S I D T G I T V S R E K

1201 V T E V A L K G E G T E E A E C K K D D A L E L Q S H A K S P P S P V E R E M
V V Q V E R E K T E A E P T H V N E E K L E H E T A V T V S E E V S K Q L L Q T V

1281 N V P I I D G A K E V S S L E G S P P P C L G Q E E A V C T K I Q V Q S S E A S F T L T A A A E E E K V L G E T A N I L E T G E T L E P A G A H L V L E E K S S

1361 E K N E D F A A H P G E D A V P T G P D C Q A K S T P V I V S A T T K K G L S S D L E G E K T T S L K W K S D E V D E Q V A C Q E V K V S V A I E D L E P E N G

1441 I L E L E T K S S K L V Q N I I Q T A V D Q F V R T E E T A T E M L T S E L Q T Q A H V I K A D S Q D A G Q E T E K E G E E P Q A S A Q D E T P I T S A K E E S

1521 E S T A V G Q A H S D I S K D M S E A S E K T M T V E V E G S T V N D Q Q L E E V V L P S E E E G G G A G T K S V P E D D G H A L L A E R I E K S L V E P K E D

1601 E K G D D V D D P E N Q N S A L A D T D A S G G L T K E S P D T N G P K Q K E K E D A Q E V E L Q E G K V H S E S D K A I T P Q A Q E E L Q K Q E R E S A K S E

1681 LTES

5.301 orosomucoid 1 precursor [Homo sapiens]

Protein Accession gi|167857790
Mean Expression Ratio 0.97
Median Expression Ratio 0.97
Credible Interval (0.666, 1.44)
Associated Peptides 3
Associated Spectra 3
Coverage NaN

| A | 2.5 | 50 | 97.5 | Sequence |
|---|-----|------|------|------------------|
| 1 | 0.63 | 0.97 | 1.5 | WFYIASAFR |
| 1 | 0.64 | 0.99 | 1.5 | EQLGEFYEALDCLR |
| 1 | 0.61 | 0.95 | 1.5 | YVGGQEHFAHLLILR |

5.302 lysozyme precursor [Homo sapiens]

Protein Accession gi|4557894
Mean Expression Ratio 0.969
Median Expression Ratio 0.97
Credible Interval (0.6, 1.57)
Associated Peptides 1
Associated Spectra 1
Coverage 0.0811

| A | 2.5 | 50 | 97.5 | Sequence |
|---|---|---|---|---|
| 1 | 0.58 | 0.96 | 1.6 | STDYGIFQINSR |

1 M K A L I V L G L V L L S V T V Q G K V F E R C E L A R T L K R L G M D G Y R G I S L A N W M C L A K W E S G Y N T R A T N Y N A G D R S T D Y G I F Q I N S R

81 Y W C N D G K T P G A V N A C H L S C S A L L Q D N I A D A V A C A K R V V R D P Q G I R A W V A W R N R C Q N R D V R Q Y V Q G C G V

5.303 vacuolar protein sorting 37C [Homo sapiens]

Protein Accession gi|57863314
Mean Expression Ratio 0.972
Median Expression Ratio 0.97
Credible Interval (0.658, 1.44)
Associated Peptides 3
Associated Spectra 3
Coverage 0.177

| A | 2.5 | 50 | 97.5 | Sequence |
|---|---|---|---|---|
| 1 | 0.62 | 0.97 | 1.5 | FLEGEVPLETFLENFSSMR |
| 1 | 0.64 | 1 | 1.6 | NLEFQGPLEISR |
| 1 | 0.6 | 0.93 | 1.4 | TLQELEELQNDSEAIDQLALESPEVQDLQLER |

1 M E T L K D K T L Q E L E E L Q N D S E A I D Q L A L E S P E V Q D L Q L E R E M A L A T N R S L A E R N L E F Q G P L E I S R S N L S D R Y Q E L R K L V E R

81 C Q E Q K A K L E K F S S A L Q P G T L L D L L Q V E G M K I E E E S E A M A E K F L E G E V P L E T F L E N F S S M R M L S H L R R V R V E K L Q E V V R K P

161 R A S Q E L A G D A P P P R P P P P V R P V P Q G T P P V V E E Q P Q P P L A M P P Y P L P Y S P S P S L P V G P T A H G A L P P A P F P V V S Q P S F Y S G P

241 L G P T Y P A A Q L G P R G A A G Y S W S P Q R S M P P R P G Y P G T P M G A S G P G Y P L R G G R A P S P G Y P Q Q S P Y P A T G G K P P Y P I Q P Q L P S F

321 PGQPQPSVPLQPPYPPGPAPPYGFPPPPGPAWPGY

5.304 NAD(P)H dehydrogenase, quinone 2 [Homo sapiens]

Protein Accession gi|156564357
Mean Expression Ratio 0.972
Median Expression Ratio 0.97

Credible Interval (0.596, 1.58)
Associated Peptides 1
Associated Spectra 1
Coverage 0.0779

| A | 2.5 | 50 | 97.5 | Sequence |
|---|-----|----|----|----------|
| 1 | 0.58 | 0.97 | 1.6 | VLAPQISFAPEIASEEER |

1 M A G K K V L I V Y A H Q E P K S F N G S L K N V A V D E L S R Q G C T V T V S
D L Y A M N L E P R A T D K D I T G T L S N P E V F N Y G V E T H E A Y K Q R S

81 L A S D I T D E Q K K V R E A D L V I F Q F P L Y W F S V P A I L K G W M D R V
L C Q G F A F D I P G F Y D S G L L Q G K L A L L S V T T G G T A E M Y T K T G

161 V N G D S R Y F L W P L Q H G T L H F C G F K V L A P Q I S F A P E I A S E E E
R K G M V A A W S Q R L Q T I W K E E P I P C T A H W H F G Q

5.305 cystatin B [Homo sapiens]

Protein Accession gi|4503117
Mean Expression Ratio 0.97
Median Expression Ratio 0.97
Credible Interval (0.603, 1.59)
Associated Peptides 1
Associated Spectra 1
Coverage 0.122

| A | 2.5 | 50 | 97.5 | Sequence |
|---|-----|----|----|----------|
| 1 | 0.58 | 0.96 | 1.6 | VHVGDEDFVHLR |

1 M M C G A P S A T Q P A T A E T Q H I A D Q V R S Q L E E K E N K K F P V F K A
V S F K S Q V V A G T N Y F I K V H V G D E D F V H L R V F Q S L P H E N K P L

81 TLSNYQTNKAKHDELTYF

5.306 triosephosphate isomerase 1 [Homo sapiens]

Protein Accession gi|4507645
Mean Expression Ratio 1.03
Median Expression Ratio 1.03
Credible Interval (0.635, 1.68)
Associated Peptides 1
Associated Spectra 1
Coverage 0.0522

| A | 2.5 | 50 | 97.5 | Sequence |
|---|---|---|---|---|
| 1 | 0.62 | 1.0 | 1.7 | TATPQQAQEVHEK |

1 M A P S R K F F V G G N W K M N G R K Q S L G E L I G T L N A A K V P A D T E V V C A P P T A Y I D F A R Q K L D P K I A V A A Q N C Y K V T N G A F T G E I S

81 P G M I K D C G A T W V V L G H S E R R H V F G E S D E L I G Q K V A H A L A E G L G V I A C I G E K L D E R E A G I T E K V V F E Q T K V I A D N V K D W S K

161 V V L A Y E P V W A I G T G K T A T P Q Q A Q E V H E K L R G W L K S N V S D A V A Q S T R I I Y G G S V T G A T C K E L A S Q P D V D G F L V G G A S L K P E

241 FVDIINAKQ

5.307 nucleoside phosphorylase [Homo sapiens]

Protein Accession gi|157168362

Mean Expression Ratio 1.03

Median Expression Ratio 1.03

Credible Interval (0.725, 1.45)

Associated Peptides 4

Associated Spectra 5

Coverage 0.208

| A | 2.5 | 50 | 97.5 | Sequence |
|---|---|---|---|---|
| 1 | 0.68 | 1.0 | 1.6 | LGADAVGMSTVPEVIVAR |
| 1 | 0.68 | 1.0 | 1.5 | LTQAQIFDYGEIPNFPR |
| 2 | 0.71 | 1.0 | 1.5 | DHINLPGF SGQNPLR |
| 1 | 0.67 | 1.0 | 1.5 | FEVGDIMLIR |

1 M E N G Y T Y E D Y K N T A E W L L S H T K H R P Q V A I I C G S G L G G L T D K L T Q A Q I F D Y G E I P N F P R S T V P G H A G R L V F G F L N G R A C V M

81 M Q G R F H M Y E G Y P L W K V T F P V R V F H L L G V D T L V V T N A A G G L N P K F E V G D I M L I R D H I N L P G F S G Q N P L R G P N D E R F G D R F P

161 A M S D A Y D R T M R Q R A L S T W K Q M G E Q R E L Q E G T Y V M V A G P S F E T V A E C R V L Q K L G A D A V G M S T V P E V I V A R H C G L R V F G F S L

241 I T N K V I M D Y E S L E K A N H E E V L A A G K Q A A Q K L E Q F V S I L M A S I P L P D K A S

5.308 guanine nucleotide binding protein (G protein), alpha 13 [Homo sapiens]

Protein Accession gi|24111250

Mean Expression Ratio 0.97

Median Expression Ratio 0.972

Credible Interval (0.686, 1.38)

Associated Peptides 4

Associated Spectra 4

Coverage 0.138

| A | 2.5 | 50 | 97.5 | Sequence |
|---|-----|------|------|----------|
| 1 | 0.61 | 0.93 | 1.4 | IIHGQDFDQR |
| 1 | 0.63 | 0.96 | 1.5 | LGEPDYIPSQQDILLAR |
| 1 | 0.65 | 0.98 | 1.5 | LTESLNIFETIVNNR |
| 1 | 0.66 | 1 | 1.5 | VFLQYLPAIR |

1 M A D F L P S R S V L S V C F P G C L L T S G E A E Q Q R K S K E I D K C L S R E K T Y V K R L V K I L L L G A G E S G K S T F L K Q M R I I H G Q D F D Q R A

81 R E E F R P T I Y S N V I K G M R V L V D A R E K L H I P W G D N S N Q Q H G D K M M S F D T R A P M A A Q G M V E T R V F L Q Y L P A I R A L W A D S G I Q N

161 A Y D R R R E F Q L G E S V K Y F L D N L D K L G E P D Y I P S Q Q D I L L A R R P T K G I H E Y D F E I K N V P F K M V D V G G Q R S E R K R W F E C F D S V

241 T S I L F L V S S S E F D Q V L M E D R L T N R L T E S L N I F E T I V N N R V F S N V S I I L F L N K T D L L E E K V Q I V S I K D Y F L E F E G D P H C L R

321 D V Q K F L V E C F R N K R R D Q Q Q K P L Y H H F T T A I N T E N I R L V F R D V K D T I L H D N L K Q L M L Q

5.309 phospholipid scramblase 3 [Homo sapiens]

Protein Accession gi|31543417
Mean Expression Ratio 1.03
Median Expression Ratio 1.03
Credible Interval (0.687, 1.54)
Associated Peptides 1
Associated Spectra 3
Coverage 0.0678

| A | 2.5 | 50 | 97.5 | Sequence |
|---|-----|-----|------|----------|
| 3 | 0.7 | 1.0 | 1.5 | EALTDADDFGLQFPLDLDVR |

1 MAGYLPPKGYAPSPPPPYPVTPGYPEPALHPGPGQAPVPAQ VPAPAPGFALFPSPGPVALGSAAPFLPLPGVPSGLEFLV

81 QIDQILIHQKAERVETFLGWETCNRYELRSGAGQPLGQAA EESNCCARLCCGARRPLRVRLADPGDREVLRLLRPLHCGC

161 SCCPCGLQEMEVQAPPGTTIGHVLQTWHPFLPKFSIQDAD RQTVLRVVGPCWTCGCGTDTNFEVKTRDESRSVGRISKQW

241 GGLVREALTDADDFGLQFPLDLDVRVKAVLLGATFLIDY MFFEKRGGAGPSAVTS

5.310 plasma glutamate carboxypeptidase [Homo sapiens]

Protein Accession gi|7706387
Mean Expression Ratio 0.974
Median Expression Ratio 0.972
Credible Interval (0.602, 1.57)
Associated Peptides 1
Associated Spectra 1
Coverage 0.0233

| A | 2.5 | 50 | 97.5 | Sequence |
|---|------|------|------|------------|
| 1 | 0.59 | 0.96 | 1.6 | LALLVDTVGPR |

1 MKFLIFAFFGGVHLLSLCSGKAICKNGISKRTFEEIKEEIAS CGDVAKAIINLAVYGKAQNRSYERLALLVDTVGPRLSG

81 SKNLEKAIQIMYQNLQQDGLEKVHLEPVRIPHWERGEESA VMLEPRIHKIAILGLGSSIGTPPEGITAEVLVVTSFDELQ

161 R R A S E A R G K I V V Y N Q P Y I N Y S R T V Q Y R T Q G A V E A A K V G A L A S L I R S V A S F S I Y S P H T G I Q E Y Q D G V P K I P T A C I T V E D A E

241 M M S R M A S H G I K I V I Q L K M G A K T Y P D T D S F N T V A E I T G S K Y P E Q V V L V S G H L D S W D V G Q G A M D D G G G A F I S W E A L S L I K D L

321 G L R P K R T L R L V L W T A E E Q G G V G A F Q Y Y Q L H K V N I S N Y S L V M E S D A G T F L P T G L Q F T G S E K A R A I M E E V M S L L Q P L N I T Q V

401 L S H G E G T D I N F W I Q A G V P G A S L L D D L Y K Y F F F H H S H G D T M T V M D P K Q M N V A A A V W A V V S Y V V A D M E E M L P R S

5.311 bone marrow stromal cell antigen 1 precursor [Homo sapiens]

Protein Accession gi|168229159
Mean Expression Ratio 1.02
Median Expression Ratio 1.03
Credible Interval (0.626, 1.66)
Associated Peptides 1
Associated Spectra 1
Coverage NaN

| A | 2.5 | 50 | 97.5 | Sequence |
|---|-----|-----|------|----------|
| 1 | 0.61 | 1.0 | 1.7 | SLFWENSHLLVNSFADNTR |

5.312 solute carrier organic anion transporter family, member 4C1 [Homo sapiens]

Protein Accession gi|38679890
Mean Expression Ratio 0.974
Median Expression Ratio 0.973
Credible Interval (0.595, 1.57)
Associated Peptides 1
Associated Spectra 1
Coverage 0.0221

| A | 2.5 | 50 | 97.5 | Sequence |
|---|-----|-----|------|----------|
| 1 | 0.58 | 0.97 | 1.6 | GIENLAFVPSSPDILR |

1 M K S A K G I E N L A F V P S S P D I L R R L S A S P S Q I E V S A L S S D P Q R E N S Q P Q E L Q K P Q E P Q K S P E P S L P S A P P N V S E E K L R S L S L

81 S E F E E G S Y G W R N F H P Q C L Q R C N T P G G F L L H Y C L L A V T Q G I
V V N G L V N I S I S T V E K R Y E M K S S L T G L I S S S Y D I S F C L L S L

161 F V S F F G E R G H K P R W L A F A A F M I G L G A L V F S L P Q F F S G E Y K
L G S L F E D T C V T T R N S T S C T S S T S S L S N Y L Y V F I L G Q L L L G

241 A G G T P L Y T L G T A F L D D S V P T H K S S L Y I G T G Y A M S I L G P A I
G Y V L G G Q L L T I Y I D V A M G E S T D V T E D D P R W L G A W W I G F L L

321 S W I F A W S L I I P F S C F P K H L P G T A E I Q A G K T S Q A H Q S N S N A
D V K F G K S I K D F P A A L K N L M K N A V F M C L V L S T S S E A L I T T G

401 F A T F L P K F I E N Q F G L T S S F A A T L G G A V L I P G A A L G Q I L G G F
L V S K F R M T C K N T M K F A L F T S G V A L T L S F V F M Y A K C E N E P

481 F A G V S E S Y N G T G E L G N L I A P C N A N C N C S R S Y Y Y P V C G D G
V Q Y F S P C F A G C S N P V A H R K P K V Y Y N C S C I E R K T E I T S T A E T

561 F G F E A K A G K C E T H C A K L P I F L C I F F I V I I F T F M A G T P I T V S I
L R C V N H R Q R S L A L G I Q F M V L R L L G T I P G P I I F G F T I D S

641 T C I L W D I N D C G I K G A C W I Y D N I K M A H M L V A I S V T C K V I T
M F F N G F A I F L Y K P P P S A T D V S F H K E N A V V T N V L A E Q D L N K I

721 VKEG

5.313 glucose transporter 14 [Homo sapiens]

Protein Accession gi|23592238
Mean Expression Ratio 1.03
Median Expression Ratio 1.03
Credible Interval (0.626, 1.68)
Associated Peptides 1
Associated Spectra 1
Coverage 0.0173

| A | 2.5 | 50 | 97.5 | Sequence |
|---|-----|-----|------|----------|
| 1 | 0.62 | 1.0 | 1.7 | QVTVLELFR |

1 M E F H N G G H V S G I G G F L V S L T S R M K P H T L A V T P A L I F A I T V A
T I G S F Q F G Y N T G V I N A P E T I I K E F I N K T L T D K A N A P P S E

81 V L L T N L W S L S V A I F S V G G M I G S F S V G L F V N R F G R R N S M L I
V N L L A A T G G C L M G L C K I A E S V E M L I L G R L V I G L F C G L C T G

161 F V P M Y I G E I S P T A L R G A F G T L N Q L G I V I G I L V A Q I F G L E L I L
G S E E L W P V L L G F T I L P A I L Q S A A L P C C P E S P R F L L I N R

241 K K E E N A T R I L Q R L W G T Q D V S Q D I Q E M K D E S A R M S Q E K Q V
T V L E L F R V S S Y R Q P I I I S I V L Q L S Q Q L S G I N A V F Y Y S T G I F

321 K D A G V Q Q P I Y A T I S A G V V N T I F T L L S L F L V E R A G R R T L H M
I G L G G M A F C S T L M T V S L L L K N H Y N G M S F V C I G A I L V F V A C

401 F E I G P G P I P W F I V A E L F S Q G P R P A A M A V A G C S N W T S N F L V
G L L F P S A A Y Y L G A Y V F I I F T G F L I T F L A F T F F K V P E T R G R

481 T F E D I T R A F E G Q A H G A D R S G K D G V M G M N S I E P A K E T T T N
V

5.314 PREDICTED: hypothetical protein [Homo sapiens]

Protein Accession gi|169218200
Mean Expression Ratio 1.03
Median Expression Ratio 1.03
Credible Interval (0.79, 1.35)
Associated Peptides 4
Associated Spectra 13
Coverage NaN

| A | 2.5 | 50 | 97.5 | Sequence |
|---|-----|-----|------|----------|
| 2 | 0.78 | 1.1 | 1.5 | ESNGQPENNYK |
| 9 | 0.83 | 1.1 | 1.3 | GFYPSDIAVEWESNGQPENNYK |
| 1 | 0.7 | 1 | 1.4 | NQVSLTCLVK |
| 1 | 0.68 | 0.98 | 1.4 | TTPPMLDSDGSFFLYSK |

5.315 serine hydroxymethyltransferase 1 (soluble) isoform 2 [Homo sapiens]; serine hydroxymethyltransferase 1 (soluble) isoform 1 [Homo sapiens]

Protein Accession gi|22547189 gi|22547186
Mean Expression Ratio 0.972
Median Expression Ratio 0.974
Credible Interval (0.599, 1.56)
Associated Peptides 1
Associated Spectra 1
Coverage NaN

| A | 2.5 | 50 | 97.5 | Sequence |
|---|---|---|---|---|
| 1 | 0.58 | 0.97 | 1.6 | VNPDTGYINYDQLEENAR |

5.316 calcium binding protein P22 [Homo sapiens]

Protein Accession gi|6005731
Mean Expression Ratio 1.02
Median Expression Ratio 1.03
Credible Interval (0.625, 1.65)
Associated Peptides 1
Associated Spectra 1
Coverage 0.082

| A | 2.5 | 50 | 97.5 | Sequence |
|---|---|---|---|---|
| 1 | 0.61 | 1.0 | 1.7 | IINAFFPEGEDQVNFR |

1 MGSRASTLLRDEELEEIKKETGFSHSQITRLYSRFTSLDKGE
NGTLSREDFQRIPELAINPLGDRIINAFFPEGEDQVNF

81 RGFMRTLAHFRPIEDNEKSKDVNGPEPLNSRSNKLHFAFR
LYDLDKDEKISRDELLQVLRMMVGVNISDEQLGSIADRTI

161 QEADQDGDSAISFTEFVKVLEKVDVEQKMSIRFLH

5.317 sushi domain containing 2 [Homo sapiens]

Protein Accession gi|10092665
Mean Expression Ratio 0.975
Median Expression Ratio 0.975
Credible Interval (0.594, 1.57)
Associated Peptides 1
Associated Spectra 1
Coverage 0.0170

| A | 2.5 | 50 | 97.5 | Sequence |
|---|---|---|---|---|
| 1 | 0.57 | 0.97 | 1.6 | GEYVLLEAALTDLR |

1 MKPALLPWALLLLATALGPGPGPTADAQESCSMRCGALDG
PCSCHPTCSGLGTCCLDFRDFCLEILPYSGSMMGGKDFVV

81 RHFKMSSPTDASVICRFKDSIQTLGHVDSSGQVHCVSPLL
YESGRIPFTVSLDNGHSFPRAGTWLAVHPNKVSMMEKSEL

161 V N E T R W Q Y Y G T A N T S G N L S L T W H V K S L P T Q T I T I E L W G Y E E T G M P Y S Q E W T A K W S Y L Y P L A T H I P N S G S F T F T P K P A P P S

241 Y Q R W R V G A L R I I D S K N Y A G Q K D V Q A L W T N D H A L A W H L S D D F R E D P V A W A R T Q C Q A W E E L E D Q L P N F L E E L P D C P C T L T Q A

321 R A D S G R F F T D Y G C D M E Q G S V C T Y H P G A V H C V R S V Q A S L R Y G S G Q Q C C Y T A D G T Q L L T A D S S G G S T P D R G H D W G A P P F R T P

401 P R V P S M S H W L Y D V L S F Y Y C C L W A P D C P R Y M Q R R P S N D C R N Y R P P R L A S A F G D P H F V T F D G T N F T F N G R G E Y V L L E A A L T D

481 L R V Q A R A Q P G T M S N G T E T R G T G L T A V A V Q E G N S D V V E V R L A N R T G G L E V L L N Q E V L S F T E Q S W M D L K G M F L S V A A G D R V S

561 I M L A S G A G L E V S V Q G P F L S V S V L L P E K F L T H T H G L L G T L N N D P T D D F T L H S G R V L P P G T S P Q E L F L F G A N W T V H N A S S L L

641 T Y D S W F L V H N F L Y Q P K H D P T F E P L F P S E T T L N P S L A Q E A A K L C G D D H F C N F D V A A T G S L S T G T A T R V A H Q L H Q R R M Q S L Q

721 P V V S C G W L A P P P N G Q K E G N R Y L A G S T I Y F H C D N G Y S L A G A E T S T C Q A D G T W S S P T P K C Q P G R S Y A V L L G I I F G G L A V V A A

801 VALVYVLLRRRKGNTHVWGAQP

5.318 occludin [Homo sapiens]

Protein Accession gi|4505487
Mean Expression Ratio 1.03
Median Expression Ratio 1.03
Credible Interval (0.708, 1.48)
Associated Peptides 2
Associated Spectra 4
Coverage 0.0766

| A | 2.5 | 50 | 97.5 | Sequence |
|---|-----|-----|------|----------|
| 2 | 0.71 | 1.1 | 1.6 | NVSAGTQDVPSPPSDYVER |
| 2 | 0.67 | 11. | 5 | STPVPEVVQELPLTSPVDDFR |

MSSRPLESPPPYRPDEFKPNHYAPSNDIYGGEMHVRPMLSQ PAYSFYPEDEILHFYKWTSPPGVIRILSMLIIVMCIAIF

81 ACVASTLAWDRGYGTSLLGGSVGYPYGGSGFGSYGSGYG YGYGYGYGYGGYTDPRAAKGFMLAMAAFCFIAALVIFVTSV

161 IRSEMSRTRRYYLSVIIVSAILGIMVFIATIVYIMGVNPTA QSSGSLYGSQIYALCNQFYTPAATGLYVDQYLYHYCVVD

241 PQEAIAIVLGFMIIVAFALIIFFAVKTRRKMDRYDKSNILW DKEHIYDEQPPNVEEWVKNVSAGTQDVPSPPSDYVERVD

321 SPMAYSSNGKVNDKRFYPESSYKSTPVPEVVQELPLTSPV DDFRQPRYSSGGNFETPSKRAPAKGRAGRSKRTEQDHYET

401 DYTTGGESCDELEEDWIREYPPITSDQQRQLYKRNFDTGL QEYKSLQSELDEINKELSRLDKELDDYREESEEYMAAADE

481 YNRLKQVKGSADYKSKKNHCKQLKSKLSHIKKMVGDYD RQKT

5.319 CD81 antigen [Homo sapiens]

Protein Accession gi|4757944
Mean Expression Ratio 1.03
Median Expression Ratio 1.03
Credible Interval (0.623, 1.68)
Associated Peptides 1
Associated Spectra 1
Coverage 0.0847

| A | 2.5 | 50 | 97.5 | Sequence |
|---|-----|-----|------|----------|
| 1 | 0.62 | 1.0 | 1.7 | QFYDQALQQAVVDDDANNAK |

MGVEGCTKCIKYLLFVFNFVFWLAGGVILGVALWLRHDPQ TTNLLYLELGDKPAPNTFYVGIYILIAVGAVMMFVGFLGC

81 YGAIQESQCLLGTFFTCLVILFACEVAAGIWGFVNKDQIA KDVKQFYDQALQQAVVDDDANNAKAVVKTFHETLDCCGSS

161 TLTALTTSVLKNNLCPSGSNIISNLFKEDCHQKIDDLFSGK LYLIGIAAIVVAVIMIFEMILSMVLCCGIRNSSVY

5.320 ectonucleotide pyrophosphatase/phosphodiesterase 6 [Homo sapiens]

Protein Accession gi|23503267
Mean Expression Ratio 0.977
Median Expression Ratio 0.976
Credible Interval (0.745, 1.27)
Associated Peptides 8
Associated Spectra 9
Coverage 0.225

| A | 2.5 | 50 | 97.5 | Sequence |
|---|-----|-----|------|----------|
| 1 | 0.72 | 1.0 | 1.5 | FVSPLTLVADEGWFITENR |
| 1 | 0.69 | 0.99 | 1.4 | GIFLAFGPDFK |
| 1 | 0.69 | 0.98 | 1.4 | IDVEGHHYGPASPQR |
| 2 | 0.7 | 0.99 | 1.4 | LLVFLLDGFR |
| 1 | 0.68 | 0.97 | 1.4 | NVPTDINFANAVSDALDSFK |
| 1 | 0.66 | 0.97 | 1.4 | YISLNDLQQVK |
| 1 | 0.68 | 0.96 | 1.4 | GWHGYDNELMDMR |
| 1 | 0.64 | 0.92 | 1.3 | AFGPDFK |

1 MAVKLGTLLLALALGLAQPASARRKLLVFLLDGFRSDYIS
DEALESLPGFKEIVSRGVKVDYLTPDFPSLSYPNYYTLMT

81 GRHCEVHQMIGNYMWDPTTNKSFDIGVNKDSLMPLWWN
GSEPLWVTLTKAKRKVYMYYWPGCEVEILGVRPTYCLEYK
NV

161 PTDINFANAVSDALDSFKSGRADLAAIYHERIDVEGHHYG
PASPQRKDALKAVDTVLKYMTKWIQERGLQDRLNVIIFSD

241 HGMTDIFWMDKVIELNKYISLNDLQQVKDRGPVVSLWPA
PGKHSEIYNKLSTVEHMTVYEKEAIPSRFYYKKGKFVSPLT

321 LVADEGWFITENREMLPFWMNSTGRREGWQRGWHGYDN
ELMDMRGIFLAFGPDFKSNFRAAPIRSVDVYNVMCNVVGIT
P

401 LPNNGSWSRVMCMLKGRASTAPPVWPSHCALALILLFLL
A

5.321 solute carrier family 5 (sodium/glucose cotransporter), member 2 [Homo sapiens]

Protein Accession gi|4507033
Mean Expression Ratio 1.02
Median Expression Ratio 1.02
Credible Interval (0.661, 1.58)
Associated Peptides 1
Associated Spectra 2
Coverage 0.0283

| A | 2.5 | 50 | 97.5 | Sequence |
|---|-----|-----|------|----------|
| 2 | 0.66 | 1.0 | 1.6 | GGVGSPPPLTQEEAAAAAR |

1 MEEHTEAGSAPEMGAQKALIDNPADILVIAAYFLLVIGVGL WSMCRTNRGTVGGYFLAGRSMVWWPVGASLFASNIGSGH

81 FVGLAGTGAASGLAVAGFEWNALFVVLLLGWLFAPVYLT AGVITMPQYLRKRFGGRRIRLYLSVLSLFLYIFTKISVDMF

161 SGAVFIQQALGWNIYASVIALLGITMIYTVTGGLAALMYT DTVQTFVILGGACILMGYAFHEVGGYSGLFDKYLGAATSL

241 TVSEDPAVGNISSFCYRPRPDSYHLLRHPVTGDLPWPALL LGLTIVSGWYWCSDQVIVQRCLAGKSLTHIKAGCILCGYL

321 KLTPMFLMVMPGMISRILYPDEVACVVPEVCRRVCGTEV GCSNIAYPRLVVKLMPNGLRGLMLAVMLAALMSSLASIFNS

401 SSTLFTMDIYTRLRPRAGDRELLLVGRLWVVFIVVVSVA WLPVVQAAQGGQLFDYIQAVSSYLAPPVSAVFVLALFVPRV

481 NEQGAFWGLIGGLLMGLARLIPEFSFGSGSCVQPSACPAF LCGVHYLYFAIVLFFCSGLLTLTVSLCTAPIPRKHLHRLV

561 FSLRHSKEEREDLDADEQQGSSLPVQNGCPESAMEMNEP QAPAPSLFRQCLLWFCGMSRGGVGSPPPLTQEEAAAAARRL

641 EDISEDPSWARVVNLNALLMMAVAVFLWGFYA

5.322 MIT, microtubule interacting and transport, domain containing 1 [Homo sapiens]

Protein Accession gi|20270349
Mean Expression Ratio 1.03
Median Expression Ratio 1.02
Credible Interval (0.627, 1.65)
Associated Peptides 1
Associated Spectra 1

Coverage 0.0723

| A | 2.5 | 50 | 97.5 | Sequence |
|---|-----|-----|------|----------|
| 1 | 0.62 | 1.0 | 1.7 | EYLNETVTEVWIEDPYIR |

1 M A K S G L R Q D P Q S T A A A T V L K R A V E L D S E S R Y P Q A L V C Y Q E G I D L L L Q V L K G T K D N T K R C N L R E K I S K Y M D R A E N I K K Y L D

81 Q E K E D G K Y H K Q I K I E E N A T G F S Y E S L F R E Y L N E T V T E V W I E D P Y I R H T H Q L Y N F L R F C E M L I K R P C K V K T I H L L T S L D E G

161 I E Q V Q Q S R G L Q E I E E S L R S H G V L L E V Q Y S S S I H D R E I R F N N G W M I K I G R G L D Y F K K P Q S R F S L G Y C D F D L R P C H E T T V D I

241 FHKKHTKNI

5.323 ring finger protein 167 [Homo sapiens]

Protein Accession gi|14149702
Mean Expression Ratio 0.976
Median Expression Ratio 0.98
Credible Interval (0.67, 1.40)
Associated Peptides 2
Associated Spectra 4
Coverage 0.0657

| A | 2.5 | 50 | 97.5 | Sequence |
|---|-----|-----|------|----------|
| 1 | 0.63 | 0.97 | 1.5 | GDEDQEEETQGQEEGDEGEPR |
| 3 | 0.67 | 0.97 | 1.4 | GPGDEDQEEETQGQEEGDEGEPR |

1 M H P A A F P L P V V V A A V L W G A A P T R G L I R A T S D H N A S M D F A D L P A L F G A T L S Q E G L Q G F L V E A H P D N A C S P I A P P P P A P V N G

81 S V F I A L L R R F D C N F D L K V L N A Q K A G Y G A A V V H N V N S N E L L N M V W N S E E I Q Q Q I W I P S V F I G E R S S E Y L R A L F V Y E K G A R V

161 L L V P D N T F P L G Y Y L I P F T G I V G L L V L A M G A V M I A R C I Q H R K R L Q R N R L T K E Q L K Q I P T H D Y Q K G D Q Y D V C A I C L D E Y E D G

241 D K L R V L P C A H A Y H S R C V D P W L T Q T R K T C P I C K Q P V H R G P G D E D Q E E E T Q G Q E E G D E G E P R D H P A S E R T P L L G S S P T L P T S

321 FGSLAPAPLVFPGPSTDPPLSPPSSPVILV

5.324 ubiquitin protein ligase E3C [Homo sapiens]

Protein Accession gi|187960100
Mean Expression Ratio 0.98
Median Expression Ratio 0.98
Credible Interval (0.6, 1.59)
Associated Peptides 1
Associated Spectra 1
Coverage NaN

| A | 2.5 | 50 | 97.5 | Sequence |
|---|-----|-----|------|----------|
| 1 | 0.58 | 0.98 | 1.6 | LNRQIRQHCLAFR |

5.325 syntaxin 8 [Homo sapiens]

Protein Accession gi|4759188
Mean Expression Ratio 0.98
Median Expression Ratio 0.98
Credible Interval (0.609, 1.57)
Associated Peptides 1
Associated Spectra 1
Coverage 0.0763

| A | 2.5 | 50 | 97.5 | Sequence |
|---|-----|-----|------|----------|
| 1 | 0.59 | 0.98 | 1.6 | IIQEQDAGLDALSSIISR |

1 M A P D P W F S T Y D S T C Q I A Q E I A E K I Q Q R N Q Y E R K G E K A P K L T
V T I R A L L Q N L K E K I A L L K D L L L R A V S T H Q I T Q L E G D R R Q

81 N L L D D L V T R E R L L L A S F K N E G A E P D L I R S S L M S E E A K R G A
P N P W L F E E P E E T R G L G F D E I R Q Q Q Q K I I Q E Q D A G L D A L S S

161 I I S R Q K Q M G Q E I G N E L D E Q N E I I D D L A N L V E N T D E K L R N E
T R R V N M V D R K S A S C G M I M V I L L L L V A I V V V A V W P T N

5.326 sorting nexin 3 [Homo sapiens]

Protein Accession gi|4507143
Mean Expression Ratio 0.981
Median Expression Ratio 0.98
Credible Interval (0.603, 1.60)
Associated Peptides 1
Associated Spectra 1
Coverage 0.0494

| A | 2.5 | 50 | 97.5 | Sequence |
|---|-----|-----|------|----------|
| 1 | 0.59 | 0.98 | 1.6 | YSDFEWLR |

1 M A E T V A D T R R L I T K P Q N L N D A Y G P P S N F L E I D V S N P Q T V G V G R G R F T T Y E I R V K T N L P I F K L K E S T V R R R Y S D F E W L R S E

81 L E R E S K V V V P P L P G K A F L R Q L P F R G D D G I F D D N F I E E R K Q G L E Q F I N K V A G H P L A Q N E R C L H M F L Q D E I I D K S Y T P S K I R

161 HA

5.327 RAB7, member RAS oncogene family [Homo sapiens]

Protein Accession gi|34147513
Mean Expression Ratio 1.02
Median Expression Ratio 1.02
Credible Interval (0.798, 1.3)
Associated Peptides 9
Associated Spectra 12
Coverage 0.483

| A | 2.5 | 50 | 97.5 | Sequence |
|---|-----|-----|------|----------|
| 2 | 0.74 | 1 | 1.4 | DEFLIQASPR |
| 1 | 0.72 | 1.0 | 1.5 | DPENFPFVVLGNK |
| 2 | 0.77 | 1.1 | 1.5 | EAINVEQAFQTIAR |
| 1 | 0.71 | 1.0 | 1.4 | FQSLGVAFYR |
| 1 | 0.72 | 1.0 | 1.4 | LVTMQIWDTAGQER |
| 2 | 0.72 | 1 | 1.4 | QETEVELYNEFPEPIK |
| 1 | 0.71 | 1 | 1.4 | TLDSWRDEFLIQASPR |
| 1 | 0.71 | 1 | 1.4 | FSNQYK |
| 1 | 0.71 | 1.0 | 1.4 | NNIPYFETSAK |

1 M T S R K K V L L K V I I L G D S G V G K T S L M N Q Y V N K K F S N Q Y K A T I G A D F L T K E V M V D D R L V T M Q I W D T A G Q E R F Q S L G V A F Y R G

81 A D C C V L V F D V T A P N T F K T L D S W R D E F L I Q A S P R D P E N F P F V V L G N K I D L E N R Q V A T K R A Q A W C Y S K N N I P Y F E T S A K E A I

161 N V E Q A F Q T I A R N A L K Q E T E V E L Y N E F P E P I K L D K N D R A K A S A E S C S C

5.328 prenylcysteine oxidase 1 [Homo sapiens]

Protein Accession gi|166795301
Mean Expression Ratio 1.02
Median Expression Ratio 1.02
Credible Interval (0.714, 1.44)
Associated Peptides 4
Associated Spectra 4
Coverage NaN

| A | 2.5 | 50 | 97.5 | Sequence |
|---|-----|-----|------|----------|
| 1 | 0.65 | 1 | 1.5 | FGLNTVLTTDNSDLFINSIGIVPSVR |
| 1 | 0.66 | 1 | 1.5 | FLNEMIAPVMR |
| 1 | 0.66 | 1.0 | 1.5 | MHMWVEDVLDK |
| 1 | 0.7 | 1.1 | 1.6 | SDFYDIVLVATPLNR |

5.329 glucose phosphate isomerase [Homo sapiens]

Protein Accession gi|18201905
Mean Expression Ratio 1.02
Median Expression Ratio 1.02
Credible Interval (0.62, 1.63)
Associated Peptides 1
Associated Spectra 1
Coverage 0.0305

| A | 2.5 | 50 | 97.5 | Sequence |
|---|-----|-----|------|----------|
| 1 | 0.61 | 1.0 | 1.7 | MIPCDFLIPVQTQHPIR |

1 M A A L T R D P Q F Q K L Q Q W Y R E H R S E L N L R R L F D A N K D R F N H F
S L T L N T N H G H I L V D Y S K N L V T E D V M R M L V D L A K S R G V E A A

81 R E R M F N G E K I N Y T E G R A V L H V A L R N R S N T P I L V D G K D V M P
E V N K V L D K M K S F C Q R V R S G D W K G Y T G K T I T D V I N I G I G G S

161 D L G P L M V T E A L K P Y S S G G P R V W Y V S N I D G T H I A K T L A Q L
N P E S S L F I I A S K T F T T Q E T I T N A E T A K E W F L Q A A K D P S A V A

241 K H F V A L S T N T T K V K E F G I D P Q N M F E F W D W V G G R Y S L W S
A I G L S I A L H V G F D N F E Q L L S G A H W M D Q H F R T T P L E K N A P V L
L

321 A L L G I W Y I N C F G C E T H A M L P Y D Q Y L H R F A A Y F Q Q G D M E S N G K Y I T K S G T R V D H Q T G P I V W G E P G T N G Q H A F Y Q L I H Q G T K

401 M I P C D F L I P V Q T Q H P I R K G L H H K I L L A N F L A Q T E A L M R G K S T E E A R K E L Q A A G K S P E D L E R L L P H K V F E G N R P T N S I V F T

481 K L T P F M L G A L V A M Y E H K I F V Q G I I W D I N S F D Q W G V E L G K Q L A K K I E P E L D G S A Q V T S H D A S T N G L I N F I K Q Q R E A R V Q

5.330 galactosidase, beta 1 isoform a preproprotein [Homo sapiens]

Protein Accession gi|119372308
Mean Expression Ratio 0.981
Median Expression Ratio 0.982
Credible Interval (0.798, 1.20)
Associated Peptides 12
Associated Spectra 17
Coverage 0.183

| A | 2.5 | 50 | 97.5 | Sequence |
|---|---|---|---|---|
| 2 | 0.74 | 1 | 1.4 | AGATLDLLVENMGR |
| 2 | 0.73 | 0.99 | 1.3 | AYVAVDGIPQGVLER |
| 2 | 0.79 | 1.1 | 1.5 | GQVWINGFNLGR |
| 1 | 0.71 | 0.99 | 1.4 | LAHELGLLVILRPGPY |
| 1 | 0.71 | 0.98 | 1.3 | TEAVASSLYDILAR |
| 1 | 0.72 | 1 | 1.4 | TVGAALDILCPSGPIK |
| 1 | 0.71 | 0.98 | 1.4 | VAVDGIPQGVLER |
| 1 | 0.7 | 0.98 | 1.4 | WLGVLLPK |
| 2 | 0.69 | 0.92 | 1.3 | HHLGDDVVLF |
| 1 | 0.7 | 0.97 | 1.3 | ACDFDYLR |
| 1 | 0.71 | 0.98 | 1.4 | VNYGAYINDFK |
| 2 | 0.67 | 0.9 | 1.2 | LAHELGLLVILR |

1 M P G F L V R I L P L L L V L L L L G P T R G L R N A T Q R M F E I D Y S R D S F L K D G Q P F R Y I S G S I H Y S R V P R F Y W K D R L L K M K M A G L N A I

81 Q T Y V P W N F H E P W P G Q Y Q F S E D H D V E Y F L R L A H E L G L L V I L R P G P Y I C A E W E M G G L P A W L L E K E S I L L R S S D P D Y L A A V D K

161 W L G V L L P K M K P L L Y Q N G G P V I T V Q V E N E Y G S Y F A C D F D Y L R F L Q K R F R H H L G D D V V L F T T D G A H K T F L K C G A L Q G L Y T T V

241 D F G T G S N I T D A F L S Q R K C E P K G P L I N S E F Y T G W L D H W G Q P
H S T I K T E A V A S S L Y D I L A R G A S V N L Y M F I G G T N F A Y W N G A

321 N S P Y A A Q P T S Y D Y D A P L S E A G D L T E K Y F A L R N I I Q K F E K V
P E G P I P P S T P K F A Y G K V T L E K L K T V G A A L D I L C P S G P I K S

401 L Y P L T F I Q V K Q H Y G F V L Y R T T L P Q D C S N P A P L S S P L N G V H
D R A Y V A V D G I P Q G V L E R N N V I T L N I T G K A G A T L D L L V E N M

481 G R V N Y G A Y I N D F K G L V S N L T L S S N I L T D W T I F P L D T E D A V
R S H L G G W G H R D S G H H D E A W A H N S S N Y T L P A F Y M G N F S I P S

561 G I P D L P Q D T F I Q F P G W T K G Q V W I N G F N L G R Y W P A R G P Q L
T L F V P Q H I L M T S A P N T I T V L E L E W A P C S S D D P E L C A V T F V D

641 RPVIGS S VTYDHP SKPVEKRLMPPPPQKNKD SWLDHV

5.331 GDP dissociation inhibitor 2 isoform 1 [Homo sapiens]

Protein Accession gi|6598323
Mean Expression Ratio 0.986
Median Expression Ratio 0.983
Credible Interval (0.61, 1.63)
Associated Peptides 1
Associated Spectra 1
Coverage 0.0337

| A | 2.5 | 50 | 97.5 | Sequence |
|---|-----|-----|------|----------|
| 1 | 0.59 | 0.98 | 1.6 | TDDYLDQPCYETINR |

1 M N E E Y D V I V L G T G L T E C I L S G I M S V N G K K V L H M D R N P Y Y G
G E S A S I T P L E D L Y K R F K I P G S P P E S M G R G R D W N V D L I P K F

81 L M A N G Q L V K M L L Y T E V T R Y L D F K V T E G S F V Y K G G K I Y K V
P S T E A E A L A S S L M G L F E K R R F R K F L V Y V A N F D E K D P R T F E G

161 I D P K K T T M R D V Y K K F D L G Q D V I D F T G H A L A L Y R T D D Y L D
Q P C Y E T I N R I K L Y S E S L A R Y G K S P Y L Y P L Y G L G E L P Q G F A R

241 L S A I Y G G T Y M L N K P I E E I I V Q N G K V I G V K S E G E I A R C K Q L I
C D P S Y V K D R V E K V G Q V I R V I C I L S H P I K N T N D A N S C Q I I

321 I P Q N Q V N R K S D I Y V C M I S F A H N V A A Q G K Y I A I V S T T V E T K E P E K E I R P A L E L L E P I E Q K F V S I S D L L V P K D L G T E S Q I F I

401 S R T Y D A T T H F E T T C D D I K N I Y K R M T G S E F D F E E M K R K K N D I Y G E D

5.332 solute carrier family 3 (activators of dibasic and neutral amino acid transport), member 2 isoform c [Homo sapiens] ; solute carrier family 3 (activators of dibasic and neutral amino acid transport), member 2 isoform f [Homo sapiens]; solute carrier family 3 (activators of dibasic and neutral amino acid transport), member 2 isoform e [Homo sapiens]; solute carrier family 3 (activators of dibasic and neutral amino acid transport), member 2 isoform d [Homo sapiens]; solute carrier family 3 (activators of dibasic and neutral amino acid transport), member 2 isoform b [Homo sapiens]; solute carrier family 3 (activators of dibasic and neutral amino acid transport), member 2 isoform a [Homo sapiens]

Protein Accession gi|65506891 gi|61744483 gi|61744481 gi|61744479 gi|61744477 gi|61744475
Mean Expression Ratio 1.02
Median Expression Ratio 1.02
Credible Interval (0.627, 1.64)
Associated Peptides 1
Associated Spectra 1
Coverage NaN

| A | 2.5 | 50 | 97.5 | Sequence |
|---|-----|-----|------|----------|
| 1 | 0.61 | 1.0 | 1.7 | LLTSFLPAQLLR |

5.333 heat shock 70kDa protein 8 isoform 1 [Homo sapiens]

Protein Accession gi|5729877
Mean Expression Ratio 1.02
Median Expression Ratio 1.02
Credible Interval (0.866, 1.20)
Associated Peptides 18
Associated Spectra 36
Coverage 0.302

| A | 2.5 | 50 | 97.5 | Sequence |
|---|-----|------|------|----------|
| 1 | 0.75 | 1.0 | 1.4 | AVVTVPAYFNDSQR |
| 2 | 0.74 | 0.98 | 1.3 | CNEIINWLDK |
| 1 | 0.75 | 1.0 | 1.4 | DAGTIAGLNVLR |
| 1 | 0.74 | 1.0 | 1.4 | FDDAVVQSDMK |
| 5 | 0.8 | 1.0 | 1.3 | FEELNADLFR |
| 1 | 0.75 | 1.0 | 1.4 | FELTGIPPAPR |
| 2 | 0.76 | 1.0 | 1.4 | LLQDFFNGK |
| 2 | 0.78 | 1.0 | 1.4 | MVNHFIAEFK |
| 1 | 0.74 | 1.0 | 1.4 | NQTAEKEEFEHQQK |

(continued)

| A | 2.5 | 50 | 97.5 | Sequence |
|---|-----|-----|------|----------|
| 1 | 0.74 | 1 | 1.4 | NQVAMNPTNTVFDAK |
| 1 | 0.75 | 1.0 | 1.4 | QTQTFTTYSDNQPGVLIQVYEGER |
| 2 | 0.77 | 1.0 | 1.4 | RFDDAVVQSDMK |
| 2 | 0.78 | 1.0 | 1.4 | SQIHDIVLVGGSTR |
| 5 | 0.76 | 0.98 | 1.3 | STAGDTHLGGEDFDNR |
| 6 | 0.77 | 0.98 | 1.2 | TVTNAVVTVPAYFNDSQR |
| 1 | 0.77 | 1.0 | 1.4 | MVVNDAGRPK |
| 1 | 0.76 | 1.0 | 1.4 | VQVEYK |
| 1 | 0.77 | 1.0 | 1.4 | HWPFMVVNDAGRPK |

1 MSKGPAVGIDLGTTYSCVGVFQHGKVEIIANDQGNRTTPSYVAFTDTERLIGDAAKNQVAMNPTNTVFDAKRLIGRRFDD

81 AVVQSDMKHWPFMVVNDAGRPKVQVEYKGETKSFYPEEVSSMVLTKMKEIAEAYLGKTVTNAVVTVPAYFNDSQRQATKD

161 AGTIAGLNVLRIINEPTAAAIAYGLDKKVGAERNVLIFDLGGGTFDVSILTIEDGIFEVKSTAGDTHLGGEDFDNRMVNH

241 FIAEFKRKHKKDISENKRAVRRLRTACERAKRTLSSSTQASIEIDSLYEGIDFYTSITRARFEELNADLFRGTLDPVEKA

321 LRDAKLDKSQIHDIVLVGGSTRIPKIQKLLQDFFNGKELNKSINPDEAVAYGAAVQAAILSGDKSENVQDLLLLDVTPLS

401 LGIETAGGVMTVLIKRNTTIPTKQTQTFTTYSDNQPGVLIQVYEGERAMTKDNNLLGKFELTGIPPAPRGVPQIEVTFDI

481 DANGILNVSAVDKSTGKENKITITNDKGRLSKEDIERMVQEAEKYKAEDEKQRDKVSSKNSLESYAFNMKATVEDEKLQG

561 KINDEDKQKILDKCNEIINWLDKNQTAEKEEFEHQQKELEKVCNPIITKLYQSAGGMPGGMPGGFPGGGAPPSGGASSGP

641 TIEEVD

5.334 RAB9A, member RAS oncogene family [Homo sapiens]

Protein Accession gi|4759012

Mean Expression Ratio 1.02
Median Expression Ratio 1.02
Credible Interval (0.627, 1.66)
Associated Peptides 1
Associated Spectra 1
Coverage 0.0697

| A | 2.5 | 50 | 97.5 | Sequence |
|---|-----|----|------|----------|
| 1 | 0.61 | 1.0 | 1.7 | DATNVAAAFEEAVR |

1 M A G K S S L F K V I L L G D G G V G K S S L M N R Y V T N K F D T Q L F H T I
G V E F L N K D L E V D G H F V T M Q I W D T A G Q E R F R S L R T P F Y R G S

81 D C C L L T F S V D D S Q S F Q N L S N W K K E F I Y Y A D V K E P E S F P F V I
L G N K I D I S E R Q V S T E E A Q A W C R D N G D Y P Y F E T S A K D A T N

161 V A A A F E E A V R R V L A T E D R S D H L I Q T D T V N L H R K P K P S S S C
C

5.335 complement component 8, gamma polypeptide [Homo sapiens]

Protein Accession gi|166197660
Mean Expression Ratio 1.02
Median Expression Ratio 1.02
Credible Interval (0.627, 1.65)
Associated Peptides 1
Associated Spectra 1
Coverage NaN

| A | 2.5 | 50 | 97.5 | Sequence |
|---|-----|----|------|----------|
| 1 | 0.61 | 1.0 | 1.7 | SLPVSDSVLSGFEQR |

5.336 MAPK scaffold protein 1 [Homo sapiens]

Protein Accession gi|11496277
Mean Expression Ratio 0.986
Median Expression Ratio 0.985
Credible Interval (0.667, 1.45)
Associated Peptides 2
Associated Spectra 3
Coverage 0.210

| A | 2.5 | 50 | 97.5 | Sequence |
|---|-----|----|------|----------|
| 2 | 0.64 | 0.97 | 1.5 | ELAPLFEELR |

(continued)

| A | 2.5 | 50 | 97.5 | Sequence |
|---|-----|-----|------|----------|
| 1 | 0.64 | 1 | 1.6 | KLPSVEGLHAIVVSDR |

1 M A D D L K R F L Y K K L P S V E G L H A I V V S D R D G V P V I K V A N D N A P E H A L R P G F L S T F A L A T D Q G S K L G L S K N K S I I C Y Y N T Y Q V

81 V Q F N R L P L V V S F I A S S S A N T G L I V S L E K E L A P L F E E L R Q V V E V S

5.337 transketolase isoform 1 [Homo sapiens]; transketolase isoform 2 [Homo sapiens]; transketolase isoform 1 [Homo sapiens]

Protein Accession gi|4507521 gi|205277465 gi|205277463
Mean Expression Ratio 1.01
Median Expression Ratio 1.01
Credible Interval (0.618, 1.61)
Associated Peptides 1
Associated Spectra 1
Coverage NaN

| A | 2.5 | 50 | 97.5 | Sequence |
|---|-----|-----|------|----------|
| 1 | 0.62 | 1 | 1.7 | LDNLVAILDINR |

5.338 complement factor B preproprotein [Homo sapiens]

Protein Accession gi|67782358
Mean Expression Ratio 0.989
Median Expression Ratio 0.988
Credible Interval (0.646, 1.52)
Associated Peptides 2
Associated Spectra 2
Coverage 0.0314

| A | 2.5 | 50 | 97.5 | Sequence |
|---|-----|------|------|----------|
| 1 | 0.62 | 0.99 | 1.6 | LLQEGQALEYVCPSGFYPYPVQTR |
| 1 | 0.62 | 1 | 1.6 | VCPSGFYPYPVQTR |

1 M G S N L S P Q L C L M P F I L G L L S G G V T T T P W S L A R P Q G S C S L E G V E I K G G S F R L L Q E G Q A L E Y V C P S G F Y P Y P V Q T R T C R S T G

81 S W S T L K T Q D Q K T V R K A E C R A I H C P R P H D F E N G E Y W P R S P Y Y N V S D E I S F H C Y D G Y T L R G S A N R T C Q V N G R W S G Q T A I C D N

161 G A G Y C S N P G I P I G T R K V G S Q Y R L E D S V T Y H C S R G L T L R G S
Q R R T C Q E G G S W S G T E P S C Q D S F M Y D T P Q E V A E A F L S S L T E

241 T I E G V D A E D G H G P G E Q Q K R K I V L D P S G S M N I Y L V L D G S D S
I G A S N F T G A K K C L V N L I E K V A S Y G V K P R Y G L V T Y A T Y P K I

321 W V K V S E A D S S N A D W V T K Q L N E I N Y E D H K L K S G T N T K K A
L Q A V Y S M M S W P D D V P P E G W N R T R H V I I L M T D G L H N M G G D P
I T

401 V I D E I R D L L Y I G K D R K N P R E D Y L D V Y V F G V G P L V N Q V N I N
A L A S K K D N E Q H V F K V K D M E N L E D V F Y Q M I D E S Q S L S L C G M

481 V W E H R K G T D Y H K Q P W Q A K I S V I R P S K G H E S C M G A V V S E
Y F V L T A A H C F T V D D K E H S I K V S V G G E K R D L E I E V V L F H P N Y
N

561 I N G K K E A G I P E F Y D Y D V A L I K L K N K L K Y G Q T I R P I C L P C T E
G T T R A L R L P P T T T C Q Q Q K E E L L P A Q D I K A L F V S E E E K K L

641 T R K E V Y I K N G D K K G S C E R D A Q Y A P G Y D K V K D I S E V V T P R
F L C T G G V S P Y A D P N T C R G D S G G P L I V H K R S R F I Q V G V I S W G

721 V V D V C K N Q K R Q K Q V P A H A R D F H I N L F Q V L P W L K E K L Q D
E D L G F L

5.339 cyclin M3 isoform 1 [Homo sapiens]; cyclin M3 isoform 2 [Homo sapiens]

Protein Accession gi|40068049 gi|40068047
Mean Expression Ratio 0.987
Median Expression Ratio 0.989
Credible Interval (0.601, 1.59)
Associated Peptides 1
Associated Spectra 1
Coverage NaN

| A | 2.5 | 50 | 97.5 | Sequence |
|---|-----|----|------|----------|
| 1 | 0.58 | 0.98 | 1.6 | AQNLPQSPENTDLQVIPGSQTR |

5.340 solute carrier family 9 (sodium/hydrogen exchanger), isoform 3 [Homo sapiens]

Protein Accession gi|194239733
Mean Expression Ratio 0.993

Median Expression Ratio 0.989
Credible Interval (0.608, 1.62)
Associated Peptides 1
Associated Spectra 1
Coverage NaN

| A | 2.5 | 50 | 97.5 | Sequence |
|---|-----|-----|------|----------|
| 1 | 0.59 | 0.99 | 1.7 | DTASDSPAGIDNPVFSPDEALDR |

5.341 solute carrier family 12 (potassium/chloride transporters), member 9 [Homo sapiens]

Protein Accession gi|31881740
Mean Expression Ratio 0.989
Median Expression Ratio 0.99
Credible Interval (0.652, 1.51)
Associated Peptides 2
Associated Spectra 2
Coverage 0.0438

| A | 2.5 | 50 | 97.5 | Sequence |
|---|-----|-----|------|----------|
| 1 | 0.62 | 0.99 | 1.6 | AEVQEVVWGEGAGAGEPEAEEEGDFVNSGR |
| 1 | 0.62 | 1 | 1.6 | YLALLETLTR |

1 M A S E S S P L L A Y R L L G E E G V A L P A N G A G G P G G A S A R K L S T F L G V V V P T V L S M F S I V V F L R I G F V V G H A G L L Q A L A M L L V A Y

81 F I L A L T V L S V C A I A T N G A V Q G G G A Y F M I S R T L G P E V G G S I G L M F Y L A N V C G C A V S L L G L V E S V L D V F G A D A T G P S G L R V L

161 P Q G Y G W N L L Y G S L L L G L V G G V C T L G A G L Y A R A S F L T F L L V S G S L A S V L I S F V A V G P R D I R L T P R P G P N G S S L P P R F G H F T

241 G F N S S T L K D N L G A G Y A E D Y T T G A V M N F A S V F A V L F N G C T G I M A G A N M S G E L K D P S R A I P L G T I V A V A Y T F F V Y V L L F F L S

321 S F T C D R T L L Q E D Y G F F R A I S L W P P L V L I G I Y A T A L S A S M S S L I G A S R I L H A L A R D D L F G V I L A P A K V V S R G G N P W A A V L Y

401 S W G L V Q L V L L A G K L N T L A A V V T V F Y L V A Y A A V D L S C L S L E W A S A P N F R P T F S L F S W H T C L L G V A S C L L M M F L I S P G A A G G

481 S L L L M G L L A A L L T A R G G P S S W G Y V S Q A L L F H Q V R K Y L L R L D V R K D H V K F W R P Q L L L L V G N P R G A L P L L R L A N Q L K K G G L Y

561 VLGHVTLGDLDSLPSDPVQPQYGAWLSLVDRAQVKAFVDLTLPSVRQGAQHLLRISLGGMKPNTLVLGFYDDAPPQDH

641 FLTDPAFSEPADSTREGSSPALSTLFPPPRAPGSPRALNPQDYVATVADALKMNKNVVLARASGALPPERLSRGSGGTSQ

721 LHHVDVWPLNLLRPRGGPGYVDVCGLFLLQMATILGMVPAWHSARLRIFLCLGPREAPGAAEGRLRALLSQLRIRAEVQE

801 VVWGEGAGAGEPEAEEEGDFVNSGRGDAEAEALARSANALVRAQQGRGTGGGPGGPEGGDAEGPITALTFLYLPRPPADP

881 ARYPRYLALLETLTRDLGPTLLVHGVTPVTCTDL

5.342 RAB5C, member RAS oncogene family isoform a [Homo sapiens]; RAB5C, member RAS oncogene family isoform b [Homo sapiens]

Protein Accession gi|41393614 gi|41393545
Mean Expression Ratio 1.01
Median Expression Ratio 1.01
Credible Interval (0.624, 1.64)
Associated Peptides 1
Associated Spectra 1
Coverage NaN

| A | 2.5 | 50 | 97.5 | Sequence |
|---|-----|----|------|----------|
| 1 | 0.6 | 1 | 1.7 | FEIWDTAGQER |

5.343 pyruvate kinase, liver and RBC isoform 1 [Homo sapiens]

Protein Accession gi|10835121
Mean Expression Ratio 1.01
Median Expression Ratio 1.01
Credible Interval (0.63, 1.67)
Associated Peptides 1
Associated Spectra 1
Coverage 0.0348

| A | 2.5 | 50 | 97.5 | Sequence |
|---|------|----|------|----------|
| 1 | 0.61 | 1 | 1.7 | GVNLPGAQVDLPGLSEQDVR |

1 M S I Q E N I S S L Q L R S W V S K S Q R D L A K S I L I G A P G G P A G Y L R R A S V A Q L T Q E L G T A F F Q Q Q Q L P A A M A D T F L E H L C L L D I D S

81 E P V A A R S T S I I A T I G P A S R S V E R L K E M I K A G M N I A R L N F S H G S H E Y H A E S I A N V R E A V E S F A G S P L S Y R P V A I A L D T K G P

161 E I R T G I L Q G G P E S E V E L V K G S Q V L V T V D P A F R T R G N A N T V W V D Y P N I V R V V P V G G R I Y I D D G L I S L V V Q K I G P E G L V T Q V

241 E N G G V L G S R K G V N L P G A Q V D L P G L S E Q D V R D L R F G V E H G V D I V F A S F V R K A S D V A A V R A A L G P E G H G I K I I S K I E N H E G V

321 K R F D E I L E V S D G I M V A R G D L G I E I P A E K V F L A Q K M M I G R C N L A G K P V V C A T Q M L E S M I T K P R P T R A E T S D V A N A V L D G A D

401 C I M L S G E T A K G N F P V E A V K M Q H A I A R E A E A A V Y H R Q L F E E L R R A A P L S R D P T E V T A I G A V E A A F K C C A A A I I V L T T T G R S

481 A Q L L S R Y R P R A A V I A V T R S A Q A A R Q V H L C R G V F P L L Y R E P P E A I W A D D V D R R V Q F G I E S G K L R G F L R V G D L V I V V T G W R P

561 GSGYTNIMRVLSIS

5.344 glutathione transferase [Homo sapiens]

Protein Accession gi|4504183
Mean Expression Ratio 1.01
Median Expression Ratio 1.01
Credible Interval (0.713, 1.41)
Associated Peptides 3
Associated Spectra 5
Coverage 0.205

| A | 2.5 | 50 | 97.5 | Sequence |
|---|-----|----|------|----------|
| 3 | 0.71 | 1.0 | 1.4 | DQQEAALVDMVNDGVEDLR |
| 1 | 0.67 | 1.0 | 1.5 | FQDGDLTLYQSNTILR |
| 1 | 0.66 | 1 | 1.5 | TVVYFPVR |

1 M P P Y T V V Y F P V R G R C A A L R M L L A D Q G Q S W K E E V V T V E T W Q E G S L K A S C L Y G Q L P K F Q D G D L T L Y Q S N T I L R H L G R T L G L Y

81 G K D Q Q E A A L V D M V N D G V E D L R C K Y I S L I Y T N Y E A G K D D Y V K A L P G Q L K P F E T L L S Q N Q G G K T F I V G D Q I S F A D Y N L L D L L

LIHEVLAPGCLDAFPLLSAYVGRLSARPKLKAFLASPEYV
NLPINGNGKQ

5.345 ATPase, H+ transporting, lysosomal 70kD, VI subunit A, isoform 1 [Homo sapiens]

Protein Accession gi|19913424
Mean Expression Ratio 1.01
Median Expression Ratio 1.01
Credible Interval (0.726, 1.41)
Associated Peptides 5
Associated Spectra 5
Coverage 0.104

| A | 2.5 | 50 | 97.5 | Sequence |
|---|-----|----|------|----------|
| 1 | 0.68 | 1.0 | 1.5 | FTMVQVWPVR |
| 1 | 0.66 | 1 | 1.5 | HFTEFVPLR |
| 1 | 0.69 | 1.0 | 1.5 | SDYAQLLEDMQNAFR |
| 1 | 0.66 | 1 | 1.5 | LPANHPLLTGQR |
| 1 | 0.7 | 1.0 | 1.6 | EILQEEEDLAEIVQLVGK |

1 MDFSKLPKILDEDKESTFGYVHGVSGPVVTACDMAGAAMY
ELVRVGHSELVGEIIRLEGDMATIQVYEETSGVSVGDPVL

81 RTGKPLSVELGPGIMGAIFDGIQRPLSDISSQTQSIYIPRGV
NVSALSRDIKWDFTPCKNLRVGSHITGGDIYGIVSENS

161 LIKHKIMLPPRNRGTVTYIAPPGNYDTSDVVLELEFEGVK
EKFTMVQVWPVRQVRPVTEKLPANHPLLTGQRVLDALFPC

241 VQGGTTAIPGAFGCGKTVISQSLSKYSNSDVIIYVGCGER
GNEMSEVLRDFPELTMEVDGKVESIMKRTALVANTSNMPV

321 AAREASIYTGITLSEYFRDMGYHVSMMADSTSRWAEALR
EISGRLAEMPADSGYPAYLGARLASFYERAGRVKCLGNPER

401 EGSVSIVGAVSPPGGDFSDPVTSATLGIVQVFWGLDKKLA
QRKHFPSVNWLISYSKYMRALDEYYDKHFTEFVPLRTKAK

481 EILQEEEDLAEIVQLVGKASLAETDKITLEVAKLIKDDFL
QQNGYTPYDRFCPFYKTVGMLSNMIAFYDMARRAVETTAQ

561 SDNKITWSIIREHMGDILYKLSSMKFKDPLKDGEAKIKSD
YAQLLEDMQNAFRSLED

5.346 DnaJ (Hsp40) homolog, subfamily A, member 1 [Homo sapiens]

Protein Accession gi|4504511
Mean Expression Ratio 0.986
Median Expression Ratio 0.99
Credible Interval (0.634, 1.49)
Associated Peptides 1
Associated Spectra 2
Coverage 0.0479

| A | 2.5 | 50 | 97.5 | Sequence |
|---|-----|----|------|----------|
| 2 | 0.63 | 0.98 | 1.5 | NVVHQLSVTLEDLYNGATR |

1 M V K E T T Y Y D V L G V K P N A T Q E E L K K A Y R K L A L K Y H P D K N P
N E G E K F K Q I S Q A Y E V L S D A K K R E L Y D K G G E Q A I K E G G A G G G

81 F G S P M D I F D M F F G G G G R M Q R E R R G K N V V H Q L S V T L E D L Y
N G A T R K L A L Q K N V I C D K C E G R G G K K G A V E C C P N C R G T G M Q
I

161 R I H Q I G P G M V Q Q I Q S V C M E C Q G H G E R I S P K D R C K S C N G R
K I V R E K K I L E V H I D K G M K D G Q K I T F H G E G D Q E P G L E P G D I I

241 I V L D Q K D H A V F T R R G E D L F M C M D I Q L V E A L C G F Q K P I S T L
D N R T I V I T S H P G Q I V K H G D I K C V L N E G M P I Y R R P Y E K G R L

321 I I E F K V N F P E N G F L S P D K L S L L E K L L P E R K E V E E T D E M D Q
V E L V D F D P N Q E R R R H Y N G E A Y E D D E H H P R G G V Q C Q T S

5.347 aquaporin 2 [Homo sapiens]

Protein Accession gi|4502179
Mean Expression Ratio 0.99
Median Expression Ratio 0.991
Credible Interval (0.645, 1.52)
Associated Peptides 2
Associated Spectra 2
Coverage 0.0886

| A | 2.5 | 50 | 97.5 | Sequence |
|---|-----|----|------|----------|
| 1 | 0.6 | 0.97 | 1.5 | QSVELHSPQSLPR |
| 1 | 0.63 | 1.0 | 1.6 | GLEPDTDWEER |

1 M W E L R S I A F S R A V F A E F L A T L L F V F F G L G S A L N W P Q A L P S V L Q I A M A F G L G I G T L V Q A L G H I S G A H I N P A V T V A C L V G C H

81 V S V L R A A F Y V A A Q L L G A V A G A A L L H E I T P A D I R G D L A V N A L S N S T T A G Q A V T V E L F L T L Q L V L C I F A S T D E R R G E N P G T P

161 A L S I G F S V A L G H L L G I H Y T G C S M N P A R S L A P A V V T G K F D D H W V F W I G P L V G A I L G S L L Y N Y V L F P P A K S L S E R L A V L K G L

241 EPDTDWEEREVRRRQSVELHSPQSLPRGTKA

5.348 serine incorporator 1 [Homo sapiens]

Protein Accession gi|24308213
Mean Expression Ratio 1.01
Median Expression Ratio 1.01
Credible Interval (0.624, 1.63)
Associated Peptides 1
Associated Spectra 1
Coverage 0.0375

| A | 2.5 | 50 | 97.5 | Sequence |
|---|-----|----|------|----------|
| 1 | 0.61 | 1 | 1.7 | LTLTSDESTLIEDGGAR |

1 M G S V L G L C S M A S W I P C L C G S A P C L L C R C C P S G N N S T V T R L I Y A L F L L V G V C V A C V M L I P G M E E Q L N K I P G F C E N E K G V V P

81 C N I L V G Y K A V Y R L C F G L A M F Y L L L S L L M I K V K S S S D P R A A V H N G F W F F K F A A A I A I I G A F F I P E G T F T T V W F Y V G M A G A

161 F C F I L I Q L V L L I D F A H S W N E S W V E K M E E G N S R C W Y A A L L S A T A L N Y L L S L V A I V L F F V Y Y T H P A S C S E N K A F I S V N M L L C

241 V G A S V M S I L P K I Q E S Q P R S G L L Q S S V I T V Y T M Y L T W S A M T N E P E T N C N P S L L S I I G Y N T T S T V P K E G Q S V Q W W H A Q G I I G

321 L I L F L L C V F Y S S I R T S N N S Q V N K L T L T S D E S T L I E D G G A R S D G S L E D G D D V H R A V D N E R D G V T Y S Y S F F H F M L F L A S L Y I

401 M M T L T N W Y R Y E P S R E M K S Q W T A V W V K I S S S W I G I V L Y V W T L V A P L V L T N R D F D

5.349 syntaxin 7 [Homo sapiens]

Protein Accession gi|170932494
Mean Expression Ratio 1.01
Median Expression Ratio 1.01
Credible Interval (0.748, 1.35)
Associated Peptides 4
Associated Spectra 8
Coverage NaN

| A | 2.5 | 50 | 97.5 | Sequence |
|---|-----|-----|------|----------|
| 1 | 0.69 | 1.0 | 1.5 | EFGSLPTTPSEQR |
| 4 | 0.72 | 0.98 | 1.3 | NLVSWESQTQPQVQVQDEEITEDDLR |
| 1 | 0.7 | 1.0 | 1.5 | QLEADIMDINEIFK |
| 2 | 0.72 | 1.0 | 1.4 | TLNQLGTPQDSPELR |

5.350 carbonic anhydrase XII isoform 2 precursor [Homo sapiens]; carbonic anhydrase XII isoform 1 precursor [Homo sapiens]

Protein Accession gi|45935383 gi|4502515
Mean Expression Ratio 0.991
Median Expression Ratio 0.993
Credible Interval (0.66, 1.49)
Associated Peptides 1
Associated Spectra 3
Coverage NaN

| A | 2.5 | 50 | 97.5 | Sequence |
|---|-----|------|------|----------|
| 3 | 0.67 | 0.99 | 1.5 | GQEAFVPGFNIEELLPER |

5.351 major histocompatibility complex, class II, DR alpha precursor [Homo sapiens]

Protein Accession gi|52426774
Mean Expression Ratio 1.00
Median Expression Ratio 1.01
Credible Interval (0.754, 1.32)
Associated Peptides 5
Associated Spectra 9
Coverage 0.209

| A | 2.5 | 50 | 97.5 | Sequence |
|---|-----|------|------|----------|
| 2 | 0.7 | 0.98 | 1.4 | FHYLPFLPSTEDVYDCR |
| 3 | 0.76 | 1.1 | 1.5 | NGKPVTTGVSETVFLPR |

(continued)

| A | 2.5 | 50 | 97.5 | Sequence |
|---|-----|-----|------|----------|
| 2 | 0.73 | 1.0 | 1.4 | VEHWGLDEPLLK |
| 1 | 0.69 | 1 | 1.5 | KFHYLPFLPSTEDVYDCR |
| 1 | 0.66 | 0.95 | 1.4 | EDHLFR |

1 M A I S G V P V L G F F I I A V L M S A Q E S W A I K E E H V I I Q A E F Y L N P D Q S G E F M F D F D G D E I F H V D M A K K E T V W R L E E F G R F A S F E

81 A Q G A L A N I A V D K A N L E I M T K R S N Y T P I T N V P P E V T V L T N S P V E L R E P N V L I C F I D K F T P P V V N V T W L R N G K P V T T G V S E T

161 V F L P R E D H L F R K F H Y L P F L P S T E D V Y D C R V E H W G L D E P L L K H W E F D A P S P L P E T T E N V V C A L G L T V G L V G I I I G T I F I I K

241 GLRKSNAAERRGPL

5.352 cytosolic phosphoenolpyruvate carboxykinase 1 [Homo sapiens]

Protein Accession gi|187281517
Mean Expression Ratio 0.996
Median Expression Ratio 0.994
Credible Interval (0.7, 1.43)
Associated Peptides 3
Associated Spectra 4
Coverage NaN

| A | 2.5 | 50 | 97.5 | Sequence |
|---|-----|-----|------|----------|
| 2 | 0.67 | 1 | 1.5 | IFHVNWFR |
| 1 | 0.65 | 1 | 1.5 | LLGQMEEEGILR |
| 1 | 0.65 | 1 | 1.5 | FLWPGFGENSR |

5.353 glutathione peroxidase 3 precursor [Homo sapiens]

Protein Accession gi|6006001
Mean Expression Ratio 0.996
Median Expression Ratio 0.994
Credible Interval (0.723, 1.37)
Associated Peptides 4
Associated Spectra 6
Coverage 0.217

| A | 2.5 | 50 | 97.5 | Sequence |
|---|-----|-----|------|----------|
| 2 | 0.7 | 1 | 1.4 | FLVGPDGIPIMR |
| 1 | 0.67 | 1 | 1.5 | FYTFLK |
| 1 | 0.66 | 0.97 | 1.4 | QEPGENSEILPTLK |
| 2 | 0.72 | 1.0 | 1.5 | YVRPGGGFVPNFQLFEK |

1 M A R L L Q A S C L L S L L L A G F V S Q S R G Q E K S K M D C H G G I S G T I Y E Y G A L T I D G E E Y I P F K Q Y A G K Y V L F V N V A S Y U G L T G Q Y I

81 E L N A L Q E E L A P F G L V I L G F P C N Q F G K Q E P G E N S E I L P T L K Y V R P G G G F V P N F Q L F E K G D V N G E K E Q K F Y T F L K N S C P P T S

161 E L L G T S D R L F W E P M K V H D I R W N F E K F L V G P D G I P I M R W H H R T T V S N V K M D I L S Y M R R Q A A L G V K R K

5.354 transmembrane 7 superfamily member 3 [Homo sapiens]

Protein Accession gi|7706575
Mean Expression Ratio 1.00
Median Expression Ratio 1.00
Credible Interval (0.7, 1.44)
Associated Peptides 4
Associated Spectra 4
Coverage 0.0842

| A | 2.5 | 50 | 97.5 | Sequence |
|---|-----|-----|------|----------|
| 1 | 0.67 | 1 | 1.5 | GRPFFPPHPYK |
| 1 | 0.68 | 1.0 | 1.6 | LQYDVYQYFLPENDLTEEMLLK |
| 1 | 0.65 | 0.99 | 1.5 | QYFLPENDLTEEMLLK |
| 1 | 0.65 | 1 | 1.5 | VTNILDPSYHIPPLR |

1 M G F L Q L L V V A V L A S E H R V A G A A E V F G N S S E G L I E F S V G K F R Y F E L N R P F P E E A I L H D I S S N V T F L I F Q I H S Q Y Q N T T V S F

81 S P T L L S N S S E T G T A S G L V F I L R P E Q S T C T W Y L G T S G I Q P V Q N M A I L L S Y S E R D P V P G G C N L E F D L D I D P N I Y L E Y N F F E T

161 T I K F A P A N L G Y A R G V D P P P C D A G T D Q D S R W R L Q Y D V Y Q Y F L P E N D L T E E M L L K H L Q R M V S V P Q V K A S A L K V V T L T A N D K T

241 S V S F S S L P G Q G V I Y N V I V W D P F L N T S A A Y I P A H T Y A C S F E A G E G S C A S L G R V S S K V F F T L F A L L G F F I C F F G H R F W K T E L

290

321 F F I G F I I M G F F F Y I L I T R L T P I K Y D V N L I L T A V T G S V G G M F L
V A V W W R F G I L S I C M L C V G L V L G F L I S S V T F F T P L G N L K

401 I F H D D G V F W V T F S C I A I L I P V V F M G C L R I L N I L T C G V I G S Y
S V V L A I D S Y W S T S L S Y I T L N V L K R A L N K D F H R A F T N V P F

481 Q T N D F I I L A V W G M L A V S G I T L Q I R R E R G R P F F P P H P Y K L W
K Q E R E R R V T N I L D P S Y H I P P L R E R L Y G R L T Q I K G L F Q K E Q

561 PAGERTPLLL

5.355 cathelicidin antimicrobial peptide [Homo sapiens]

Protein Accession gi|39753970
Mean Expression Ratio 0.997
Median Expression Ratio 0.996
Credible Interval (0.668, 1.49)
Associated Peptides 1
Associated Spectra 3
Coverage 0.0529

| A | 2.5 | 50 | 97.5 | Sequence |
|---|-----|----|------|----------|
| 3 | 0.67 | 1 | 1.5 | FALLGDFFR |

1 M K T Q R D G H S L G R W S L V L L L L G L V M P L A I I A Q V L S Y K E A V L
R A I D G I N Q R S S D A N L Y R L L D L D P R P T M D G D P D T P K P V S F T

81 V K E T V C P R T T Q Q S P E D C D F K K D G L V K R C M G T V T L N Q A R G
S F D I S C D K D N K R F A L L G D F F R K S K E K I G K E F K R I V Q R I K D F

161 LRNLVPRTES

5.356 transmembrane protein 55B isoform 1 [Homo sapiens]; transmembrane protein 55B isoform 2 [Homo sapiens]

Protein Accession gi|154816186 gi|154816184
Mean Expression Ratio 0.995
Median Expression Ratio 0.996
Credible Interval (0.652, 1.53)
Associated Peptides 2
Associated Spectra 2
Coverage NaN

| A | 2.5 | 50 | 97.5 | Sequence |
|---|-----|----|------|----------|
| 1 | 0.63 | 1 | 1.6 | NTFLWTEFTDR |

(continued)

| A | 2.5 | 50 | 97.5 | Sequence |
|---|---|---|---|---|
| 1 | 0.61 | 0.98 | 1.6 | IINLGPVHPGPLSPEPQPMGVR |

5.357 aconitase 1 [Homo sapiens]

Protein Accession gi|8659555
Mean Expression Ratio 0.996
Median Expression Ratio 0.997
Credible Interval (0.645, 1.56)
Associated Peptides 1
Associated Spectra 2
Coverage 0.0214

| A | 2.5 | 50 | 97.5 | Sequence |
|---|---|---|---|---|
| 2 | 0.65 | 0.99 | 1.6 | VILQDFTGVPAVVDFAAMR |

1 M S N P F A H L A E P L D P V Q P G K K F F N L N K L E D S R Y G R L P F S I R V
L L E A A I R N C D E F L V K K Q D I E N I L H W N V T Q H K N I E V P F K P

81 A R V I L Q D F T G V P A V V D F A A M R D A V K K L G G D P E K I N P V C P
A D L V I D H S I Q V D F N R R A D S L Q K N Q D L E F E R N R E R F E F L K W G

161 S Q A F H N M R I I P P G S G I I H Q V N L E Y L A R V V F D Q D G Y Y Y P D S
L V G T D S H T T M I D G L G I L G W G V G G I E A E A V M L G Q P I S M V L P

241 Q V I G Y R L M G K P H P L V T S T D I V L T I T K H L R Q V G V V G K F V E F
F G P G V A Q L S I A D R A T I A N M C P E Y G A T A A F F P V D E V S I T Y L

321 V Q T G R D E E K L K Y I K K Y L Q A V G M F R D F N D P S Q D P D F T Q V V
E L D L K T V V P C C S G P K R P Q D K V A V S D M K K D F E S C L G A K Q G F K

401 G F Q V A P E H H N D H K T F I Y D N T E F T L A H G S V V I A A I T S C T N T
S N P S V M L G A G L L A K K A V D A G L N V M P Y I K T S L S P G S G V V T Y

481 Y L Q E S G V M P Y L S Q L G F D V V G Y G C M T C I G N S G P L P E P V V E
A I T Q G D L V A V G V L S G N R N F E G R V H P N T R A N Y L A S P P L V I A Y

561 A I A G T I R I D F E K E P L G V N A K G Q Q V F L K D I W P T R D E I Q A V E
R Q Y V I P G M F K E V Y Q K I E T V N E S W N A L A T P S D K L F F W N S K S

641 T Y I K S P P F F E N L T L D L Q P P K S I V D A Y V L L N L G D S V T T D H I S
P A G N I A R N S P A A R Y L T N R G L T P R E F N S Y G S R R G N D A V M A

721 RGTFANIRLLNRFLNKQAPQTIHLPSGEILDVFDAAERYQ QAGLPLIVLAGKEYGAGSSRDWAAKGPFLLGIKAVLAESY

801 ERIHRSNLVGMGVIPLEYLPGENADALGLTGQERYTIIPE NLKPQMKVQVKLDTGKTFQAVMRFDTDVELTYFLNGGIL

881 NYMIRKMAK

5.358 nicastrin precursor [Homo sapiens]

Protein Accession gi|24638433
Mean Expression Ratio 0.997
Median Expression Ratio 0.997
Credible Interval (0.656, 1.51)
Associated Peptides 2
Associated Spectra 2
Coverage 0.031

| A | 2.5 | 50 | 97.5 | Sequence |
|---|---|---|---|---|
| 1 | 0.63 | 1 | 1.6 | GKFPVQLENVDSFVELGQVALR |
| 1 | 0.63 | 1 | 1.6 | FPVQLENVDSFVELGQVALR |

1 MATAGGGSGADPGSRGLLRLLSFCVLLAGLCRGNSVERKI YIPLNKTAPCVRLLNATHQIGCQSSISGDTGVIHVVEKEE

81 DLQWVLTDGPNPPYMVLLESKHFTRDLMEKLKGRTSRIA GLAVSLTKPSPASGFSPSVQCPNDGFGVYSNSYGPEFAHCR

161 EIQWNSLGNGLAYEDFSFPIFLLEDENETKVIKQCYQDHN LSQNGSAPTFPLCAMQLFSHMHAVISTATCMRRSSIQSTF

241 SINPEIVCDPLSDYNVWSMLKPINTTGTLKPDDRVVVAAT RLDSRSFFWNVAPGAESAVASFVTQLAAAEALQKAPDVTT

321 LPRNVMFVFFQGETFDYIGSSRMVYDMEKGKFPVQLENV DSFVELGQVALRTSLELWMHTDPVSQKNESVRNQVEDLLAT

401 LEKSGAGVPAVILRRPNQSQPLPPSSLQRFLRARNISGVV LADHSGAFHNKYYQSIYDTAENINVSYPEWLSPEEDLNFV

481 TDTAKALADVATVLGRALYELAGGTNFSDTVQADPQTVT RLLYGFLIKANNSWFQSILRQDLRSYLGDGPLQHYIAVSSP

561 T N T T Y V V Q Y A L A N L T G T V V N L T R E Q C Q D P S K V P S E N K D L Y E Y S W V Q G P L H S N E T D R L P R C V R S T A R L A R A L S P A F E L S Q W

641 S S T E Y S T W T E S R W K D I R A R I F L I A S K E L E L I T L T V G F G I L I F S L I V T Y C I N A K A D V L F I A P R E P G A V S Y

5.359 carboxymethylenebutenolidase [Homo sapiens]

Protein Accession gi|20270371
Mean Expression Ratio 0.995
Median Expression Ratio 0.997
Credible Interval (0.672, 1.45)
Associated Peptides 3
Associated Spectra 3
Coverage 0.135

| A | 2.5 | 50 | 97.5 | Sequence |
|---|-----|----|------|----------|
| 1 | 0.64 | 1 | 1.6 | A VIVIQDIFGWQLPNTR |
| 1 | 0.63 | 1 | 1.5 | EVQVEHIK |
| 1 | 0.63 | 0.98 | 1.5 | NLIEWLNK |

1 M A N E A Y P C P C D I G H R L E Y G G L G R E V Q V E H I K A Y V T K S P V D A G K A V I V I Q D I F G W Q L P N T R Y I A D M I S G N G Y T T I V P D F F V

81 G Q E P W D P S G D W S I F P E W L K T R N A Q K I D R E I S A I L K Y L K Q Q C H A Q K I G I V G F C W G G T A V H H L M M K Y S E F R A G V S V Y G I V K D

161 S E D I Y N L K N P T L F I F A E N D V V I P L K D V S L L T Q K L K E H C K V E Y Q I K T F S G Q T H G F V H R K R E D C S P A D K P Y I D E A R R N L I E W

241 LNKYM

5.360 major histocompatibility complex, class II, DR beta 3 precursor [Homo sapiens]

Protein Accession gi|17986005
Mean Expression Ratio 1
Median Expression Ratio 1
Credible Interval (0.679, 1.47)
Associated Peptides 2
Associated Spectra 3
Coverage 0.094

| A | 2.5 | 50 | 97.5 | Sequence |
|---|-----|----|------|----------|
| 1 | 0.66 | 1.0 | 1.6 | SVSGFYPGSIEVR |

(continued)

| A | 2.5 | 50 | 97.5 | Sequence |
|---|-----|----|------|----------|
| 2 | 0.66 | 1 | 1.5 | TQPLQHHNLLVCSVSGFYPGSIEVR |

1 MVCLKLPGGSSLAALTVTLMVLSSRLAFAGDTRPRFLELR KSECHFFNGTERVRYLDRYFHNQEEFLRFDSDVGEYRAVT

81 ELGRPVAESWNSQKDLLEQKRGRVDNYCRHNYGVGESFT VQRRVHPQVTVYPAKTQPLQHHNLLVCSVSGFYPGSIEVRW

161 FRNGQEEKAGVVSTGLIQNGDWTFQTLVMLETVPRSGEV YTCQVEHPSVTSALTVEWRARSESAQSKMLSGVGGFVLGLL

241 FLGAGLFIYFRNQKGHSGLQPTGFLS

5.361 hypothetical protein LOC51571 [Homo sapiens]

Protein Accession gi|42734438
Mean Expression Ratio 0.997
Median Expression Ratio 1
Credible Interval (0.648, 1.52)
Associated Peptides 2
Associated Spectra 2
Coverage 0.0988

| A | 2.5 | 50 | 97.5 | Sequence |
|---|-----|----|------|----------|
| 1 | 0.61 | 1 | 1.6 | INNVP AEGENEVNNELANR |
| 1 | 0.63 | 1 | 1.6 | DAEGILEDLQSYR |

1 MGNLLKVLTCTDLEQGPNFFLDFENAQPTESEKEIYNQVN VVLKDAEGILEDLQSYRGAGHEIREAIQHPADEKLQEKAW

81 GAVVPLVGKLKKFYEFSQRLEAALRGLLGALTSTPYSPTQ HLEREQALAKQFAEILHFTLRFDELKMTNPAIQNDFSYYR

161 RTLSRMRINNVPAEGENEVNNELANRMSLFYAEATPMLK TLSDATTKFVSENKNLPIENTTDCLSTMASVCRVMLETPEY

241 RSRFTNEETVSFCLRVMVGVIILYDHVHPVGAFAKTSKID MKGCIKVLKDQPPNSVEGLLNALRYTTKHLNDETTS

## III. Renal Status Assay Measurments

The ability of a particular renal biomarker assay measurement to distinguish between two populations can be established using ROC analysis. For example, ROC curves established from a "first" subpopulation (i.e., for example, a population predisposed to one or more future changes in renal status) and a "second" subpopulation (i.e., for example, a population not predisposed to one or more future changes in renal status). Calculation of these ROC curves and establising the area under these ROC curves quantitate the predictive power of the specific assay measurement. In some embodiments, predictive power established by assay measurements described herein comprise an AUC ROC greater than 0.5, preferably at least 0.6, more preferably 0.7, still more preferably at least 0.8, even more preferably at least 0.9, and most preferably at least 0.95.

## A. Immunoassays

In general, immunoassays involve contacting a sample containing, or suspected of containing, a biomarker of interest with at least one antibody that specifically binds to the biomarker. A detectable signal is then generated indicative of the presence or amount of complexes formed by the binding of polypeptides in the sample to the antibody. The detectable signal is then related to the presence or amount of the biomarker in the sample. Numerous methods and devices have been reported regarding the detection and analysis of biological biomarkers. See, e.g., U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, each of which is herein incorporated by reference in its entirety, including all tables, figures and claims.

Numerous immunoassay devices and methods can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. See, e.g., U.S. Patents 5,631,171; and 5,955,377, each of which is herein incorporated by reference in its entirety, including all tables, figures and claims. Robotic instrumentation for performing these immunoassays are commercially available including, but not limited to, Beckman ACCESS®, Abbott AXSYM®, Roche ELECSYS®, Dade Behring STRATUS® systems. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in immunoassays. Solid phases that may be used to immobilize specific binding members include, but are not limited to those developed and/or used as solid phases in solid phase binding assays. Examples of suitable solid phases include, but are not limited to, membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. For example, an assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

In certain embodiments, a urinary renal biomarker assay method comprises an immunoassay. For example, antibodies for use in such assays may specifically bind an epitope of a renal biomarker of interest, and may also bind one or more polypeptides that are "related" thereto, as that term is defined hereinafter. In one embodiment, the renal biomarker of interest is a fully length marker (i.e., for example, a protein). In one embodiment, the renal biomarker of interest is a protein fragment marker (i.e., for example, a peptide). Numerous immunoassay formats are available compatible with body fluid samples including, but not limited to, urine, blood, serum, saliva, tears, and plasma.

In this regard, detectable signals obtained from an immunoassay may be a direct result of complexes formed between one or more antibodies and the target biomolecule (i.e., for example, an analyte) and polypeptides containing the necessary epitope(s) to which the antibodies bind. While such assays may detect the full length biomarker and the assay result be expressed as a concentration of a biomarker of interest, the signal from the assay may actually be a result of all such "immunoreactive" polypeptides present in the sample. Expression of biomarkers may also be determined by means other than immunoassays, including protein measurements (i.e., for example, dot blots, western blots, chromatographic methods, mass spectrometry, etc.) and nucleic acid measurements (mRNA quantitation). This list is not meant to be limiting.

The foregoing method steps should not be interpreted to mean that the renal biomarker assay measurements is/are used in isolation in the methods described herein. Rather, additional variables or other clinical indicia may be included in the methods described herein. For example, risk stratification, diagnostic, classification, monitoring, etc. methods as described herein may be combined with one or more clinical indicia relevant to the patient population including, but not limited to, demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more renal biomarker assay measurements are described hereinafter. In: Harrison's Principles of Internal Medicine, 17th Ed., McGraw Hill, New York, pages 1741-1830; and In: Current Medical Diagnosis & Treatment 2008, 47th Ed, McGraw Hill, New York, pages 785-815, each of which are herein incorporated by reference in their entirety.

When more than one biomarker is measured, the individual biomarkers may be measured in samples obtained at the same time, or may be determined from samples obtained at different (e.g., an earlier or later) times. The individual biomarkers may also be measured on the same or different body fluid samples. For example, one renal biomarker may be measured in a serum or plasma sample and another renal biomarker may be measured in a urine sample. In addition, assignment of a likelihood may combine a renal biomarker assay measurement with temporal changes in one or more additional variables.

### B. Detectable Labels

Generation of a detectable signal from the detectable label can be performed using various optical, acoustical, and electrochemical methods. Examples of detection modes include, but are not limited to, fluorescence, radiochemical detection, reflectance, absorbance, amperometry, conductance, impedance, interferometry, ellipsometry, etc. In certain of these methods, the solid phase antibody may be coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

Biological assays require methods for detection, and one of the most common methods for quantitation of assay measurements is to conjugate a detectable label to a protein or nucleic acid that has affinity for one of the components in the biological system being studied. Detectable labels used in the immunoassays described above may include, but are not limited to, molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents may involve at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups and are believed to react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

### D. Assay Correlations

In some embodiments, the renal biomarker assay measurement is/are correlated to one or more future changes in renal function. In one embodiment, risk stratification comprises determining a subject's likelihood (i.e., for example, probability) for a future improvement in renal function.

In one embodiment, the renal biomarker assay measurement is/are correlated to a likelihood of such a future improvement in renal function. In one embodiment, the method correlates a likelihood of such a future injury to renal function. In one embodiment, the risk stratification comprises determining a subject's risk for progression to acute renal failure (ARF).

In one embodiment, the renal biomarker assay measurement is/are correlated to a likelihood of such progression to acute renal failure (ARF). In one embodiment, the risk stratification method comprises determining a subject's outcome risk.

In one embodiment, the assay measurement is/are correlated to a likelihood of the occurrence of a clinical outcome related to a renal injury suffered by the subject.

Consequently, the measured concentration value(s) may each be compared to a threshold value, wherein either a "positive going kidney injury marker", or a "negative going kidney injury marker" is identified. In one embodiment, the risk stratification comprises determining a subject's risk for future reduced renal function. In some embodiments, the method assigns a likelihood, risk, or probability that such that an event of interest is more or less likely to occur within 180 Days of the time at which the body fluid sample is obtained from the subject. In some embodiments, the assigned likelihood, risk, or probability relates to an event of interest occurring within a time period including, but not limited to, 18 months, 120 Days, 90 Days, 60 Days, 45 Days, 30 Days, 21 Days, 14 Days, 7 Days, 5 Days, 96 hours, 72 hours, 48 hours, 36 hours, 24 hours, 12 hours, or less. Alternatively, assigning a risk at 0 hours of the time at which the body fluid sample is obtained from the subject is equivalent to diagnosis of a current condition.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, a cost/benefit analysis is involved in selecting a diagnostic threshold.

1. Thresholds

Suitable thresholds may be determined in a variety of ways. For example, one recommended diagnostic threshold for the diagnosis of acute myocardial infarction uses cardiac troponin, wherein the diagnostic threshold is set at the 97.5th percentile of the cardiac troponin concentration measured in a normal population. Another method to determine a diagnostic threshold comprises measuring serial samples from the same patient, where a prior "baseline" result is used to monitor for temporal changes in a biomarker level.

Population studies may also be used to select thresholds. For example, Reciever Operating Characteristic ("ROC") arose from the field of signal dectection theriory developed during World War II for the analysis of radar images, and ROC analysis is often used to select a threshold to distinguish a "diseased" subpopulation from a "nondiseased" subpopulation. Predictive power balances the occurences of false positives (i.e., for example, when the person tests positive, but actually does not have the disease) and false negatives (i.e., for example, when the person tests negative, suggesting they are healthy, when they actually do have the disease). To draw a ROC curve, the true positive rate (TPR) and false positive rate (FPR) are determined as the decision threshold is varied continuously. Since TPR is equivalent with sensitivity and FPR is equal to (1 - specificity), the ROC graph is sometimes called the sensitivity vs (1 - specificity) plot. A perfect test will have an area under the ROC curve of 1.0; a random test will have an area of 0.5. A threshold value is selected to provide an acceptable level of specificity and sensitivity usually determined by summing specificity values with sensitivity values. Consequently, the larger the calculated threshold value the greater the predicitive power of the specific assay measurement under analysis.

In this context, "diseased" is meant to refer to a population having one characteristic (i.e., for example, the presence of a disease or condition or the occurrence of some outcome) and "nondiseased" population lacking the same characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold value comparisons, other methods for correlating assay measurements to a patient classification (i.e., for example, occurrence or nonoccurrence of disease, likelihood of an outcome, etc.) include, but are not limited to, decision trees, rule sets, Bayesian methods, and neural network methods. These methods can produce probability values representing the degree to which a subject or patient belongs to one classification out of a plurality of classifications.

Multiple thresholds may also be used to assess renal status in a subject and/or patient. For example, a multiple thresholding method may combine a "first" subpopulation which is predisposed to one or more future changes in renal status, the occurrence of an injury, a classification, etc., with a "second" subpopulation which is not so predisposed into a single group. This combination group is then subdivided into three or more equal parts (i.e., for example, tertiles, quartiles, quintiles, etc., depending on the number of subdivisions). An odds ratio is assigned to subjects based on which subdivision they fall into. If one considers a tertile embodiment, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

2. Specificity And Sensitivity

In some embodiments, a measured concentration of one or more renal biomarkers, or a composite of such biomarkers, may be treated as continuous variables. For example, any particular biomarker concentration can be converted into a corresponding probability of a future reduction in renal function for the subject, the occurrence of an injury, a classification, etc. Alternatively, a threshold value can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed.

In one embodiment, a threshold value is selected to separate a first and a second population by one or more of the following measures of test accuracy:

i) an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

ii) a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

iii) a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

iv) at least about 75% sensitivity, combined with at least about 75% specificity; a positive likelihood ratio (calculated as sensitivity/(1-specificity)) of greater than 1, at least about 2, more preferably at least about 3, still more preferably at least about 5, and most preferably at least about 10; or

v) a negative likelihood ratio (calculated as (1-sensitivity)/specificity) of less than 1, less than or equal to about 0.5, more preferably less than or equal to about 0.3, and most preferably less than or equal to about 0.1.

Various measures of test accuracy have been reported and used to determine the effectiveness of a given biomarker. Fischer et al., Intensive Care Med. 29:1043-1051 (2003). These accuracy measures include, but are not limited to, sensitivity and specificity, predictive values, likelihood ratios, diagnostic odds ratios, and AUC ROC values. For example, AUC ROC values are equal to the probability that a classifier will rank a randomly chosen positive instance higher than a randomly chosen negative one. Consequently, an AUC ROC value may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity;

a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1.

## G. Conventional Renal Diagnostics

As noted above, the terms "acute renal (or kidney) injury" and "acute renal (or kidney) failure" as used herein are generally defined, in part, in terms of changes in serum creatinine from a baseline value. Most conventional definitions of ARF have common elements, including but not limited to the use of serum creatinine and, often, urine output. Patients may present with renal dysfunction without an available baseline measure of renal function for use in this comparison. In such an event, one may estimate a baseline serum creatinine value by assuming the patient initially had a normal GFR.

1.. Glomerular Filtration Rate And Creatinine

Glomerular filtration rate (GFR) is generally definded as the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:
By normalizing the GFR to the body surface area, a GFR of approximately 75-100 ml/min per 1.73 m$^2$ can be assumed. The rate therefore measured is the quantity of the substance in the urine that originated from a calculable volume of blood.
There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.
Creatinine clearance (CCr) can be calculated if values for creatinine's urine concentration (UCr), urine flow rate (V), and creatinine's plasma concentration (PCr) are known. Since the product of urine concentration and urine flow rate yields creatinine's excretion rate, creatinine clearance is also said to be its excretion rate (UCr×V) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m$^2$. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a mL/kg/hr basis, hourly urine collection and measurement is adequate. In the case where, for example, only a cumulative 24-h output was available and no patient weights are provided, minor modifications of the RIFLE urine output criteria have been described. For example, some have assumed an average patient weight of 70 kg, wherein patients are assigned a RIFLE classification based on the following: <35 mL/h (Risk), <21 mL/h (Injury) or <4 mL/h (Failure). Bagshaw et al., Nephrol. Dial. Transplant. 23:1203-1210 (2008).

2. Treatment Regimen Selection

Once a renal diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. Various appropriate treatments for numerous diseases have been previously discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the renal biomarkers of the present invention may be used to monitor a course of treatment. For example, an improved prognostic state or a worsened prognostic state may indicate that a particular treatment is or is not efficacious.

**IV. Antibodies**

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In some embodiments, antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity may be calculated as $K_d = k_{off}/k_{on}$ ($k_{off}$ is the dissociation rate constant, $K_{on}$ is the association rate constant and Kd is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. See, e.g., van Erp et al., J. Immunoassay 12:425-443 (1991); and Nelson et al., Comput. Methods Programs Biomed. 27: 65-68 (1988).

Numerous publications discuss the use of phage display technology to produce and screen libraries of polypeptides for binding to a selected analyte. See, e.g, Cwirla et al., Proc. Natl. Acad. Sci. USA 87: 6378-6382 (1990); Devlin et al., Science 249:404-406 (1990); Scott et al., Science 249:386-388 (1990); and Ladner et al., U.S. Pat. No. 5,571,698 (all references herein incorporated by reference). A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. See, e.g., U.S. Patent No. 6,057,098, which is hereby incorporated in its entirety, including all tables, figures, and claims.

Antibodies generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

Antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

**V. Kits**

In some embodiments, the present invention also contemplates devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

In some embodiments, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

**Experimental**

Example I

BioMaRK Data Collection Method

*Patients And Study Design*

BioMaRK was an observational cohort study conducted as an ancillary study to the Veterans Affairs/National Institutes of Health (VA/NIH) Acute Renal Failure Trial Network study (ATN study). The ATN study was a multicenter, prospective trial of two strategies for renal replacement therapy in critically ill patients with acute kidney injury. Coca et al., "Biomarkers for the diagnosis and risk stratification of acute kidney injury: a systematic review" Kidney Int, 73:1008-1016 (2008). Adult patients (18 years or older) with AKI and requiring renal-replacement therapy (RRT), as well as failure of one or more non-renal organ systems or sepsis were eligible. As a sub-study to the ATN study, 109 patients were enrolled at The University of Pittsburgh Medical Center, The VA Pittsburgh Healthcare System, The Cleveland Clinical Foundation, The University of Texas Health Science Center at Houston, and Washington University Medical Center to undergo serial blood and urine sampling. Incomplete data including unavailability of urine samples precluded inclusion of 33 subjects; consequently the remaining 76 formed the analysis cohort. Approval from the Institutional Review Boards was received from the University of Pittsburgh and all participating sites.

*Data Collection/Laboratory Measurements*

Medical records of study participants were prospectively reviewed to retrieve hospitalization data including baseline demographic characteristics, serial renal function, daily urine volume, and severity of illness scores. The presence of sepsis was defined by international consensus criteria. Levy et al., "2001 SCCM/ESICM/ACCP/ATS/SIS International Sepsis Definitions Conference" Crit Care Med, 31:1250-1256 (2003). Recovery of renal function was defined by survival and dialysis independence at Day 60 post AKI. For purposes of primary analysis, partial recovery (i.e. failure to return to baseline renal function but free of dialysis) was included in the recovery group. Similarly, all deaths were included in the non-recovery group.

Fresh urine samples were obtained on Days 1, 7, and 14 post AKI. Immediately upon obtaining a well-mixed 30 ml sample, a protease inhibitor tablet (Roche Diagnostics Corporation, IN, USA) was added. After processing, the sample was frozen (at -80°C) until analyzed. Samples were assayed in duplicate, and data were analyzed using Bio-Rad Bio-Plex Manager Software (version 4.1). Urine creatinine concentrations were measured using a non-enzymatic assay (DICT-500, BioAssay Systems, CA, USA).

Example II

Proteomics Analysis

Urine collected in accordance with Example I from fourteen (14) patients with severe AKI was evaluated with an unbiased proteomics discovery platform.

Data collected from seven (7) patients that did not recover renal function after AKI was compared to data collected from seven (7) patients that did recover renal function after AKI. The two groups were matched for age (e.g., +/- 5 yrs) and gender.

The data presented herein show that approximately thirty (30) proteins were differentially expressed between the Recovery Group and the Non-Recovery Group. A preliminary analysis has categorized these proteins into groups including, but not limited to:

1. Ferritin, alpha and beta globin, or catalase that may be involved in providing protection from reactive oxygen species
2. Complement factor H or complement component 4 BP that may be involved in regulation of complement activation
3. Olfactomedin-4, leucine rich alpha-2 glycoprotein or ring finger protein 167 that may be involved in cell survival and proliferation.
4. Inter-alpha globulin inhibitor H4, heparan sulfate proteoglycan 2, (N-acylsphingosine aminohydrolase and saposin) that may be involved in basement membrane, matrix proteins or sphingolipid turnover.

Although it is not necessary to understand the mechanism of an invention, it is believed that the differential expression of proteins categorized in Group 3 and Group 4 might be directly involved in renal recovery because of their involvement with cell proliferation and/or rebuilding of the basement membrane.

The present invention will now be further defined with reference to the following numbered clauses:

1. A composition comprising an renal injury biomarker, wherein said biomarker comprises at least a fragment of a protein selected from the group consisting of ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preproprotien, carbonic anhydrase, and uromodulin precursor.

2. The composition of Clause 1, wherein said composition further comprises a urine sample.

3. The composition of Clause 2, wherein said urine sample is collected between 1 day and 14 days after a kidney injury.

4. The composition of Clause 2, wherein said urine sample is a human urine sample.

5. The composition of Clause 1, wherein said biomarker is at least 2.5 fold higher as compared to an expected level in a renal recovery group.

6. The composition of Clause 1, wherein said biomarker is at least 2.0 fold higher as compared to an expected level in a renal recovery group.

7. The composition of Clause 1, wherein said biomarker is at least 1.5 fold higher as compared to an expected level in a renal recovery group.

8. The composition of Clause 1, wherein said biomarker is at least 1.25 fold higher as compared to an expected level in a renal recovery group.

9. The composition of Clause 1, wherein said biomarker is at least 2.5 fold lower as compared to an expected level in a renal recovery group.

14. The composition of Clause 9, wherein said biomarker is at least 2.0 fold lower as compared to an expected level in a renal recovery group.

15. The composition of Clause 9, wherein said biomarker is at least 1.5 fold lower as compared to an expected level in a renal recovery group.

16. The composition of Clause 9, wherein said biomarker is at least 1.25 fold lower as compared to an expected level in a renal recovery group.

17. A method, comprising:

a) providing;

i) a patient exhibiting at least one symptom of an acute renal injury; and
ii) a biological fluid sample obtained from said patient, wherein said sample comprises a renal injury biomarker;

b) measuring a renal recovery biomarker value;
c) comparing said said renal biomarker value to an expected value from a renal recovery group; and
d) predicting a probability of renal recovery for said patient based upon said comparison.

18. The method of Clause 17, wherein said probability of renal recovery is greater than 90%.

19. The method of Clause 17, wherein said probability of renal recovery is greater than 75%.

20. The method of Clause 17, wherein said probability of renal recovery is greater than 50%.

21. The method of Clause 17, wherein said probability of renal recovery is less than 50%.

22. The method of Clause 17, wherein said probability of renal recovery is less than 25%.

23. The method of Clause 17, wherein said probability of renal recovery is less than 10%.

24. The method of Clause 17, wherein said biomarker comprises at least a fragment of a protein selected from the group consisting of ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preprotien, carbonic anhydrase, and uromodulin precursor.

25. A kit, comprising:

a) a first container comprising an antibody specifically directed to an renal injury biomarker, wherein said biomarker comprises at least a fragment of a protein selected from the group consisting of ferritin, beta globin, catalase, alpha globin, epidermal growth factor receptor pathway substrate 8, mucin isoform precursor, ezrin, delta globin, moesin, phosphoprotein isoform, annexin A2, myoglobin, hemopexin, serine proteinase inhibitor, serpine peptidase inhibitor, CD14 antigen precursor, fibronectin isoform preprotein, angiotensinogen preprotein, complement component precursor, carbonic anhydrase, uromodulin precursor, complement factor H, complement component 4 BP, heparan sulfate proteoglycan 2, olfactomedian-4, leucine rich alpha-2 glycoprotein, ring finger protein 167, inter-alpha globulin inhibitor H4, heparan sulfate proteoglycan 2, N-acylshingosine aminohydrolase, serine proteinase inhibitor clade A member 1, mucin 1, clusterin isoform 1, brain abundant membrane attached signal protein 1, dipeptidase 1, fibronectin 1 isoform 5 preprotein, angiotensinogen preproprotien, carbonic anhydrase, and uromodulin precursor;
b) instructions for determining whether said biomarker is overexpressed as compared to an expected value from a renal recovery group;
c) instructions for determining whether said biomarker is underexpressed as compared to an expected value from a renal recovery group; and
d) instructions for determining the probability of renal recovery.

38. The kit of Clause 23, wherein said antibody is a monoclonal antibody.

39. The kit of Clause 24, wherein said monoclonal antibody is specifically directed to said biomarker protein fragment.

**Claims**

1. A method for the prediction of a probability of renal recovery of a patient exhibiting at least one symptom of an acute renal injury, comprising:

a) measuring in vitro, in a sample of human urine from the patient, a value of at least one fragment of a detectable protein biomarker selected from the group consisting of moesin, ferritin, alpha and beta globulin, catalase, complement factor H, complement component 4 BP, olfactormedin-4, leucine rich alpha-2 glycoprotein, ring finger protein 167, inter-alpha globulin inhibitor H4, heparan sulphate proteoglycan 2, and N-acylsphingosine aminohydrolase;
b) comparing said value of at least one fragment of said detectable protein biomarker to at least one predetermined threshold value of a protein biomarker selected from the group consisting of moesin, ferritin, alpha and beta globulin, catalase, complement factor H, complement component 4 BP, olfactormedin-4, leucine rich alpha-2 glycoprotein, ring finger protein 167, inter-alpha globulin inhibitor H4, heparan sulphate proteoglycan 2, and N-acylsphingosine aminohydrolase from a renal recovery group that survived an acute renal injury, recovered renal function and is dialysis independent at day 60 post acute renal injury; and
c) predicting a probability of renal recovery of said patient based upon said comparison.

2. The method of Claim 1, wherein said at least one fragment of said detectable protein biomarker is a surrogate for said protein biomarker.

3. A method for the prediction of a probability of renal recovery of a patient exhibiting at least one symptom of an acute renal injury, comprising:

a) measuring in vitro, in a sample of human urine from the patient, a value of at least one fragment of a detectable protein biomarker comprising at least one fragment of a moesin detectable protein biomarker;
b) comparing said value of said at least one fragment of said moesin detectable protein biomarker to at least one predetermined threshold value of a moesin protein biomarker from a renal recovery group that survived an acute renal injury, recovered renal function and is dialysis independent at day 60 post acute renal injury; and
c) predicting a probability of renal recovery of said patient based upon said comparison.

4. The method of Claim 3, wherein said at least one fragment of said detectable protein biomarker is further selected from the group consisting of a ferritin, alpha and beta globulin, catalase, complement factor H, complement component 4 BP, olfactormedin-4, leucine rich alpha-2 glycoprotein, ring finger protein 167, inter-alpha globulin inhibitor H4, heparan sulphate proteoglycan 2, and N-acylsphingosine aminohydrolase.

5. The method of Claim 3, wherein said at least one predetermined threshold value is of a protein biomarker further selected from the group consising of a ferritin, alpha and beta globulin, catalase, complement factor H, complement component 4 BP, olfactormedin-4, leucine rich alpha-2 glycoprotein, ring finger protein 167, inter-alpha globulin inhibitor H4, heparan sulphate proteoglycan 2, and N-acylsphingosine aminohydrolase.

6. The method of Claim 5, wherein said at least one fragment of said detectable protein biomarker is a surrogate for said protein biomarker.

Figure 1

1/7

Arthur A vs B

| 0.25 | 0.5 | 1 | 2 | 4 |
| --- | --- | --- | --- | --- |

| Peptides | Spectra | Coverage | Protein |
| --- | --- | --- | --- |
| 7 | 29 | 0.51 | beta globin [Homo sapiens] |
| 13 | 20 | 0.279 | catalase [Homo sapiens] |
| 3 | 5 | NaN | myoglobin [Homo sapiens]; myoglobin [Homo sapiens]; myoglobin [Homo sapiens] |
| 12 | 36 | NaN | alpha 1 globin [Homo sapiens]; alpha 2 globin [Homo sapiens] |
| 12 | 17 | 0.297 | hemopexin [Homo sapiens] |
| 27 | 68 | NaN | serine proteinase inhibitor, clade A, member 1 [Homo sapiens]; serine proteinase inhibitor, clade A, member 1 [Hom |
| 6 | 22 | NaN | mucin 1 isoform 3 precursor [Homo sapiens]; mucin 1 isoform 2 precursor [Homo sapiens]; mucin 1 isoform 1 prec |
| 15 | 21 | 0.388 | serpin peptidase inhibitor, clade A, member 3 precursor [Homo sapiens] |
| 9 | 16 | NaN | CD14 antigen precursor [Homo sapiens]; CD14 antigen precursor [Homo sapiens] |
| 5 | 7 | 0.396 | brain abundant, membrane attached signal protein 1 [Homo sapiens] |
| 5 | 6 | NaN | dipeptidase 1 [Homo sapiens]; dipeptidase 1 [Homo sapiens] |
| 11 | 17 | 0.0758 | fibronectin 1 isoform 5 preproprotein [Homo sapiens] |
| 10 | 25 | 0.293 | angiotensinogen preproprotein [Homo sapiens] |
| 39 | 69 | 0.301 | complement component 3 precursor [Homo sapiens] |
| 13 | 28 | NaN | carbonic anhydrase I [Homo sapiens]; carbonic anhydrase I [Homo sapiens]; carbonic anhydrase I [Homo sapiens]; |
| 25 | 50 | NaN | uromodulin precursor [Homo sapiens]; uromodulin precursor [Homo sapiens] |
| 9 | 16 | 0.0239 | heparan sulfate proteoglycan 2 [Homo sapiens] |
| 9 | 12 | 0.194 | clusterin isoform 1 [Homo sapiens] |
| 7 | 8 | NaN | ezrin [Homo sapiens]; ezrin [Homo sapiens] |
| 3 | 4 | 0.306 | delta globin [Homo sapiens] |
| 14 | 27 | 0.291 | fibrinogen, beta chain preproprotein [Homo sapiens] |
| 5 | 9 | 0.109 | moesin [Homo sapiens] |
| 12 | 22 | 0.216 | transferrin [Homo sapiens] |
| 4 | 5 | NaN | secreted phosphoprotein 1 isoform c [Homo sapiens]; secreted phosphoprotein 1 isoform a [Homo sapiens]; secret |
| 8 | 14 | 0.213 | pancreatic amylase alpha 2A precursor [Homo sapiens] |
| 3 | 5 | 0.129 | annexin A2 isoform 1 [Homo sapiens] |
| 5 | 5 | 0.104 | complement component 4 binding protein, alpha chain precursor [Homo sapiens] |
| 65 | 259 | 0.744 | albumin preproprotein [Homo sapiens] |
| 9 | 11 | 0.153 | epidermal growth factor receptor pathway substrate 8 [Homo sapiens] |
| 27 | 30 | 0.073 | apolipoprotein B precursor [Homo sapiens] |
| 3 | 5 | 0.146 | peroxiredoxin 2 isoform a [Homo sapiens] |
| 2 | 3 | 0.0285 | sphingomyelin phosphodiesterase 1, acid lysosomal isoform 1 precursor [Homo sapiens] |
| 3 | 4 | 0.224 | proteasome beta 2 subunit [Homo sapiens] |
| 9 | 13 | NaN | manganese superoxide dismutase isoform A precursor [Homo sapiens]; manganese superoxide dismutase isoform |
| 6 | 17 | 0.49 | transthyretin [Homo sapiens] |
| 9 | 13 | NaN | haptoglobin isoform 1 preproprotein [Homo sapiens]; haptoglobin isoform 2 preproprotein [Homo sapiens] |
| 2 | 2 | 0.0327 | fibulin 1 isoform D [Homo sapiens] |
| 5 | 5 | 0.194 | serine (or cysteine) proteinase inhibitor, clade C (antithrombin), member 1 [Homo sapiens] |
| 5 | 5 | 0.141 | hypothetical protein LOC148362 [Homo sapiens] |
| 9 | 21 | 0.41 | ferritin, heavy polypeptide 1 [Homo sapiens] |
| 3 | 4 | NaN | serum amyloid A1 preproprotein [Homo sapiens]; serum amyloid A1 preproprotein [Homo sapiens] |
| 4 | 10 | 0.319 | programmed cell death 6 [Homo sapiens] |
| 8 | 12 | NaN | complement component 1 inhibitor precursor [Homo sapiens]; complement component 1 inhibitor precursor [Homo |
| 3 | 3 | NaN | chromatin modifying protein 5 [Homo sapiens] |
| 4 | 5 | 0.146 | vacuolar protein sorting factor 4B [Homo sapiens] |
| 9 | 15 | 0.158 | prominin 1 [Homo sapiens] |
| 6 | 7 | 0.0353 | alpha 3 type VI collagen isoform 1 precursor [Homo sapiens] |
| 10 | 13 | NaN | actin, gamma 1 propeptide [Homo sapiens]; beta actin [Homo sapiens] |
| 2 | 3 | 0.0388 | solute carrier family 5 (sodium/glucose cotransporter), member 12 [Homo sapiens] |
| 4 | 5 | NaN | annexin A11 [Homo sapiens]; annexin A11 [Homo sapiens]; annexin A11 [Homo sapiens] |
| 3 | 3 | 0.147 | chromatin modifying protein 4B [Homo sapiens] |
| 2 | 4 | 0.242 | fatty acid binding protein 4, adipocyte [Homo sapiens] |
| 2 | 2 | NaN | proteasome alpha 3 subunit isoform 1 [Homo sapiens]; proteasome alpha 3 subunit isoform 2 [Homo sapiens] |
| 5 | 6 | NaN | gelsolin isoform a precursor [Homo sapiens]; gelsolin isoform b [Homo sapiens]; gelsolin isoform c [Homo sapiens |
| 12 | 20 | 0.192 | programmed cell death 6 interacting protein [Homo sapiens] |
| 5 | 8 | 0.0954 | prosaposin isoform a preproprotein [Homo sapiens] |
| 16 | 66 | 0.634 | ferritin, light polypeptide [Homo sapiens] |
| 19 | 57 | 0.369 | galectin 3 binding protein [Homo sapiens] |
| 3 | 4 | 0.276 | peptidoglycan recognition protein 1 [Homo sapiens] |
| 5 | 12 | NaN | ubiquitin and ribosomal protein S27a precursor [Homo sapiens]; ubiquitin and ribosomal protein S27a precursor [H |
| 3 | 8 | 0.282 | hypothetical protein LOC169693 [Homo sapiens] |
| 2 | 3 | 0.0921 | CD9 antigen [Homo sapiens] |
| 2 | 2 | 0.047 | solute carrier family 5 (sodium/glucose cotransporter), member 10 isoform 2 [Homo sapiens] |
| 2 | 2 | NaN | sorting nexin 18 isoform a [Homo sapiens]; sorting nexin 18 isoform b [Homo sapiens] |
| 2 | 4 | NaN | syntenin isoform 1 [Homo sapiens]; syntenin isoform 1 [Homo sapiens] |
| 25 | 39 | 0.268 | glutamyl aminopeptidase (aminopeptidase A) [Homo sapiens] |
| 2 | 3 | 0.0947 | guanine nucleotide binding protein (G protein), q polypeptide [Homo sapiens] |

**Figure 2**

| Peptides | Spectra | Coverage | Protein |
|---|---|---|---|
| 2 | 3 | NaN | syndecan 1 precursor [Homo sapiens]; syndecan 1 precursor [Homo sapiens] |
| 3 | 4 | 0.0782 | copine III [Homo sapiens] |
| 3 | 5 | NaN | annexin VII isoform 2 [Homo sapiens]; annexin VII isoform 1 [Homo sapiens] |
| 10 | 14 | 0.0461 | cubilin [Homo sapiens] |
| 2 | 3 | 0.0947 | guanine nucleotide binding protein (G protein), alpha 11 (Gq class) [Homo sapiens] |
| 3 | 4 | NaN | proteasome alpha 1 subunit isoform 2 [Homo sapiens]; proteasome alpha 1 subunit isoform 1 [Homo sapiens] |
| 9 | 11 | 0.172 | myeloperoxidase [Homo sapiens] |
| 2 | 4 | NaN | solute carrier family 5 (iodide transporter), member 8 [Homo sapiens] |
| 2 | 2 | NaN | ras homolog gene family, member C precursor [Homo sapiens]; ras homolog gene family, member C precursor [Hor |
| 8 | 10 | 0.0812 | complement factor H isoform a precursor [Homo sapiens] |
| 7 | 9 | 0.234 | flotillin 1 [Homo sapiens] |
| 2 | 3 | 0.0841 | WIRE protein [Homo sapiens] |
| 3 | 3 | 0.0833 | putative MAPK activating protein PM28 [Homo sapiens] |
| 8 | 15 | 0.238 | flotillin 2 [Homo sapiens] |
| 15 | 29 | 0.456 | cathepsin D preproprotein [Homo sapiens] |
| 2 | 3 | 0.358 | hypothetical protein LOC58527 [Homo sapiens] |
| 3 | 3 | 0.141 | annexin 5 [Homo sapiens] |
| 3 | 3 | 0.293 | profilin 1 [Homo sapiens] |
| 2 | 2 | NaN | cortactin isoform b [Homo sapiens]; cortactin isoform a [Homo sapiens] |
| 8 | 10 | NaN | solute carrier family 3, member 1 [Homo sapiens] |
| 5 | 7 | NaN | cell division cycle 42 isoform 1 [Homo sapiens]; cell division cycle 42 isoform 1 [Homo sapiens] |
| 3 | 3 | 0.249 | vacuolar protein sorting 28 isoform 1 [Homo sapiens] |
| 3 | 3 | 0.0415 | mannosidase, alpha, class 2B, member 1 precursor [Homo sapiens] |
| 4 | 7 | 0.224 | chloride intracellular channel 1 [Homo sapiens] |
| 3 | 3 | 0.241 | complement factor D preproprotein [Homo sapiens] |
| 11 | 17 | 0.247 | scavenger receptor class B, member 2 [Homo sapiens] |
| 9 | 15 | NaN | prolylcarboxypeptidase isoform 1 preproprotein [Homo sapiens]; prolylcarboxypeptidase isoform 2 preproprotein [H |
| 2 | 3 | 0.078 | annexin I [Homo sapiens] |
| 2 | 2 | 0.0440 | immunoglobulin superfamily, member 8 [Homo sapiens] |
| 3 | 6 | 0.00833 | Fc fragment of IgG binding protein [Homo sapiens] |
| 2 | 3 | NaN | hypothetical protein LOC284422 [Homo sapiens] |
| 3 | 6 | NaN | annexin VI isoform 2 [Homo sapiens]; annexin VI isoform 1 [Homo sapiens] |
| 9 | 37 | 0.425 | cystatin C precursor [Homo sapiens] |
| 8 | 21 | 0.222 | tweety 3 [Homo sapiens] |
| 2 | 2 | 0.278 | hypothetical protein LOC84418 [Homo sapiens] |
| 2 | 2 | NaN | CD82 antigen isoform 2 [Homo sapiens]; CD82 antigen isoform 1 [Homo sapiens] |
| 6 | 13 | 0.207 | napsin A preproprotein [Homo sapiens] |
| 4 | 7 | 0.132 | acid phosphatase 2, lysosomal isoform 1 precursor [Homo sapiens] |
| 2 | 2 | 0.119 | apolipoprotein E precursor [Homo sapiens] |
| 3 | 8 | NaN | transmembrane protein 176B isoform b [Homo sapiens]; transmembrane protein 176B isoform a [Homo sapiens]; tr |
| 2 | 2 | NaN | mercaptopyruvate sulfurtransferase isoform 2 [Homo sapiens]; mercaptopyruvate sulfurtransferase isoform 2 [Hom |
| 2 | 2 | 0.0475 | mannosidase, alpha, class 1A, member 1 [Homo sapiens] |
| 2 | 2 | 0.109 | integral membrane protein 2B [Homo sapiens] |
| 4 | 8 | 0.112 | tetraspan 1 [Homo sapiens] |
| 9 | 23 | 0.344 | apolipoprotein D precursor [Homo sapiens] |
| 9 | 18 | 0.417 | stomatin isoform a [Homo sapiens] |
| 3 | 3 | 0.0937 | kininogen 1 isoform 2 [Homo sapiens] |
| 3 | 4 | 0.101 | guanine nucleotide binding protein (G protein), alpha inhibiting activity polypeptide 2 [Homo sapiens] |
| 39 | 112 | 0.657 | olfactomedin 4 precursor [Homo sapiens] |
| 3 | 3 | 0.150 | brain creatine kinase [Homo sapiens] |
| 3 | 4 | NaN | chromatin modifying protein 2A [Homo sapiens]; chromatin modifying protein 2A [Homo sapiens] |
| 2 | 2 | 0.0329 | solute carrier family 4, sodium bicarbonate cotransporter, member 4 isoform 2 [Homo sapiens] |
| 20 | 27 | 0.232 | ceruloplasmin precursor [Homo sapiens] |
| 4 | 4 | 0.186 | glycerophosphodiester phosphodiesterase domain containing 3 [Homo sapiens] |
| 4 | 4 | 0.0707 | glucuronidase, beta [Homo sapiens] |
| 2 | 2 | 0.0329 | solute carrier family 4, anion exchanger, member 1 [Homo sapiens] |
| 2 | 2 | NaN | arylsulfatase A isoform a precursor [Homo sapiens]; arylsulfatase A isoform a precursor [Homo sapiens]; arylsulfat |
| 2 | 2 | 0.0642 | keratin 9 [Homo sapiens] |
| 4 | 6 | 0.0923 | pyruvate kinase, muscle isoform M2 [Homo sapiens] |
| 3 | 3 | 0.0836 | coagulation factor II preproprotein [Homo sapiens] |
| 2 | 2 | 0.122 | proteasome alpha 6 subunit [Homo sapiens] |
| 2 | 3 | 0.0742 | tubulin, beta, 2 [Homo sapiens] |
| 4 | 5 | 0.0489 | solute carrier family 6, member 19 [Homo sapiens] |
| 2 | 2 | NaN | decay accelerating factor for complement isoform 2 precursor [Homo sapiens] |
| 54 | 111 | 0.483 | membrane alanine aminopeptidase precursor [Homo sapiens] |
| 3 | 3 | 0.106 | toll interacting protein [Homo sapiens] |
| 2 | 2 | 0.0593 | arylsulfatase F precursor [Homo sapiens] |

**Figure 2 (cont'd)**

| Peptides | Spectra | Coverage | Protein |
|---|---|---|---|
| 16 | 25 | 0.234 | polymeric immunoglobulin receptor [Homo sapiens] |
| 6 | 13 | NaN | cathepsin C isoform a preproprotein [Homo sapiens] |
| 2 | 5 | 0.144 | prostatic binding protein [Homo sapiens] |
| 4 | 4 | 0.144 | vacuolar protein sorting factor 4A [Homo sapiens] |
| 2 | 2 | 0.0996 | transmembrane protein 192 [Homo sapiens] |
| 3 | 3 | 0.09 | vitronectin precursor [Homo sapiens] |
| 7 | 13 | 0.0387 | von Willebrand factor preproprotein [Homo sapiens] |
| 2 | 3 | NaN | tripartite motif protein TRIM14 isoform alpha [Homo sapiens]; tripartite motif protein TRIM14 isoform alpha [Homo s |
| 3 | 3 | 0.0486 | ectonucleotide pyrophosphatase/phosphodiesterase 4 (putative function) [Homo sapiens] |
| 2 | 3 | NaN | PREDICTED: similar to kappa immunoglobulin (subgroup V kappa I) [Homo sapiens] |
| 8 | 15 | NaN | CD63 antigen isoform B [Homo sapiens]; CD63 antigen isoform A [Homo sapiens] |
| 2 | 3 | 0.061 | MARVEL domain containing 3 isoform 1 [Homo sapiens] |
| 4 | 5 | 0.0848 | sialin [Homo sapiens] |
| 2 | 2 | 0.0532 | tubulin, alpha 1a [Homo sapiens] |
| 8 | 14 | 0.212 | PDZ domain containing 1 [Homo sapiens] |
| 2 | 3 | NaN | target of myb1 isoform 1 [Homo sapiens]; target of myb1 isoform 2 [Homo sapiens] |
| 2 | 3 | 0.241 | cystin 1 [Homo sapiens] |
| 5 | 8 | 0.135 | CNDP dipeptidase 2 [Homo sapiens] |
| 2 | 2 | NaN | syntaxin binding protein 2 isoform b [Homo sapiens]; syntaxin binding protein 2 isoform a [Homo sapiens] |
| 2 | 2 | 0.120 | matrix metalloproteinase 7 preproprotein [Homo sapiens] |
| 3 | 6 | 0.0637 | keratin 1 [Homo sapiens] |
| 12 | 16 | 0.217 | lactotransferrin precursor [Homo sapiens] |
| 3 | 4 | 0.125 | aminoacylase 1 [Homo sapiens] |
| 9 | 9 | 0.207 | cathepsin A isoform a precursor [Homo sapiens] |
| 4 | 4 | NaN | pleckstrin homology domain containing, family B (evectins) member 2 isoform 3 [Homo sapiens]; pleckstrin homolo |
| 5 | 7 | 0.184 | enolase 1 [Homo sapiens] |
| 2 | 2 | NaN | hypothetical protein LOC64855 isoform 2 [Homo sapiens]; hypothetical protein LOC64855 isoform 1 [Homo sapiens |
| 2 | 2 | 0.0561 | urate anion exchanger 1 isoform a [Homo sapiens] |
| 3 | 4 | 0.109 | hypothetical protein LOC26970 [Homo sapiens] |
| 2 | 2 | NaN | chloride channel 7 isoform b [Homo sapiens]; chloride channel 7 isoform a [Homo sapiens] |
| 5 | 7 | 0.058 | solute carrier family 34 (sodium phosphate), member 2 [Homo sapiens] |
| 3 | 4 | 0.110 | vacuolar H+ATPase B2 [Homo sapiens] |
| 5 | 7 | 0.0745 | angiotensin I converting enzyme 2 precursor [Homo sapiens] |
| 3 | 10 | 0.145 | aquaporin 1 [Homo sapiens] |
| 4 | 6 | 0.170 | phospholipid scramblase 1 [Homo sapiens] |
| 4 | 4 | NaN | ATPase, H+ transporting, lysosomal V0 subunit a1 isoform c [Homo sapiens]; ATPase, H+ transporting, lysosomal V |
| 2 | 2 | 0.0763 | guanine nucleotide binding protein (G protein), alpha inhibiting activity polypeptide 3 [Homo sapiens] |
| 2 | 4 | 0.065 | dipeptidyl peptidase 7 preproprotein [Homo sapiens] |
| 3 | 4 | NaN | keratin 10 [Homo sapiens] |
| 2 | 2 | NaN | villin 1 [Homo sapiens] |
| 2 | 3 | 0.0439 | aldehyde dehydrogenase 1A1 [Homo sapiens] |
| 4 | 5 | 0.161 | phosphoglycerate kinase 1 [Homo sapiens] |
| 12 | 21 | NaN | heat shock 70kDa protein 1A [Homo sapiens]; heat shock 70kDa protein 1B [Homo sapiens] |
| 2 | 3 | NaN | hypothetical protein LOC126321 isoform b [Homo sapiens]; hypothetical protein LOC126321 isoform c [Homo sapie |
| 2 | 2 | 0.0238 | mannosidase, alpha, class 2B, member 2 [Homo sapiens] |
| 2 | 2 | NaN | CD74 antigen isoform a [Homo sapiens]; CD74 antigen isoform c [Homo sapiens]; CD74 antigen isoform b [Homo s |
| 9 | 12 | 0.141 | dipeptidylpeptidase IV [Homo sapiens] |
| 3 | 4 | 0.247 | lipocalin 2 [Homo sapiens] |
| 2 | 2 | 0.0253 | angiotensin I converting enzyme isoform 1 precursor [Homo sapiens] |
| 2 | 4 | 0.105 | carbonyl reductase 1 [Homo sapiens] |
| 10 | 19 | 0.26 | sodium/hydrogen exchanger regulatory factor 1 [Homo sapiens] |
| 2 | 2 | NaN | argininosuccinate synthetase 1 [Homo sapiens]; argininosuccinate synthetase 1 [Homo sapiens] |
| 2 | 3 | NaN | spinster homolog 1 isoform 1 [Homo sapiens]; spinster homolog 1 isoform 1 [Homo sapiens] |
| 2 | 2 | 0.1 | superoxide dismutase 3, extracellular precursor [Homo sapiens] |
| 4 | 4 | 0.0971 | hexosaminidase B preproprotein [Homo sapiens] |
| 2 | 3 | 0.069 | disrupted in renal carcinoma 2 [Homo sapiens] |
| 4 | 7 | 0.0501 | GNAS complex locus XLas [Homo sapiens] |
| 2 | 2 | 0.0232 | A kinase (PRKA) anchor protein 12 isoform 2 [Homo sapiens] |
| 3 | 3 | NaN | orosomucoid 1 precursor [Homo sapiens] |
| 3 | 3 | 0.177 | vacuolar protein sorting 37C [Homo sapiens] |
| 4 | 5 | 0.208 | nucleoside phosphorylase [Homo sapiens] |
| 4 | 4 | 0.138 | guanine nucleotide binding protein (G protein), alpha 13 [Homo sapiens] |
| 4 | 13 | NaN | PREDICTED: hypothetical protein [Homo sapiens] |
| 2 | 4 | 0.0766 | occludin [Homo sapiens] |
| 8 | 9 | 0.225 | ectonucleotide pyrophosphatase/phosphodiesterase 6 [Homo sapiens] |
| 2 | 4 | 0.0657 | ring finger protein 167 [Homo sapiens] |
| 9 | 12 | 0.483 | RAB7, member RAS oncogene family [Homo sapiens] |

Scale markers: 0.25    0.5    1    2    4

**Figure 2 (cont'd)**

| Peptides | Spectra | Coverage | Protein |
|---|---|---|---|
| 4 | 4 | NaN | prenylcysteine oxidase 1 [Homo sapiens] |
| 12 | 17 | 0.183 | galactosidase, beta 1 isoform a preproprotein [Homo sapiens] |
| 18 | 36 | 0.302 | heat shock 70kDa protein 8 isoform 1 [Homo sapiens] |
| 2 | 3 | 0.210 | MAPK scaffold protein 1 [Homo sapiens] |
| 2 | 2 | 0.0314 | complement factor B preproprotein [Homo sapiens] |
| 2 | 2 | 0.0438 | solute carrier family 12 (potassium/chloride transporters), member 9 [Homo sapiens] |
| 3 | 5 | 0.205 | glutathione transferase [Homo sapiens] |
| 5 | 5 | 0.104 | ATPase, H+ transporting, lysosomal 70kD, V1 subunit A, isoform 1 [Homo sapiens] |
| 2 | 2 | 0.0886 | aquaporin 2 [Homo sapiens] |
| 4 | 8 | NaN | syntaxin 7 [Homo sapiens] |
| 5 | 9 | 0.209 | major histocompatibility complex, class II, DR alpha precursor [Homo sapiens] |
| 3 | 4 | NaN | cytosolic phosphoenolpyruvate carboxykinase 1 [Homo sapiens] |
| 4 | 6 | 0.217 | glutathione peroxidase 3 precursor [Homo sapiens] |
| 4 | 4 | 0.0842 | transmembrane 7 superfamily member 3 [Homo sapiens] |
| 2 | 2 | NaN | transmembrane protein 55B isoform 1 [Homo sapiens]; transmembrane protein 55B isoform 2 [Homo sapiens] |
| 2 | 2 | 0.031 | nicastrin precursor [Homo sapiens] |
| 3 | 3 | 0.135 | carboxymethylenebutenolidase [Homo sapiens] |
| 2 | 3 | 0.094 | major histocompatibility complex, class II, DR beta 3 precursor [Homo sapiens] |
| 2 | 2 | 0.0968 | hypothetical protein LOC51571 [Homo sapiens] |

| Peptides | Spectra | Coverage | Protein |
|---|---|---|---|
| 1 | 1 | NaN | complement component 4B preproprotein [Homo sapiens] |
| 1 | 2 | 0.0852 | vacuolar protein sorting 25 [Homo sapiens] |
| 1 | 1 | 0.0219 | chloride intracellular channel 6 [Homo sapiens] |
| 1 | 3 | 0.0912 | vacuolar protein sorting 37B [Homo sapiens] |
| 1 | 2 | 0.0115 | complement component 4A preproprotein [Homo sapiens] |
| 1 | 2 | 0.0217 | nidogen 1 precursor [Homo sapiens] |
| 1 | 1 | NaN | histone cluster 2, H4b [Homo sapiens]; histone cluster 2, H4a [Homo sapiens]; histone cluster 1, H4i [Homo sapiens |
| 1 | 2 | 0.122 | hypothetical protein LOC91894 [Homo sapiens] |
| 1 | 1 | 0.143 | ring finger protein 152 [Homo sapiens] |
| 1 | 1 | 0.0184 | ephrin receptor EphA1 [Homo sapiens] |
| 1 | 1 | 0.0878 | proteasome beta 3 subunit [Homo sapiens] |
| 1 | 2 | NaN | carboxypeptidase M precursor [Homo sapiens]; carboxypeptidase M precursor [Homo sapiens]; carboxypeptidase |
| 1 | 5 | NaN | CD59 antigen preproprotein [Homo sapiens]; CD59 antigen preproprotein [Homo sapiens]; CD59 antigen preproprot |
| 1 | 3 | 0.0353 | amnionless protein precursor [Homo sapiens] |
| 1 | 1 | 0.0404 | von Willebrand factor A domain containing 1 isoform 1 [Homo sapiens] |
| 1 | 1 | 0.0272 | plasminogen [Homo sapiens] |
| 1 | 2 | 0.0671 | ficolin 2 isoform a precursor [Homo sapiens] |
| 1 | 1 | NaN | glycophorin C isoform 2 [Homo sapiens]; glycophorin C isoform 1 [Homo sapiens] |
| 1 | 1 | 0.0175 | collagen, type VI, alpha 1 precursor [Homo sapiens] |
| 1 | 1 | NaN | calmodulin 1 [Homo sapiens]; calmodulin 3 [Homo sapiens]; calmodulin 2 [Homo sapiens] |
| 1 | 1 | 0.089 | ras homolog gene family, member G [Homo sapiens] |
| 1 | 1 | 0.0558 | vacuolar protein sorting 37D [Homo sapiens] |
| 1 | 5 | 0.0324 | protease, serine, 1 preproprotein [Homo sapiens] |
| 1 | 2 | 0.03 | prominin 2 [Homo sapiens] |
| 1 | 2 | NaN | cathepsin B preproprotein [Homo sapiens]; cathepsin B preproprotein [Homo sapiens]; cathepsin B preproprotein [ |
| 1 | 3 | 0.0292 | radixin [Homo sapiens] |
| 1 | 1 | 0.104 | GM2 ganglioside activator precursor [Homo sapiens] |
| 1 | 1 | 0.106 | eukaryotic translation initiation factor 6 isoform c [Homo sapiens] |
| 1 | 2 | NaN | syntenin isoform 3 [Homo sapiens]; syntenin isoform 3 [Homo sapiens] |
| 1 | 1 | 0.0423 | guanine nucleotide binding protein, alpha z polypeptide [Homo sapiens] |
| 1 | 1 | 0.0231 | cytochrome P450, family 4, subfamily A, polypeptide 11 [Homo sapiens] |
| 1 | 1 | 0.0226 | peptidoglycan recognition protein 2 precursor [Homo sapiens] |
| 1 | 1 | NaN | SMAD family member 9 isoform b [Homo sapiens]; SMAD family member 9 isoform a [Homo sapiens] |
| 1 | 2 | 0.0304 | phospholipid transfer protein isoform a precursor [Homo sapiens] |
| 1 | 1 | 0.0341 | guanine nucleotide binding protein (G protein) alpha 12 [Homo sapiens] |
| 1 | 1 | 0.0344 | solute carrier family 1, member 1 [Homo sapiens] |

**Figure 3**

0.25　0.5　1　2　4

| Peptides | Spectra | Coverage | Protein |
|---|---|---|---|
| 1 | 2 | NaN | solute carrier family 13 member 3 isoform b [Homo sapiens]; solute carrier family 13 member 3 isoform a [Homo sap |
| 1 | 1 | 0.140 | GTPase Rab14 [Homo sapiens] |
| 1 | 1 | 0.104 | transmembrane protein 9 [Homo sapiens] |
| 1 | 1 | NaN | calcium binding protein 39 [Homo sapiens]; calcium binding protein 39 [Homo sapiens]; calcium binding protein 39 |
| 1 | 1 | 0.00284 | hemicentin 1 [Homo sapiens] |
| 1 | 1 | 0.0813 | prostate stem cell antigen preproprotein [Homo sapiens] |
| 1 | 2 | 0.0246 | solute carrier family 47, member 1 [Homo sapiens] |
| 1 | 1 | 0.0728 | biliverdin reductase B (flavin reductase (NADPH)) [Homo sapiens] |
| 1 | 1 | NaN | proteasome alpha 4 subunit isoform 1 [Homo sapiens]; proteasome alpha 4 subunit isoform 1 [Homo sapiens] |
| 1 | 1 | NaN | palate, lung and nasal epithelium associated precursor [Homo sapiens]; palate, lung and nasal epithelium associate |
| 1 | 5 | NaN | PREDICTED: similar to hCG1642538 [Homo sapiens]; PREDICTED: similar to hCG1642538 [Homo sapiens] |
| 1 | 1 | 0.0424 | actin, alpha 1, skeletal muscle [Homo sapiens] |
| 1 | 1 | 0.0656 | RAP2B, member of RAS oncogene family [Homo sapiens] |
| 1 | 8 | 0.045 | transmembrane BAX inhibitor motif containing 1 [Homo sapiens] |
| 1 | 1 | 0.0483 | mucolipin 1 [Homo sapiens] |
| 1 | 1 | 0.0343 | annexin IV [Homo sapiens] |
| 1 | 1 | NaN | diazepam binding inhibitor isoform 2 [Homo sapiens]; diazepam binding inhibitor isoform 3 [Homo sapiens]; diazep |
| 1 | 2 | 0.0335 | tubulin, alpha 4a [Homo sapiens] |
| 1 | 1 | 0.0203 | solute carrier family 1 (neutral amino acid transporter), member 5 [Homo sapiens] |
| 1 | 1 | 0.0652 | claudin 2 [Homo sapiens] |
| 1 | 1 | NaN | myoferlin isoform a [Homo sapiens]; myoferlin isoform b [Homo sapiens] |
| 1 | 1 | NaN | deleted in malignant brain tumors 1 isoform c precursor [Homo sapiens]; deleted in malignant brain tumors 1 isofor |
| 1 | 1 | 0.0234 | galactosylceramidase isoform a precursor [Homo sapiens] |
| 1 | 1 | NaN | hypothetical protein LOC55194 [Homo sapiens]; PREDICTED: hypothetical protein [Homo sapiens] |
| 1 | 3 | NaN | CD151 antigen [Homo sapiens]; CD151 antigen [Homo sapiens]; CD151 antigen [Homo sapiens]; CD151 antigen [Ho |
| 1 | 2 | 0.066 | RAB2A, member RAS oncogene family [Homo sapiens] |
| 1 | 1 | 0.0132 | actinin, alpha 4 [Homo sapiens] |
| 1 | 1 | NaN | tumor differentially expressed protein 1 [Homo sapiens]; tumor differentially expressed protein 1 [Homo sapiens] |
| 1 | 1 | NaN | phosphodiesterase 8A isoform 2 [Homo sapiens]; phosphodiesterase 8A isoform 1 [Homo sapiens] |
| 1 | 1 | 0.0112 | SH3 domain and tetratricopeptide repeats 1 [Homo sapiens] |
| 1 | 1 | NaN | BH3 interacting domain death agonist isoform 2 [Homo sapiens]; BH3 interacting domain death agonist isoform 3 [H |
| 1 | 1 | NaN | ribophorin II isoform 2 precursor [Homo sapiens] |
| 1 | 1 | 0.0687 | chromatin modifying protein 4C [Homo sapiens] |
| 1 | 1 | NaN | sacsin [Homo sapiens] |
| 1 | 1 | 0.0444 | lumican precursor [Homo sapiens] |
| 1 | 1 | 0.0302 | chromatin modifying protein 1B [Homo sapiens] |
| 1 | 1 | NaN | SHC (Src homology 2 domain containing) transforming protein 1 isoform 2 [Homo sapiens]; SHC (Src homology 2 d |
| 1 | 1 | NaN | ATPase, H+ transporting, lysosomal V0 subunit a4 [Homo sapiens]; ATPase, H+ transporting, lysosomal V0 subunit |
| 1 | 1 | 0.0544 | UBX domain protein 6 [Homo sapiens] |
| 1 | 1 | 0.0192 | tetratricopeptide repeat domain 38 [Homo sapiens] |
| 1 | 2 | 0.0629 | crystallin, alpha B [Homo sapiens] |
| 1 | 1 | 0.00775 | dual oxidase 2 precursor [Homo sapiens] |
| 1 | 3 | 0.126 | DnaJ (Hsp40) homolog, subfamily C, member 5 [Homo sapiens] |
| 1 | 1 | 0.11 | secretoglobin, family 1A, member 1 (uteroglobin) [Homo sapiens] |
| 1 | 1 | NaN | structural maintenance of chromosomes 2 [Homo sapiens]; structural maintenance of chromosomes 2 [Homo sapie |
| 1 | 1 | 0.091 | superoxide dismutase 1, soluble [Homo sapiens] |
| 1 | 1 | 0.0227 | solute carrier family 44, member 2 [Homo sapiens] |
| 1 | 2 | NaN | PREDICTED: hypothetical protein [Homo sapiens] |
| 1 | 1 | 0.0403 | tropomyosin 4 [Homo sapiens] |
| 1 | 1 | 0.0101 | myosin VI [Homo sapiens] |
| 1 | 1 | 0.0176 | heat shock protein 90kDa alpha (cytosolic), class A member 1 isoform 1 [Homo sapiens] |
| 1 | 1 | NaN | RAB1A, member RAS oncogene family isoform 1 [Homo sapiens]; RAB1B, member RAS oncogene family [Homo sa |
| 1 | 2 | NaN | PREDICTED: similar to hCG1992647, partial [Homo sapiens] |
| 1 | 1 | NaN | eukaryotic translation initiation factor 5A isoform A [Homo sapiens] |
| 1 | 3 | 0.149 | PDZK1 interacting protein 1 [Homo sapiens] |
| 1 | 1 | NaN | hexosaminidase A preproprotein [Homo sapiens] |
| 1 | 1 | 0.0491 | paralemmin isoform 1 [Homo sapiens] |
| 1 | 1 | 0.00904 | zinc finger protein 262 [Homo sapiens] |
| 1 | 1 | 0.0196 | skeletal muscle receptor tyrosine kinase [Homo sapiens] |
| 1 | 1 | NaN | spastin isoform 2 [Homo sapiens]; spastin isoform 1 [Homo sapiens] |
| 1 | 3 | 0.0748 | allograft inflammatory factor 1 isoform 3 [Homo sapiens] |
| 1 | 1 | 0.0539 | quinolinate phosphoribosyltransferase [Homo sapiens] |
| 1 | 1 | NaN | transmembrane protein 106A [Homo sapiens]; PREDICTED: similar to Transmembrane protein 106A [Homo sapiens |
| 1 | 1 | 0.062 | uroplakin 1A [Homo sapiens] |
| 1 | 1 | 0.075 | secretory carrier membrane protein 3 isoform 1 [Homo sapiens] |
| 1 | 1 | 0.225 | guanine nucleotide binding protein (G protein), gamma 2 [Homo sapiens] |
| 1 | 1 | 0.0476 | sorbitol dehydrogenase [Homo sapiens] |

**Figure 3 (cont'd)**

| | Peptides | Spectra | Coverage | Protein |
|---|---|---|---|---|
| 0.25  0.5  1  2  4 | 1 | 1 | 0.0258 | major facilitator superfamily domain containing 1 [Homo sapiens] |
| | 1 | 1 | 0.110 | nuclear transport factor 2 [Homo sapiens] |
| | 1 | 4 | 0.00515 | nucleoporin 188kDa [Homo sapiens] |
| | 1 | 1 | 0.0323 | hypothetical protein LOC54978 [Homo sapiens] |
| | 1 | 1 | 0.097 | adenine phosphoribosyltransferase isoform b [Homo sapiens] |
| | 1 | 1 | 0.0811 | lysozyme precursor [Homo sapiens] |
| | 1 | 1 | 0.0779 | NAD(P)H dehydrogenase, quinone 2 [Homo sapiens] |
| | 1 | 1 | 0.122 | cystatin B [Homo sapiens] |
| | 1 | 1 | 0.0522 | triosephosphate isomerase 1 [Homo sapiens] |
| | 1 | 3 | 0.0678 | phospholipid scramblase 3 [Homo sapiens] |
| | 1 | 1 | 0.0233 | plasma glutamate carboxypeptidase [Homo sapiens] |
| | 1 | 1 | NaN | bone marrow stromal cell antigen 1 precursor [Homo sapiens] |
| | 1 | 1 | 0.0221 | solute carrier organic anion transporter family, member 4C1 [Homo sapiens] |
| | 1 | 1 | 0.0173 | glucose transporter 14 [Homo sapiens] |
| | 1 | 1 | NaN | serine hydroxymethyltransferase 1 (soluble) isoform 2 [Homo sapiens]; serine hydroxymethyltransferase 1 (soluble) |
| | 1 | 1 | 0.082 | calcium binding protein P22 [Homo sapiens] |
| | 1 | 1 | 0.0170 | sushi domain containing 2 [Homo sapiens] |
| | 1 | 1 | 0.0847 | CD81 antigen [Homo sapiens] |
| | 1 | 2 | 0.0283 | solute carrier family 5 (sodium/glucose cotransporter), member 2 [Homo sapiens] |
| | 1 | 1 | 0.0723 | MIT, microtubule interacting and transport, domain containing 1 [Homo sapiens] |
| | 1 | 1 | NaN | ubiquitin protein ligase E3C [Homo sapiens] |
| | 1 | 1 | 0.0763 | syntaxin 8 [Homo sapiens] |
| | 1 | 1 | 0.0494 | sorting nexin 3 [Homo sapiens] |
| | 1 | 1 | 0.0305 | glucose phosphate isomerase [Homo sapiens] |
| | 1 | 1 | 0.0337 | GDP dissociation inhibitor 2 isoform 1 [Homo sapiens] |
| | 1 | 1 | NaN | solute carrier family 3 (activators of dibasic and neutral amino acid transport), member 2 isoform c [Homo sapiens] |
| | 1 | 1 | 0.0697 | RAB9A, member RAS oncogene family [Homo sapiens] |
| | 1 | 1 | NaN | complement component 8, gamma polypeptide [Homo sapiens] |
| | 1 | 1 | NaN | transketolase isoform 1 [Homo sapiens]; transketolase isoform 2 [Homo sapiens]; transketolase isoform 1 [Homo sa |
| | 1 | 1 | NaN | cyclin M3 isoform 1 [Homo sapiens]; cyclin M3 isoform 2 [Homo sapiens] |
| | 1 | 1 | NaN | solute carrier family 9 (sodium/hydrogen exchanger), isoform 3 [Homo sapiens] |
| | 1 | 1 | NaN | RAB5C, member RAS oncogene family isoform a [Homo sapiens]; RAB5C, member RAS oncogene family isoform b |
| | 1 | 1 | 0.0348 | pyruvate kinase, liver and RBC isoform 1 [Homo sapiens] |
| | 1 | 2 | 0.0479 | DnaJ (Hsp40) homolog, subfamily A, member 1 [Homo sapiens] |
| | 1 | 1 | 0.0375 | serine incorporator 1 [Homo sapiens] |
| | 1 | 3 | NaN | carbonic anhydrase XII isoform 2 precursor [Homo sapiens]; carbonic anhydrase XII isoform 1 precursor [Homo sap |
| | 1 | 3 | 0.0529 | cathelicidin antimicrobial peptide [Homo sapiens] |
| | 1 | 2 | 0.0214 | aconitase 1 [Homo sapiens] |

**Figure 3 (cont'd)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- **ALI et al.** Incidence and outcomes in acute kidney injury: a comprehensive population-based study. *J Am Soc Nephrol,* 2007, vol. 18, 1292-1298
- **UCHINO et al.** Acute renal failure in critically ill patients: a multinational, multicenter study. *JAMA,* 2005, vol. 294, 813-818
- **PALEVSKY et al.** Intensity of renal support in critically ill patients with acute kidney injury. *N Engl J Med,* 2008, vol. 359, 7-20
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- Current Medical Diagnosis & Treatment. McGraw Hill, 2008, 785-815
- Fundamental Immunology. Raven Press, 1993
- **WILSON et al.** *J. Immunol. Methods,* 1994, vol. 175, 267-273
- **YARMUSH et al.** *J. Biochem. Biophys. Methods,* 1992, vol. 25, 85-97
- **WARD et al.** *Nature,* 1989, vol. 341, 544-546
- Harrison's Principles of Internal Medicine. McGraw Hill, 1741-1830
- Merck Manual
- **PRAUGHT et al.** *Curr Opin Nephrol Hypertens,* 2005, vol. 14, 265-270
- **CHERTOW et al.** *J Am Soc Nephrol,* 2005, vol. 16, 3365-3370
- **LASSNIGG et al.** *J Am Soc Nephrol,* 2004, vol. 15, 1597-1605
- **BELLOMO et al.** *Crit Care,* 2004, vol. 8 (4), R204-212
- **MCCOLLOUGH et al.** *Rev Cardiovasc Med.,* 2006, vol. 7 (4), 177-197
- **PECOITS-FILHO et al.** Associations between circulating inflammatory markers and residual renal function in CRF patients. *Am J Kidney Dis.,* 2003, vol. 41 (6), 1212-1218
- **MANNS et al.** Cost of acute renal failure requiring dialysis in the intensive care unit: clinical and resource implications of renal recovery. *Crit Care Med,* 2003, vol. 31, 449-455
- **BHANDARI et al.** Survivors of acute renal failure who do not recover renal function. *QJM,* 1996, vol. 89, 415-421
- **MEHTA et al.** A randomized clinical trial of continuous versus intermittent dialysis for acute renal failure. *Kidney Int,* 2001, vol. 60, 1154-1163
- **AUGUSTINE et al.** A randomized controlled trial comparing intermittent with continuous dialysis in patients with ARF. *Am J Kidney Dis,* 2004, vol. 44, 1000-1007
- **UEHLINGER et al.** Comparison of continuous and intermittent renal replacement therapy for acute renal failure. *Nephrol Dial Transplant,* 2005, vol. 20, 1630-1637
- **UCHINO et al.** Discontinuation of continuous renal replacement therapy: a post hoc analysis of a prospective multicenter observational study. *Crit Care Med,* 2009, vol. 37, 2576-2582
- **SUPAVEKIN et al.** Differential gene expression following early renal ischemia/reperfusion. *Kidney Int,* 2003, vol. 63, 1714-1724
- **MISHRA et al.** Kidney NGAL is a novel early marker of acute injury following transplantation. *Pediatr Nephrol,* 2006, vol. 21, 856-863
- **HIRSCH et al.** NGAL is an early predictive biomarker of contrast-induced nephropathy in children. *Pediatr Nephrol,* 2007, vol. 22, 2089-2095

- **ZAPPITELLI et al.** Urine neutrophil gelatinase-associated lipocalin is an early marker of acute kidney injury in critically ill children: a prospective cohort study. *Crit Care,* 2007, vol. 11, R84
- **RONCO et al.** Matrix metalloproteinases in kidney disease progression and repair: a case of flipping the coin. *Semin Nephrol,* 2007, vol. 27, 352-362
- **PARIKH et al.** Urine IL-18 is an early diagnostic marker for acute kidney injury and predicts mortality in the intensive care unit. *J Am Soc Nephrol,* 2005, vol. 16, 3046-3052
- **PARIKH et al.** Urinary IL-18 is an early predictive biomarker of acute kidney injury after cardiac surgery. *Kidney Int,* 2006, vol. 70, 199-203
- **LIU et al.** Hepatocyte growth factor: new arsenal in the fights against renal fibrosis?. *Kidney Int,* 2006, vol. 70, 238-240
- **HERGET-ROSENTHAL et al.** Measurement of urinary cystatin C by particle-enhanced nephelometric immunoassay: precision, interferences, stability and reference range. *Ann Clin Biochem,* 2004, vol. 41, 111-118
- **COCA et al.** Biomarkers for the diagnosis and risk stratification of acute kidney injury: a systematic review. *Kidney Int,* 2008, vol. 73, 1008-1016
- The Immunoassay Handbook. Stockton Press, 1994
- **FISCHER et al.** *Intensive Care Med.,* 2003, vol. 29, 1043-1051
- **BAGSHAW et al.** *Nephrol. Dial. Transplant.,* 2008, vol. 23, 1203-1210
- Merck Manual of Diagnosis and Therapy. Merck Research Laboratories, 1999
- **VAN ERP et al.** *J. Immunoassay,* 1991, vol. 12, 425-443
- **NELSON et al.** *Comput. Methods Programs Biomed.,* 1988, vol. 27, 65-68
- **CWIRLA et al.** *Proc. Natl. Acad. Sci.,* 1990, vol. 87, 6378-6382
- **DEVLIN et al.** *Science,* 1990, vol. 249, 404-406
- **SCOTT et al.** *Science,* 1990, vol. 249, 386-388
- **LEVY et al.** 2001 SCCM/ESICM/ACCP/ATS/SIS International Sepsis Definitions Conference. *Crit Care Med,* 2003, vol. 31, 1250-1256